# EUROPEAN PATENT SPECIFICATION

(11) **EP 3 663 308 B1**
(45) Date of publication and mention of the grant of the patent: **14.07.2021**
(21) Application number: 19211446.0
(22) Date of filing: 26.11.2019
(51) Int. Cl.: C07F 15/00, C09K 11/02, C09K 11/06, H01L 51/00

(54) **ORGANOMETALLIC COMPOUND, ORGANIC LIGHT-EMITTING DEVICE INCLUDING THE ORGANOMETALLIC COMPOUND, AND DIAGNOSTIC COMPOSITION INCLUDING THE ORGANOMETALLIC COMPOUND**
ORGANOMETALLISCHE VERBINDUNG, ORGANISCHE LICHTEMITTIERENDE VORRICHTUNG MIT DER ORGANOMETALLISCHEN VERBINDUNG UND DIAGNOSTISCHE ZUSAMMENSETZUNG MIT DER ORGANOMETALLISCHEN VERBINDUNG
COMPOSÉ ORGANOMÉTALLIQUE, DISPOSITIF ÉLECTROLUMINESCENT ORGANIQUE LE COMPRENANT ET COMPOSITION DE DIAGNOSTIC COMPRENANT LE COMPOSÉ ORGANOMÉTALLIQUE

(30) Priority: 05.12.2018 KR 20180155457
(43) Date of publication of application: 10.06.2020
(73) Proprietor: Samsung Electronics Co., Ltd., Gyeonggi-do 16677 (KR)
(72) Inventor: HWANG, Kyuyoung, 16678 Gyeonggi-do (KR); KWAK, Seungyeon, 16678 Gyeonggi-do (KR); KWAK, Yoonhyun, 16678 Gyeonggi-do (KR); LEE, Kum Hee, 16678 Gyeonggi-do (KR); LEE, Sunghun, 16678 Gyeonggi-do (KR); CHOI, Whail, 16678 Gyeonggi-do (KR); JEON, Aram, 16678 Gyeonggi-do (KR); CHOI, Byoungki, 16678 Gyeonggi-do (KR); CHOI, Hyeonho, 16678 Gyeonggi-do (KR)
(74) Representative: Elkington and Fife LLP

(56) References cited:
- EP-A1- 3 000 815
- US-A1- 2016 155 963

## Description

### FIELD OF THE INVENTION

One or more embodiments of the present disclosure relate to an organometallic compound, an organic light-emitting device including the organometallic compound, and a diagnostic composition including the organometallic compound.

### BACKGROUND OF THE INVENTION

Organic light-emitting devices (OLEDs) are self-emission devices, which have better characteristics in terms of viewing angle, response time, brightness, driving voltage, and response speed, and produce full-color images.

In an example, an organic light-emitting device includes an anode, a cathode, and an organic layer disposed between the anode and the cathode, wherein the organic layer includes an emission layer. A hole transport region may be disposed between the anode and the emission layer, and an electron transport region may be disposed between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state, thereby generating light.

Meanwhile, luminescence compounds may be used to monitor, sense, or detect a variety of biological materials including cells and proteins. An example of the luminescence compounds includes a phosphorescent luminescence compound.

Various types of organic light emitting devices are known. However, there still remains a need in OLEDs having low driving voltage, high efficiency, high brightness, and long lifespan.

US 2016/0155963 discloses organometallic compounds represented by M(L₁)ₙ₁(L₂)ₙ₂.

### SUMMARY OF THE INVENTION

Provided are an organometallic compound, an organic light-emitting device including the organometallic compound, and a diagnostic composition including the organometallic compound.
organometallic compound.

Additional aspects will be set forth in part in the description which follows and, in part, will be apparent from the description, or may be learned by practice of the presented embodiments.

According to an aspect of an embodiment, organometallic compounds are represented by Formula 1:

Formula 1 M(L₁)ₙ₁(L₂)ₙ₂

In Formula 1,
M may be a transition metal,
L₁ may be a ligand represented by Formula 2A,
n1 may be 1 or 2, wherein, when n1 is 2, two groups L₁ may be identical to or different from each other,
L₂ may be a ligand represented by Formula 2B,
n2 may be 1 or 2, wherein, when n2 is 2, two groups L₂ may be identical to or different from each other,
the sum of n1 and n2 may be 2 or 3, and
each L₁ and L₂ may be different from each other:

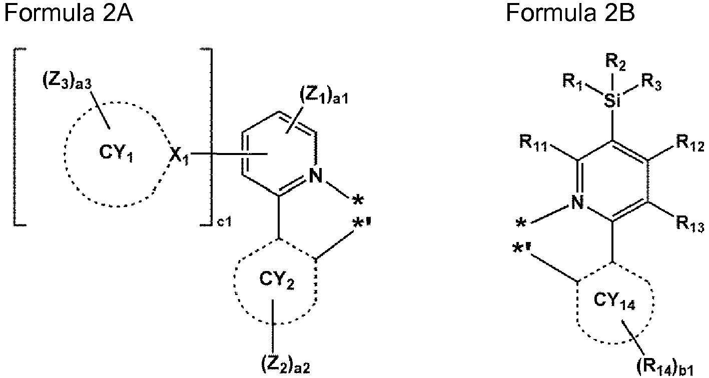

In Formulae 2A and 2B,
X₁ may be C, N, Si, or P,
ring CY₁, ring CY₂, and ring CY₁₄ may each independently be a C₅-C₃₀ carbocyclic group or a C₁-C₃₀ heterocyclic group,
R₁ to R₃ may each independently be a C₁-C₆₀ alkyl group or a C₆-C₆₀ aryl group, each independently unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, and a C₁-C₁₀ alkyl group,
Z₁ to Z₃ and R₁₁ to R₁₄ may each independently be selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), - B(Q₆)(Q₇), and -P(=O)(Q₈)(Q₉), wherein R₁₂ may be neither hydrogen nor a methyl group,
a1 may be an integer from 0 to 3, wherein, when a1 is two or more, two or more groups Z₁ may be identical to or different from each other,
a2, a3, and b1 may each independently be an integer from 0 to 20, wherein, when a2 is two or more, two or more groups Z₂ may be identical to or different from each other, when a3 is two or more, two or more groups Z₃ may be identical to or different from each other, and when b1 is two or more, two or more groups R₁₄ may be identical to or different from each other,
c1 may be an integer from 1 to 4, wherein, when c1 is two or more, two or more groups represented by 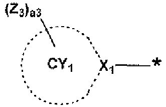 may be identical to or different from each other,
when a1 is two or more, two or more groups Z₁ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
when a2 is two or more, two or more groups Z₂ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
when a3 is two or more, two or more groups Z₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₂ and R₁₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
when b1 is two or more, two or more groups R₁₄ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more substituents selected from Z₁ and Z₂ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more substituents selected from R₁₁ to R₁₄ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₀ₐ may be selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, - N(Q₁)(Q₂), -Si(Q₃)(Q₄)(Q₅), -Ge(Q₃)(Q₄)(Q₅), -B(Q₆)(Q₇), and -P(=O)(Q₈)(Q₉),
* and *' in Formulae 2A and 2B each indicate a binding site to M in Formula 1,
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C60 aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and substituted monovalent non-aromatic condensed heteropolycyclic group may each independently be selected from:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C60 alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -B(Q₁₆)(Q₁₇), and -P(=O)(Q₁₈)(Q₁₉);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, or a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, or a monovalent non-aromatic condensed heteropolycyclic group, each substituted with at least one selected from deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C60 aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -B(Q₂₆)(Q₂₇), and
   -P(=O)(Q₂₈)(Q₂₉); and
-N(Q₃₁)(Q₃₂), -Si(Q₃₃)(Q₃₄)(Q₃₅), -B(Q₃₆)(Q₃₇), or -P(=O)(Q₃₈)(Q₃₉), and
Q₁ to Q₉, Q₁₁ to Q₁₉, Q₂₁ to Q₂₉, and Q₃₁ to Q₃₉ may each independently be selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group and a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

Another aspect of the present disclosure provides an organic light-emitting device including: a first electrode; a second electrode; and an organic layer disposed between the first electrode and the second electrode and including an emission layer, wherein the organic layer includes at least one organometallic compound represented by Formula 1.

The organometallic compound in the emission layer of the organic layer may act as a dopant.

Another aspect of the present disclosure provides a diagnostic composition including at least one organometallic compound represented by Formula 1.

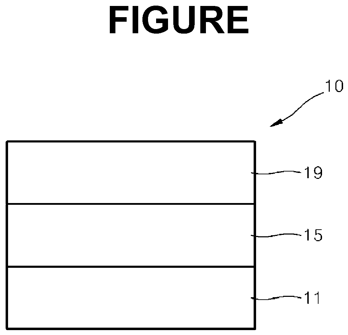

### BRIEF DESCRIPTION OF THE DRAWING

These and/or other aspects will become apparent and more readily appreciated from the following description of the embodiments, taken in conjunction with FIGURE which is a schematic view of an organic light-emitting device according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly in contact with the other element or intervening elements may be present therebetween. In contrast, when an element is referred to as being "directly on" another element, there are no intervening elements present.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

The terminology used herein is for the purpose of describing particular embodiments only and is not intended to be limiting. As used herein, the singular forms "a," "an," and "the" are intended to include the plural forms as well, unless the context clearly indicates otherwise.

The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

"About" or "approximately" as used herein is inclusive of the stated value and means within an acceptable range of deviation for the particular value as determined by one of ordinary skill in the art, considering the measurement in question and the error associated with measurement of the particular quantity (*i.e.,* the limitations of the measurement system). For example, "about" can mean within one or more standard deviations, or within ± 30%, 20%, 10%, 5% of the stated value.

An organometallic compound according to an embodiment is represented by Formula 1:

Formula 1 M(L₁)ₙ₁(L₂)ₙ₂

In Formula 1, M may be a transition metal. For example, M may be selected from a first-row transition metal, a second-row transition metal, and a third-row transition metal, of the Periodic Table of Elements.

In an embodiment, M may be iridium (Ir), platinum (Pt), osmium (Os), titanium (Ti), zirconium (Zr), hafnium (Hf), europium (Eu), terbium (Tb), thulium (Tm), or rhodium (Rh).

In an embodiment, M may be Ir, Pt, Os, or Rh, but embodiments of the present disclosure are not limited thereto.

In Formula 1, L₁ may be a ligand represented by Formula 2A. In Formula 1, n1 indicates the number of groups L₁, and may be 1 or 2. When n1 is two, two groups L₁ may be identical to or different from each other.

In Formula 1, L₂ may be a ligand represented by Formula 2B. In Formula 1, n2 indicates the number of groups L₂, and may be 1 or 2. When n2 is two, two groups L₂ may be identical to or different from each other.

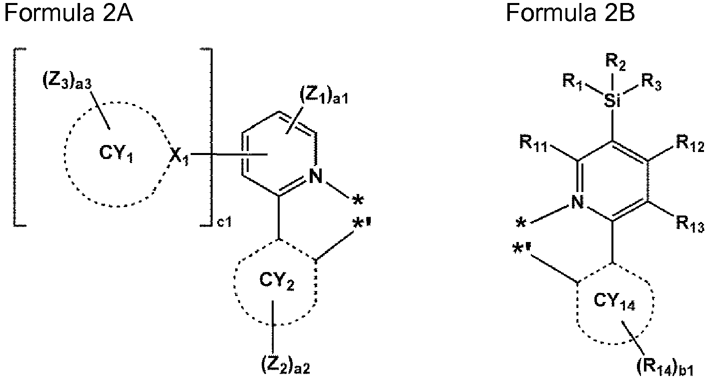

Formulae 2A and 2B will be described below.

In Formula 1, L₁ and L₂ may be different from each other.

In an embodiment, M may be Ir, and the sum of n1 and n2 may be 3; or M may be Pt, and the sum of n1 and n2 may be 2.

In Formula 2A, X₁ may be C, N, Si, or P. For example, X₁ may be C, but embodiments of the present disclosure are not limited thereto.

In Formulae 2A and 2B, ring CY₁, ring CY₂, and ring CY₁₄ may each independently be a C₅-C₃₀ carbocyclic group or a C₁-C₃₀ heterocyclic group.

For example, ring CY₁, ring CY₂, and ring CY₁₄ may each independently be i) a first ring, ii) a second ring, iii) a condensed ring in which at least two first rings are condensed, iv) a condensed ring in which at least two second rings are condensed, or v) a condensed ring in which at least one first ring and at least one second ring are condensed, wherein
the first ring may be a cyclopentane group, a cyclopentadiene group, a furan group, a thiophene group, a pyrrole group, a silole group, an indene group, a benzofuran group, a benzothiophene group, an indole group, a benzosilole group, an oxazole group, an isoxazole group, an oxadiazole group, an isoxadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, or a triazasilole group, and
the second ring may be an adamantane group, a norbornane group, a norbornene group, a cyclohexane group, a cyclohexene group, a benzene group, a pyridine group, a pyrimidine group, a pyrazine group, a pyridazine group, or a triazine group.

In an embodiment, ring CY₁, ring CY₂, and ring CY₁₄ may each independently be selected from a cyclopentane group, a cyclohexane group, a cycloheptane group, a cyclopentene group, a cyclohexene group, a cycloheptene group, a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, and a 5,6,7,8-tetrahydroquinoline group, but embodiments of the present disclosure are not limited thereto.

In an embodiment, ring CY₁, ring CY₂, and ring CY₁₄ may each independently be selected from a benzene group, a naphthalene group, a 1,2,3,4-tetrahydronaphthalene group, a phenanthrene group, a pyridine group, a pyrimidine group, a pyrazine group, a triazine group, a benzofuran group, a benzothiophene group, a fluorene group, a carbazole group, a dibenzofuran group, a dibenzothiophene group, a dibenzosilole group, an azafluorene group, an azacarbazole group, an azadibenzofuran group, an azadibenzothiophene group, and an azadibenzosilole group, but embodiments of the present disclosure are not limited thereto.

In Formula 2B, R₁ to R₃ may each independently be a C₁-C₆₀ alkyl group or a C₆-C₆₀ aryl group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, and a C₁-C₁₀ alkyl group.

For example, R₁ to R₃ may each independently be selected from a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, a phenyl group, a biphenyl group, or a naphthyl group, each unsubstituted or substituted with at least one selected from deuterium, -F, -Cl, -Br, -I, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof and a C₁-C₁₀ alkyl group.

In an embodiment, R₁ to R₃ may each independently be -CH₃, -CH₂CH₃, - CD₃,
-CD₂H, -CDH₂, -CH₂CD₃, or -CD₂CH₃.

In Formula 2B, R₁ to R₃ may be identical to or different from each other.

In an embodiment, R₁ to R₃ may be identical to each other, but embodiments of the present disclosure are not limited thereto.

In Formulae 2A and 2B, Z₁ to Z₃ and R₁₁ to R₁₄ may each independently be selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a C₇-C₆₀ arylalkyl group, a substituted or unsubstituted C₁-C₆₀ heteroaryl group, a C₁-C₆₀ heteroaryloxy group, a C₁-C₆₀ heteroarylthio group, a C₂-C₆₀ heteroarylalkyl group, a substituted or unsubstituted monovalent non-aromatic condensed polycyclic group, a substituted or unsubstituted monovalent non-aromatic condensed heteropolycyclic group, - N(Q₁)(Q₂), -Si(Q₃)(Q₄)(Q₅), -Ge(Q₃)(Q₄)(Q₅), -B(Q₆)(Q₇), and -P(=O)(Q₈)(Q₉), wherein R₁₂ may be neither hydrogen nor a methyl group, and Q₁ to Q₉ are the same as described above.

In an embodiment, Z₁ to Z₃ and R₁₁ to R₁₄ may each independently be selected from:
hydrogen, deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, or a C₁-C₂₀ alkoxy group;
a C₁-C₂₀ alkyl group or a C₁-C₂₀ alkoxy group, each substituted with at least one selected from deuterium, -F, -Cl, -Br, -I, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a pyridinyl group, or a pyrimidinyl group;
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, and - Si(Q₃₃)(Q₃₄)(Q₃₅); and
-N(Q₁)(Q₂), -Si(Q₃)(Q₄)(Q₅), -Ge(Q₃)(Q₄)(Q₅), -B(Q₆)(Q₇), -P(=O)(Q₈)(Q₉), or - P(Q₈)(Q₉), wherein R₁₂ is neither hydrogen nor a methyl group, and
Q₁ to Q₉ and Q₃₃ to Q₃₅ may each independently be selected from:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or -CD₂CDH₂; and
   an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with at least one selected from deuterium, a C₁-C₁₀ alkyl group, and a phenyl group.

In an embodiment, in Formula 2B, R₁₂ may be selected from:
a C₂-C₂₀ alkyl group or a C₂-C₂₀ alkoxy group;
a methyl group or a methoxy group, each substituted with at least one selected from a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₁₄ aryl group, a C₁-C₁₄ heteroaryl group, a monovalent non-aromatic condensed polycyclic group, and a monovalent non-aromatic condensed heteropolycyclic group;
a C₂-C₂₀ alkyl group or a C₂-C₂₀ alkoxy group, each substituted with at least one selected from deuterium, -F, -Cl, -Br, -I, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₁₄ aryl group, a C₁-C₁₄ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₁₄ aryl group, a C₁-C₁₄ heteroaryl group, a monovalent non-aromatic condensed polycyclic group, or a monovalent non-aromatic condensed heteropolycyclic group, each unsubstituted or substituted with at least one selected from deuterium, -F, -Cl, -Br, -I, -CD₃, -CD₂H, - CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₁₄ aryl group, a C₁-C₁₄ heteroaryl group, a monovalent non-aromatic condensed polycyclic group, and a monovalent non-aromatic condensed heteropolycyclic group; and
-N(Q₁)(Q₂), -Si(Q₃)(Q₄)(Q₅), -Ge(Q₃)(Q₄)(Q₅), -B(Q₆)(Q₇), or -P(=O)(Q₈)(Q₉).

In an embodiment, in Formula 2B, R₁₂ may be selected from:
a C₂-C₂₀ alkyl group or a C₂-C₂₀ alkoxy group;
methyl group or a methoxy group, each substituted with at least one selected from a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a naphthyl group, a pyridinyl group, and a pyrimidinyl group;
a methyl group or a methoxy group, each substituted with i) at least one selected from a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a naphthyl group, a pyridinyl group, and a pyrimidinyl group, and ii) at least one deuterium;
a C₂-C₂₀ alkyl group or a C₂-C₂₀ alkoxy group, each substituted with at least one selected from deuterium, -F, -Cl, -Br, -I, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a naphthyl group, a pyridinyl group, and a pyrimidinyl group; and
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group or azadibenzothiophenyl group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C20 alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, and an azadibenzothiophenyl group.

Examples of the C₁-C₆₀ alkyl group, the C₁-C₂₀ alkyl group, and the C₁-C₁₀ alkyl group include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, or a tert-decyl group, and examples of the C₁-C₆₀ alkoxy group, the C₁-C₂₀ alkoxy group, and the C₁-C₁₀ alkoxy group include a methoxy group, an ethoxy group, a propoxy group, a butoxy group, and a pentoxy group.

In an embodiment, in Formulae 2A and 2B, Z₁ to Z₃, R₁₁, R₁₃, and R₁₄ may each independently be selected from hydrogen, deuterium, -F, a cyano group, a nitro group, -SF₅, -CH₃, -CD₃, -CD₂H, -CDH₂, -CF₃, -CF₂H, -CFH₂, groups represented by Formulae 9-1 to 9-66, groups represented by Formulae 9-1 to 9-66 in which at least one hydrogen is substituted with deuterium, groups represented by Formulae 10-1 to 10-249, groups represented by Formulae 10-1 to 10-249 in which at least one hydrogen is substituted with deuterium, -N(Q₁)(Q₂), -Si(Q₃)(Q₄)(Q₅), and - Ge(Q₃)(Q₄)(Q₅) (wherein Q₁ to Q₅ are each independently the same as described above); and/or
R₁₂ in Formula 2B may be selected from groups represented by Formulae 9-1 to 9-66, groups represented by Formulae 9-1 to 9-66 in which at least one hydrogen is substituted with deuterium, groups represented by Formulae 10-1 to 10-249, and groups represented by Formulae 10-1 to 10-249 in which at least one hydrogen is substituted with deuterium.

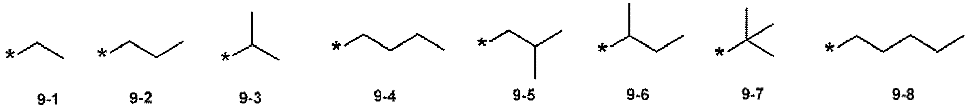

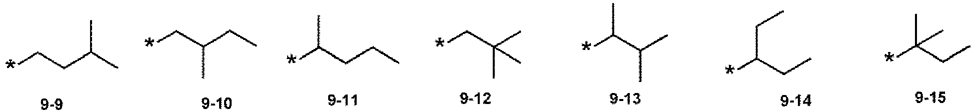

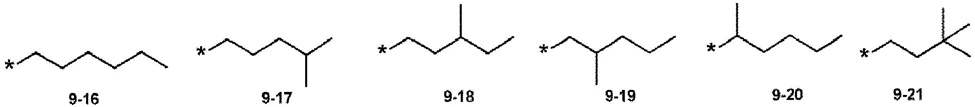

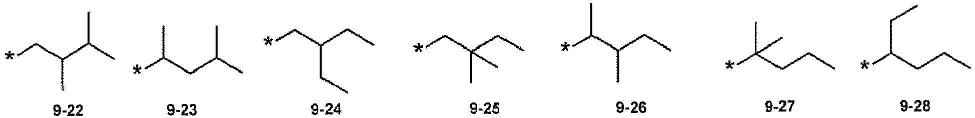

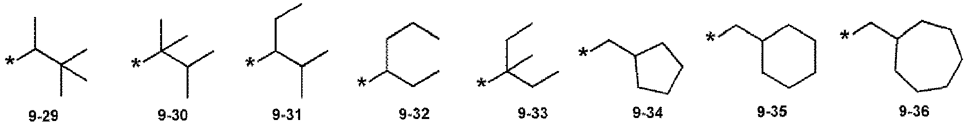

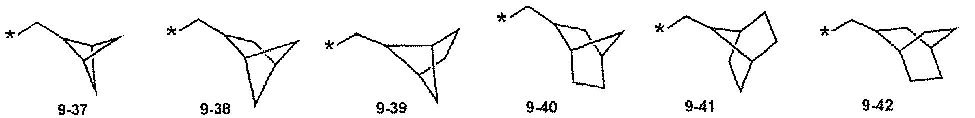

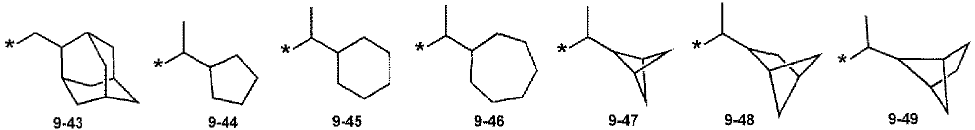

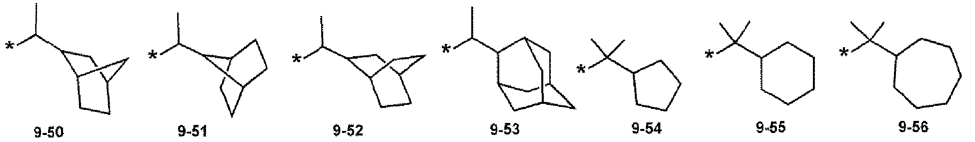

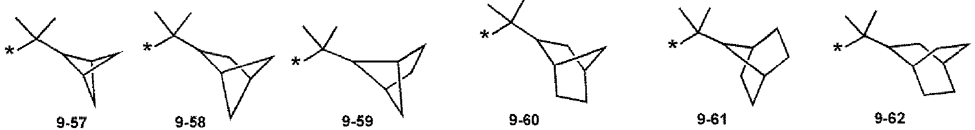

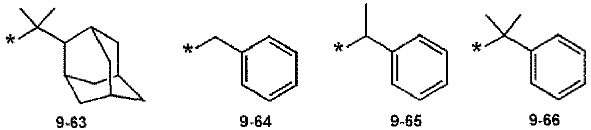

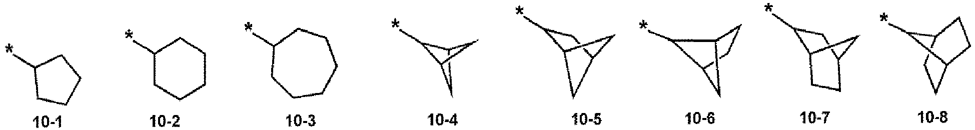

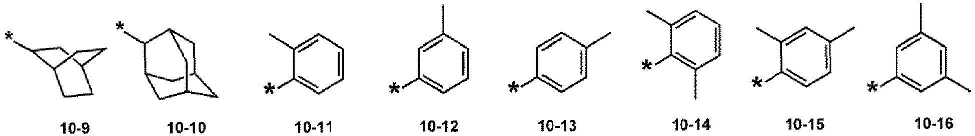

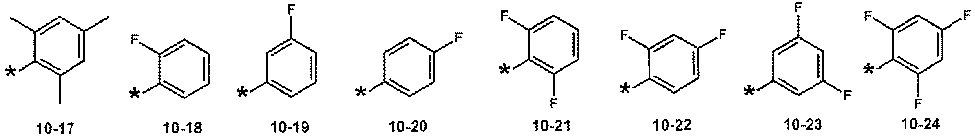

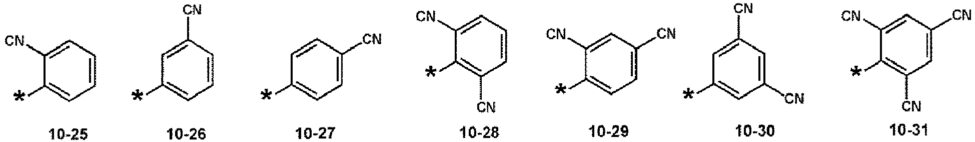

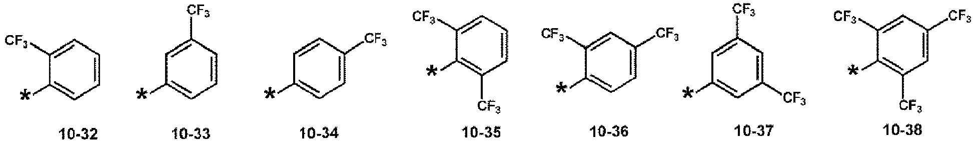

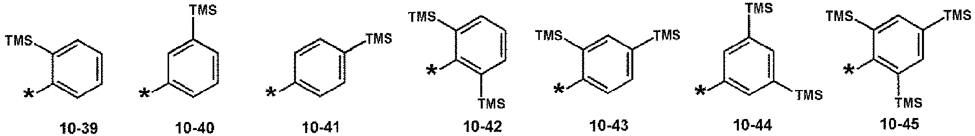

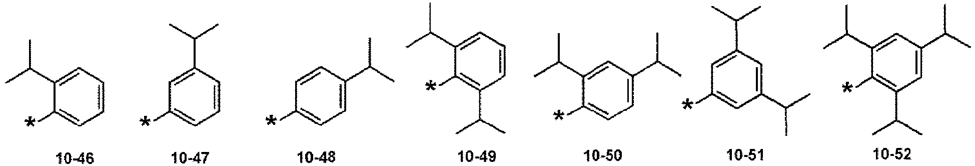

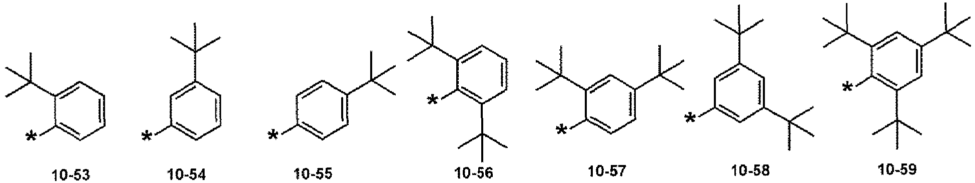

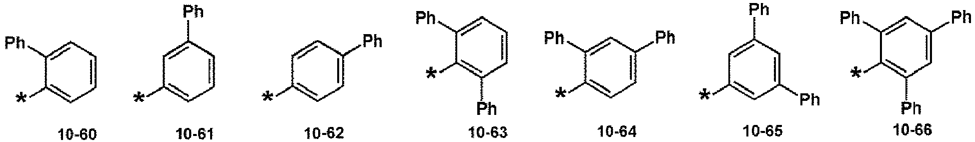

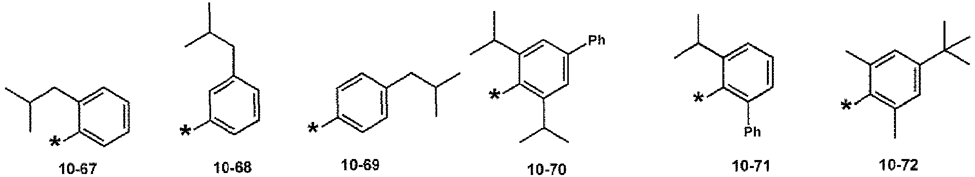

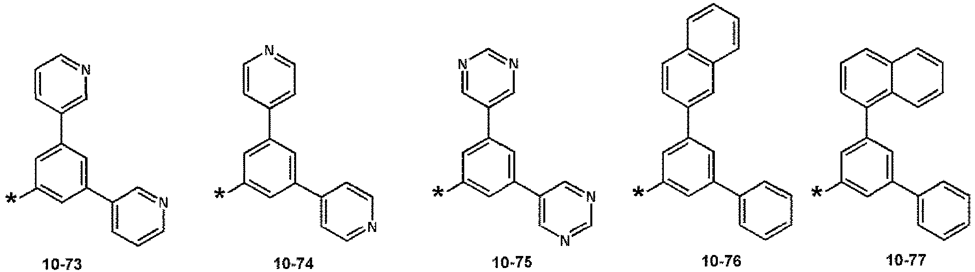

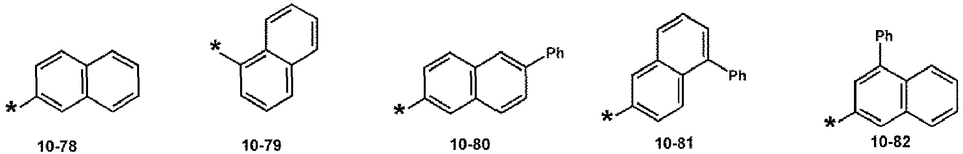

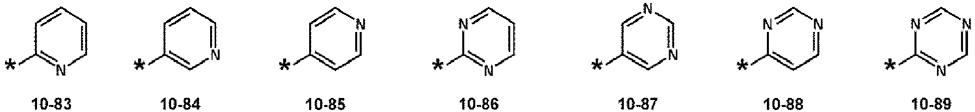

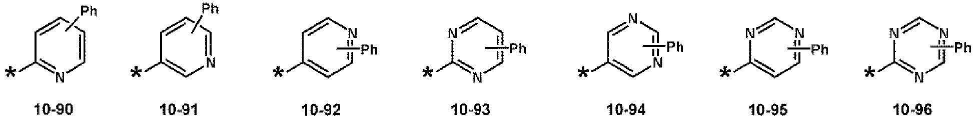

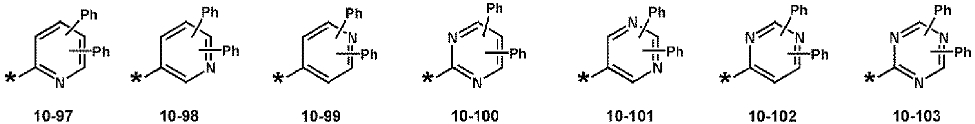

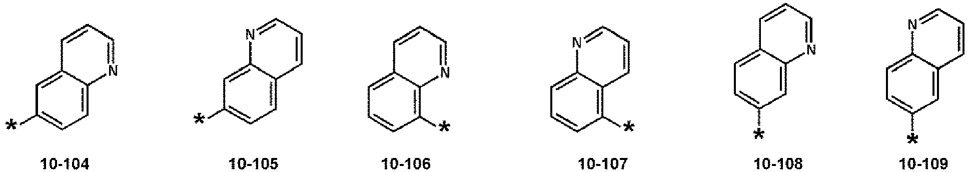

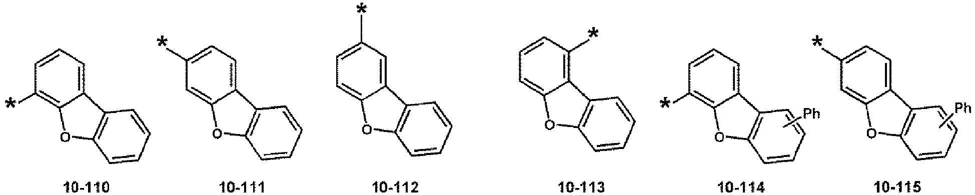

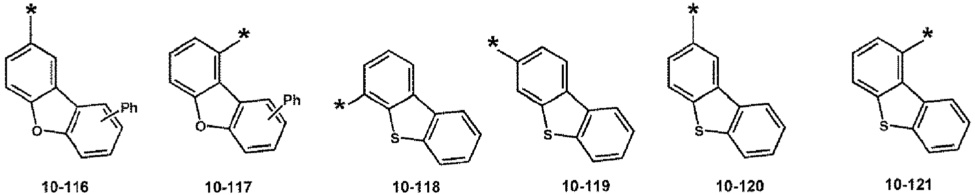

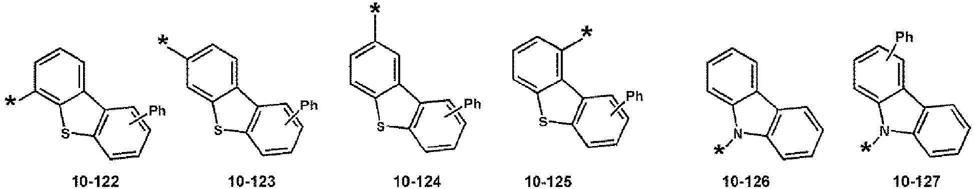

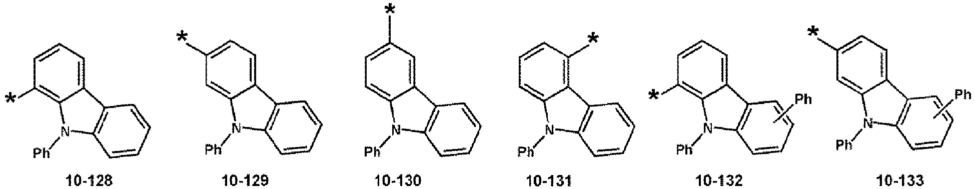

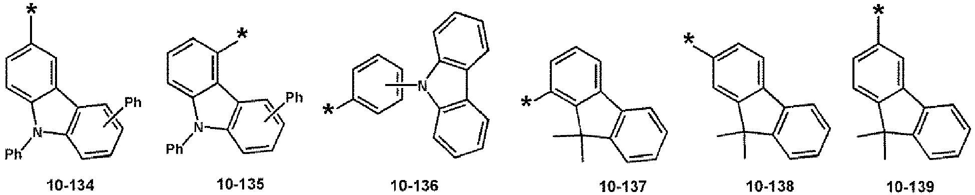

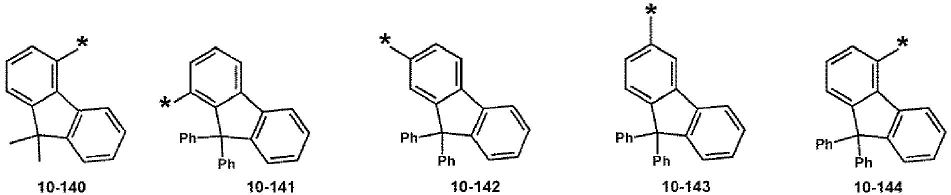

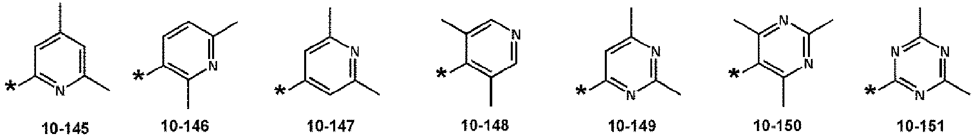

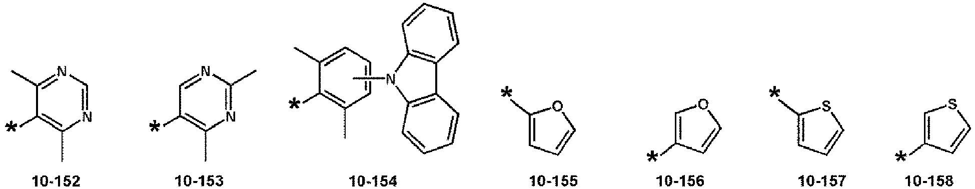

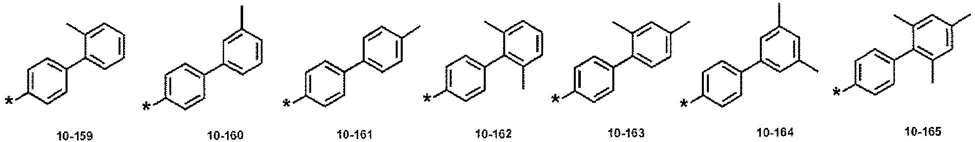

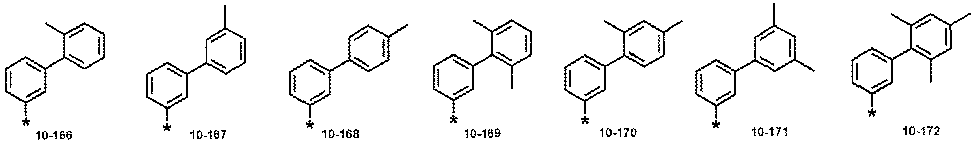

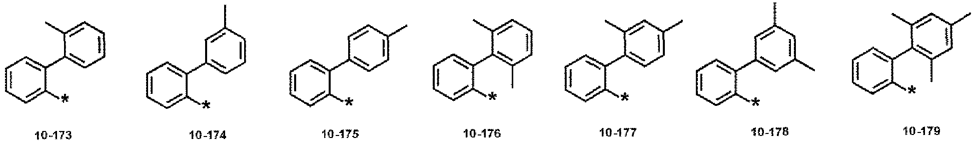

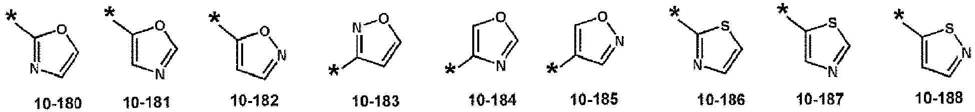

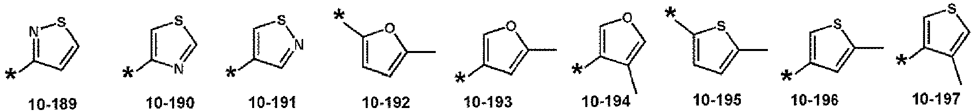

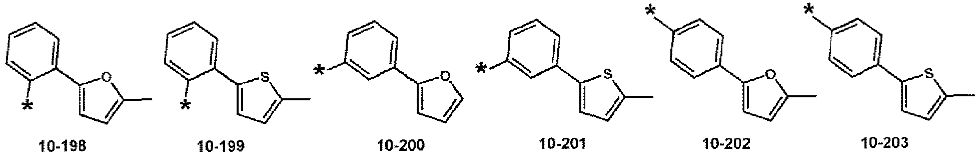

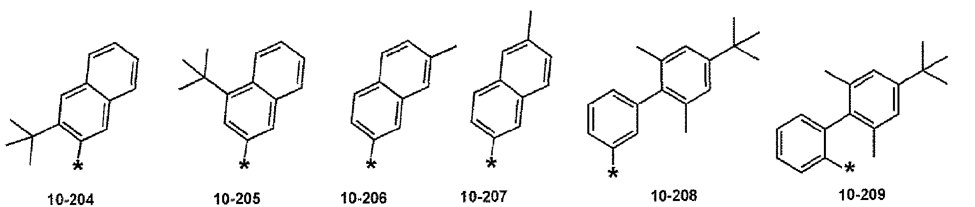

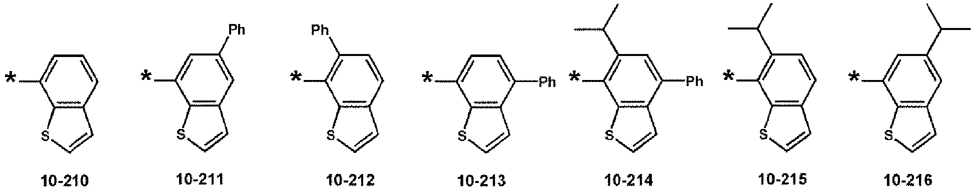

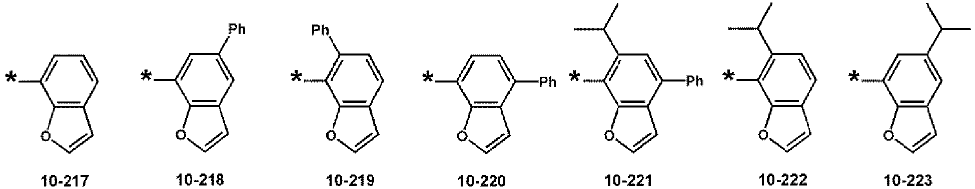

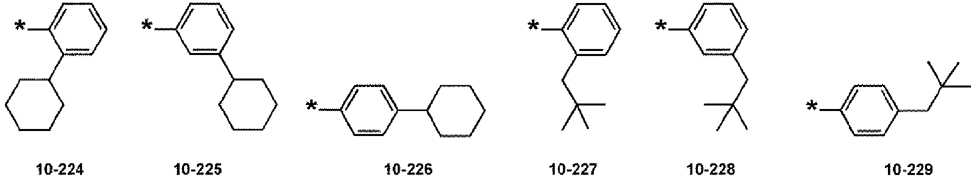

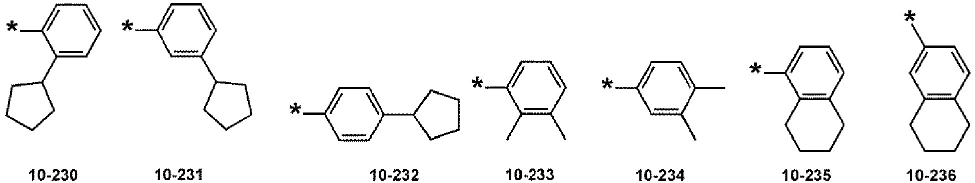

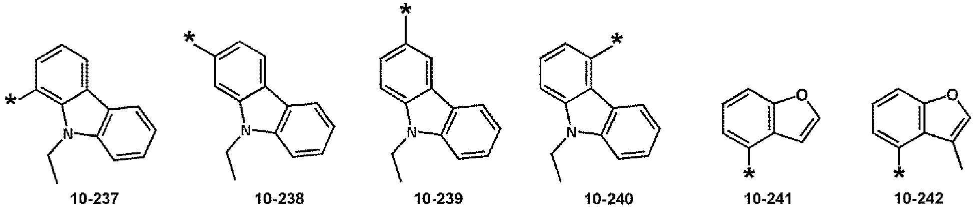

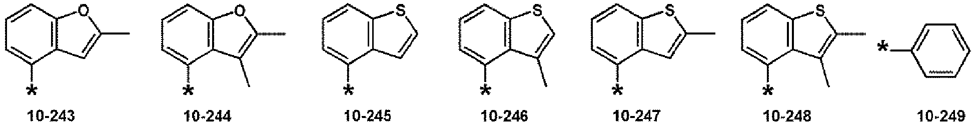

In Formulae 9-1 to 9-66 and Formulae 10-1 to 10-249, * indicates a binding site to a neighboring atom, Ph indicates a phenyl group, and TMS indicates a trimethylsilyl group. As used herein, "The groups represented by Formulae 9-1 to 9-66 in which at least one hydrogen is substituted with deuterium" may be, for example, groups represented by Formulae 9-501 to 9-552:

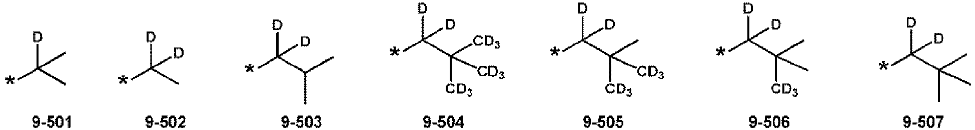

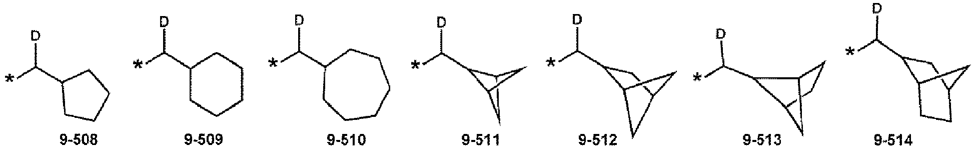

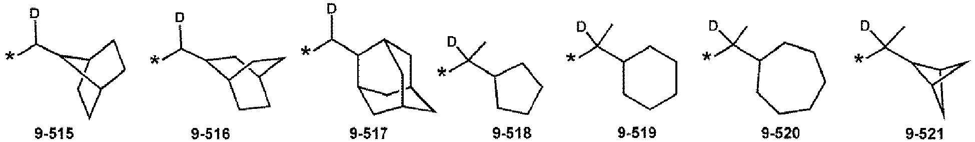

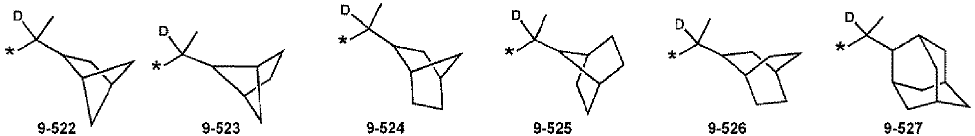

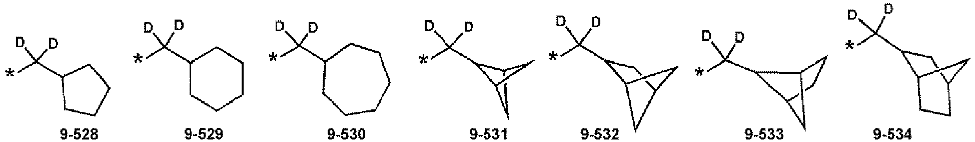

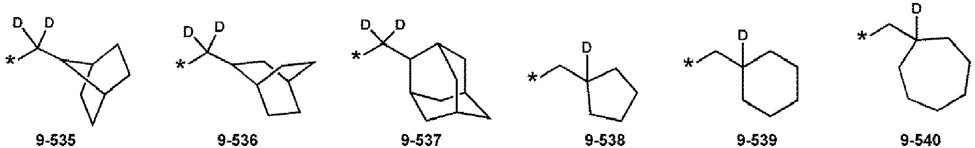

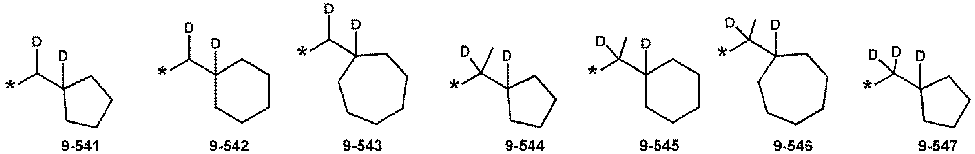

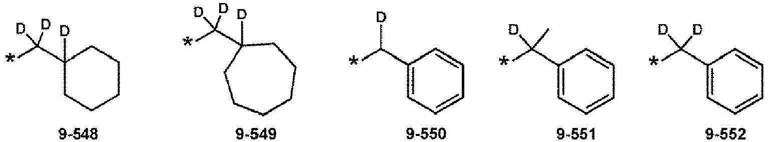

As used herein, "The groups represented by Formulae 10-1 to 10-249 in which at least one hydrogen is substituted with deuterium" may be, for example, groups represented by Formulae 10-501 to 10-510:

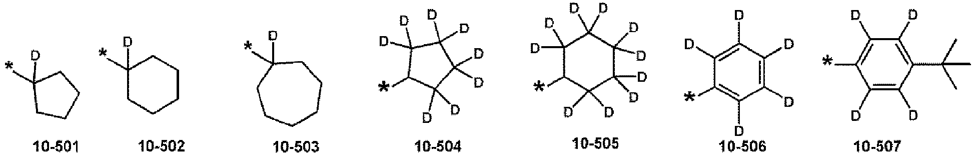

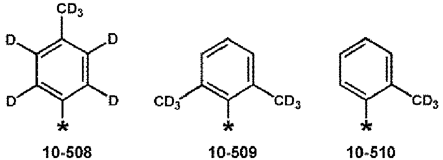

In an embodiment, in Formula 2A, Z₁ may be selected from hydrogen, deuterium, -F, a cyano group, a substituted or unsubstituted C₁-C₆₀ alkyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, - N(Q₁)(Q₂), -Si(Q3)(Q4)(Q5), and -Ge(Q3)(Q4)(Q5), wherein Q₁ to Q₅ are the same as described above.

In Formula 2A, a1 indicates the number of groups Z₁, and may be an integer of 0 to 3. When a1 is two or more, two or more groups Z₁ may be identical to or different from each other.

In Formulae 2A and 2B, a2, a3, and b1 each indicate the number of groups Z₂, Z₃, and R₁₄, respectively, and may each independently be an integer of 0 to 20. When a2 is two or more, two or more groups Z₂ may be identical to or different from each other, when a3 is two or more, two or more groups Z₃ may be identical to or different from each other, and when b1 is two or more, two or more groups R₁₄ may be identical to or different from each other. For example, a2, a3, and b1 may each independently be an integer of 0 to 10, but embodiments of the present disclosure are not limited thereto.

c1 may be an integer of 1 to 4, wherein, when c1 is two or more, two or more groups represented by 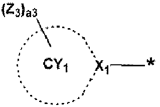 may be identical to or different from each other. For example, c1 may be 1 or 2.

In Formulae 2A and 2B, i) two or more groups selected from a plurality of groups Z₁ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, ii) two or more groups selected from a plurality of groups Z₂ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, iii) two or more groups selected from a plurality of groups Z₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, iv) R₁₂ and R₁₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, v) two or more groups selected from a plurality of groups R₁₄ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, or vi) two or more groups selected from groups Z₁, Z₂, and R₁₁ to R₁₄ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ. Here, examples of the C₅-C₃₀ carbocyclic group that is unsubstituted or substituted with at least one R₁₀ₐ or the C₁-C₃₀ heterocyclic group that is unsubstituted or substituted with at least one R₁₀ₐ include a benzene group, a cyclopentane group, a cyclopentadiene group, a bicyclo[2.2.1]heptane group, a furan group, a thiophene group, a pyrrole group, a silole group, an indene group, a benzofuran group, a benzothiophene group, an indole group, or a benzosilole group, each unsubstituted or substituted with at least one R₁₀ₐ, wherein R₁₀ₐ is defined the same as Z₁. The C₅-C₃₀ carbocyclic group and the C₁-C₃₀ heterocyclic group will be additionally described below.

In Formulae 2A and 2B, * and *' each indicate a binding site to M in Formula 1.

In an embodiment, a group represented by 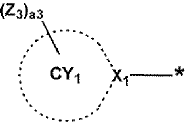 in Formula 2A may be selected from a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, each unsubstituted or substituted with group Z₃ in the number of a3.

a3, Z₃, and X₁ are the same as described above.

In an embodiment, the group represented by 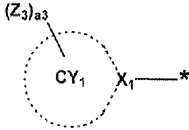 in Formula 2A may be selected from a phenyl group, a biphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, and an imidazopyrimidinyl group, each unsubstituted or substituted with group Z₃ in the number of a3.

In an embodiment, a group represented by 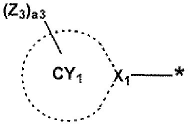 in Formula 2A may be a group represented by one selected from Formulae CY1(1) to CY1(20):

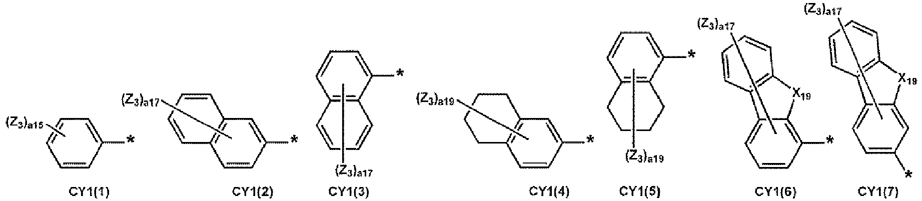

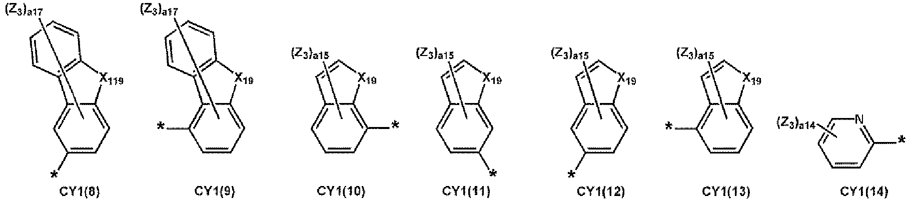

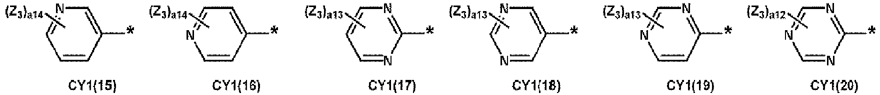

In Formulae CY1(1) to CY1(20),
Z₃ may be the same as described above,
X₁₉ may be O, S, N(Z₄), C(Z₄)(Z₅), or Si(Z₄)(Z₅),
Z₄ and Z₅ may each independently be the same as described in connection with Z₃,
a12 may be an integer from 0 to 2,
a13 may be an integer from 0 to 3,
a14 may be an integer from 0 to 4,
a15 may be an integer from 0 to 5,
a17 may be an integer from 0 to 7,
a19 may be an integer from 0 to 9, and
* indicates a binding site to a neighboring carbon atom.

In an embodiment, a group represented by 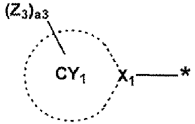 in Formula 2A may be a group represented by one selected from Formulae 10-10(1) to 10-10(18) and 10-249:

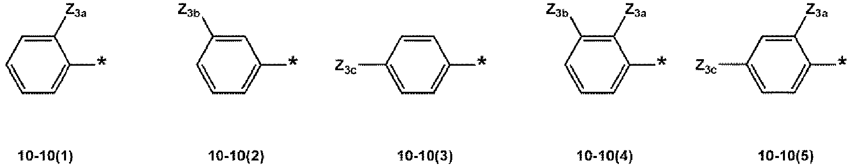

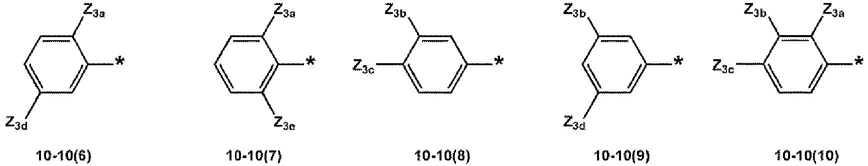

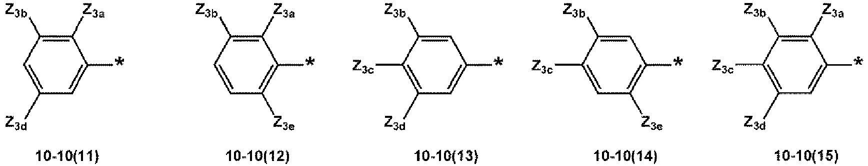

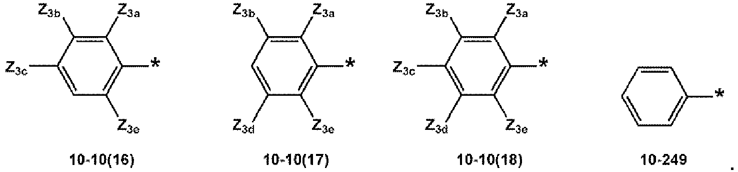

In Formulae 10-10(1) to 10-10(18), Z₃ₐ to Z₃ₑ may each independently be the same as described in connection with Z₃, wherein Z₃ₐ to Z₃ₑ may not be hydrogen, and * indicates a binding site to a neighboring carbon atom.

In an embodiment, a group represented by 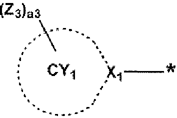 in Formula 2A may be selected from groups represented by Formulae 10-1 to 10-249 and groups represented by Formulae 10-1 to 10-249 in which at least one hydrogen is substituted with deuterium, but embodiments of the present disclosure are not limited thereto.

In an embodiment, a group represented by 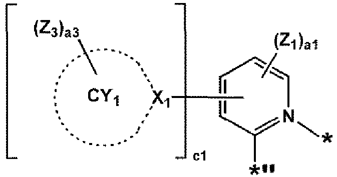 in Formula 2A may be a group represented by one selected from Formulae CY1-1 to CY1-56:

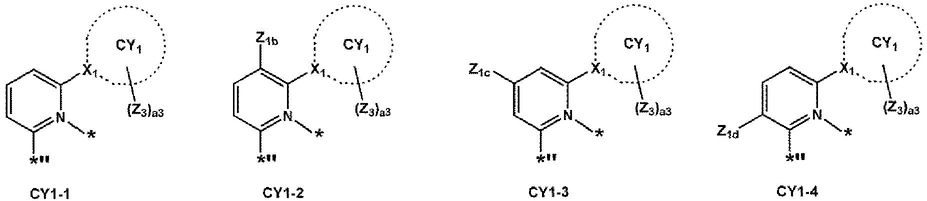

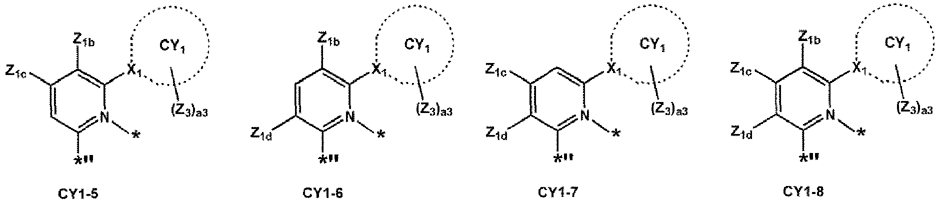

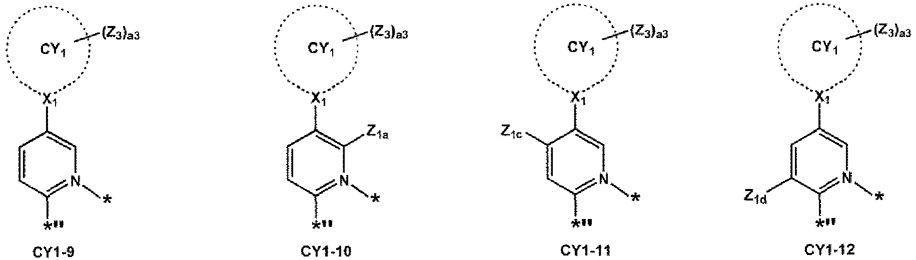

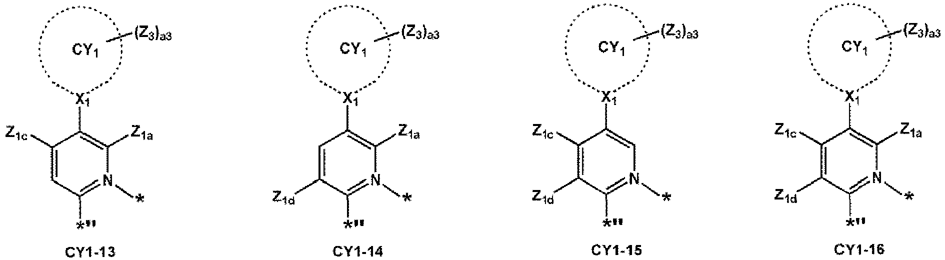

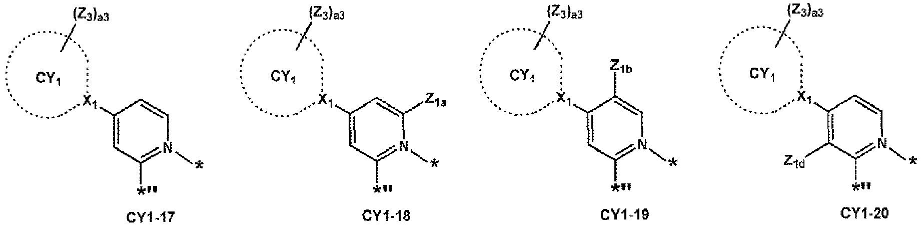

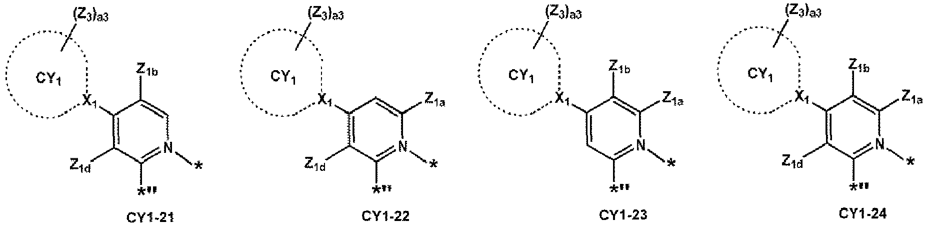

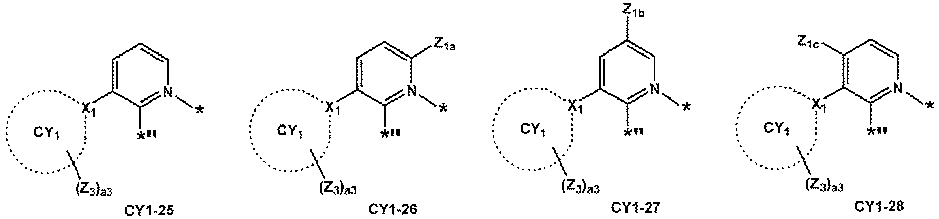

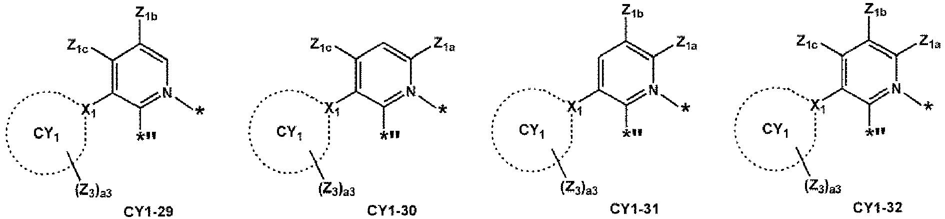

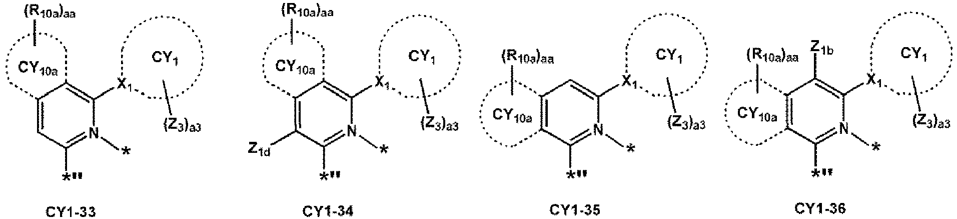

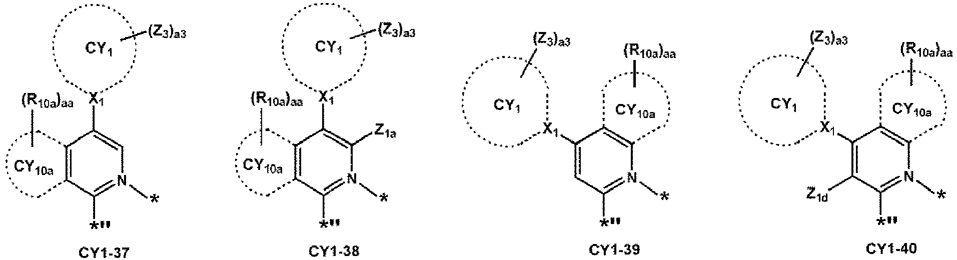

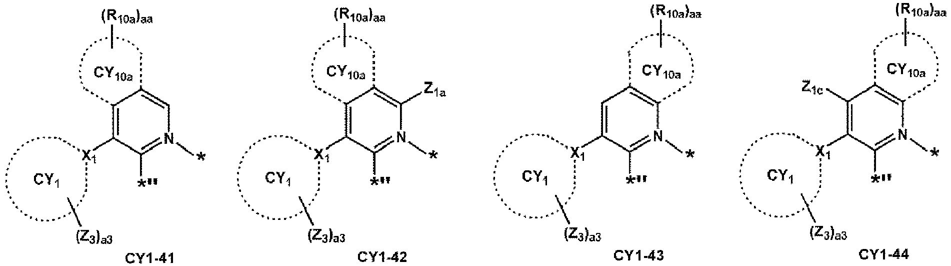

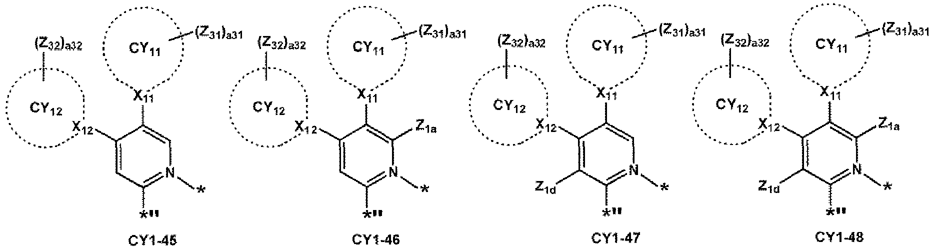

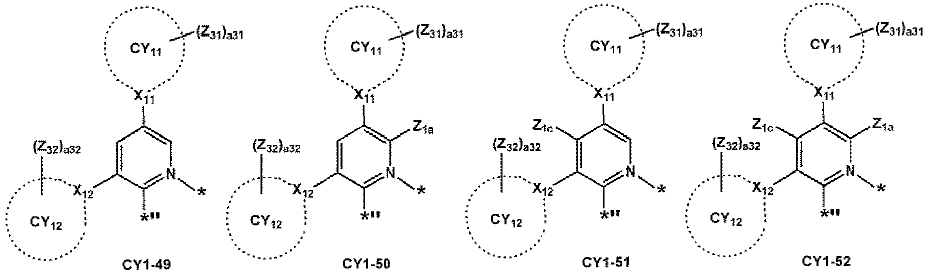

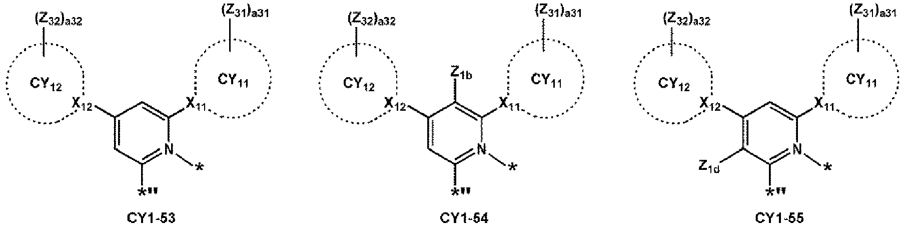

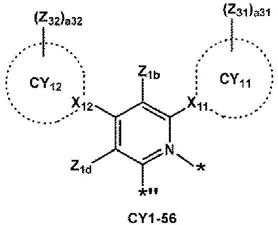

In Formulae CY1-1 to CY1-56,
X₁, ring CY₁, Z₃, and a3 may each independently be the same as described herein,
Z₁ₐ to Z_{1d} may each independently be the same as described in connection with Z₁, wherein Z₁ₐ to Z_{1d} may not be hydrogen,
X₁₁ and X₁₂ may each independently be the same as described in connection with X₁,
ring CY₁₁ and ring CY₁₂ may each independently be the same as described in connection with ring CY₁,
Z₃₁ and Z₃₂ may each independently be the same as described in connection with Z₃,
a31 and a32 may each independently be the same as described in connection with a3,
ring CY₁₀ₐ may be a C₅-C₃₀ carbocyclic group or a C₁-C₃₀ heterocyclic group,
R₁₀ₐ may be the same as described above,
aa may be an integer from 0 to 10, and
* indicates a binding site to M in Formula 1, and *" indicates a binding site to ring CY₂ in Formula 2A.

In an embodiment, a group represented by 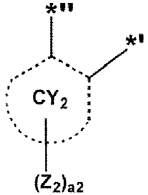 in Formula 2A may be a group represented by one selected from Formulae CY2-1 to CY2-64:

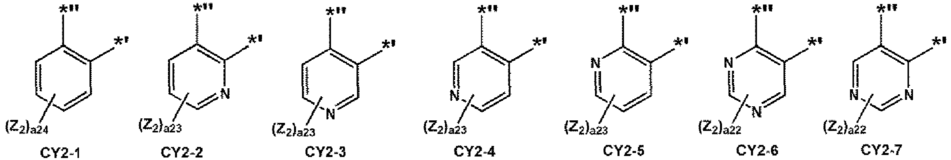

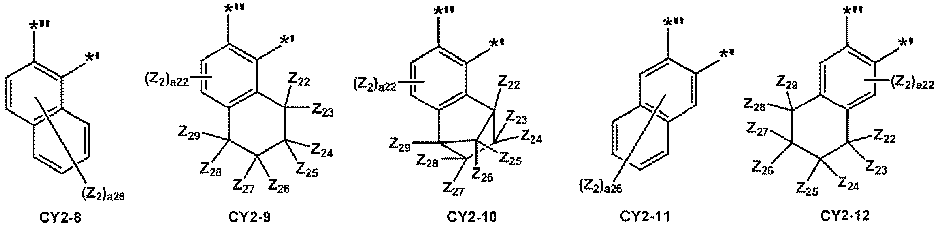

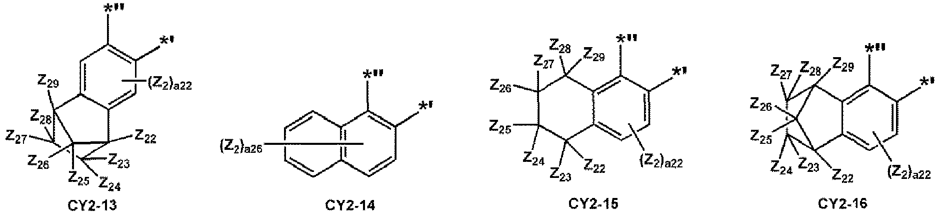

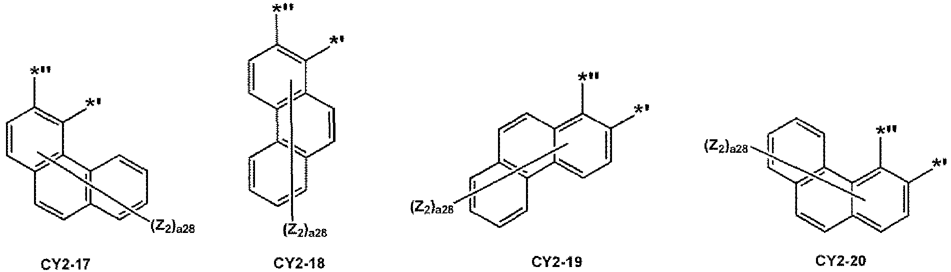

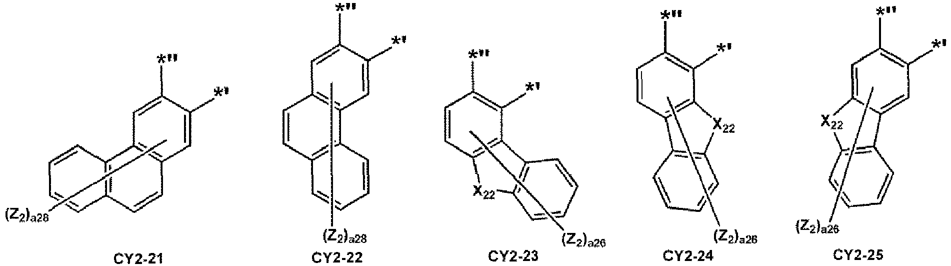

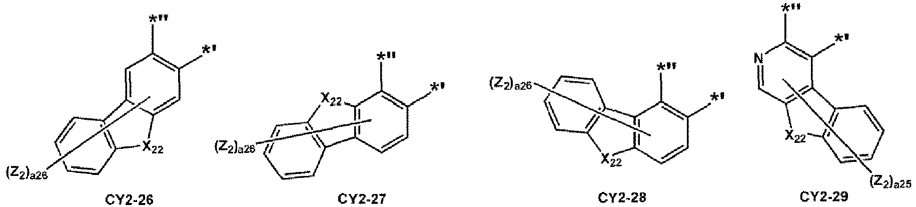

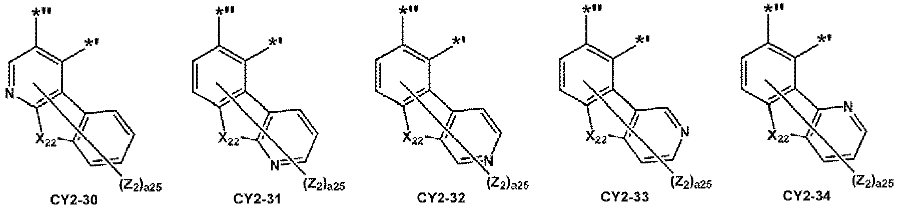

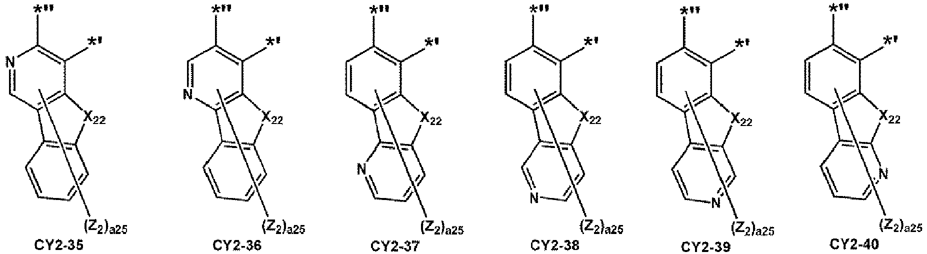

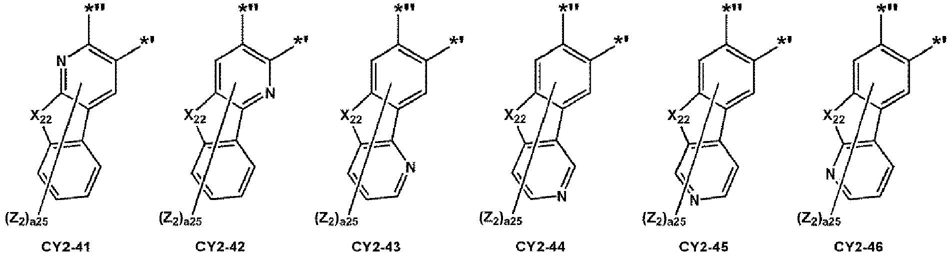

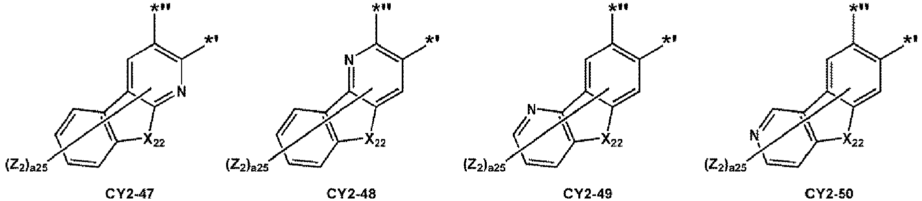

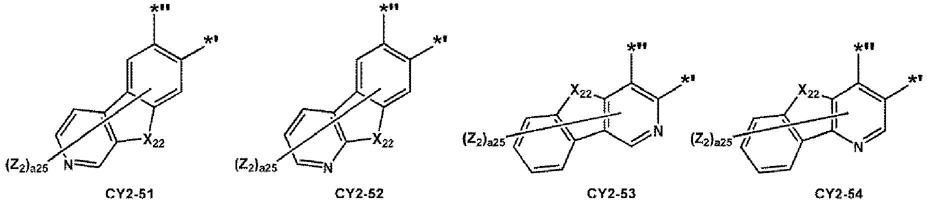

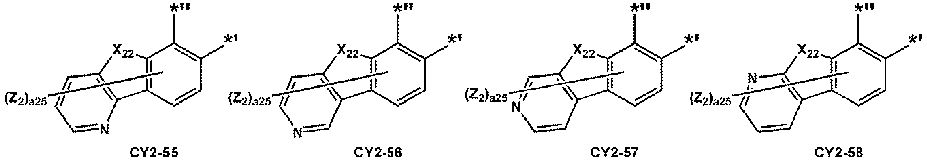

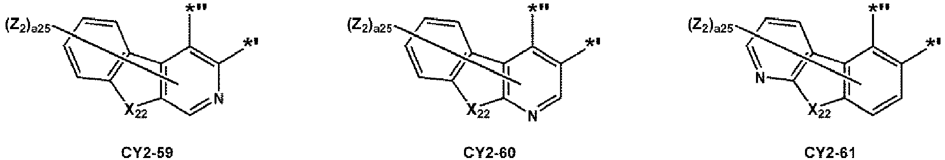

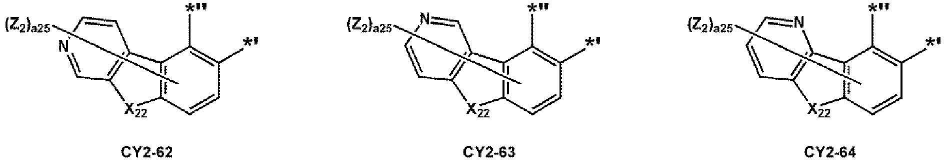

In Formulae CY2-1 to CY2-64,
Z₂ may be the same as described above,
X₂₂ may be C(Z₂₂)(Z₂₃), N(Z₂₂), O, S, or Si(Z₂₂)(Z₂₃),
Z₂₂ to Z₂₉ may each independently be the same as described in connection with Z₂,
a28 may be an integer from 0 to 8,
a26 may be an integer from 0 to 6,
a25 may be an integer from 0 to 5,
a24 may be an integer from 0 to 4,
a23 may be an integer from 0 to 3,
a22 may be an integer from 0 to 2,

*" indicates a binding site to a carbon atom of a neighboring pyridine ring in Formula 2A, and
*' indicates a binding site to M in Formula 1.

In an embodiment, a group represented by 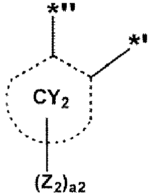 in Formula 2A may be a group represented by one selected from Formulae CY2(1) to CY2(16):

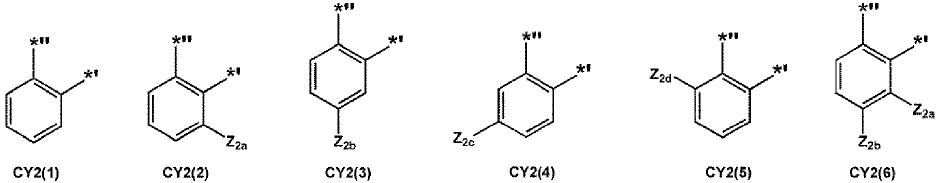

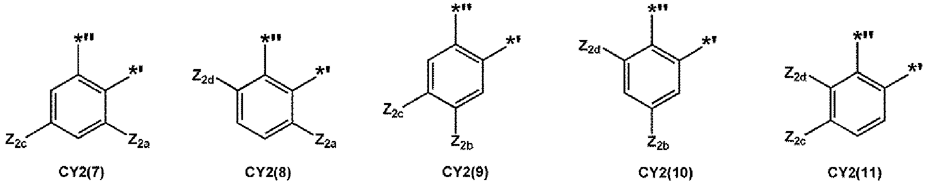

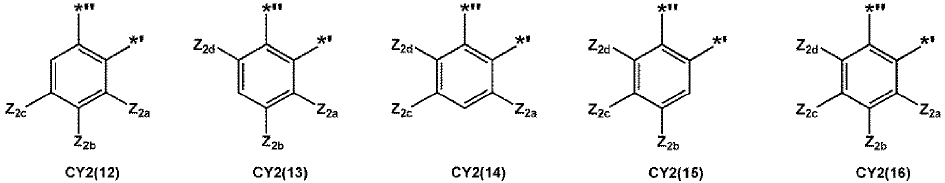

In Formulae CY2(1) to CY2(16),
Z₂ₐ to Z_{2d} may each independently be the same as described in connection with Z₂, wherein Z₂ₐ to Z_{2d} may not be hydrogen,
*" indicates a binding site to a carbon atom of a neighboring pyridine ring in Formula 2A, and
*' indicates a binding site to M in Formula 1.

In an embodiment, a group represented by 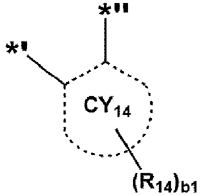 in Formula 2B may be a group represented by one selected from Formulae CY14-1 to CY14-64:

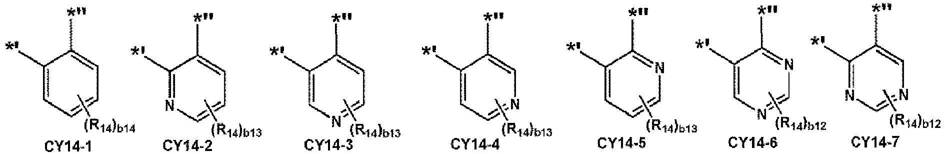

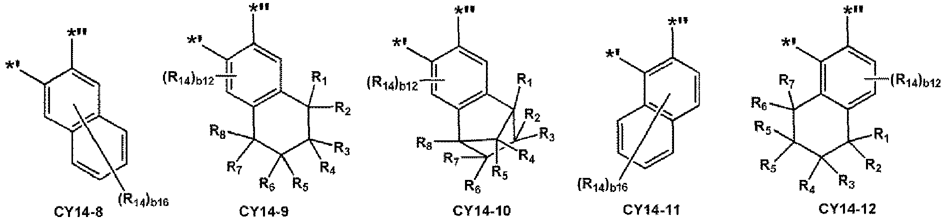

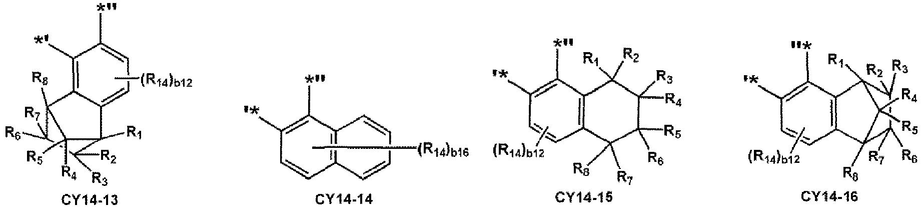

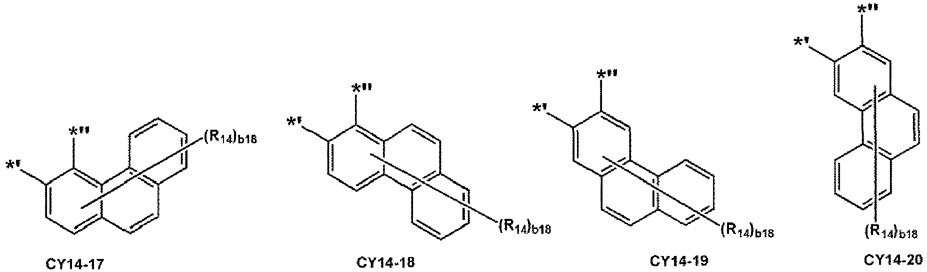

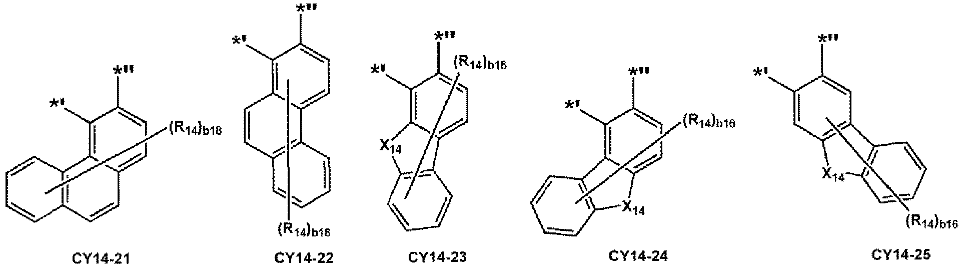

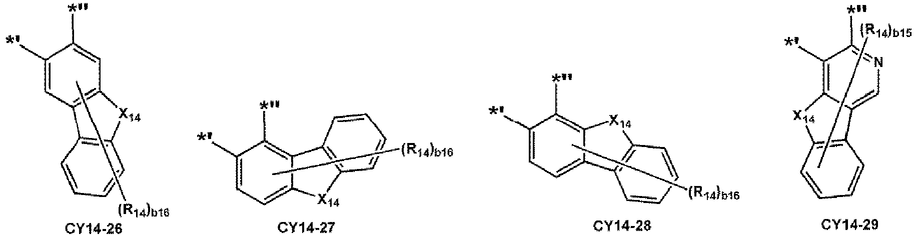

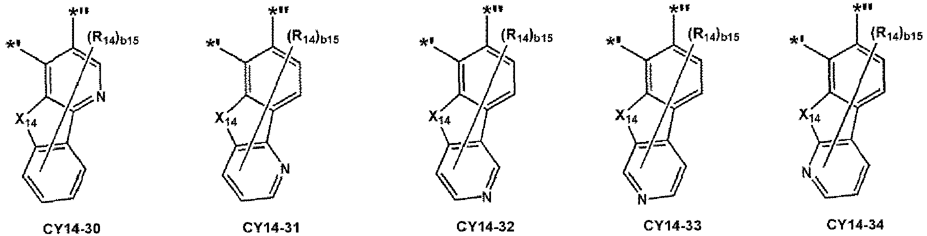

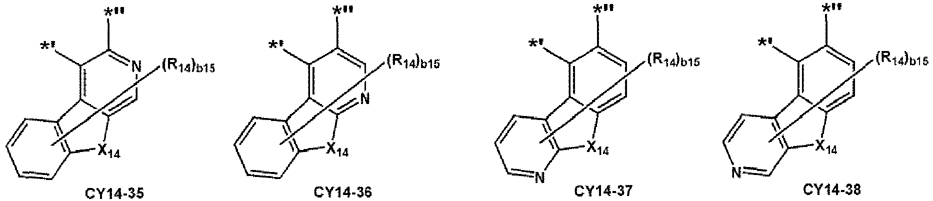

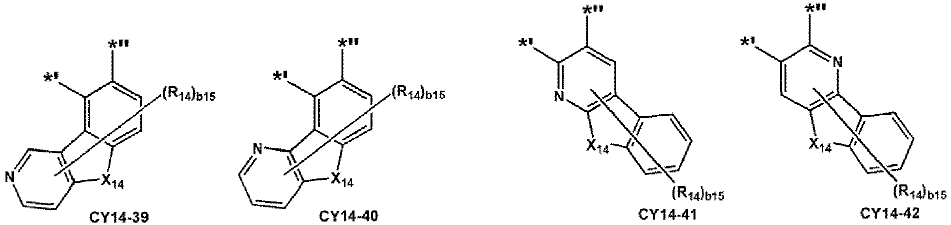

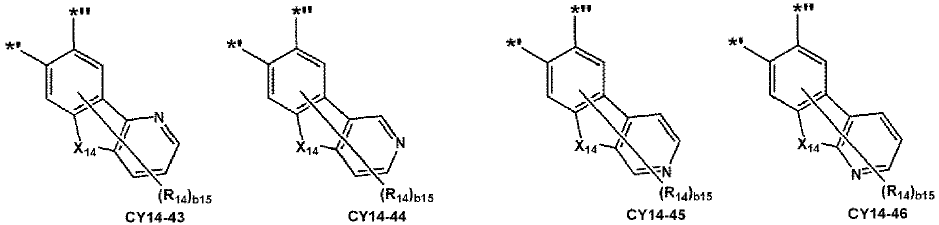

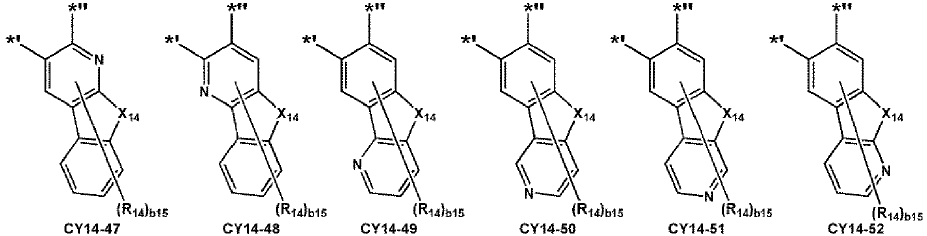

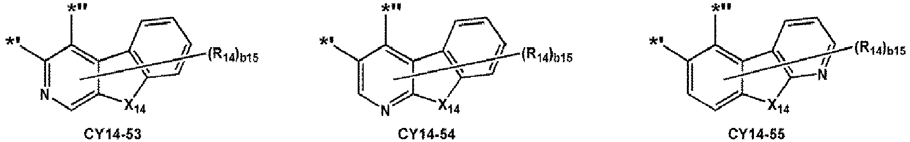

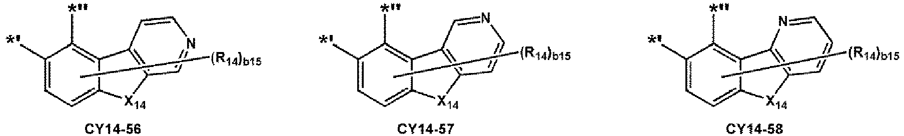

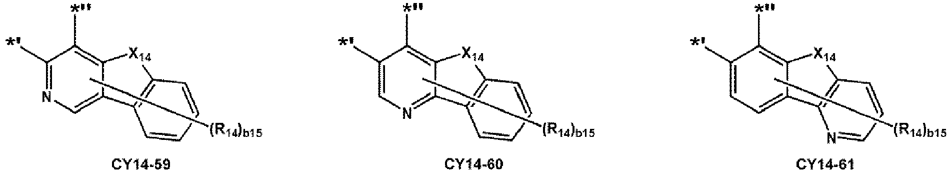

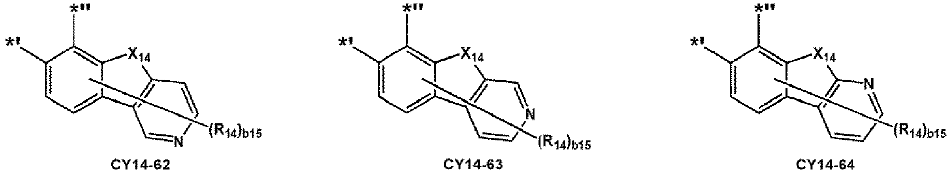

In Formulae CY14-1 to CY14-64,
R₁₄ may be the same as described above,
X₁₄ may be C(R₁)(R₂), N(R₁), O, S, or Si(R₁)(R₂),
R₁ to R₈ may each independently be the same as described in connection with R₁₄,
b18 may be an integer from 0 to 8,
b16 may be an integer from 0 to 6,
b15 may be an integer from 0 to 5,
b14 may be an integer from 0 to 4,
b13 may be an integer from 0 to 3,
b12 may be an integer from 0 to 2,
*" indicates a binding site to a carbon atom of a neighboring pyridine ring in Formula 2B, and
*' indicates a binding site to M in Formula 1.

In an embodiment, a group represented by 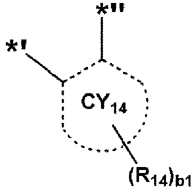 in Formula 2B may be a group represented by one selected from Formulae CY14(1) to CY14(16):

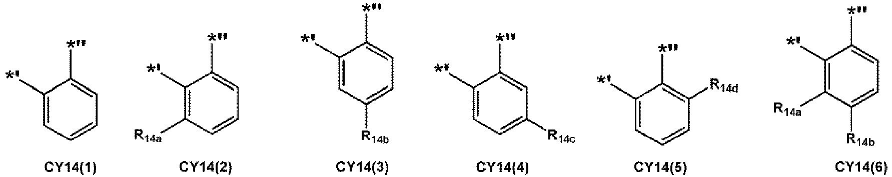

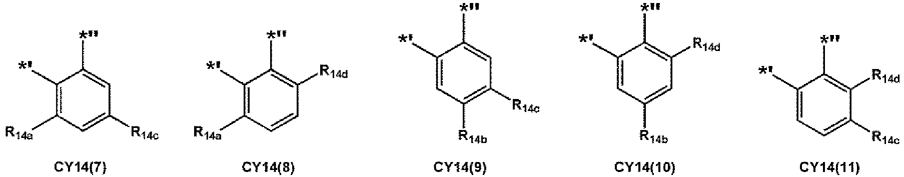

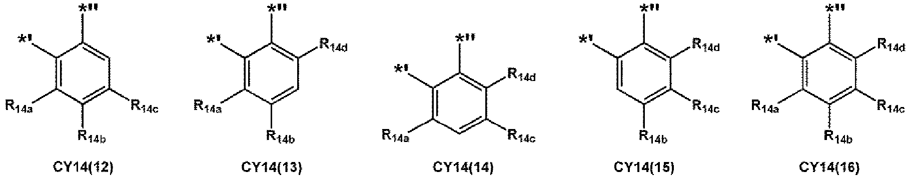

In Formulae CY14(1) to CY14(16),
R₁₄ₐ to R_{14d} may each independently be the same as described in connection with R₁₄, wherein R₁₄ₐ to R_{14d} may not be hydrogen,
*" indicates a binding site to a carbon atom of a neighboring pyridine ring in Formula 2B, and
*' indicates a binding site to M in Formula 1.

In an embodiment, the organometallic compound may be represented by Formula 1A:

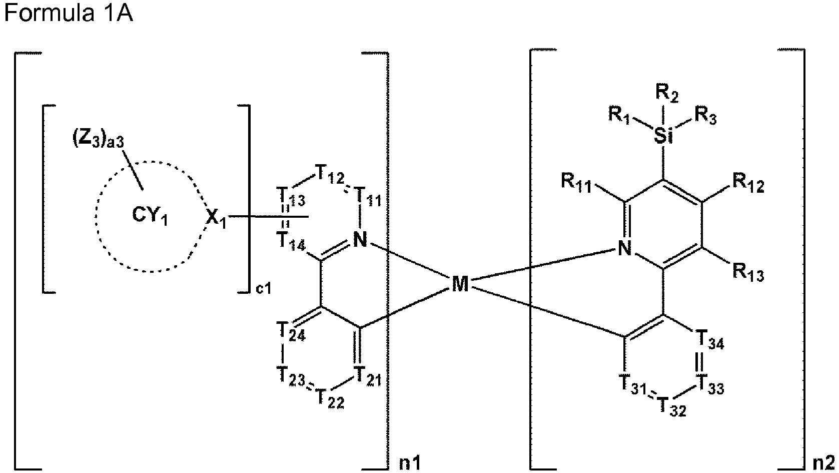

In Formula 1A,
M, n1, n2, X₁, ring CY₁, R₁ to R₃, Z₃, R₁₁ to R₁₃, and c1 may each independently be the same as described herein,
T₁₁ may be N, C(Z₁ₐ), or carbon linked to ring CY₁, T₁₂ may be N, C(Z_{1b}), or carbon linked to ring CY₁, T₁₃ may be N, C(Z_{1c}), or carbon linked to ring CY₁, T₁₄ may be N, C(Z_{1d}), or carbon linked to ring CY₁, at least one selected from T₁₁ to T₁₄ may be carbon linked to ring CY₁, and Z₁ₐ to Z_{1d} may each independently be the same as described in connection with Z₁,
T21 may be N or C(Z₂ₐ), T₂₂ may be N or C(Z_{2b}), T₂₃ may be N or C(Z2c), T₂₄ may be N or C(Z_{2d}), and Z₂ₐ to Z_{2d} may each independently be the same as described in connection with Z₂,
T31 may be N or C(R₁₄ₐ), T₃₂ may be N or C(R_{14b}), T₃₃ may be N or C(R_{14c}), T₃₄ may be N or C(R_{14d}), and R₁₄ₐ to R_{14d} may each independently be the same as described in connection with R₁₄,
two or more groups Z₁ₐ to Z_{1d} may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more groups Z₂ₐ to Z_{2d} may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more groups Z₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₂ and R₁₃ may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more groups R₁₄ₐ to R_{14d} may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more groups Z₁ₐ to Z_{1d}, Z₂ₐ to Z_{2d}, R₁₁ to R₁₃, and R₁₄ₐ to R_{14d} may optionally be linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ, and
R₁₀ₐ may be the same as described above.

In an embodiment, the organometallic compound represented by Formula 1 may satisfy at least one selected from Condition 1 to Condition 5:
Condition 1
   Z₁ in Formula 2A is not -Si(Q₃)(Q₄)(Q₅)
Condition 2
   Z₂ in Formula 2A is not -Si(Q₃)(Q₄)(Q₅)
Condition 3
   Z₃ in Formula 2A is not -Si(Q₃)(Q₄)(Q₅)
Condition 4
   None of R₁₁ to R₁₃ in Formula 2B is -Si(Q₃)(Q₄)(Q₅)
Condition 5
   R₁₄ in Formula 2B is not -Si(Q3)(Q4)(Q5).

In an embodiment, the number of silicon (Si) atoms in the organometallic compound represented by Formula 1 may be 1, 2, or 3.

In an embodiment, the number of silicon (Si) atoms in the organometallic compound represented by Formula 1 may be 1 or 2.

The terms "an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, and an azadibenzothiophene 5,5-dioxide group" as used herein each refer to a heterocyclic group having the same structural backbone as that of each of "an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, and a dibenzothiophene 5,5-dioxide group", respectively, wherein at least one of ring-forming carbons of the rings above is substituted with nitrogen.

For example, the organometallic compound may be one selected from Compounds 1 to 420, but embodiments of the present disclosure are not limited thereto:

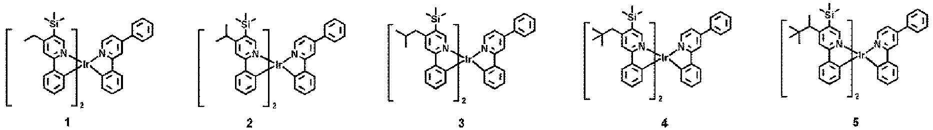

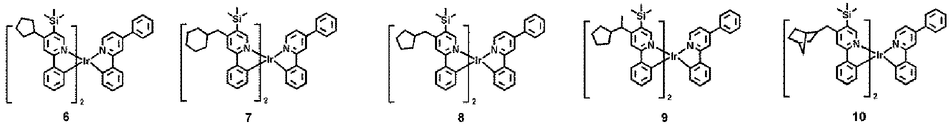

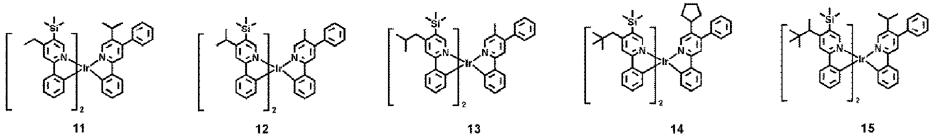

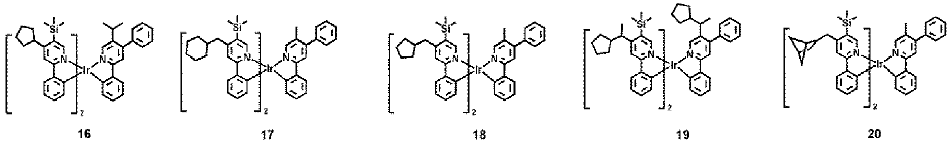

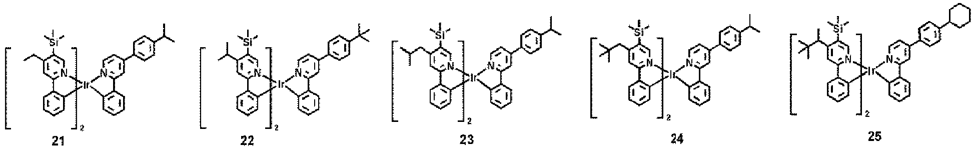

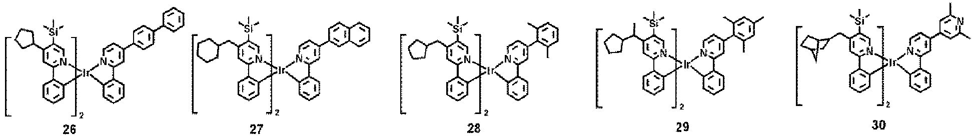

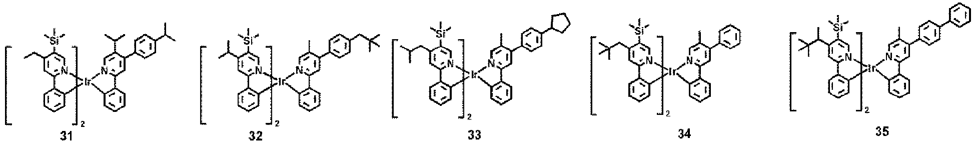

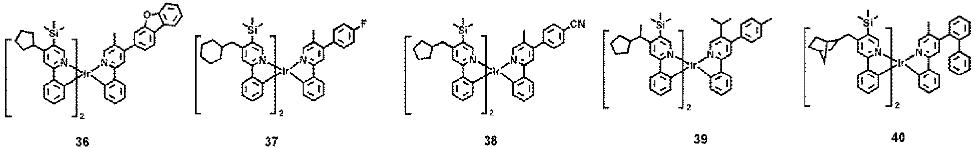

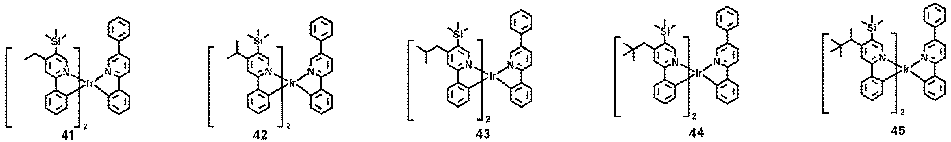

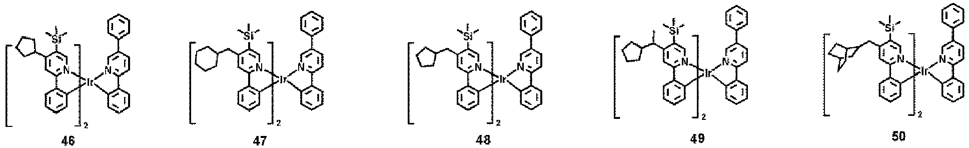

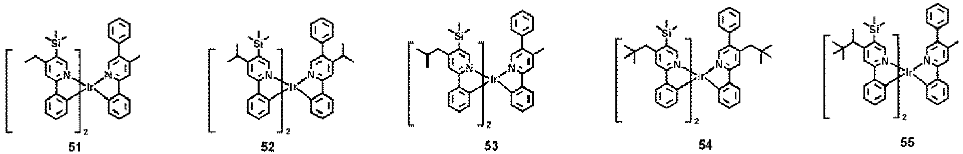

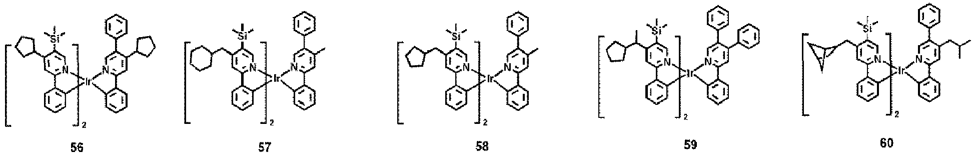

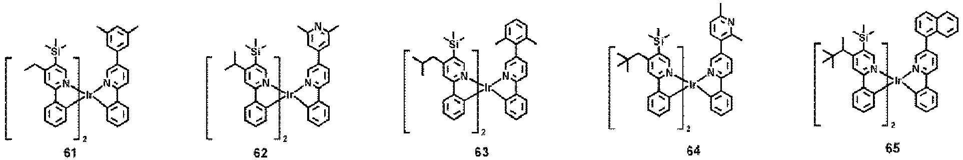

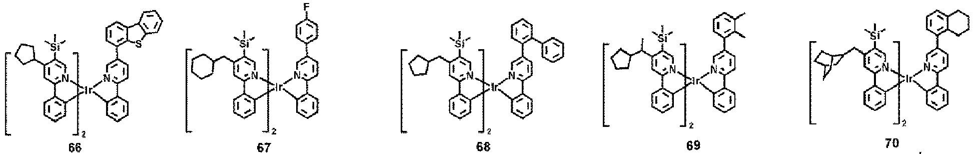

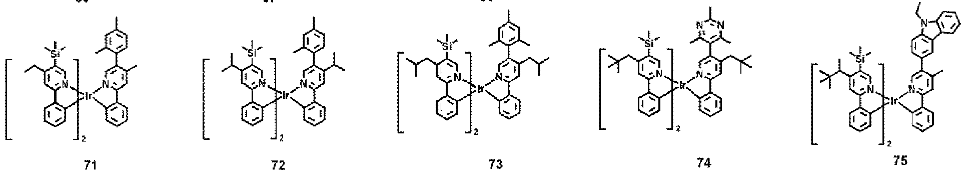

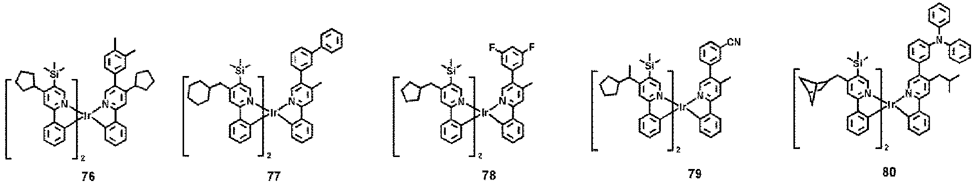

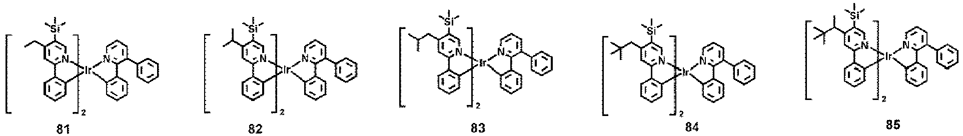

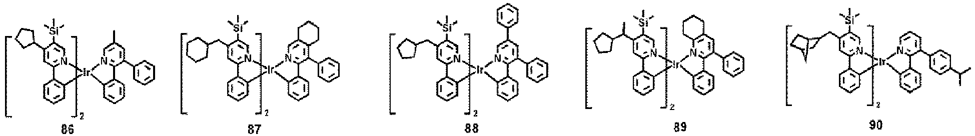

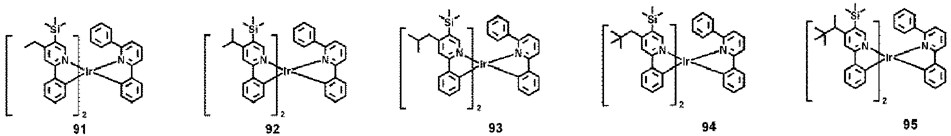

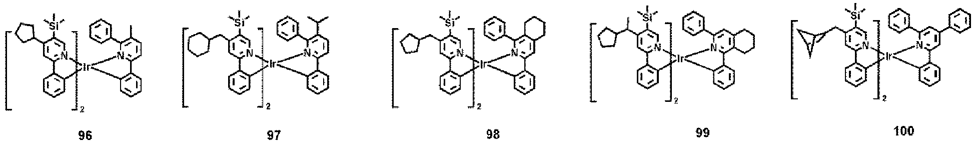

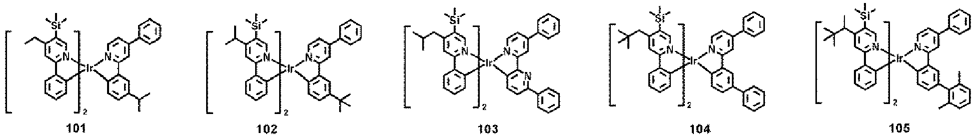

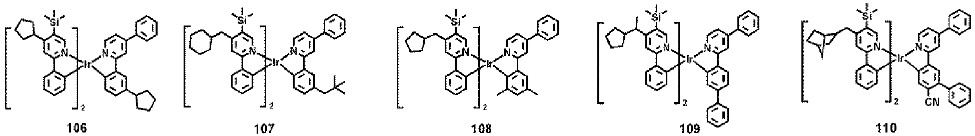

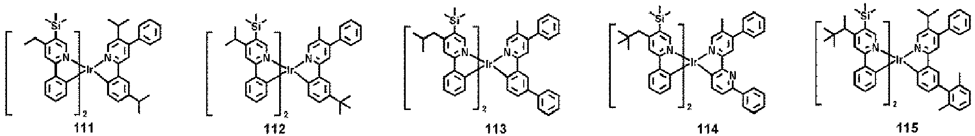

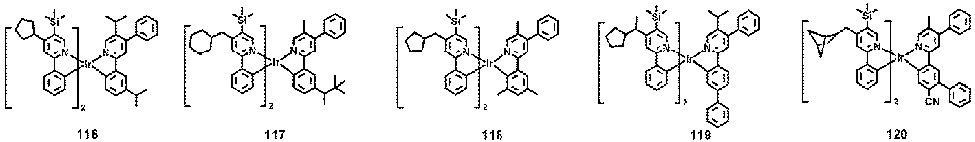

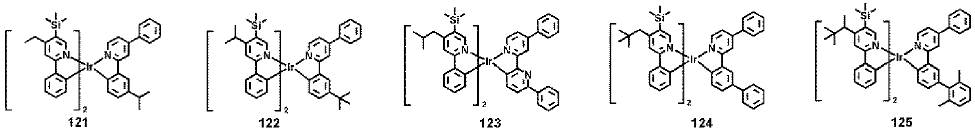

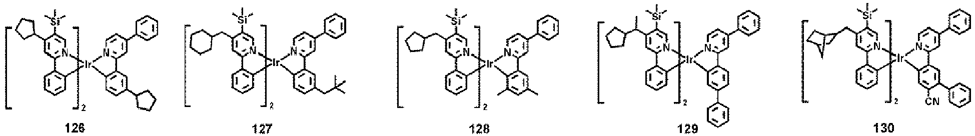

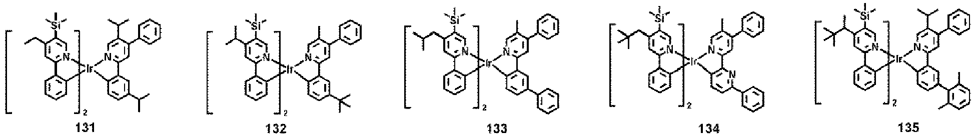

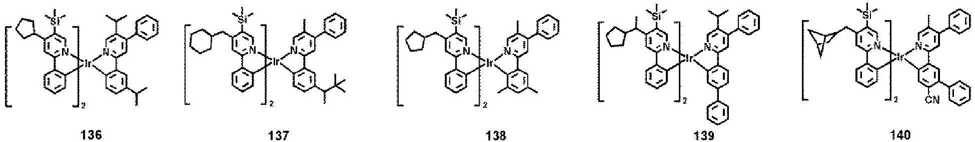

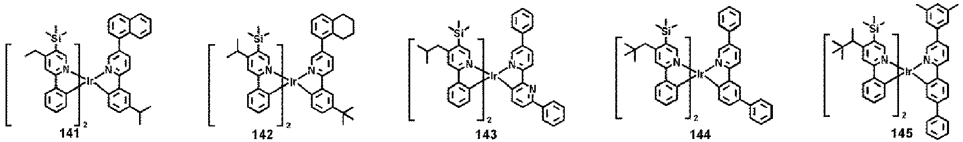

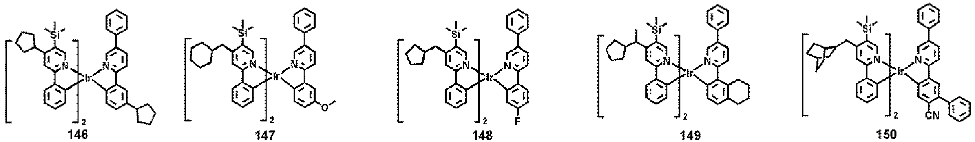

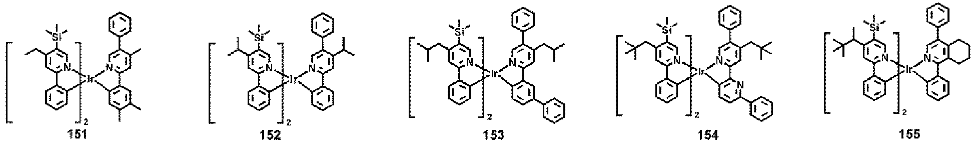

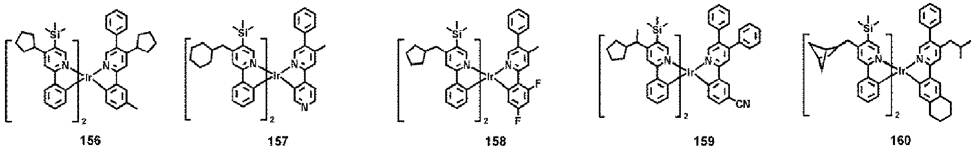

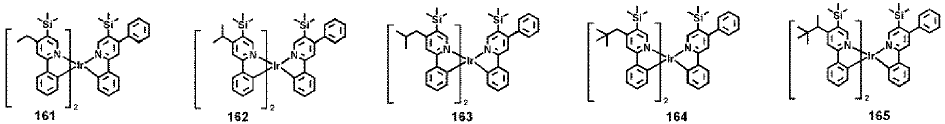

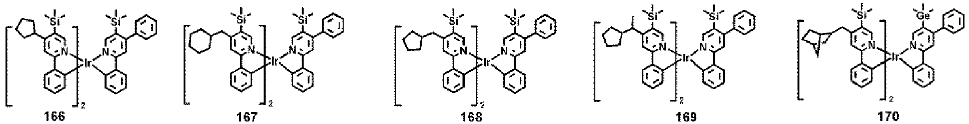

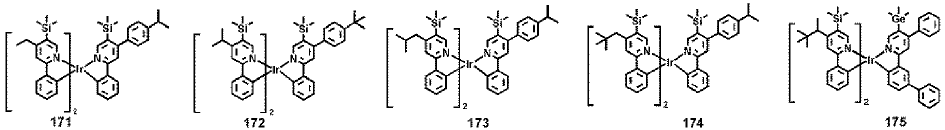

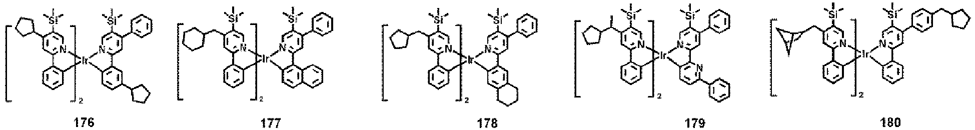

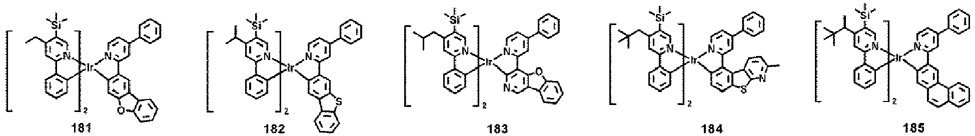

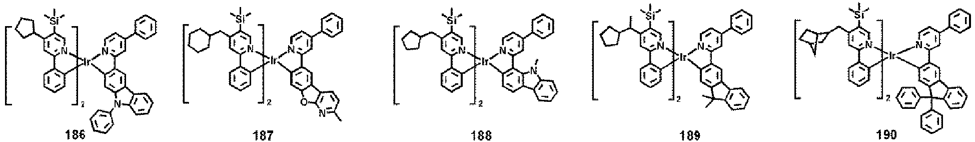

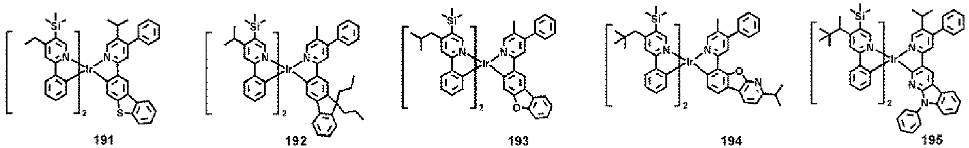

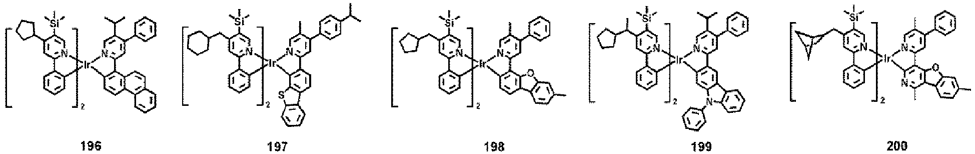

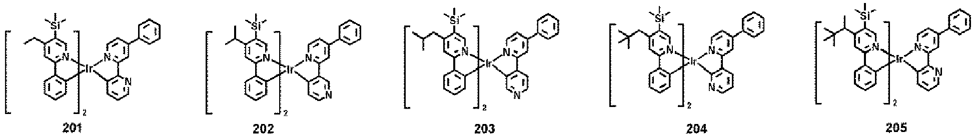

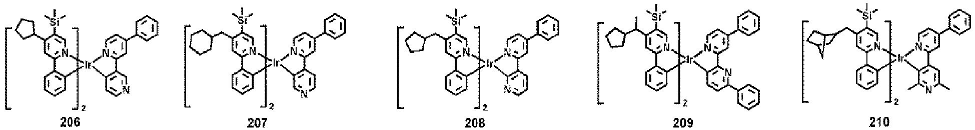

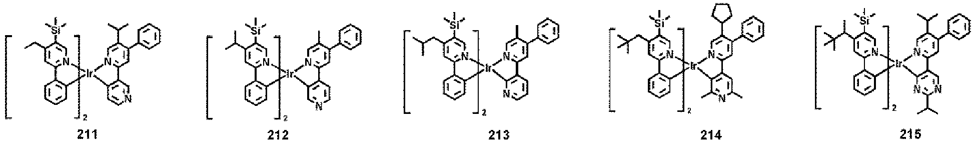

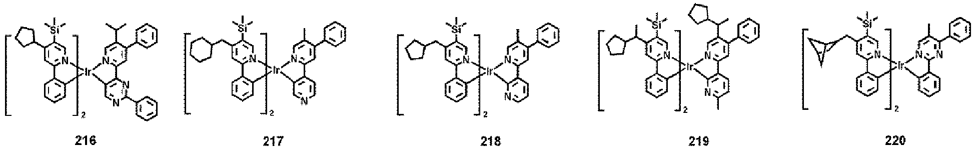

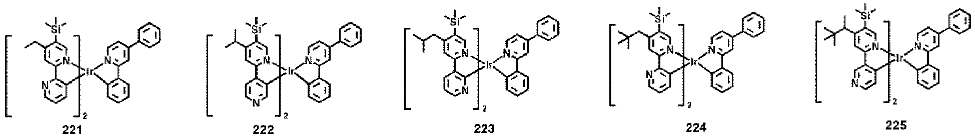

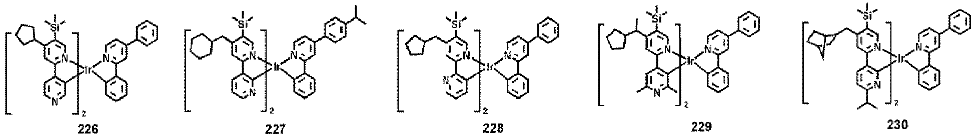

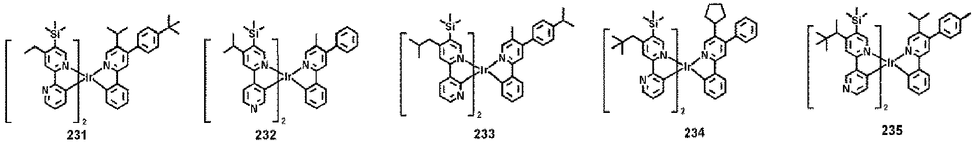

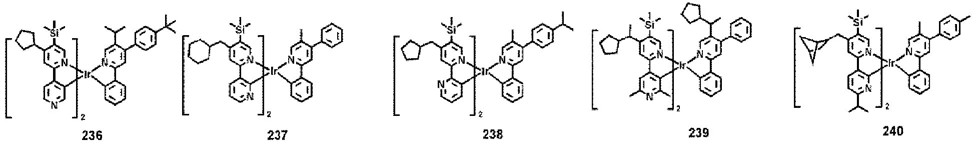

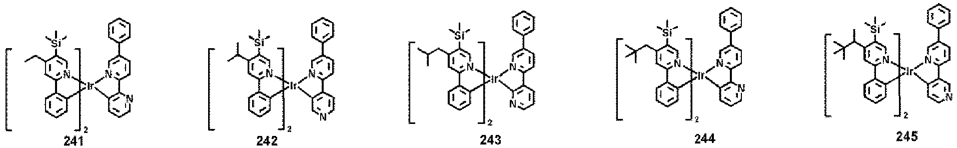

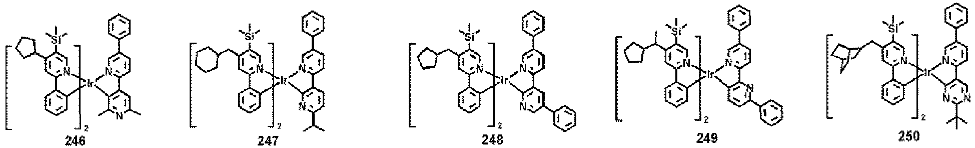

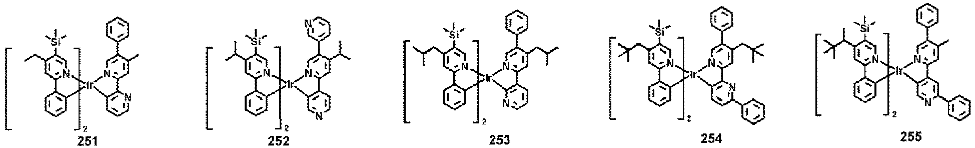

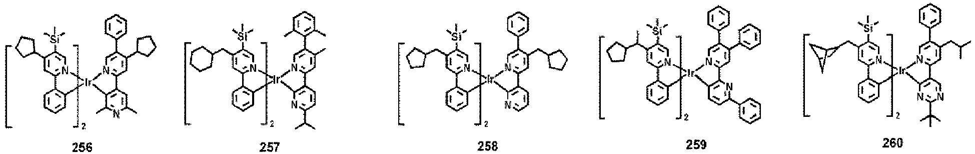

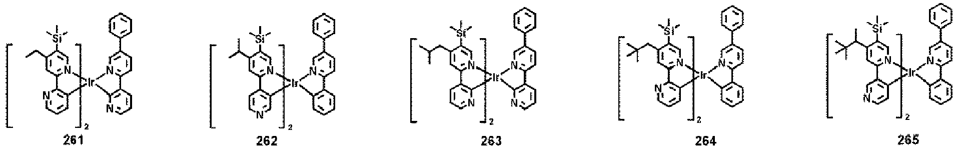

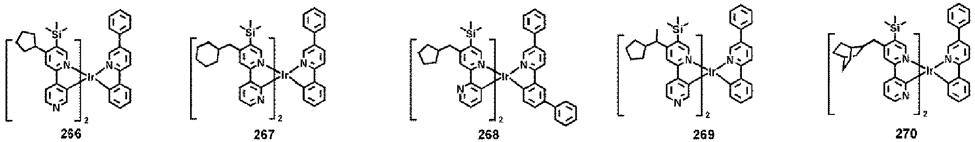

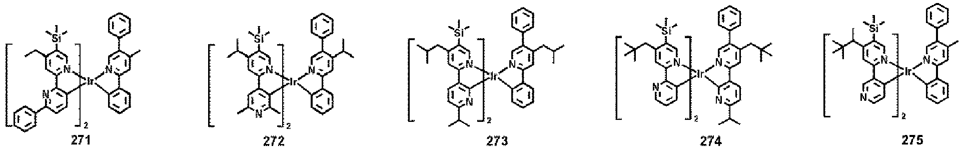

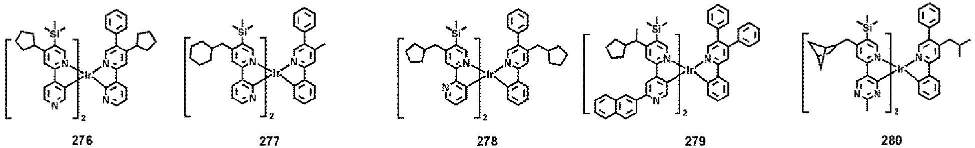

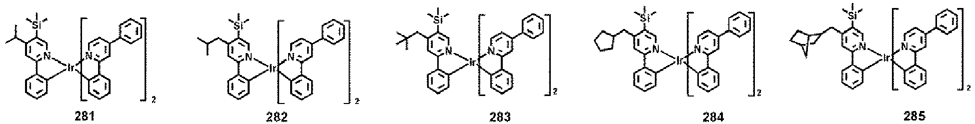

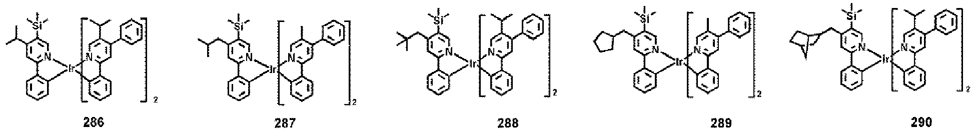

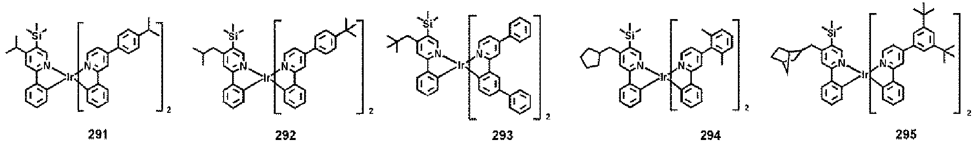

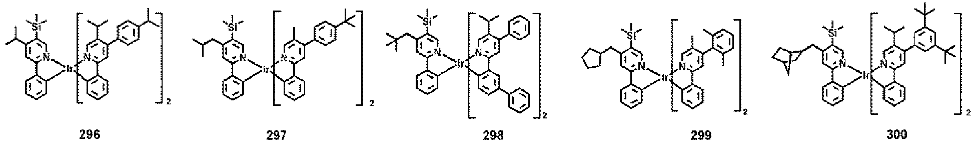

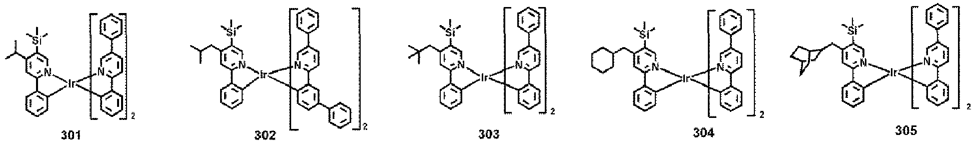

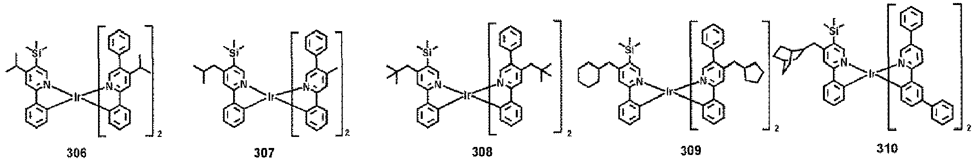

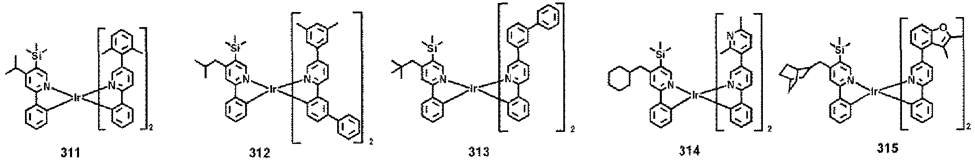

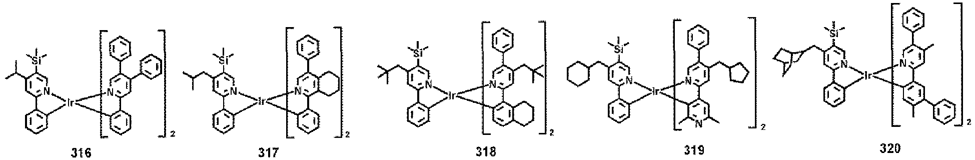

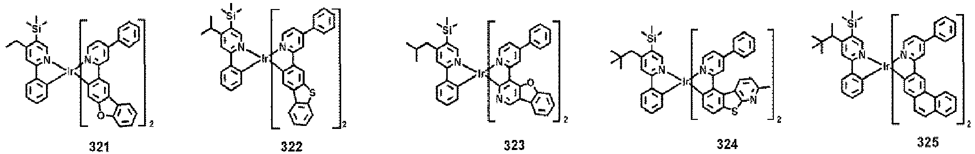

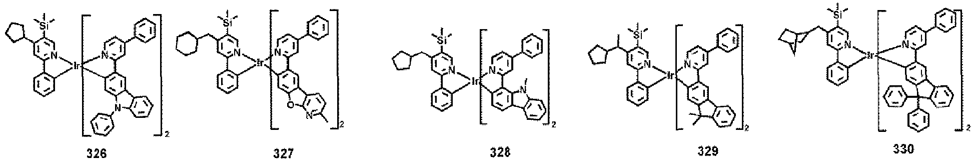

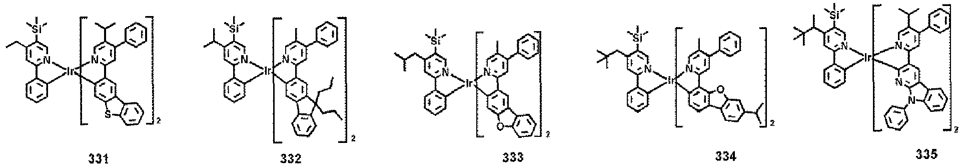

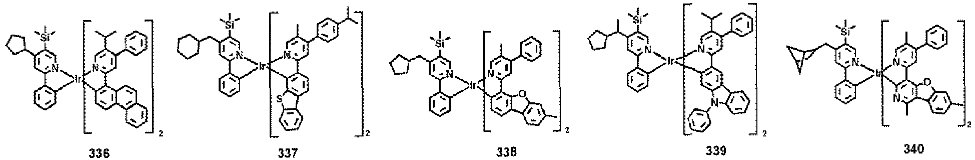

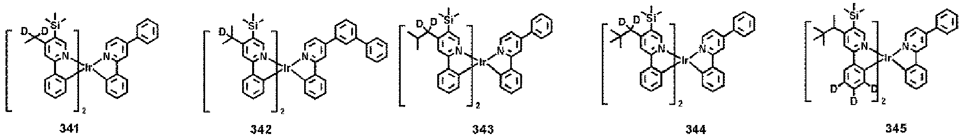

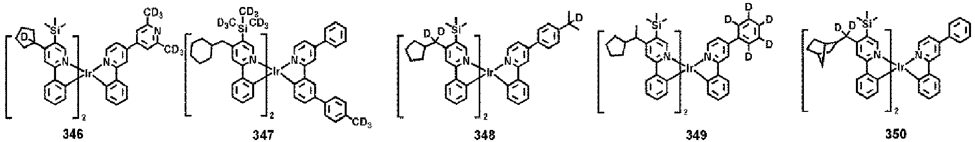

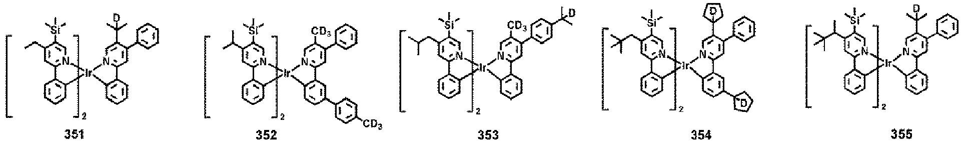

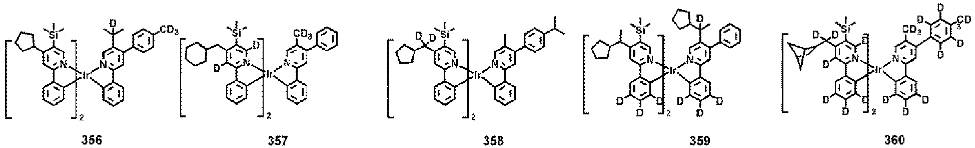

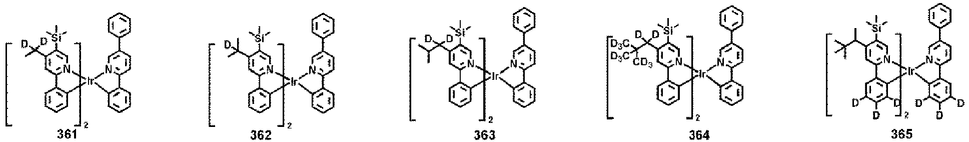

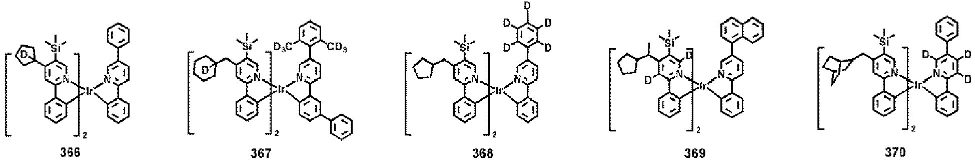

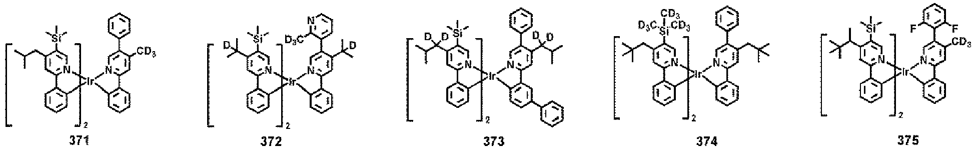

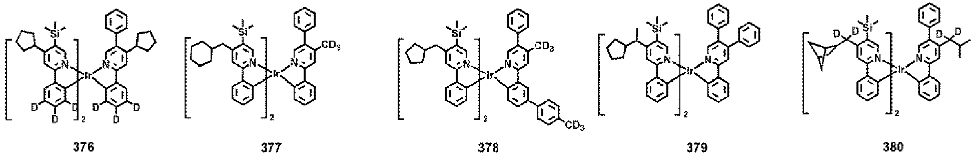

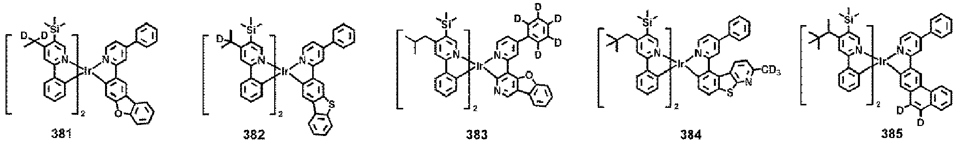

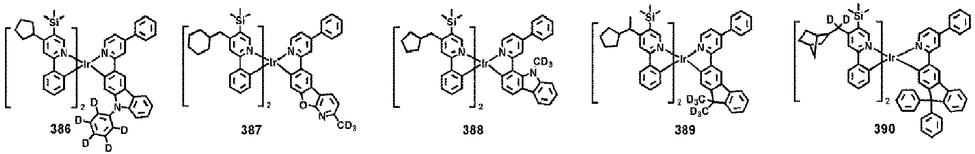

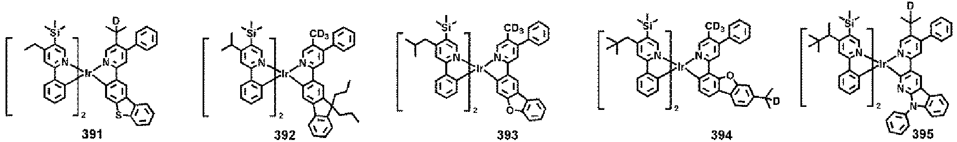

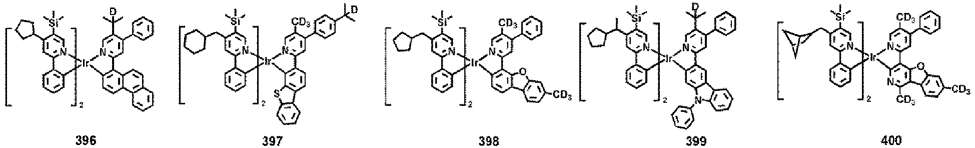

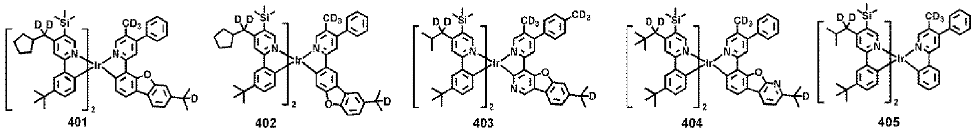

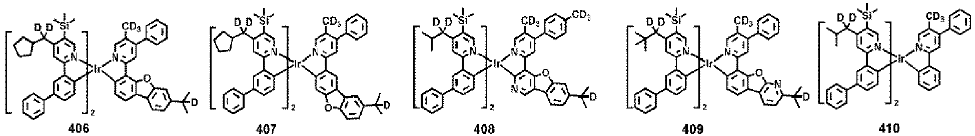

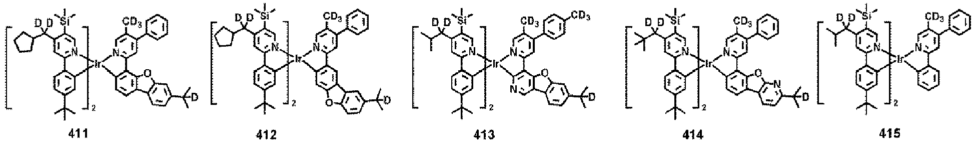

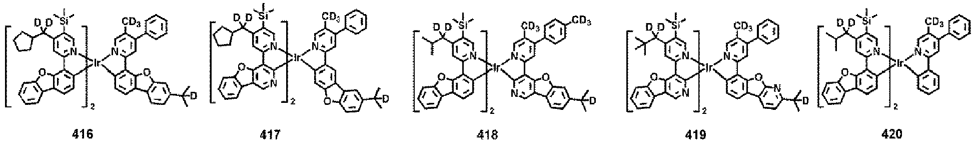

L₁ in the organometallic compound represented by Formula 1 may be a ligand represented by Formula 2A, n1 is the number of groups L₁ and may be 1 or 2, L₂ may be a ligand represented by Formula 2B, n2 is the number of groups L₂ and may be 1 or 2, and L₁ and L₂ may be different from each other. That is, the organometallic compound may be a heteroleptic complex essentially including at least one ligand represented by Formula 2A and at least one ligand represented by Formula 2B as a ligand linked to a metal M.

Without wishing to be bound by theory, since L₂ in Formula 1 is a ligand represented by Formula 2B, the organometallic compound represented by Formula 1 may have a highest occupied molecular orbital (HOMO) energy level, a lowest unoccupied molecular orbital (LUMO) energy level, and a T₁ energy level that are suitable for a material for an electronic device, for example, a material for an organic light-emitting device. Therefore, an electronic device, for example, an organic light-emitting device, which includes the organometallic compound represented by Formula 1, may have excellent luminescence efficiency and lifespan characteristics.

R₁₂ in Formula 2B is neither hydrogen nor a methyl group. As such, the organometallic compound represented by Formula 1 may emit light that is shifted toward relatively shorter wavelengths, for example, blue light, green light, or greenish blue light, and an electronic device, for example, an organic light-emitting device, including the organometallic compound may have an excellent out-coupling effect as well as high emission efficiency.

In Formula 2B, a silyl group is bonded to the fifth position of a pyridine ring (see Formula 2B). Without wishing to be bound by theory, due to this bonding, the organometallic compound including the ligand represented by Formula 2B has excellent heat resistance and decomposition resistance characteristics. Accordingly, an electronic device, for example, an organic light-emitting device, including the organometallic compound has high stability during manufacturing, preserving, and/or driving, and a long lifespan.

Furthermore, the pyridine ring in Formula 2A may be substituted with ring CY₁(s) in the number of c1. Accordingly, the orientation characteristics of the organometallic compound represented by Formula 1 are improved, thereby providing high luminescence efficiency for an electronic device, for example, an organic light-emitting device, which includes the organometallic compound.

Highest occupied molecular orbital (HOMO) energy level, lowest unoccupied molecular orbital (LUMO) energy level, singlet (S₁) energy level, and triplet (T₁) energy level of some of the organometallic compound represented by Formula 1 was evaluated by using a Gaussian 09 program accompanied with optimization of molecular structure according to B3LYP-based density functional theory (DFT). Results thereof are shown in Table 1, where the values are in electron volts (eV).

**Table 1**

| Compound No. | HOMO (eV) | LUMO (eV) | S₁(eV) | T₁(eV) |
|---|---|---|---|---|
| 13 | -4.712 | -1.232 | 2.783 | 2.555 |
| 53 | -4.717 | -1.197 | 2.827 | 2.559 |
| 163 | -4.735 | -1.232 | 2.787 | 2.543 |
| 405 | -4.657 | -1.228 | 2.746 | 2.534 |

From Table 1, it is confirmed that the organometallic compound represented by Formula 1 has such electric characteristics that are suitable for use in an electronic device, for example, for use as a dopant for an organic light-emitting device.

Synthesis methods of the organometallic compound represented by Formula 1 may be understood by one of ordinary skill in the art by referring to Synthesis Examples provided below.

The organometallic compound represented by Formula 1 is suitable for use in an organic layer of an organic light-emitting device, for example, for use as a dopant in an emission layer of the organic layer. Thus, another aspect provides an organic light-emitting device that includes: a first electrode; a second electrode; and an organic layer that is disposed between the first electrode and the second electrode and includes an emission layer, wherein the organic layer includes at least one organometallic compound represented by Formula 1.

The organic light-emitting device may have, due to the inclusion of an organic layer including the organometallic compound represented by Formula 1, a low driving voltage, high efficiency, high power, high quantum efficiency, a long lifespan, a low roll-off ratio, and excellent color purity.

The organometallic compound of Formula 1 may be used between a pair of electrodes of an organic light-emitting device. For example, the organometallic compound represented by Formula 1 may be included in the emission layer. In this regard, the organometallic compound may act as a dopant, and the emission layer may further include a host (that is, an amount of the organometallic compound represented by Formula 1 is smaller than an amount of the host). The emission layer may emit, for example, green light or blue light.

The expression "(an organic layer) includes at least one organometallic compounds" used herein may include a case in which "(an organic layer) includes identical organometallic compounds represented by Formula 1" and a case in which "(an organic layer) includes two or more different organometallic compounds represented by Formula 1."

For example, the organic layer may include, as the organometallic compound, only Compound 1. In this regard, Compound 1 may exist in an emission layer of the organic light-emitting device. In another embodiment, the organic layer may include, as the organometallic compound, Compound 1 and Compound 2. In this regard, Compound 1 and Compound 2 may exist in the same layer (for example, Compound 1 and Compound 2 all may exist in the emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

In an embodiment, in the organic light-emitting device, the first electrode is an anode, and the second electrode is a cathode, and the organic layer further includes a hole transport region disposed between the first electrode and the emission layer and an electron transport region disposed between the emission layer and the second electrode, and the hole transport region includes a hole injection layer, a hole transport layer, an electron blocking layer, a buffer layer, or any combination thereof, and the electron transport region includes a hole blocking layer, an electron transport layer, an electron injection layer, or any combination thereof.

The term "organic layer" as used herein refers to a single layer and/or a plurality of layers between the first electrode and the second electrode of the organic light-emitting device. The "organic layer" may include, in addition to an organic compound, an organometallic complex including a metal.

FIGURE is a schematic view of an organic light-emitting device 10 according to an embodiment. Hereinafter, the structure of an organic light-emitting device according to an embodiment and a method of manufacturing an organic light-emitting device according to an embodiment will be described in connection with the FIGURE. The organic light-emitting device 10 includes a first electrode 11, an organic layer 15, and a second electrode 19, which are sequentially stacked.

A substrate may be additionally disposed under the first electrode 11 or above the second electrode 19. For use as the substrate, any substrate that is used in general organic light-emitting devices may be used, and the substrate may be a glass substrate or a transparent plastic substrate, each having excellent mechanical strength, thermal stability, transparency, surface smoothness, ease of handling, and water resistance.

The first electrode 11 may be formed, for example, by depositing or sputtering a material for forming the first electrode 11 on the substrate. The first electrode 11 may be an anode. The material for forming the first electrode 11 may be selected from materials with a high work function to facilitate hole injection. The first electrode 11 may be a reflective electrode, a semi-transmissive electrode, or a transmissive electrode. The material for forming the first electrode may be, for example, indium tin oxide (ITO), indium zinc oxide (IZO), tin oxide (SnO₂), and zinc oxide (ZnO). In an embodiment, magnesium (Mg), aluminum (Al), aluminum-lithium (Al-Li), calcium (Ca), magnesium-indium (Mg-In), or magnesium-silver (Mg-Ag) may be used as the material for forming the first electrode.

The first electrode 11 may have a single-layered structure or a multi-layered structure including two or more layers. For example, the first electrode 11 may have a three-layered structure of ITO/Ag/ITO, but the structure of the first electrode 110 is not limited thereto.

The organic layer 15 may be disposed on the first electrode 11.

The organic layer 15 may include a hole transport region, an emission layer, and an electron transport region.

The hole transport region may be disposed between the first electrode 11 and the emission layer.

The hole transport region may include a hole injection layer, a hole transport layer, an electron blocking layer, a buffer layer, or any combination thereof.

The hole transport region may include only one of a hole injection layer or a hole transport layer. In another embodiment, the hole transport region may have a hole injection layer/hole transport layer structure or a hole injection layer/hole transport layer/electron blocking layer structure, which are sequentially stacked in this stated order in a direction extending from the first electrode 11.

A hole injection layer may be formed on the first electrode 11 by using one or more exemplary methods selected from vacuum deposition, spin coating, casting, or Langmuir-Blodgett (LB) deposition.

When a hole injection layer is formed by vacuum deposition, the deposition conditions may vary according to a compound that is used to form the hole injection layer, and the structure and thermal characteristics of the hole injection layer. For example, the deposition conditions may include a deposition temperature of about 100 °C to about 500 °C, a vacuum pressure of about 10⁻⁸ torr to about 10⁻³ torr, and a deposition rate of about 0.01 Å/sec to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 rpm to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include at least one selected from m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201 below, and a compound represented by Formula 202 below:

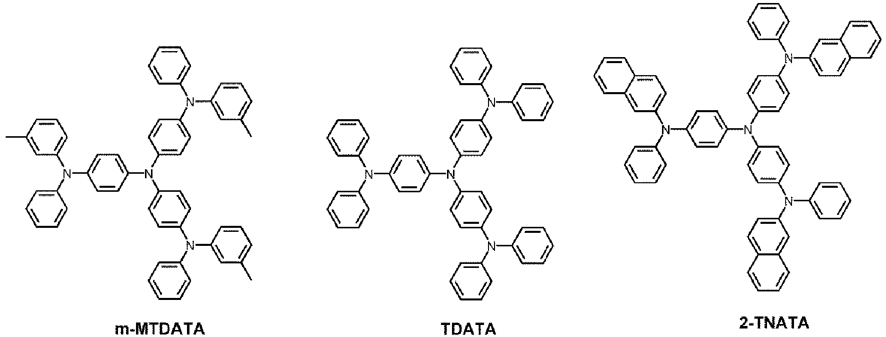

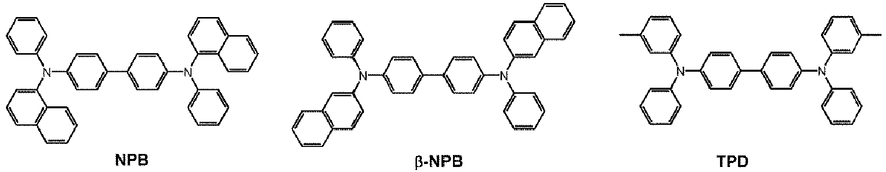

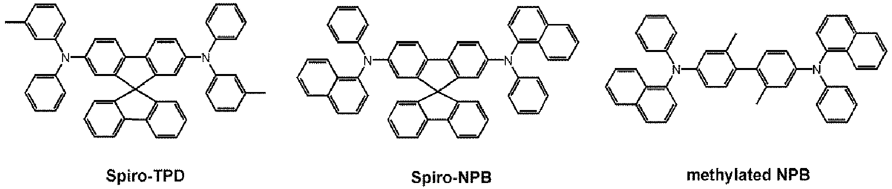

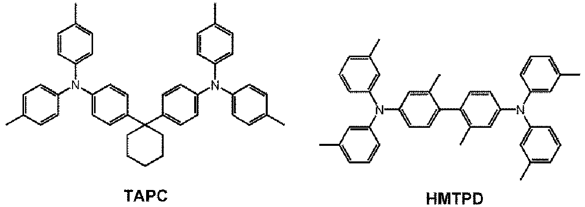

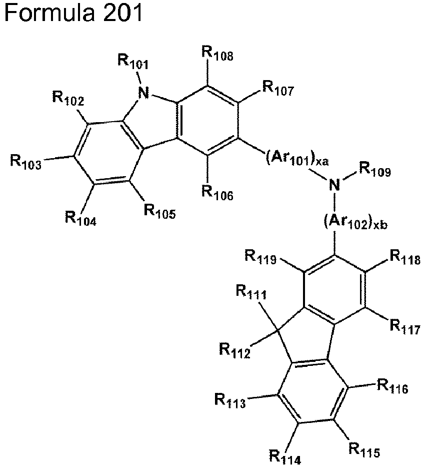

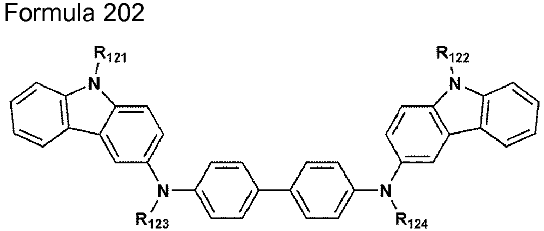

Ar₁₀₁ and Ar₁₀₂ in Formula 201 may each independently be selected from:
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group; and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group, each substituted with at least one selected from deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group.

In Formula 201, xa and xb may each independently be an integer of 0 to 5, or may be 0, 1, or 2. For example, xa is 1 and xb is 0, but xa and xb are not limited thereto.

R₁₀₁ to R₁₀₈, R₁₁₁ to R₁₁₉, and R₁₂₁ to R₁₂₄ in Formulae 201 and 202 may each independently be selected from:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, a pentyl group, a hexyl group, and the like), or a C₁-C₁₀ alkoxy group (for example, a methoxy group, an ethoxy group, a propoxy group, a butoxy group, a pentoxy group, and the like);
a C₁-C₁₀ alkyl group or a C₁-C₁₀ alkoxy group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, or a phosphoric acid group or a salt thereof;
a phenyl group, a naphthyl group, an anthracenyl group, a fluorenyl group, or a pyrenyl group; and
a phenyl group, a naphthyl group, an anthracenyl group, a fluorenyl group, and a pyrenyl group, each substituted with at least one selected from deuterium, -F, - Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, or a C₁-C₁₀ alkoxy group,
but embodiments of the present disclosure are not limited thereto.

R₁₀₉ in Formula 201 may be selected from:
a phenyl group, a naphthyl group, an anthracenyl group, or a pyridinyl group; and
a phenyl group, a naphthyl group, an anthracenyl group, and a pyridinyl group, each substituted with at least one selected from deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C20 alkyl group, a C₁-C₂₀ alkoxy group, a phenyl group, a naphthyl group, an anthracenyl group, or a pyridinyl group.

In an embodiment, the compound represented by Formula 201 may be represented by Formula 201A, but embodiments of the present disclosure are not limited thereto:

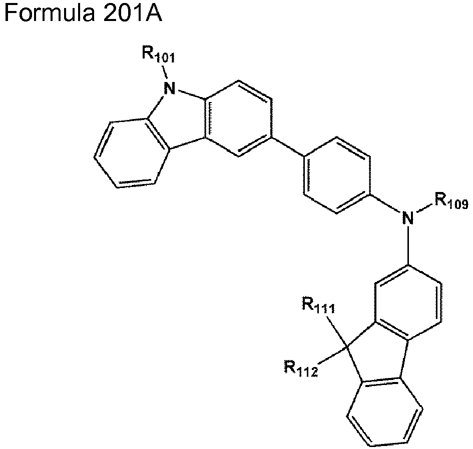

R₁₀₁, R₁₁₁, R₁₁₂, and R₁₀₉ in Formula 201A may be understood by referring to the description provided herein.

For example, the compound represented by Formula 201, and the compound represented by Formula 202 may include compounds HT1 to HT20 illustrated below, but are not limited thereto.

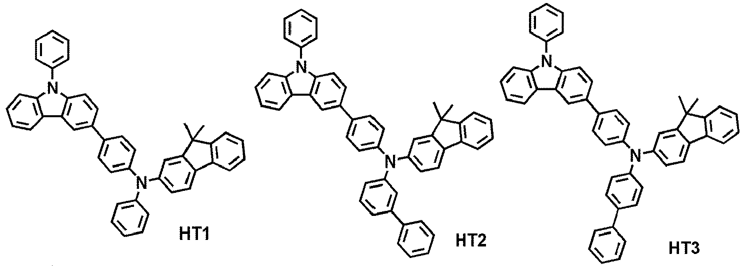

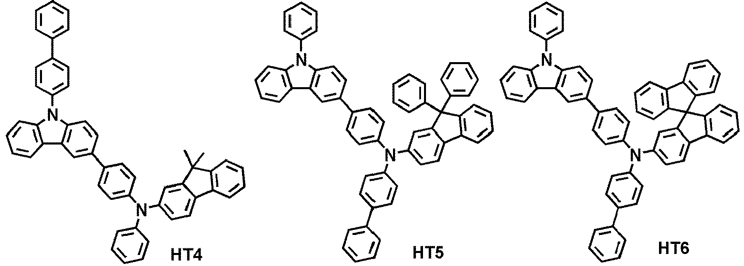

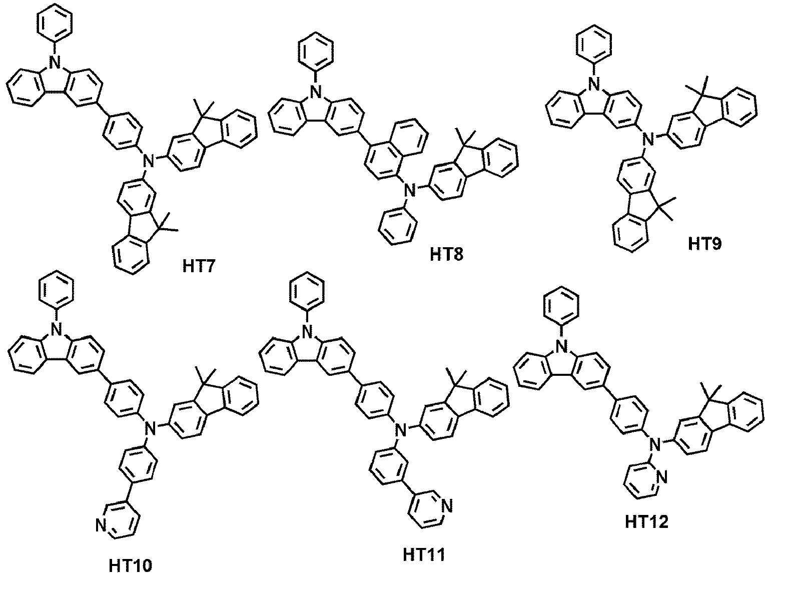

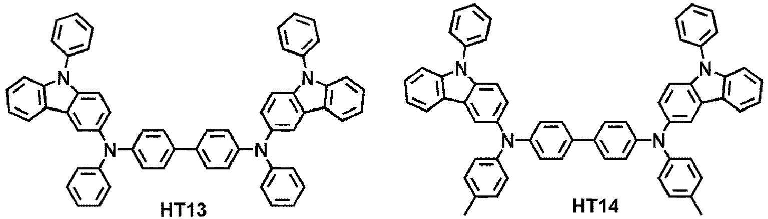

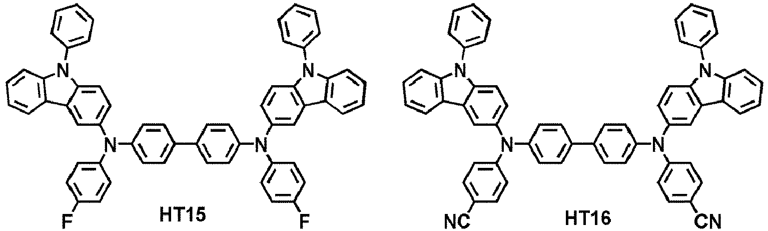

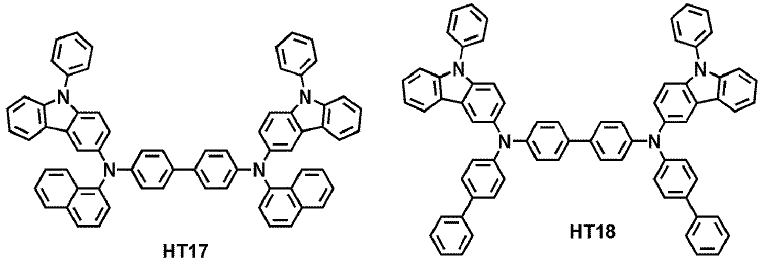

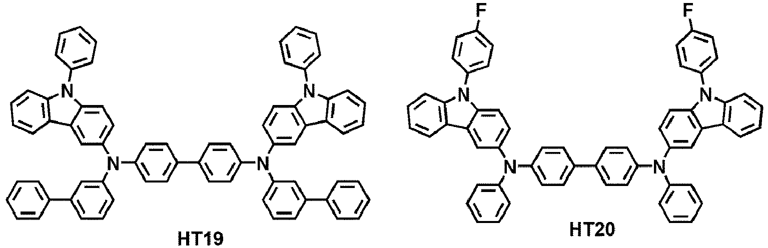

A thickness of the hole transport region may be in a range of about 100 Angstroms (Å) to about 10,000 Å, for example, about 100 Å to about 1,000 Å. When the hole transport region includes at least one of a hole injection layer and a hole transport layer, the thickness of the hole injection layer may be in a range of about 100 Å to about 10,000 Å, and for example, about 100 Å to about 1,000 Å, and the thickness of the hole transport layer may be in a range of about 50 Å to about 2,000 Å, and for example, about 100 Å to about 1500 Å. When the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one selected from a quinone derivative, a metal oxide, and a cyano group-containing compound, but embodiments of the present disclosure are not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenum oxide; and a cyano group-containing compound, such as Compound HT-D1 below, but are not limited thereto.

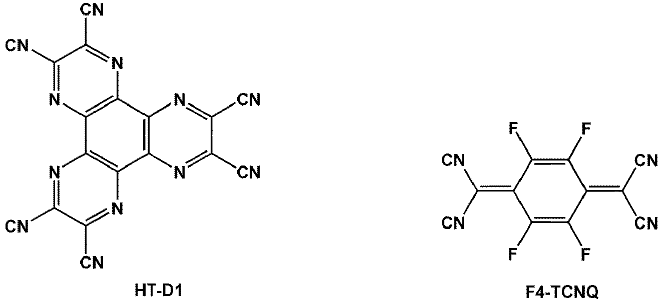

The hole transport region may include a buffer layer.

In an embodiment, the buffer layer may compensate for an optical resonance distance according to a wavelength of light emitted from the emission layer, and thus, efficiency of a formed organic light-emitting device may be improved.

In an embodiment, an emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a compound that is used to form the emission layer.

In an embodiment, when the hole transport region includes an electron blocking layer, a material for the electron blocking layer may be selected from materials for the hole transport region described above and materials for a host to be explained later. However, the material for the electron blocking layer is not limited thereto. For example, when the hole transport region includes an electron blocking layer, a material for the electron blocking layer may be mCP, which will be explained later.

The emission layer may include a host and a dopant, and the dopant may include the organometallic compound represented by Formula 1.

The host may include at least one selected from TPBi, TBADN, ADN (also referred to as "DNA"), CBP, CDBP, TCP, mCP, and Compounds H50 to H52:

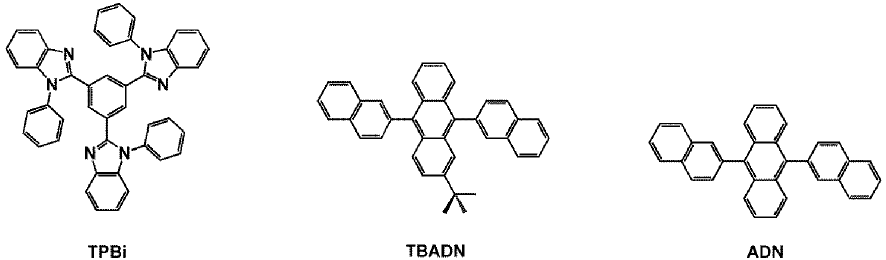

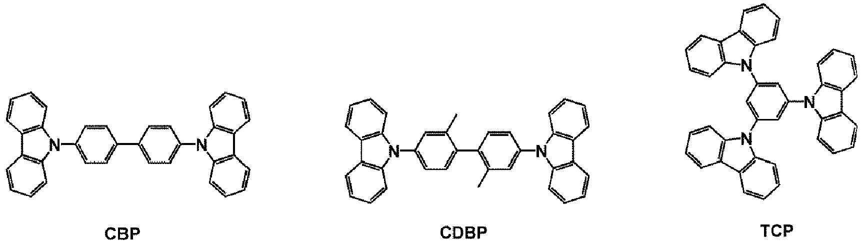

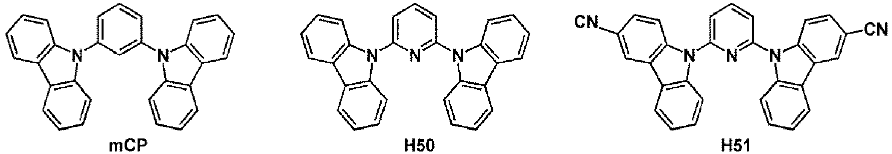

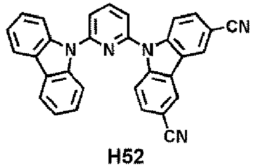

When the organic light-emitting device is a full-color organic light-emitting device, the emission layer may be patterned into a red emission layer, a green emission layer, and a blue emission layer. In an embodiment, due to a stacked structure including a red emission layer, a green emission layer, and/or a blue emission layer, the emission layer may emit white light.

When the emission layer includes a host and a dopant, an amount of the dopant may be in a range of about 0.01 parts by weight to about 15 parts by weight based on 100 parts by weight of the host, but embodiments of the present disclosure are not limited thereto.

A thickness of the emission layer may be in a range of about 100 Å to about 1,000 Å, for example, about 200 Å to about 600 Å. When the thickness of the emission layer is within this range, excellent light-emission characteristics may be obtained without a substantial increase in driving voltage.

Then, an electron transport region may be disposed on the emission layer.

The electron transport region may include a hole blocking layer, an electron transport layer, an electron injection layer, or any combination thereof.

For example, the electron transport region may have a hole blocking layer/electron transport layer/electron injection layer structure or an electron transport layer/electron injection layer structure, but the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, at least one of BCP, Bphen, and BAlq but embodiments of the present disclosure are not limited thereto.

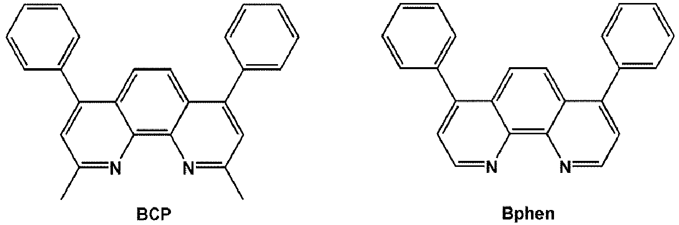

A thickness of the hole blocking layer may be in a range of about 20 Å to about 1,000 Å, for example, about 30 Å to about 300 Å. When the thickness of the hole blocking layer is within these ranges, the hole blocking layer may have improved hole blocking ability without a substantial increase in driving voltage.

The electron transport layer may further include at least one selected from BCP, Bphen, Alq₃, BAlq, TAZ, and NTAZ.

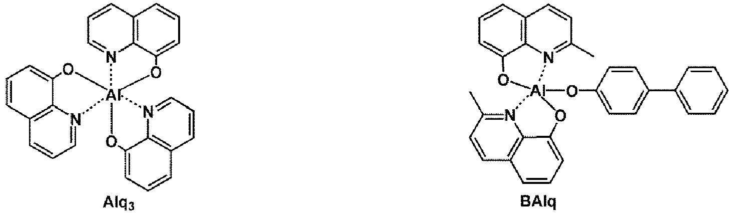

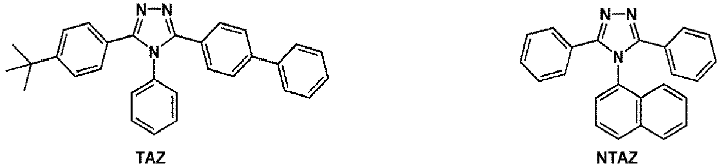

In an embodiment, the electron transport layer may include at least one selected from ET1 to ET25, but are not limited thereto:

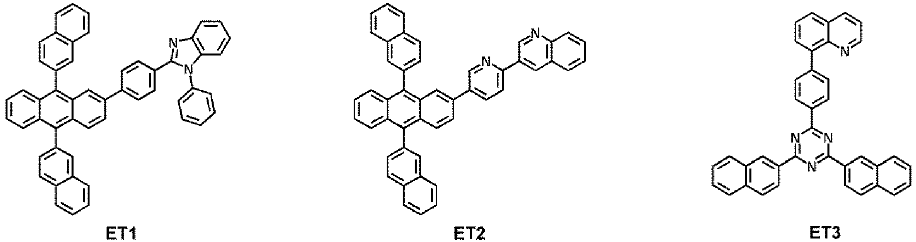

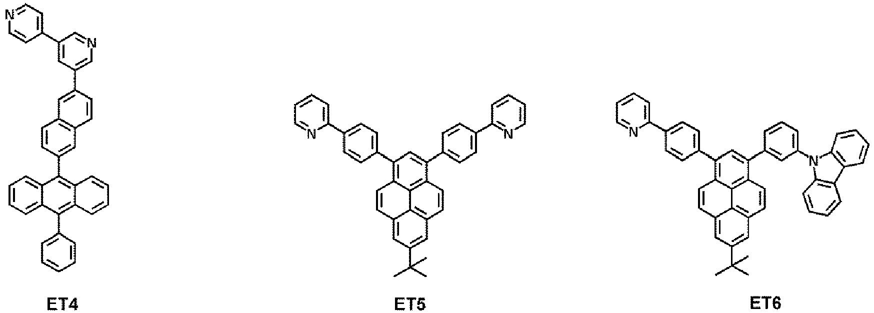

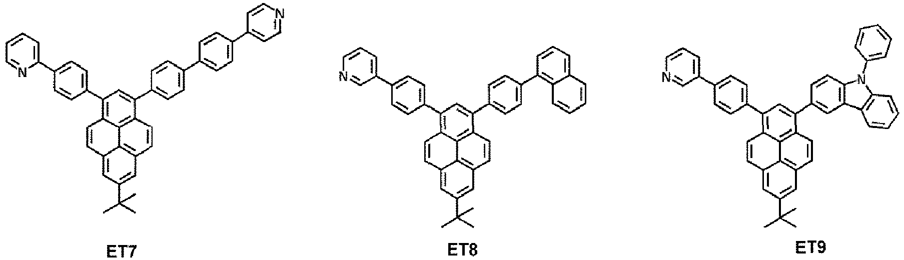

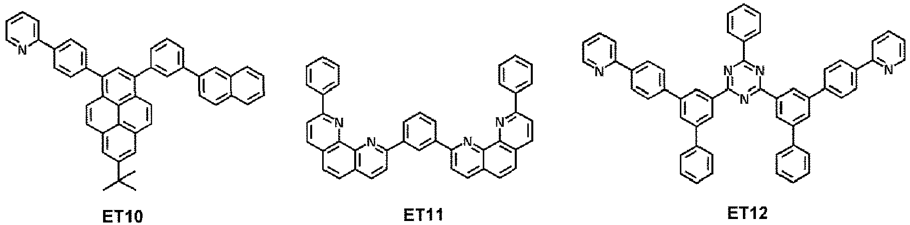

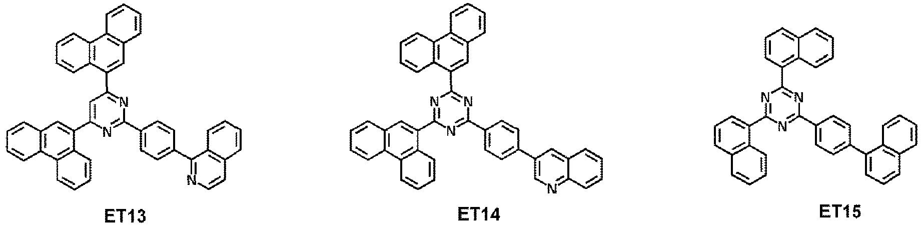

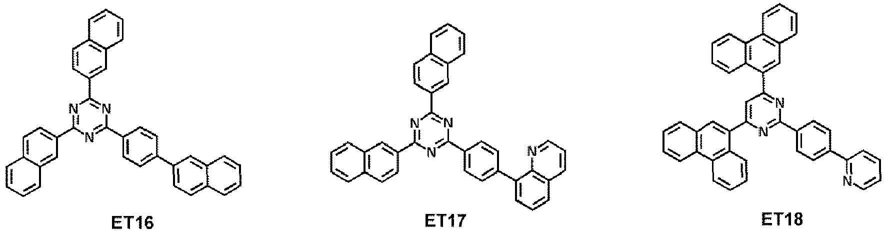

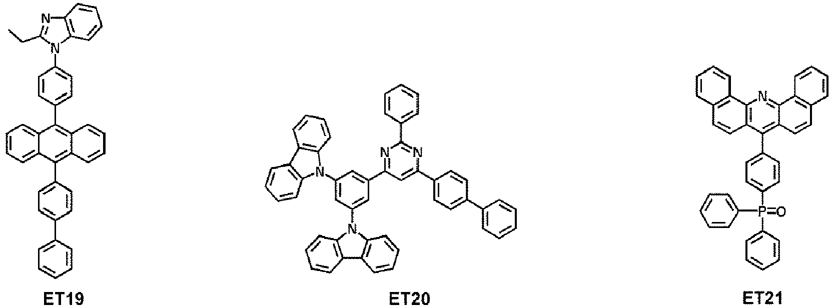

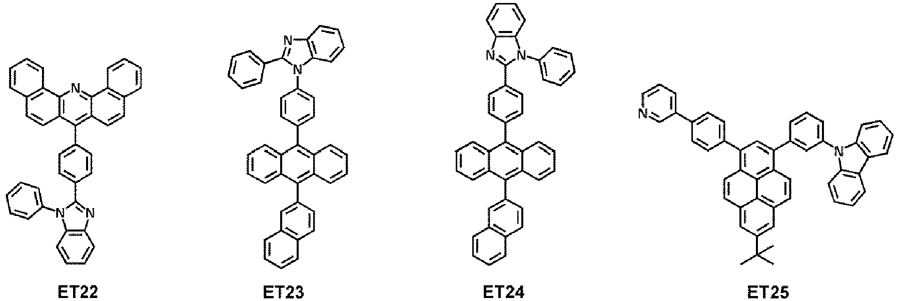

A thickness of the electron transport layer may be in a range of about 100 Å to about 1,000 Å, for example, about 150 Å to about 500 Å. When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium 8-hydroxylquinolate, LiQ) or ET-D2.

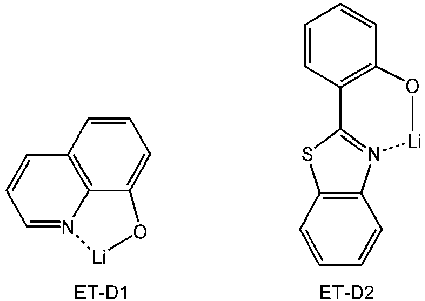

The electron transport region may include an electron injection layer that promotes flow of electrons from the second electrode 19 thereinto.

The electron injection layer may include at least one selected from LiF, NaCl, CsF, Li₂O, and BaO.

The thickness of the electron injection layer may be in a range of about 1 Å to about 100 Å, for example, about 3 Å to about 90 Å. When the thickness of the electron injection layer is within the range described above, the electron injection layer may have satisfactory electron injection characteristics without a substantial increase in driving voltage.

The second electrode 19 may be disposed on the organic layer 15. The second electrode 19 may be a cathode. The material for forming the second electrode 19 may be selected from a metal, an alloy, an electrically conductive compound, or a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as a material for forming the second electrode 19. In an embodiment, to manufacture a top-emission type light-emitting device, a transmissive electrode formed using ITO or IZO may be used as the second electrode 19.

Another aspect of the present disclosure provides a diagnostic composition including at least one organometallic compound represented by Formula 1.

The organometallic compound represented by Formula 1 provides high luminescence efficiency. Accordingly, a diagnostic composition including the organometallic compound may have high diagnostic efficiency.

The diagnostic composition may be used in various applications including a diagnosis kit, a diagnosis reagent, a biosensor, and a biomarker.

Hereinbefore, the organometallic compound, the organic light-emitting device as described with reference to the FIGURE, and a diagnostic composition including the organometallic compound have been described, but embodiments of the present disclosure are not limited thereto.

The term "C₁-C₆₀ alkyl group" as used herein refers to a linear or branched saturated aliphatic hydrocarbon monovalent group having 1 to 60 carbon atoms, and non-limiting examples thereof include a methyl group, an ethyl group, a propyl group, an isobutyl group, a sec-butyl group, a tert-butyl group, a pentyl group, an isoamyl group, and a hexyl group. The term "C₁-C₆₀ alkylene group" as used herein refers to a divalent group having the same structure as the C₁-C₆₀ alkyl group.

The term "C₁-C₆₀ alkoxy group" as used herein refers to a monovalent group represented by -OA₁₀₁ (wherein A₁₀₁ is the C₁-C₆₀ alkyl group), and non-limiting examples thereof include a methoxy group, an ethoxy group, and an isopropyloxy group.

The term "C₂-C₆₀ alkenyl group" as used herein refers to a hydrocarbon group having at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group having at least one carbon-carbon triple bond in the middle or at the terminus of the C₂-C₆₀ alkyl group, and examples thereof include an ethynyl group, and a propynyl group. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon monocyclic group having 3 to 10 carbon atoms, and non-limiting examples thereof include a cyclopropyl group, a cyclobutyl group, a cyclopentyl group, a cyclohexyl group, and a cycloheptyl group. The term "C₃-C₁₀ cycloalkylene group" as used herein refers to a divalent group having the same structure as the C₃-C₁₀ cycloalkyl group.

The term "C₁-C₁₀ heterocycloalkyl group" as used herein refers to a monovalent saturated monocyclic group having at least one heteroatom selected from N, O, P, Si and S as a ring-forming atom and 1 to 10 carbon atoms, and non-limiting examples thereof include a tetrahydrofuranyl group, and a tetrahydrothiophenyl group. The term "C₁-C₁₀ heterocycloalkylene group" as used herein refers to a divalent group having the same structure as the C₁-C₁₀ heterocycloalkyl group.

The term "C₃-C₁₀ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group that has at least one heteroatom selected from N, O, P, Si, and S as a ring-forming atom, 1 to 10 carbon atoms, and at least one double bond in its ring. Examples of the C₁-C₁₀ heterocycloalkenyl group are a 2,3-dihydrofuranyl group, and a 2,3-dihydrothiophenyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

The term "C₁-C₆₀ heteroaryl group" as used herein refers to a monovalent group having a heterocyclic aromatic system that has at least one heteroatom selected from N, O, P, Si, and S as a ring-forming atom, and 1 to 60 carbon atoms. The term "C₁-C₆₀ heteroarylene group" as used herein refers to a divalent group having a heterocyclic aromatic system that has at least one heteroatom selected from N, O, P, Si and S as a ring-forming atom, and 1 to 60 carbon atoms. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other.

The term "C₆-C₆₀ aryloxy group" used herein indicates -OA₁₀₂ (wherein A₁₀₂ is the C₆-C₆₀ aryl group), and a C₆-C₆₀ arylthio group used herein indicates -SA₁₀₃ (wherein A₁₀₃ is the C₆-C₆₀ aryl group).

The term "monovalent non-aromatic condensed polycyclic group" as used herein refers to a monovalent group (for example, having 8 to 60 carbon atoms) having two or more rings condensed with each other, only carbon atoms are used as ring-forming atoms, and having no aromaticity present in the entire molecular structure. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group (for example, having 2 to 60 carbon atoms) having two or more rings condensed with each other, a heteroatom selected from N, O, P, Si, and S, other than carbon atoms, as a ring-forming atom, and having no aromaticity present in its entire molecular structure. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

The term "C₅-C₃₀ carbocyclic group" as used herein refers to a saturated or unsaturated cyclic group having, as a ring-forming atom, 5 to 30 carbon atoms only. The C₅-C₃₀ carbocyclic group may be a monocyclic group or a polycyclic group.

The term "C₁-C₃₀ heterocyclic group" as used herein refers to a saturated or unsaturated cyclic group having, as a ring-forming atom, at least one heteroatom selected from N, O, Si, P, and S other than 1 to 30 carbon atoms. The C₁-C₃₀ heterocyclic group may be a monocyclic group or a polycyclic group.

At least one substituent of the substituted C₅-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group may each independently be:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂),-Si(Q₁₃)(Q₁₄)(Q₁₅), -B(Q₁₆)(Q₁₇), and -P(=O)(Q₁₈)(Q₁₉);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, or a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, or a monovalent non-aromatic condensed heteropolycyclic group, each substituted with at least one selected from deuterium, -F, -Cl, -Br, -I, -CD₃, -CD₂H, -CDH₂, -CF₃,-CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₂₁)(Q₂₂),-Si(Q₂₃)(Q₂₄)(Q₂₅), -B(Q₂₆)(Q₂₇), and -P(=O)(Q₂₈)(Q₂₉); or
-N(Q₃₁)(Q₃₂), -Si(Q₃₃)(Q₃₄)(Q₃₅), -B(Q₃₆)(Q₃₇) or -P(=O)(Q₃₈)(Q₃₉), and
Q1 to Q₉, Q₁₁ to Q₁₉, Q₂₁ to Q₂₉, and Q₃₁ to Q₃₉, as used herein, may each independently be selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group and a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

When a group containing a specified number of carbon atoms is substituted with any of the groups listed in the preceding paragraphs, the number of carbon atoms in the resulting "substituted" group is defined as the sum of the carbon atoms contained in the original (unsubstituted) group and the carbon atoms (if any) contained in the substituent. For example, when the term "substituted C₁-C₃₀ alkyl" refers to a C₁-C₃₀ alkyl group substituted with C₆-C₃₀ aryl group, the total number of carbon atoms in the resulting aryl substituted alkylene group is C₇-C₆₀.

The expressions * and *' as used herein each indicate a binding site to a neighboring atom, unless otherwise stated.

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that an amount of A used was identical to an amount of B used, in terms of a molar equivalent.

### EXAMPLES

### Synthesis Example 1 (Compound 13)

### Synthesis of Compound 13A

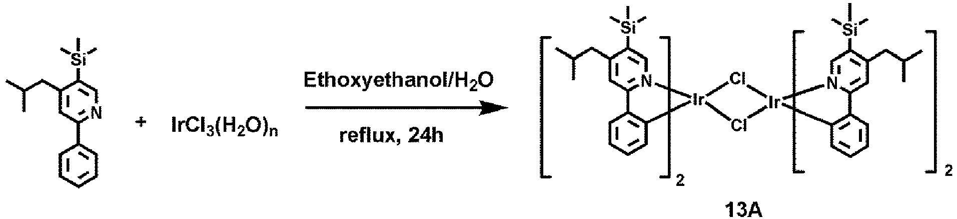

4-isobutyl-2-phenyl-5-(trimethylsilyl)pyridine (8.0 grams (g), 28.2 millimoles (mmol)) and iridium chloride trihydrate (4.4 g, 12.5 mmol) were mixed with 120 mL of ethoxyethanol and 40 mL of distilled water and stirred for 24 hours under reflux, and the reaction mixture was cooled to room temperature. A solid generated therefrom was filtered, separated, and sufficiently washed with water, methanol, and hexane in order. The solid was dried in a vacuum oven to obtain 5.5 g (yield of 55 %) of Compound 13A.

### Synthesis of Compound 13B

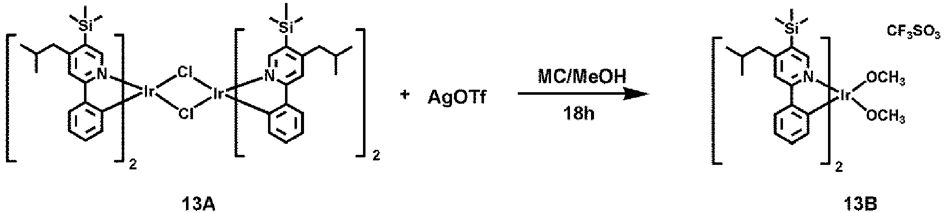

Compound 13A (1.7 g, 1.0 mmol) and 45 mL of methylene chloride were mixed, and AgOTf (0.54 g, 2.1 mmol) was dissolved in 15 mL of methanol and added thereto. Then, the reaction mixture was stirred at room temperature for 18 hours in a state in which light was blocked with an aluminium foil. A solid generated by filtering the reaction mixture by using celite was removed, and a filtrate was evaporated under a reduced pressure to obtain a solid (Compound 13B), which was used for a next reaction without additional purification.

### Synthesis of Compound 13

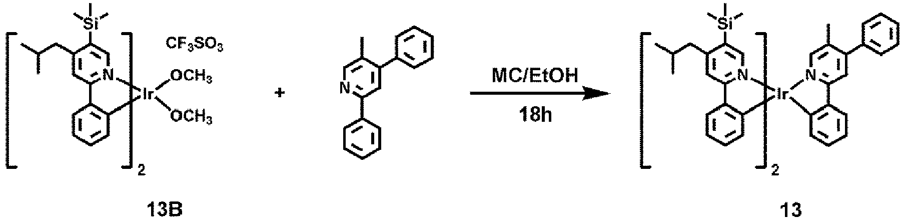

Compound 13B (1.8 g, 1.9 mmol) and 5-methyl-2,4-diphenylpyridine (0.6 g, 2.3 mmol) were mixed with 40 mL of ethanol and stirred for 18 hours under reflux, and the reaction mixture was cooled. A mixture obtained therefrom was filtered to obtain a solid. The solid was sufficiently washed with ethanol and hexane, and column chromatography was performed in a methylene chloride:hexane solvent system to obtain 0.8 g (yield of 41 %) of Compound 13. Compound 13 was identified by high resolution mass spectrometry (HRMS) and HPLC analysis.

HRMS (matrix assisted laser desorption ionization (MALDI)) calculated for C₅₄H₆₂IrN₃Si₂: m/z 1001.4112, found: 1001.4107.

### Synthesis Example 2 (Compound 163)

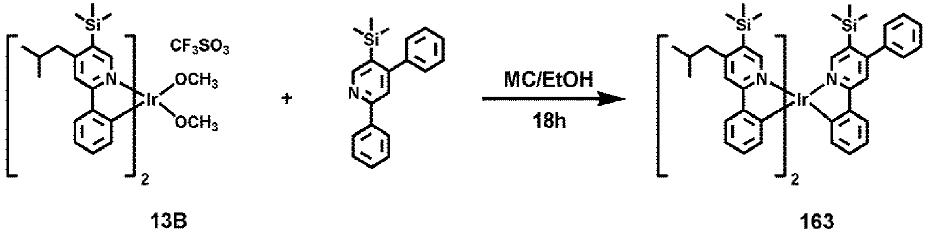

0.6 g (yield of 32 %) of Compound 163 was obtained in the same manner as used to synthesize Compound 13 of Synthesis Example 1, except that 2,4-diphenyl-5-(trimethylsilyl)pyridine was used instead of 5-methyl-2,4-diphenylpyridine. Compound 163 was identified by HRMS and HPLC analysis.

HRMS (MALDI) calculated for C₅₆H₆₈IrN₃Si₃: m/z 1059.4350, found: 1059.4358.

### Synthesis Example 3 (Compound 193)

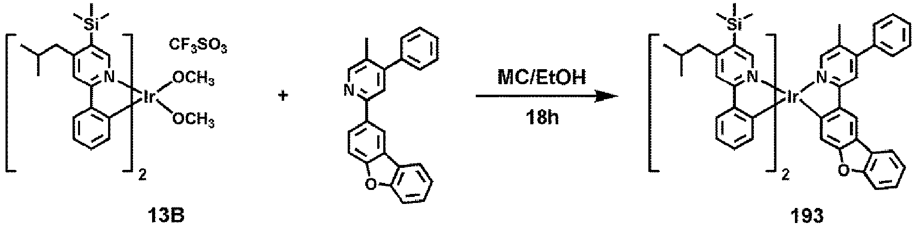

0.9 g (yield of 47 %) of Compound 193 was obtained in the same manner as used to synthesize Compound 13 of Synthesis Example 1, except that 2-(dibenzo[b,d]furan-2-yl)-5-methyl-4-phenylpyridine was used instead of 5-methyl-2,4-diphenylpyridine. Compound 193 was identified by HRMS and HPLC analysis.

### Synthesis Example 4: Synthesis of Compound 358

### Synthesis of Compound 358A

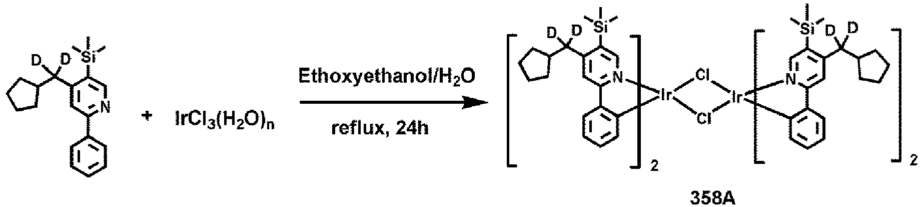

3.2 g (yield of 52 %) of Compound 358A was obtained in the same manner as used to synthesize Compound 13A of Synthesis Example 1, except that 4-(cyclopentylmethyl-D2)-2-phenyl-5-(trimethylsilyl)pyridine was used instead of 4-isobutyl-2-phenyl-5-(trimethylsilyl)pyridine. Compound 358A was identified by HRMS and HPLC analysis.

### Synthesis of Compound 358B

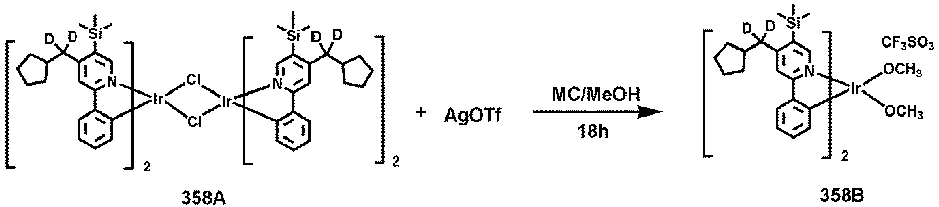

Compound 358B was obtained in the same manner as used to synthesize Compound 13B of Synthesis Example 1, except that Compound 358A was used instead of Compound 13A. Compound 358B was used for a next reaction without additional purification.

### Synthesis of Compound 358

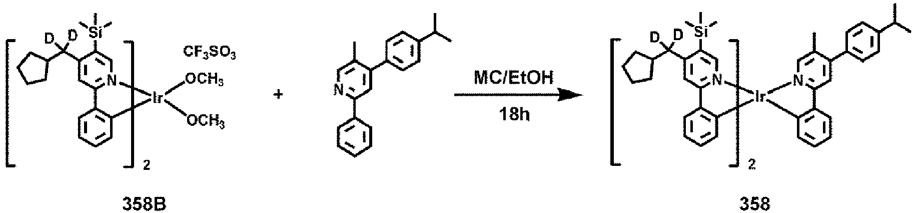

0.5 g (yield of 26 %) of Compound 358 was obtained in the same manner as used to synthesize Compound 13 of Synthesis Example 1, except that Compound 358B was used instead of Compound 13B, and 4-isopropylphenyl)-5-methyl-2-phenylpyridine was used instead of 5-methyl-2,4-diphenylpyridine. Compound 358 was identified by HRMS and HPLC analysis.

HRMS (MALDI) calculated for C₆₁H₆₈D₄IrN₃Si₂: m/z 1099.5145, found: 1099.5152.

### Synthesis Example 5: Synthesis of Compound 371

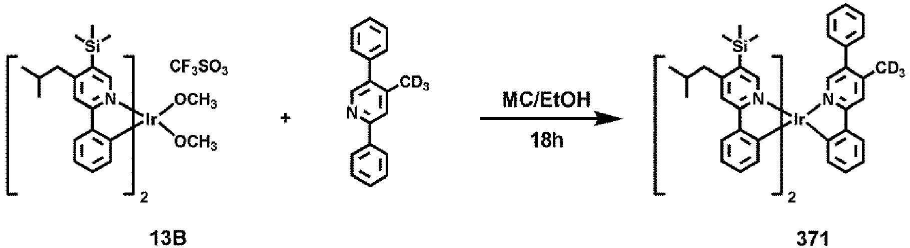

0.7 g (yield of 37 %) of Compound 371 was obtained in the same manner as used to synthesize Compound 13 of Synthesis Example 1, except that 4-methyl-D3-2,5-diphenylpyridine was used instead of 5-methyl-2,4-diphenylpyridine. Compound 371 was identified by HRMS and HPLC analysis.

HRMS (MALDI) calculated for C₆₀H₆₄IrN₃Si₂: m/z 1091.4217, found: 1091.4210.

### Example 1

As an anode, a glass substrate, on which ITO/Ag/ITO were respectively deposited to thicknesses of 70 Å /1,000 Å /70 Å, was cut to a size of 50 mm x 50 mm x 0.5 mm, sonicated with isopropyl alcohol and deionized water each for 5 minutes, and then cleaned by exposure to ultraviolet irradiation and ozone for 30 minutes. Then, the glass substrate was provided to a vacuum deposition apparatus.

N-(2-Naphthyl)-N',N'-bis{4-[2-naphthyl(phenyl)amino]phenyl}-N-phenyl-1,4-benzenediamine (2-TNATA) was vacuum-deposited on the anode to form a hole injection layer having a thickness of 600 Å, and 4,4'-bis[N-(1-naphthyl)-N-phenylamino]biphenyl (NPB) was vacuum-deposited on the hole injection layer to form a hole transport layer having a thickness of 1,350 Å.

Then, 9,9'-(4,4'-Biphenyldiyl)bis(9H-carbazole) (CBP) (host) and Compound 13 (dopant) were co-deposited on the hole transport layer at a weight ratio of 98:2 to form an emission layer having a thickness of 400 Å.

Then, 2,9-Dimethyl-4,7-diphenyl-1,10-phenanthroline (BCP) was vacuum-deposited on the emission layer to form a hole blocking layer having a thickness of 50 Å, Alq₃ was vacuum-deposited on the hole blocking layer to form an electron transport layer having a thickness of 350 Å, LiF was vacuum-deposited on the electron transport layer to form an electron injection layer having a thickness of 10 Å, and Mg and Ag were co-deposited on the electron injection layer at a weight ratio of 90:10 to form a cathode having a thickness of 120 Å, thereby completing the manufacture of an organic light-emitting device.

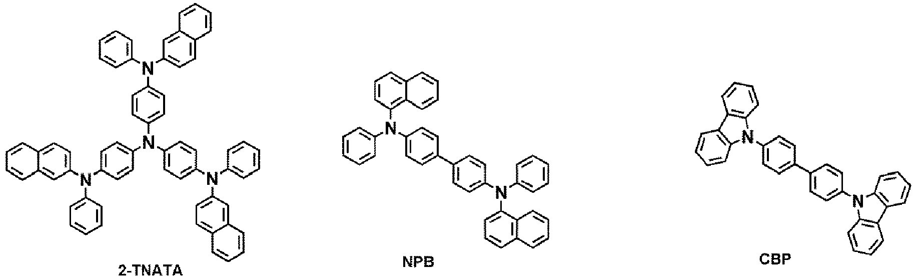

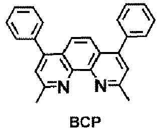

### Examples 2 to 5 and Comparative Examples A and B

Organic light-emitting devices were manufactured in the same manner as in Example 1, except that Compounds shown in Table 2 were each used instead of Compound 13 as a dopant in forming an emission layer.

### Evaluation Example 2: Evaluation of characteristics of organic light-emitting devices

The driving voltage (Volts, V), maximum value (Max EQE) of external quantum efficiency (%), roll-off ratio, full width at half maximum (FWHM) of the main peak of electroluminescence (EL) spectrum (nanometers, nm), maximum emission wavelength (nm), and lifespan (T₉₇) of the organic light-emitting devices manufactured according to Examples 1 to 5 and Comparative Examples A and B were evaluated, and results thereof are shown in Table 2. A current-voltage meter (Keithley 2400) and a luminance meter (Minolta Cs-1000A) were used as an evaluation apparatus. The lifespan (T₉₇) (at 3,500 nit or 3,500 candela per square meter) indicates an amount of time (hours, hr) that lapsed when luminance was 97 % of initial luminance (100 %), and was expressed as a relative value (%). The roll-off ratio was calculated by using Equation 20: $Roll off ratio = \left\{1-\left(Efficiency \left(at 3,500 nit\right)/Maximum emission efficiency\right)\right\} \times 100 %$

**Table 2**

| | Dopant compound | Driving voltage (V) | Max EQE (%) | Roll-Off ratio (%) | FWHM (nm) | Maximum emission wavelength (nm) | Lifespan (relative value, %) (at 3,500 nit) |
|---|---|---|---|---|---|---|---|
| Example 1 | 13 | 3.9 | 23.9 | 18 | 66 | 522 | 130 |
| Example 2 | 163 | 3.9 | 24.2 | 16 | 65 | 518 | 150 |
| Example 3 | 193 | 3.8 | 22.9 | 19 | 69 | 521 | 118 |
| Example 4 | 358 | 4.0 | 23.8 | 16 | 62 | 516 | 150 |
| Example 5 | 371 | 4.0 | 22.3 | 21 | 68 | 520 | 105 |
| Comparative Example A | A | 4.6 | 17.3 | 28 | 81 | 524 | 47 |
| Comparative Example B | B | 4.5 | 19.4 | 26 | 73 | 519 | 96 |

### Dopant compounds:

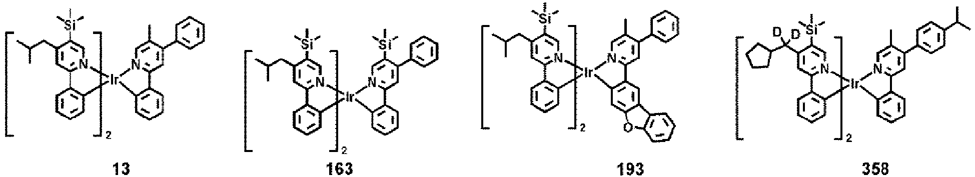

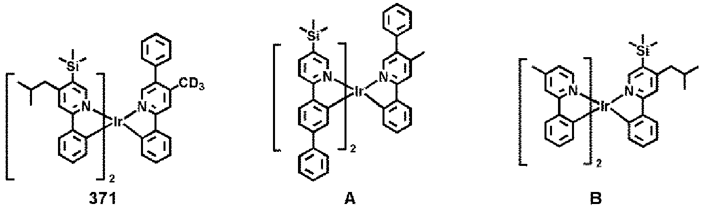

From Table 2, it is confirmed that the organic light-emitting devices of Examples 1 to 5 have improved driving voltage, external quantum efficiency, roll-off ratio, and lifespan characteristics, as compared with those of the organic light-emitting devices of Comparative Examples A and B.

Since the organometallic compound has excellent electric characteristics and heat resistance, an electronic device, for example, an organic light-emitting device, which includes the organometallic compound, may have improved driving voltage, current density, efficiency, power, color purity, and/or lifespan characteristics. In addition, because the organometallic compound has excellent phosphorescence characteristics, a diagnostic composition having high diagnostic efficiency may be provided by using the organometallic compound.

## Claims

1. An organometallic compound represented by Formula 1:
Formula 1 M(L₁)ₙ₁(L₂)ₙ₂,
wherein, in Formula 1,
M is a transition metal,
L₁ is a ligand represented by Formula 2A,
n1 is 1 or 2, wherein, when n1 is 2, two groups L₁ are identical to or different from each other,
L₂ is a ligand represented by Formula 2B,
n2 is 1 or 2, wherein, when n2 is 2, two group L₂ are identical to or different from each other,
the sum of n1 and n2 is 2 or 3, and
L₁ and L₂ are different from each other: 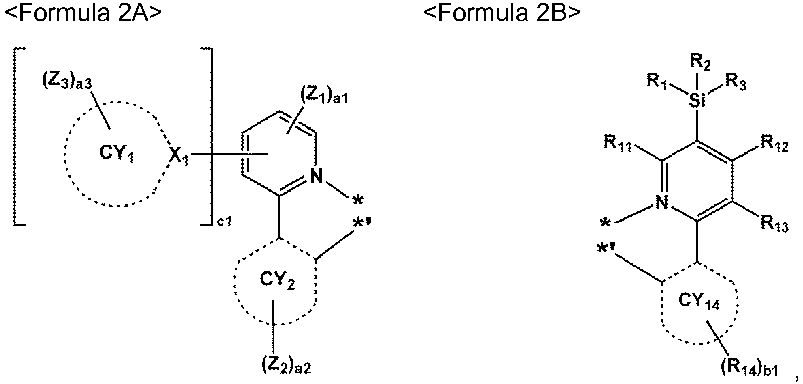 wherein, in Formulae 2A and 2B,
X₁ is C, N, Si, or P,
ring CY₁, ring CY₂, and ring CY₁₄ are each independently a C₅-C₃₀ carbocyclic group or a C₁-C₃₀ heterocyclic group,
R₁ to R₃ are each independently a C₁-C₆₀ alkyl group or a C₆-C₆₀ aryl group, each independently unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, and a C₁-C₁₀ alkyl group,
Z₁ to Z₃ and R₁₁ to R₁₄ are each independently selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, -N(Q₁)(Q₂),
-Si(Q₃)(Q₄)(Q₅), -Ge(Q₃)(Q₄)(Q₅), -B(Q₆)(Q₇), and-P(=O)(Q₈)(Q₉), wherein R₁₂ is neither hydrogen nor a methyl group,
a1 is an integer from 0 to 3, wherein, when a1 is two or more, two or more groups Z₁ are identical to or different from each other,
a2, a3, and b1 are each independently an integer from 0 to 20, wherein, when a2 is two or more, two or more groups Z₂ are identical to or different from each other, when a3 is two or more, two or more groups Z₃ are identical to or different from each other, and when b1 is two or more, two or more groups R₁₄ are identical to or different from each other,
c1 is an integer from 1 to 4, wherein, when c1 is 2 or more, two or more groups represented by 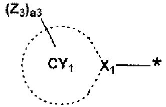 are identical to or different from each other,
when a1 is two or more, two or more groups Z₁ in the number of c1 are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
when a2 is two or more, two or more groups Z₂ in the number of a2 are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
when a3 is two or more, two or more groups Z₃ in the number of a3 are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₂ and R₁₃ are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
when b1 is two or more, two or more groups R₁₄(s) in the number of b1 are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more substituents selected from Z₁ and Z₂ are optionally linked to form a C₅-C₃₀carbocyclic group that is unsubstituted or substituted with at least one R₁₀ₐ or a C₁-C₃₀ heterocyclic group that is unsubstituted or substituted with at least one R₁₀ₐ,
two or more substituents selected from R₁₁ to R₁₄ are optionally linked to form a C₅-C₃₀ carbocyclic group that is unsubstituted or substituted with at least one of R₁₀ₐ or a C₁-C₃₀ heterocyclic group that is unsubstituted or substituted with at least one R₁₀ₐ,
R₁₀ₐ is selected from hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₇-C₆₀ arylalkyl group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group,-N(Q₁)(Q₂), -Si(Q₃)(Q₄)(Q₅), -Ge(Q₃)(Q₄)(Q₅), -B(Q₆)(Q₇), and -P(=O)(Q₈)(Q₉),
* and *' in Formulae 2A and 2B each indicate a binding site to M in Formula 1,
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₇-C₆₀ arylalkyl group, the substituted C₁-C₆₀ heteroaryl group, the substituted C₁-C₆₀ heteroaryloxy group, the substituted C₁-C₆₀ heteroarylthio group, the substituted C₂-C₆₀ heteroarylalkyl group, the substituted monovalent non-aromatic condensed polycyclic group, and the substituted monovalent non-aromatic condensed heteropolycyclic group are each independently selected from:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -B(Q₁₆)(Q₁₇), and -P(=O)(Q₁₈)(Q₁₉);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, or a monovalent non-aromatic condensed heteropolycyclic group, each substituted with at least one selected from deuterium, -F, -Cl, -Br, -I, -CD₃, -CD₂H, -CDH₂, -CF₃,-CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C60 aryloxy group, a C₆-C₆₀ arylthio group, a C₇-C₆₀ arylalkyl group, a C₁-C₆₀ heteroaryl group, a C₁-C₆₀ heteroaryloxy group, a C₁-C₆₀ heteroarylthio group, a C₂-C₆₀ heteroarylalkyl group, a monovalent non-aromatic condensed polycyclic group, a monovalent non-aromatic condensed heteropolycyclic group, -N(Q₂₁)(Q₂₂),-Si(Q₂₃)(Q₂₄)(Q₂₅), -B(Q₂₆)(Q₂₇), and
-P(=O)(Q₂₈)(Q₂₉); and
-N(Q₃₁)(Q₃₂), -Si(Q₃₃)(Q₃₄)(Q₃₅), -B(Q₃₆)(Q₃₇), or -P(=O)(Q₃₈)(Q₃₉); and
Q₁ to Q₉, Q₁₁ to Q₁₉, Q₂₁ to Q₂₉, and Q₃₁ to Q₃₉ are each independently selected from hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryl group substituted with at least one selected from a C₁-C₆₀ alkyl group and a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group.

2. The organometallic compound of claim 1, wherein,
in Formula 1, M is Ir, and the sum of n1 and n2 is 3; or
in Formula 1, M is Pt, and the sum of n1 and n2 is 2.

3. The organometallic compound of claims 1 or 2, wherein,
in Formulae 2A and 2B, ring CY₁, ring CY₂, and ring CY₁₄ are each independently selected from a cyclopentane group, a cyclohexane group, a cycloheptane group, a cyclopentene group, a cyclohexene group, a cycloheptene group, a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, and a 5,6,7,8-tetrahydroquinoline group; and/or
wherein,
in Formula 2B, R₁ to R₃ are each independently selected from a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, a phenyl group, a biphenyl group, or a naphthyl group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof and a C₁-C₁₀ alkyl group.

4. The organometallic compound of any of claims 1-3, wherein,
in Formulae 2A and 2B, Z₁ to Z₃ and R₁₁ to R₁₄ are each independently selected from:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, or a C₁-C₂₀ alkoxy group;
a C₁-C₂₀ alkyl group and a C₁-C₂₀ alkoxy group, each substituted with at least one selected from deuterium, -F, -Cl, -Br, -I, -CD₃, -CD₂H, -CDH₂, -CF₃, -CF₂H,-CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, a naphthyl group, a pyridinyl group, or a pyrimidinyl group;
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, and-Si(Q₃₃)(Q₃₄)(Q₃₅); and
-N(Q₁)(Q₂), -Si(Q₃)(Q₄)(Q₅), -Ge(Q₃)(Q₄)(Q₅), -B(Q₆)(Q₇), -P(=O)(Q₈)(Q₉), or -P(Q₈)(Q₉), wherein R₁₂ is neither hydrogen nor a methyl group, and
Q₁ to Q₉ and Q₃₃ to Q₃₅ are each independently selected from:
-CH₃, -CD₃, -CD₂H, -CDH₂, -CH₂CH₃, -CH₂CD₃, -CH₂CD₂H, -CH₂CDH₂,-CHDCH₃, -CHDCD₂H, -CHDCDH₂, -CHDCD₃, -CD₂CD₃, -CD₂CD₂H, or -CD₂CDH₂; and
an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with at least one selected from deuterium, a C₁-C₁₀ alkyl group, or a phenyl group; and/or
wherein
R₁₂ in Formula 2B is selected from:
a C₂-C₂₀ alkyl group or a C₂-C₂₀ alkoxy group;
a methyl group or a methoxy group, each substituted with at least one selected from a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a naphthyl group, a pyridinyl group, and a pyrimidinyl group;
a methyl group or a methoxy group, each substituted with i) at least one selected from a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a naphthyl group, a pyridinyl group, and a pyrimidinyl group, and ii) at least one deuterium;
a C₂-C₂₀ alkyl group or a C₂-C₂₀ alkoxy group, each substituted with at least one selected from deuterium, -F, -CI, -Br, -I, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a pyridinyl group, and a pyrimidinyl group; and
a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with at least one selected from deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo(1.1.1)pentyl group, a bicyclo(2.1.1)hexyl group, a bicyclo(2.2.1)heptyl group, a bicyclo(2.2.2)octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, and an azadibenzothiophenyl group.

5. The organometallic compound of any of claims 1-4, wherein,
in Formulae 2A and 2B, Z₁ to Z₃, R₁₁, R₁₃, and R₁₄ are each independently selected from hydrogen, deuterium, -F, a cyano group, a nitro group, -SF₅, -CH₃,-CD₃, -CD₂H, -CDH₂, -CF₃, -CF₂H, -CFH₂, groups represented by Formulae 9-1 to 9-66, groups represented by Formulae 9-1 to 9-66 wherein at least one hydrogen is substituted with deuterium, groups represented by Formulae 10-1 to 10-249, and groups represented by Formulae 10-1 to 10-249 wherein at least one hydrogen is substituted with deuterium, -N(Q₁)(Q₂), -Si(Q₃)(Q₄)(Q₅), and -Ge(Q₃)(Q₄)(Q₅), wherein Q₁ to Q₅ are each independently the same as in claim 1, and
R₁₂ in Formula 2B is selected from groups represented by Formulae 9-1 to 9-66, groups represented by Formulae 9-1 to 9-66 wherein at least one hydrogen is substituted with deuterium, groups represented by Formulae 10-1 to 10-249, groups represented by Formulae 10-1 to 10-249 wherein at least one hydrogen is substituted with deuterium: 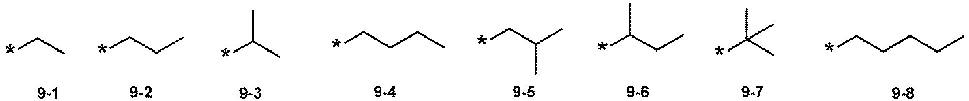 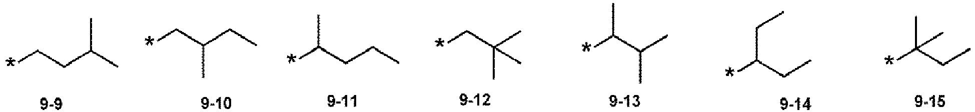 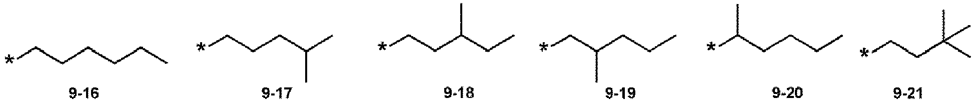 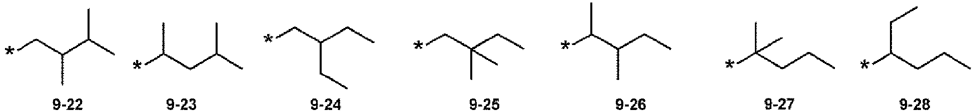 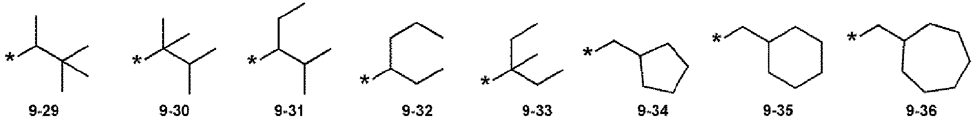 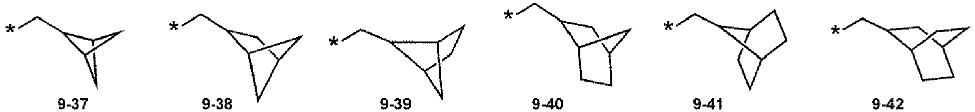 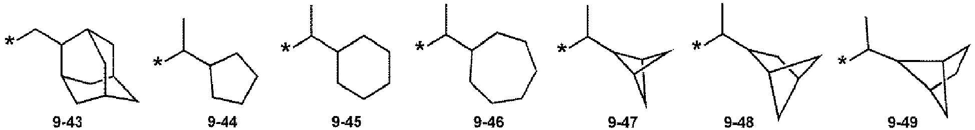 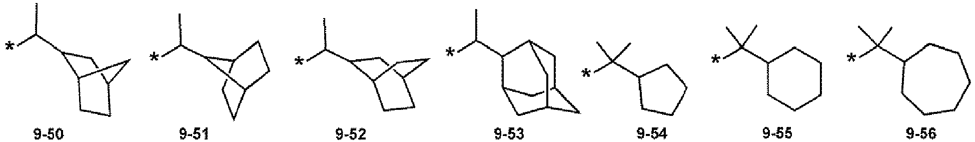 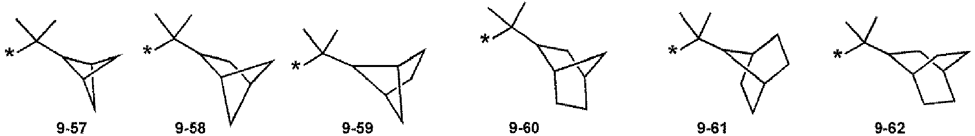 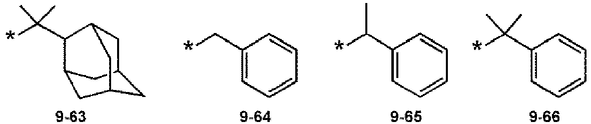 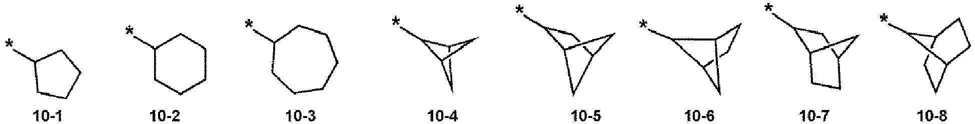 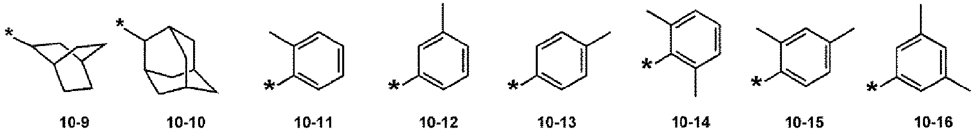 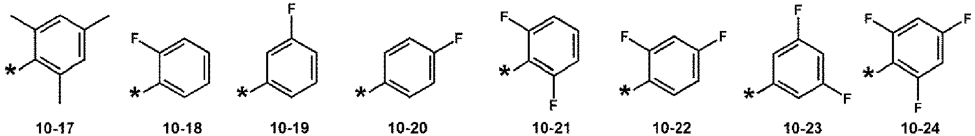 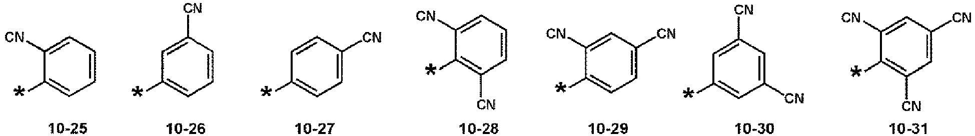 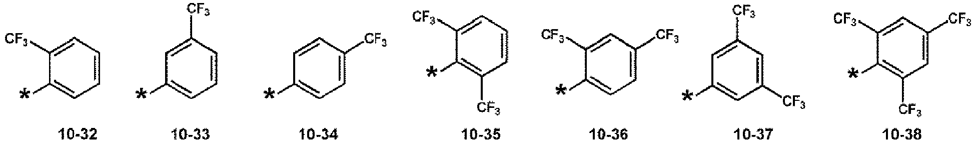 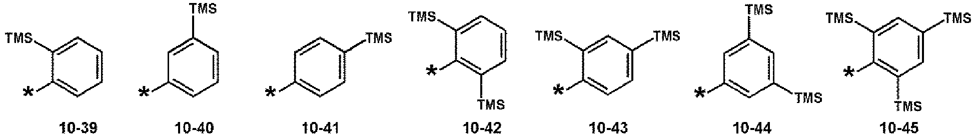 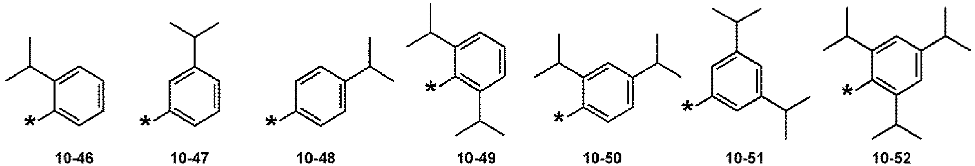 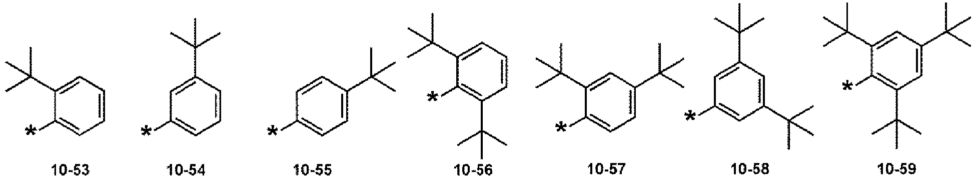 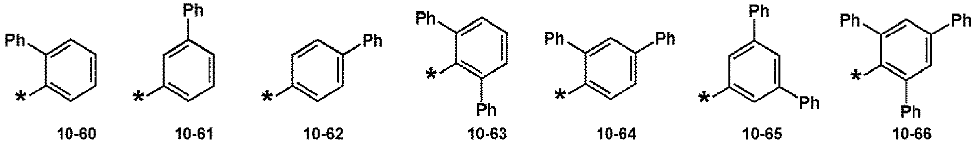 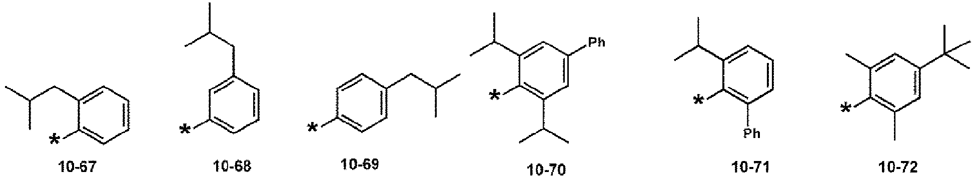 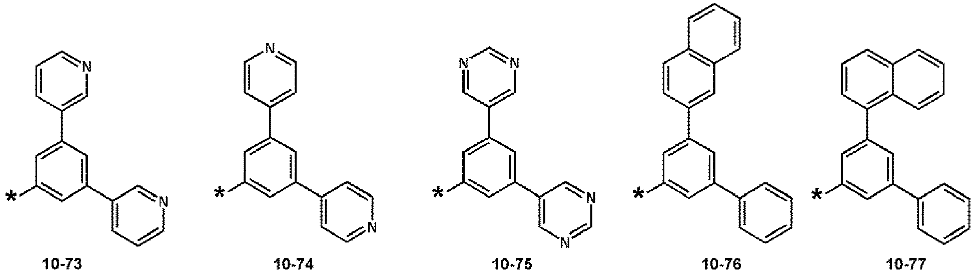 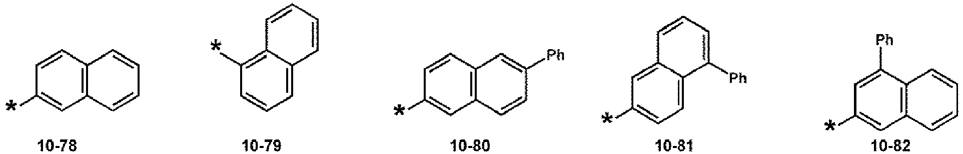 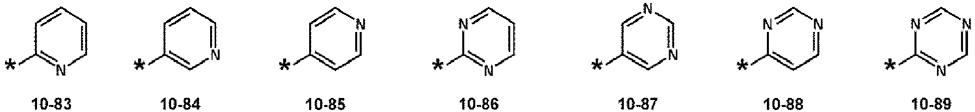 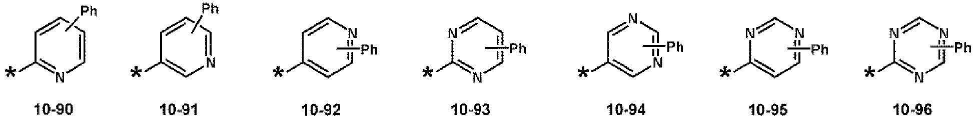 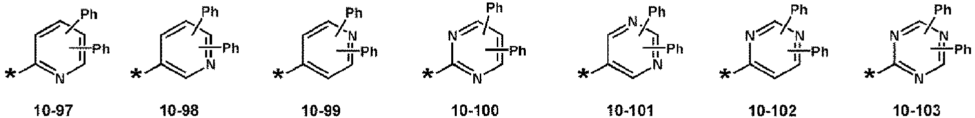 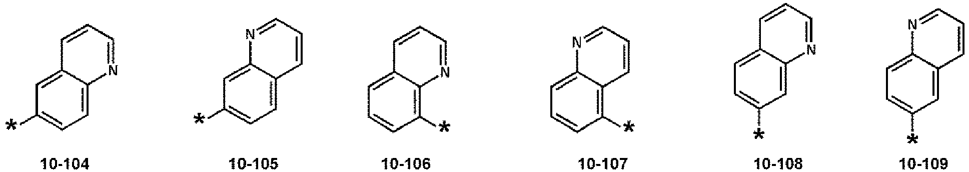 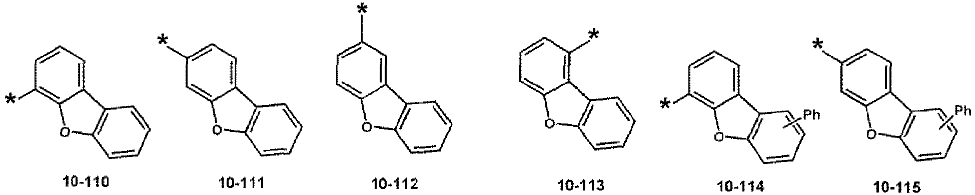 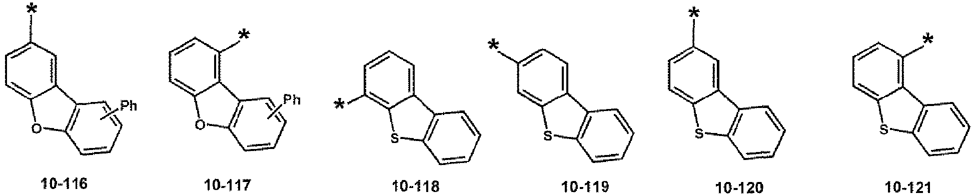 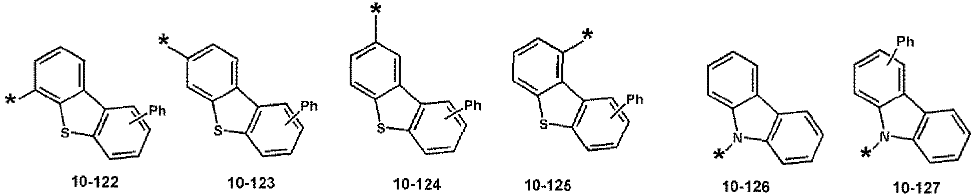 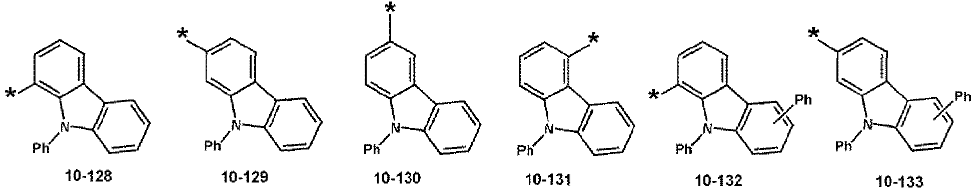 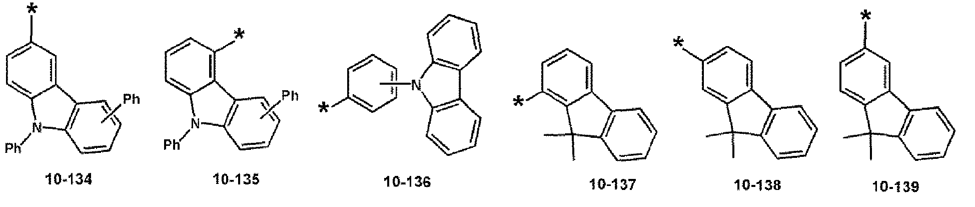 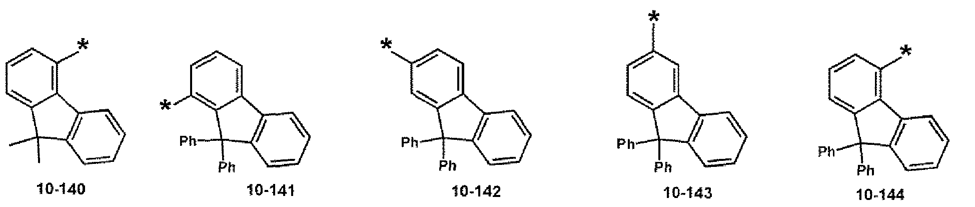 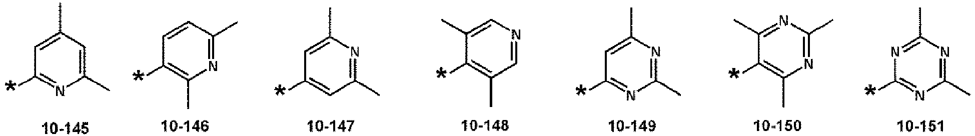 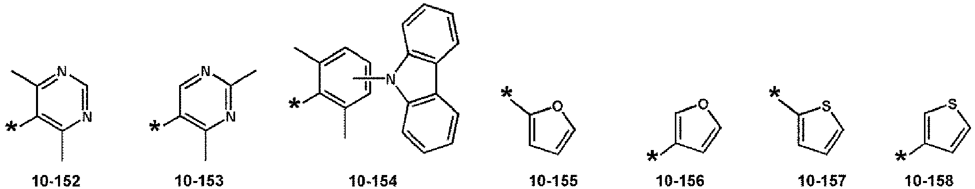 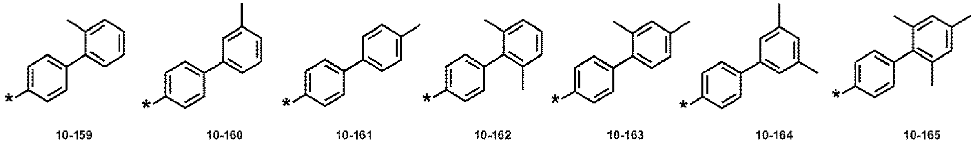 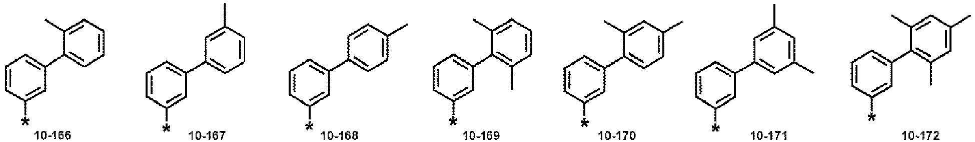 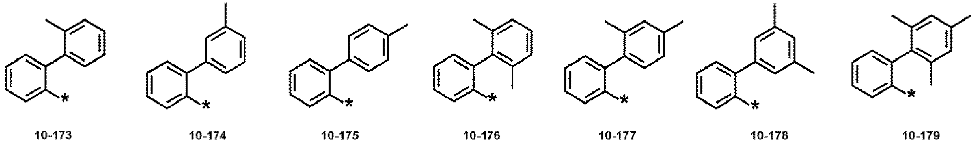 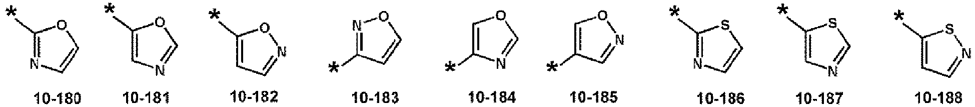 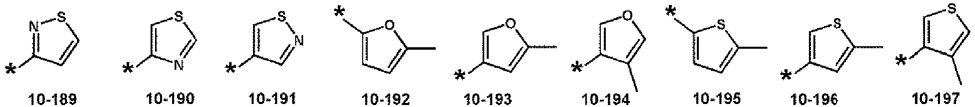 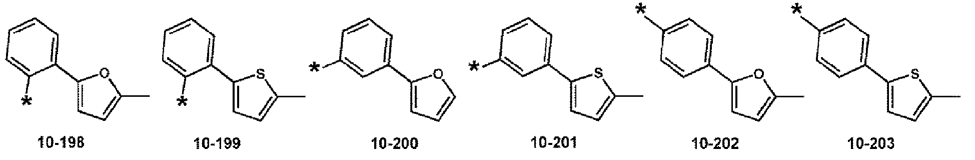 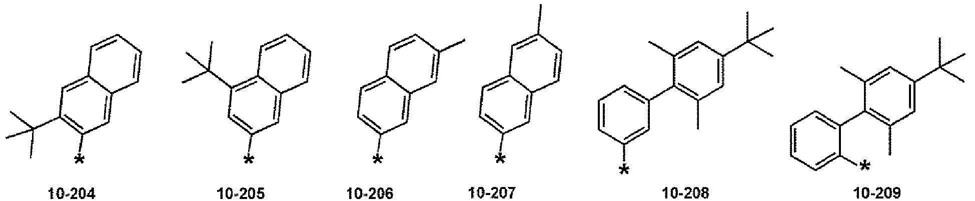 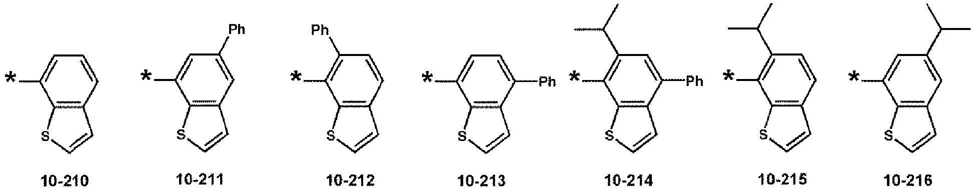 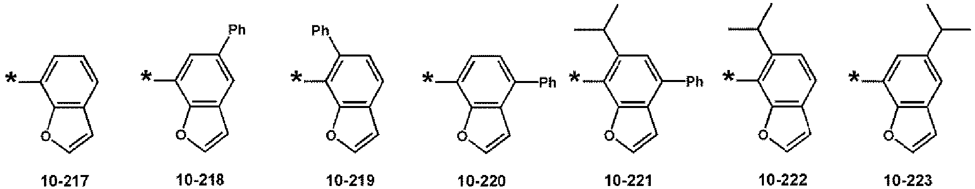 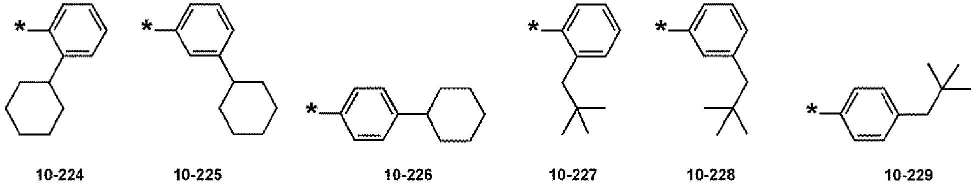 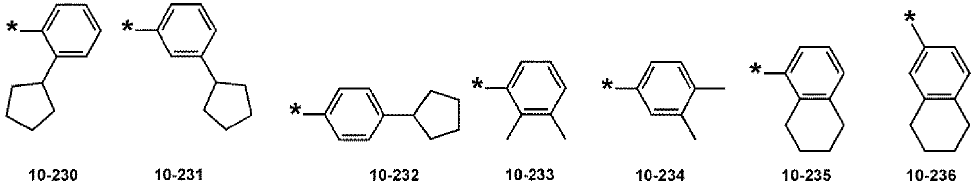 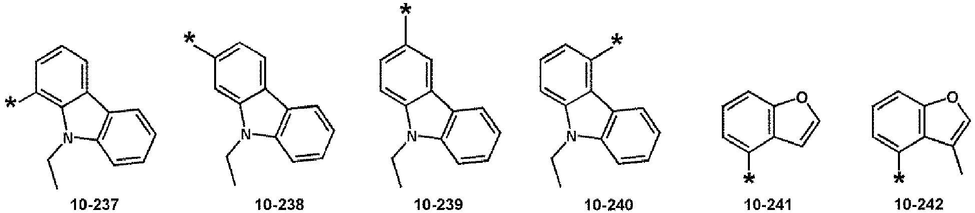 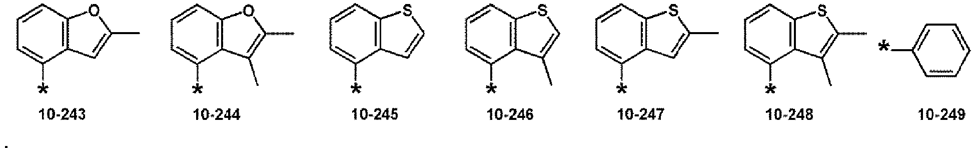 wherein, in Formulae 9-1 to 9-66 and 10-1 to 10-249, * indicates a binding site to a neighboring atom, Ph indicates a phenyl group, and TMS indicates a trimethylsilyl group.

6. The organometallic compound of any of claims 1-5, wherein,
in Formula 2A, Z₁ is selected from hydrogen, deuterium, -F, a cyano group, a substituted or unsubstituted C₁-C₆₀ alkyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, -N(Q₁)(Q₂), -Si(Q₃)(Q₄)(Q₅), and -Ge(Q₃)(Q₄)(Q₅), wherein Q₁ to Q₅ are each independently the same as in claim 1.

7. The organometallic compound of any of claims 1-6, wherein c1 in Formula 2A is 1 or 2; and/or
wherein
the group represented by 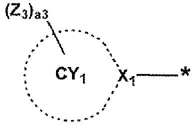 in Formula 2A is a group represented by one selected from Formulae CY1(1) to CY1(20): 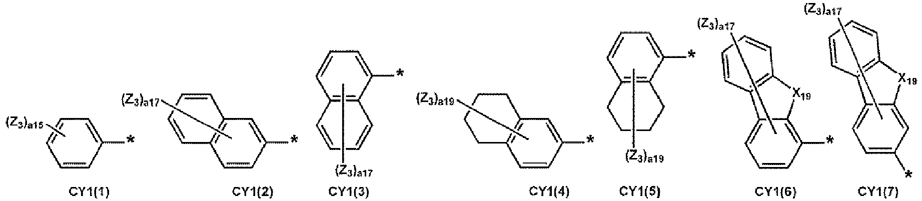 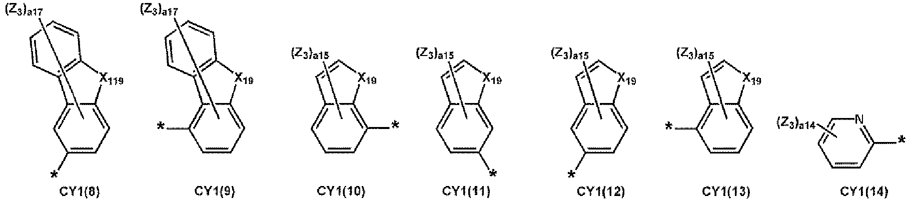 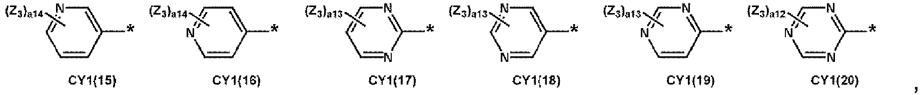 wherein, in Formulae CY1(1) to CY1(20),
Z₃ is the same as described in claim 1,
X₁₉ is O, S, N(Z₄), C(Z₄)(Z₅), or Si(Z₄)(Z₅),
Z₄ and Z₅ are each independently the same as described in connection with Z₃,
a12 is an integer from 0 to 2,
a13 is an integer from 0 to 3,
a14 is an integer from 0 to 4,
a15 is an integer from 0 to 5,
a17 is an integer from 0 to 7,
a19 is an integer from 0 to 9, and
* indicates a binding site to a neighboring carbon atom.

8. The organometallic compound of any of claims 1-7, wherein
the group represented by 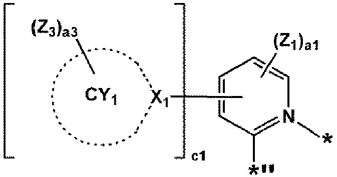 in Formula 2A is a group represented by one selected from Formulae CY1-1 to CY1-56: 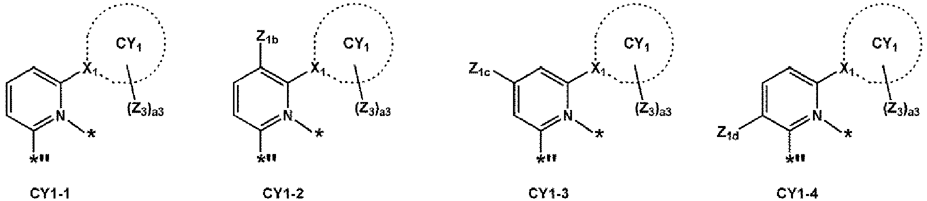 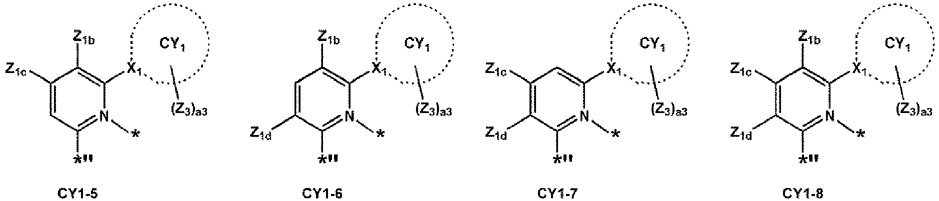 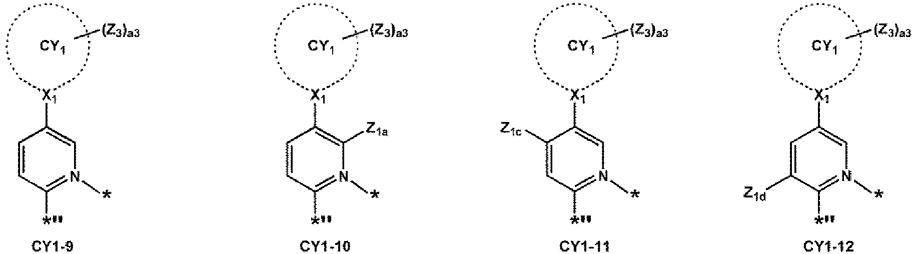 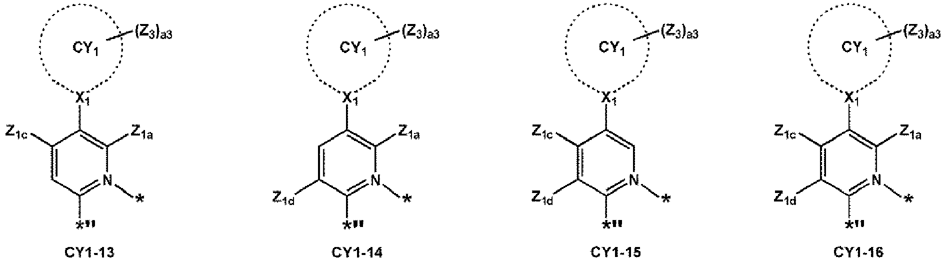 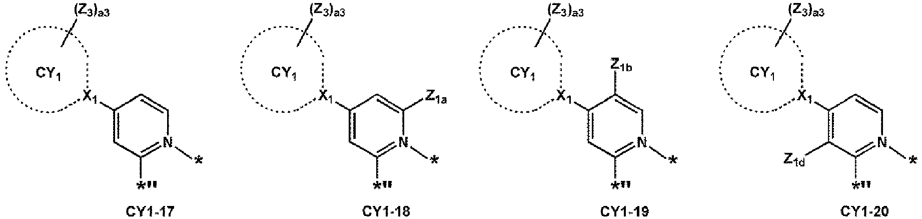 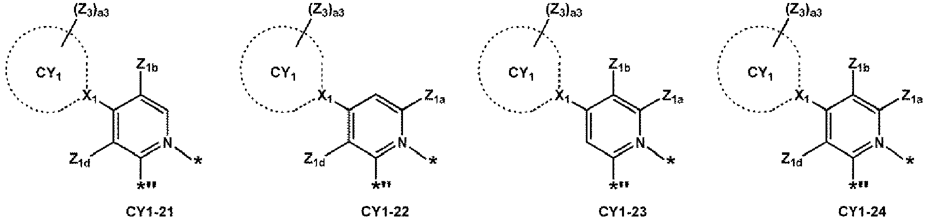 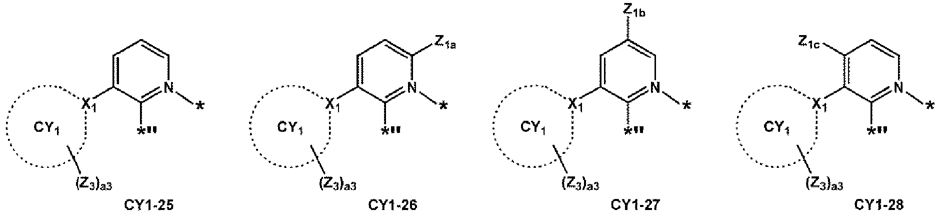 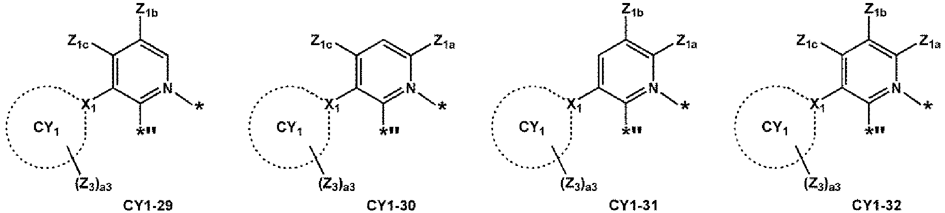 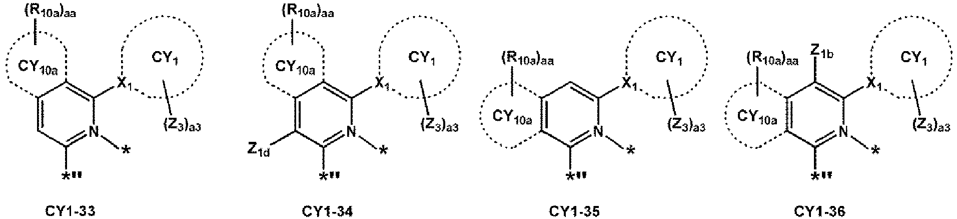 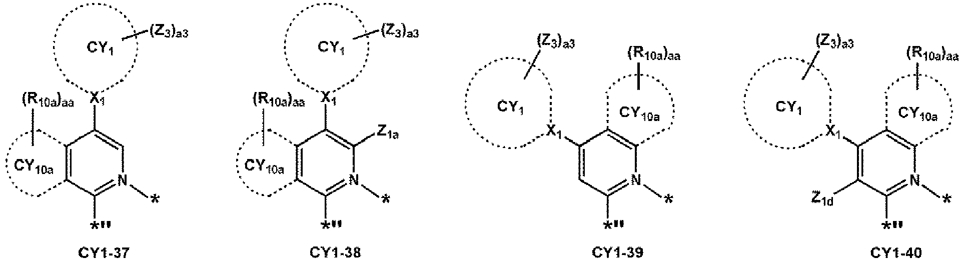 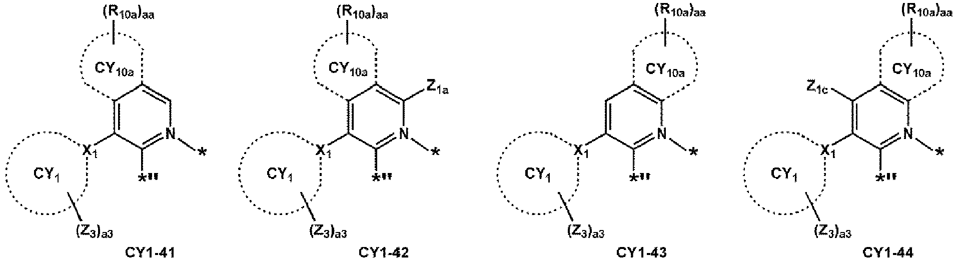 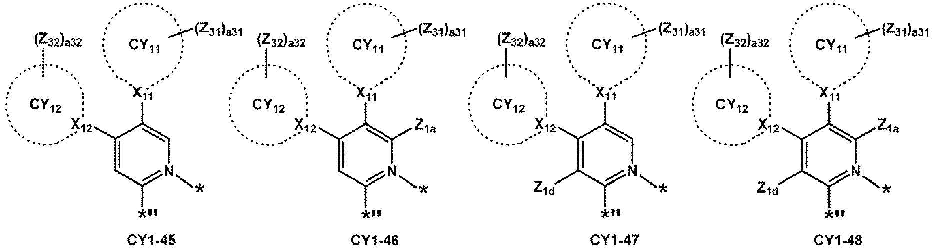 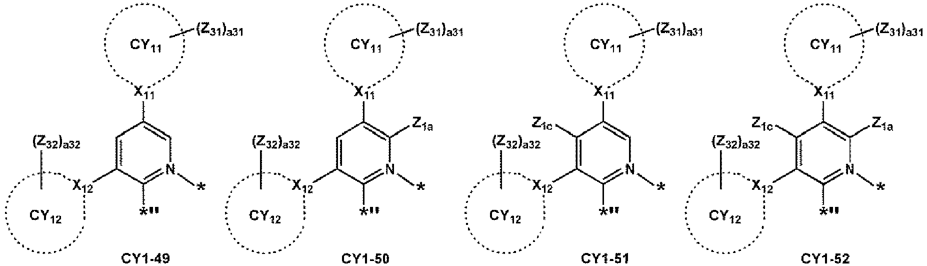 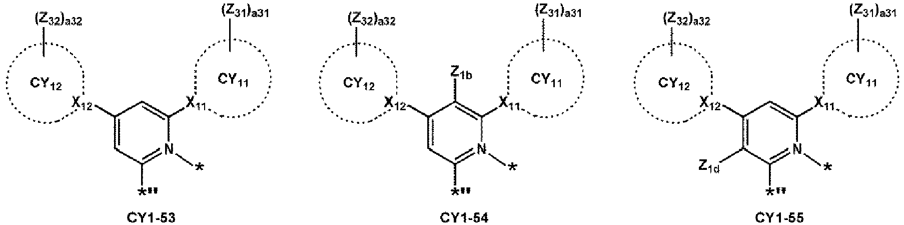 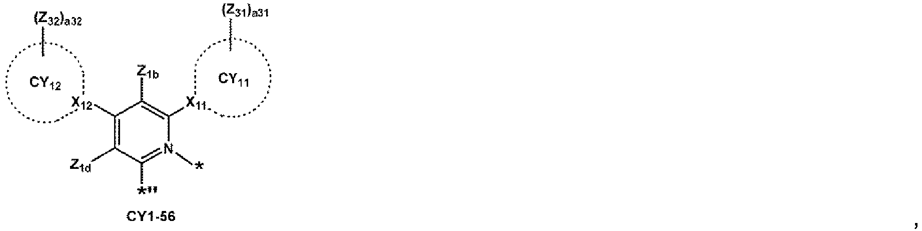 wherein, in Formulae CY1-1 to CY1-56,
X₁, ring CY₁, Z₃, and a3 are each independently the same as described in claim 1,
Z₁ₐ to Z_{1d} are each independently the same as described in connection with Z₁ in claim 1, wherein Z₁ₐ to Z_{1d} are not hydrogen,
X₁₁ and X₁₂ are each independently the same as described in connection with X₁ in claim 1,
ring CY₁₁ and ring CY₁₂ are each independently the same as described in connection with ring CY₁ in claim 1,
Z₃₁ and Z₃₂ are each independently the same as described in connection with Z₃ in claim 1,
a31 and a32 are each independently the same as described in connection with a3 in claim 1,
ring CY₁₀ₐ is a C₅-C₃₀ carbocyclic group or a C₁-C₃₀ heterocyclic group,
R₁₀ₐ is the same as described in claim 1,
aa is an integer from 0 to 10,
* indicates a binding site to M in Formula 1, and
*" indicates a binding site to ring CY₂ in Formula 2A.

9. The organometallic compound of any of claims 1-8, wherein
the group represented by 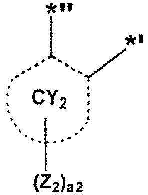 in Formula 2A is a group represented by one selected from Formulae CY2-1 to CY2-64: 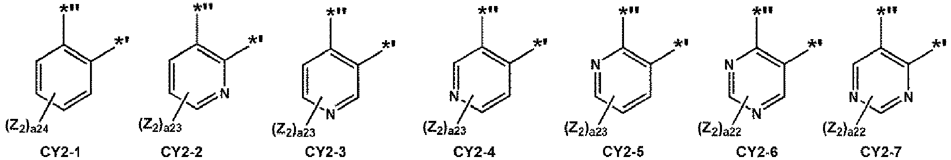 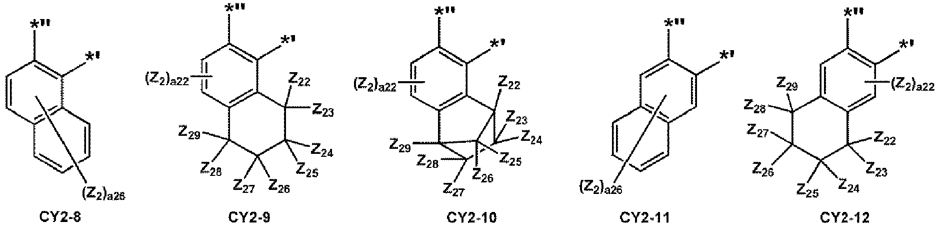 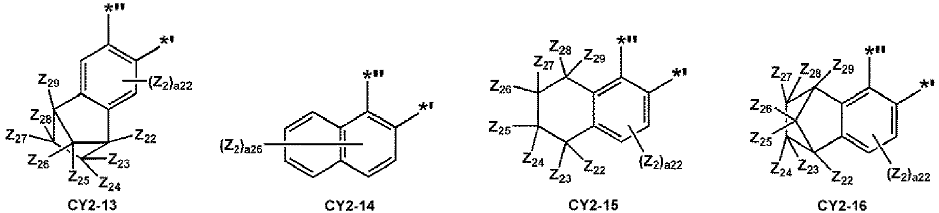 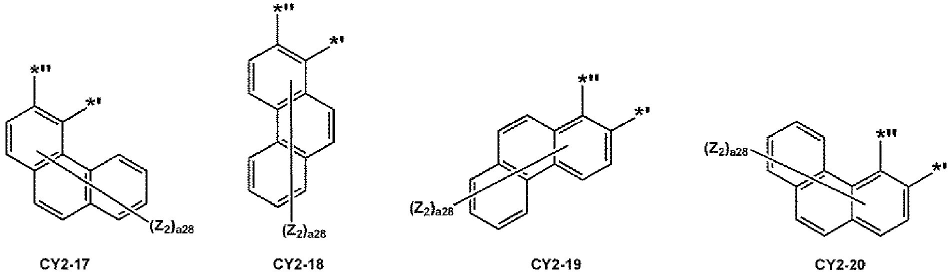 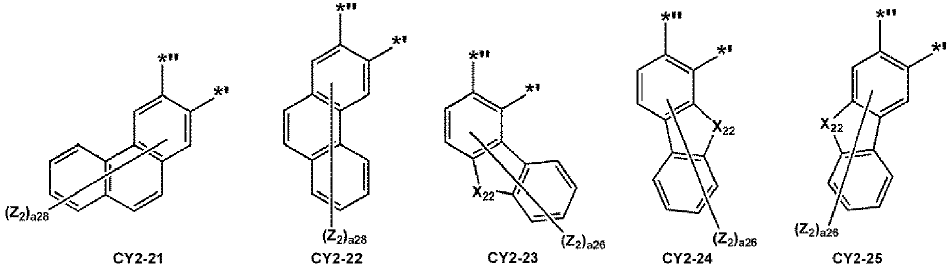 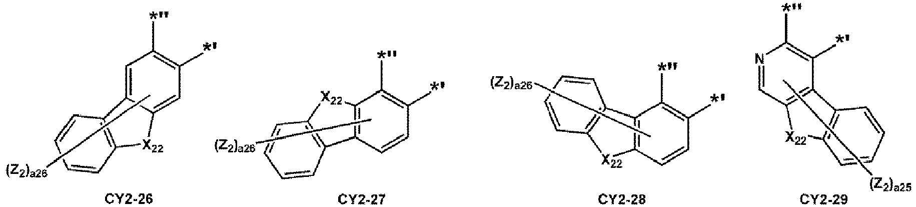 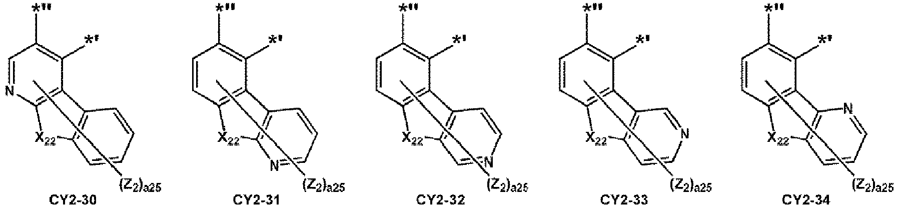 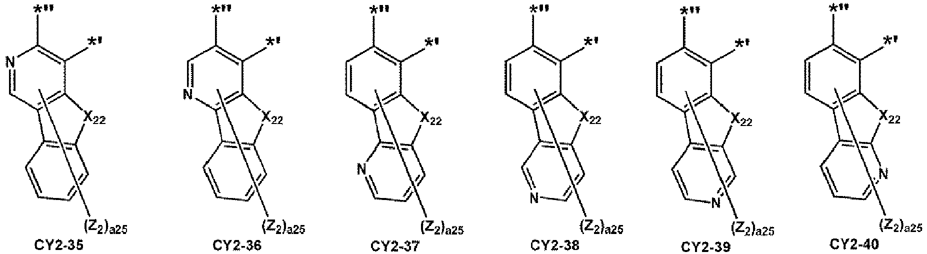 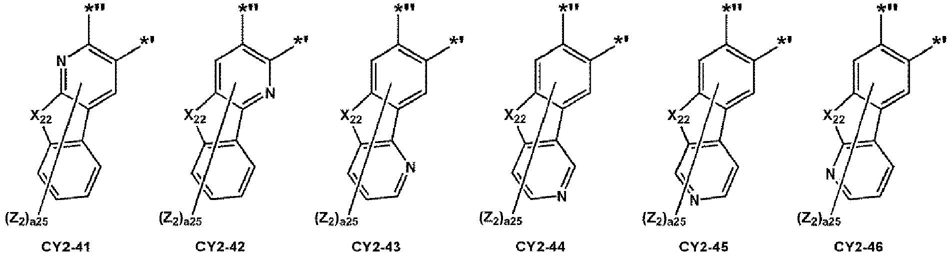 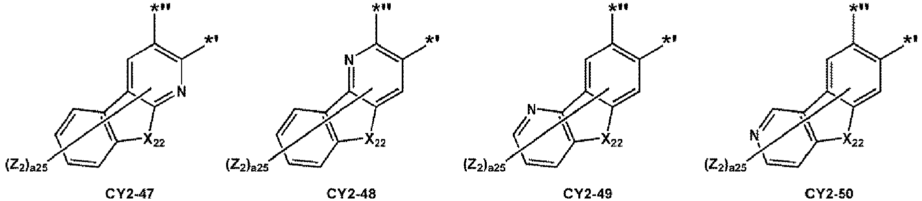 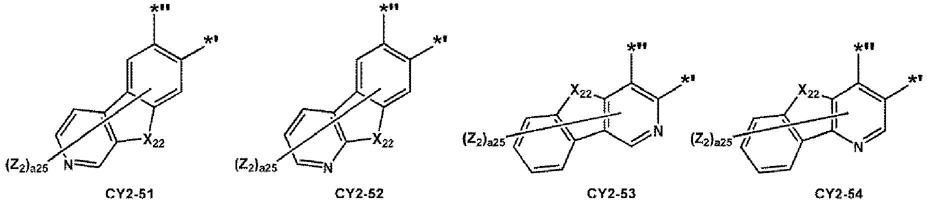 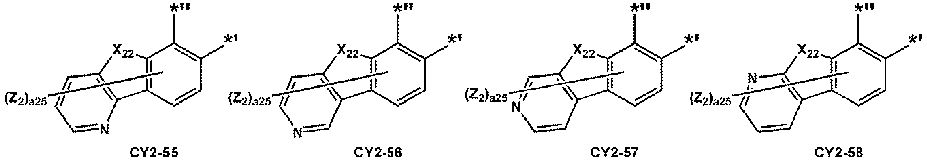 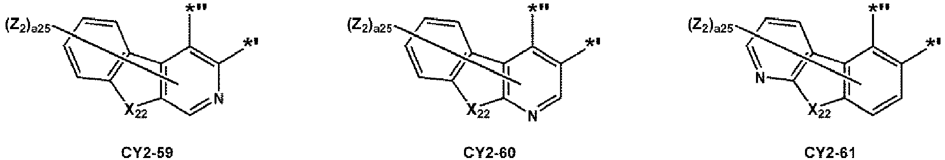 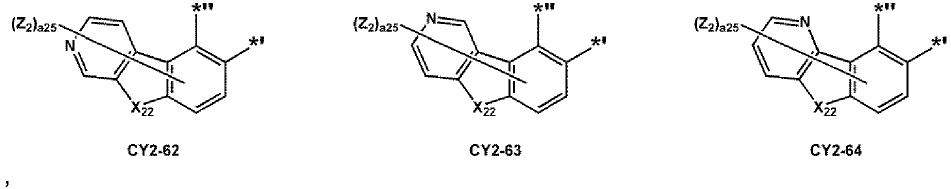 wherein, in Formulae CY2-1 to CY2-64,
Z₂ is the same as described in claim 1,
X₂₂ is C(Z₂₂)(Z₂₃), N(Z₂₂), O, S, or Si(Z₂₂)(Z₂₃),
Z₂₂ to Z₂₉ are each independently the same as described in connection with Z₂ in claim 1,
a28 is an integer from 0 to 8,
a26 is an integer from 0 to 6,
a25 is an integer from 0 to 5,
a24 is an integer from 0 to 4,
a23 is an integer from 0 to 3,
a22 is an integer from 0 to 2,
*" indicates a binding site to a carbon atom of a neighboring pyridine ring in Formula 2A, and
*' indicates a binding site to M in Formula 1.

10. The organometallic compound of any of claims 1-9, wherein
the group represented by 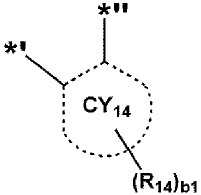 in Formula 2B is a group represented by one selected from Formulae CY14-1 to CY14-64: 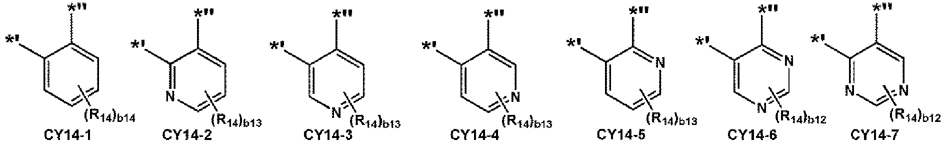 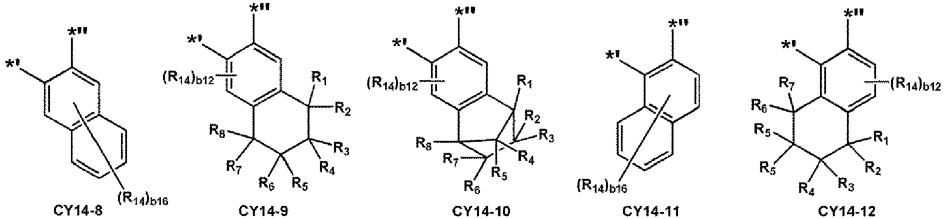 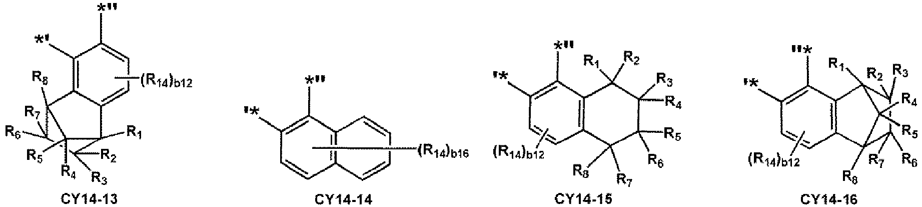 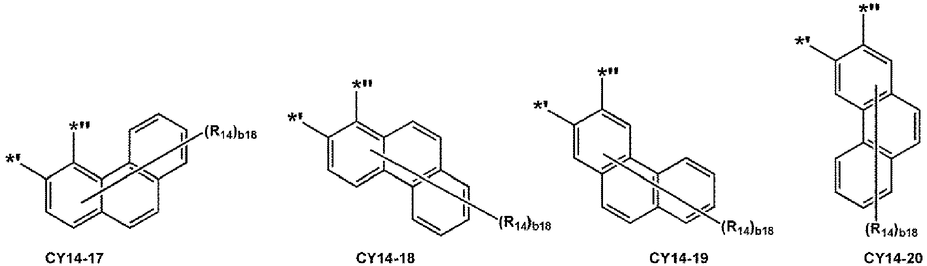 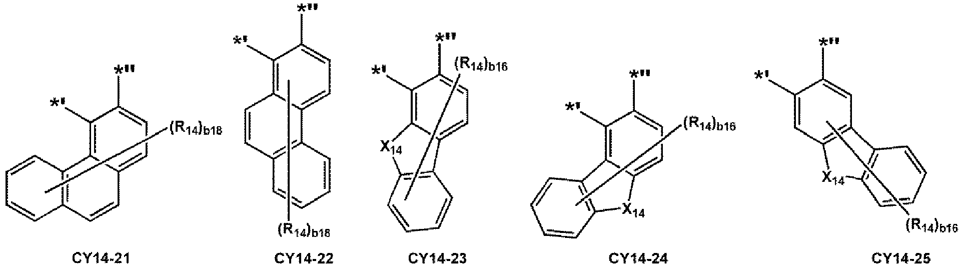 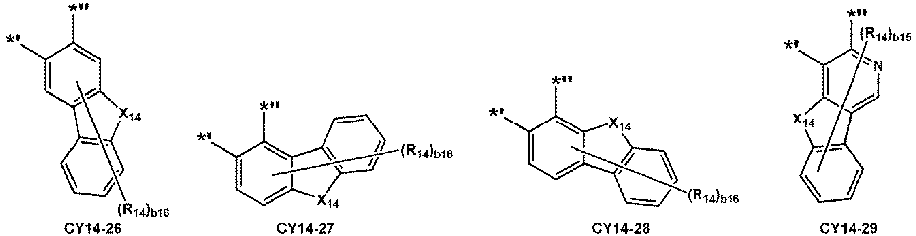 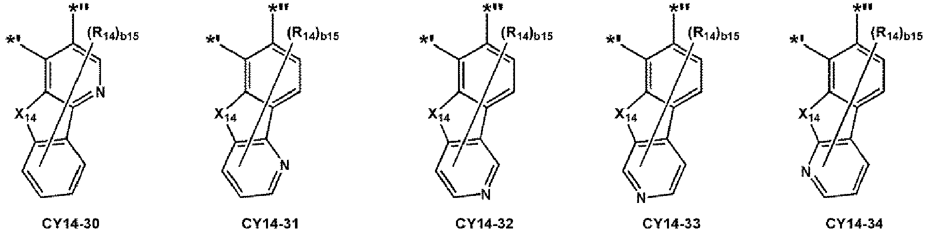 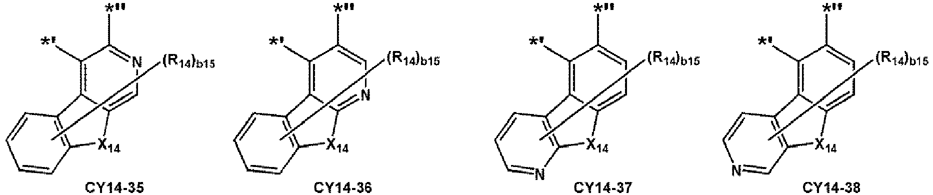 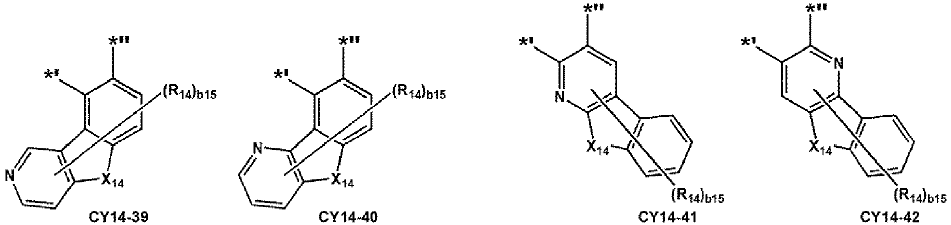 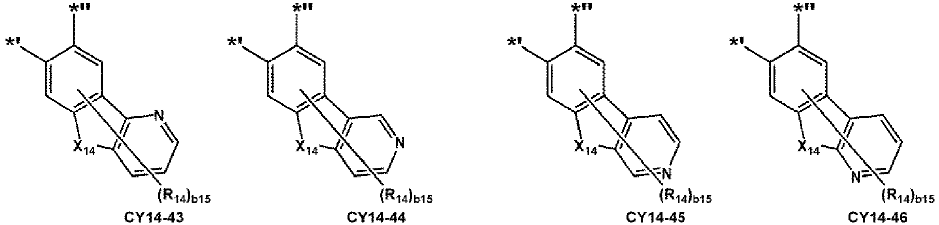 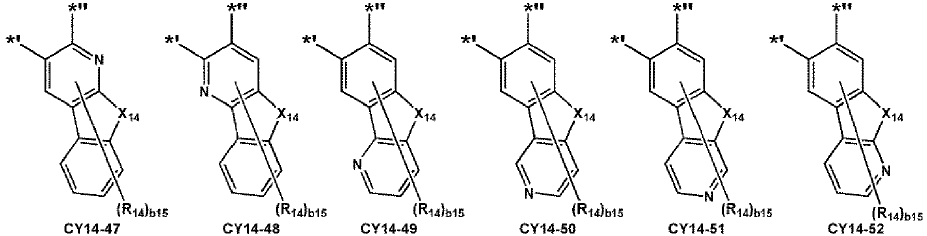 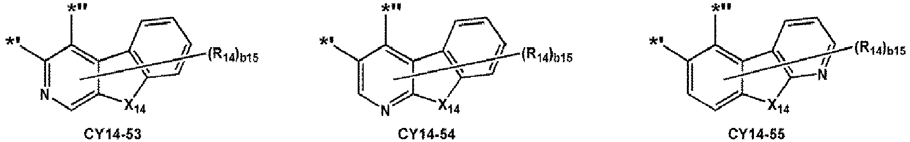 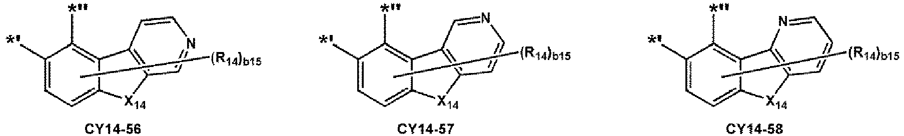 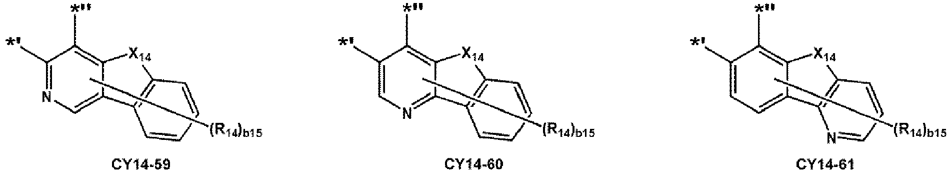 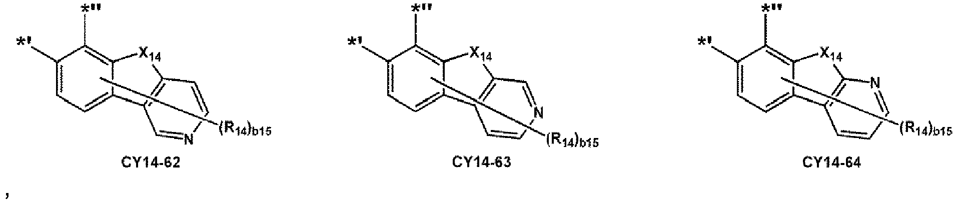 wherein, in Formulae CY14-1 to CY14-64,
R₁₄ is the same as described in claim 1,
X₁₄ is C(R₁)(R₂), N(R₁), O, S, or Si(R₁)(R₂),
R₁ to R₈ are each independently the same as described in connection with R₁₄ in claim 1,
b18 is an integer from 0 to 8,
b16 is an integer from 0 to 6,
b15 is an integer from 0 to 5,
b14 is an integer from 0 to 4,
b13 is an integer from 0 to 3,
b12 is an integer from 0 to 2,
*" indicates a binding site to a carbon atom of a neighboring pyridine ring in Formula 2B, and
*' indicates a binding site to M in Formula 1.

11. The organometallic compound of any of claims 1-10, wherein
the organometallic compound is represented by Formula 1A: 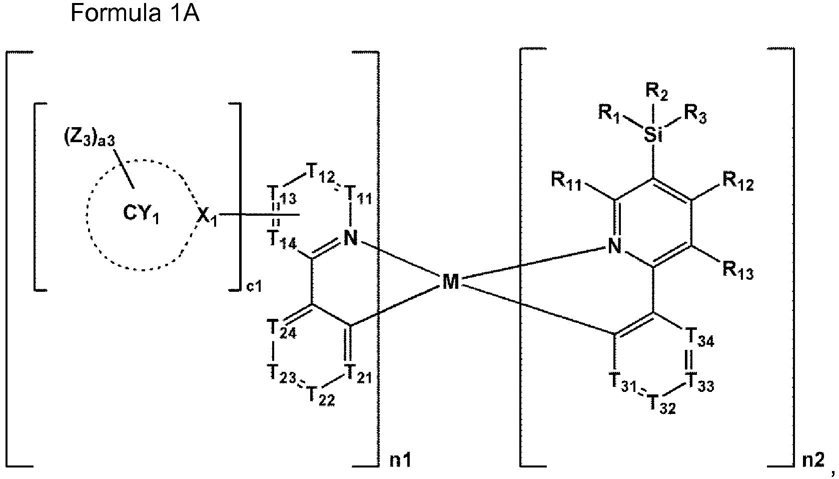 wherein, in Formula 1A,
M, n1, n2, X₁, ring CY₁, R₁ to R₃, Z₃, R₁₁ to R₁₃, and c1 are each independently the same as described in claim 1,
T₁₁ is N, C(Z₁ₐ), or carbon linked to ring CY₁, T₁₂ is N, C(Z_{1b}), or carbon linked to ring CY₁, T₁₃ is N, C(Z_{1c}), or carbon linked to ring CY₁, T₁₄ is N, C(Z_{1d}), or carbon linked to ring CY₁, at least one selected from T₁₁ to T₁₄ is carbon linked to ring CY₁, and Z₁ₐ to Z_{1d} are each independently the same as described in connection with Z₁ in claim 1,
T₂₁ is N or C(Z₂ₐ), T₂₂ is N or C(Z_{2b}), T₂₃ is N or C(Z_{2c}), T₂₄ is N or C(Z_{2d}), and Z₂ₐ to Z_{2d} are each independently the same as described in connection with Z₂ in claim 1,
T₃₁ is N or C(R₁₄ₐ), T₃₂ is N or C(R_{14b}), T₃₃ is N or C(R_{14c}), T₃₄ is N or C(R_{14d}), and R₁₄ₐ to R_{14d} are each independently the same as described in connection with R₁₄ in claim 1,
two or more groups Z₁ₐ to Z_{1d} are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more groups Z₂ₐ to Z_{2d} are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
when a3 is two or more, two or more groups Z₃ are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₂ and R₁₃ are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more groups R₁₄ₐ to R_{14d} are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
two or more groups Z₁ₐ to Z_{1d}, Z₂ₐ to Z_{2d}, R₁₁ to R₁₃, and R₁₄ₐ to R_{14d} are optionally linked to form a C₅-C₃₀ carbocyclic group that is unsubstituted or substituted with at least one R₁₀ₐ or a C₁-C₃₀ heterocyclic group that is unsubstituted or substituted with at least one R₁₀ₐ,
R₁₀ₐ is the same as described in claim 1,
the bonds beteween N and M are coordinate bonds, and
the bonds between C and M are covalent bonds.

12. The organometallic compound of claim 1, wherein
the organometallic compound is one selected from Compounds 1 to 420:

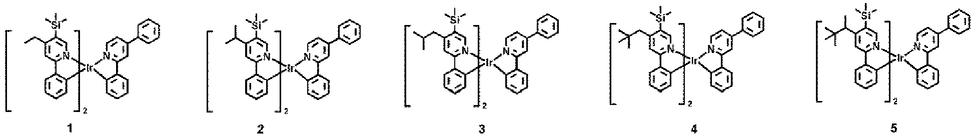

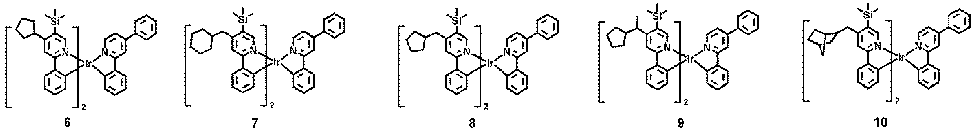

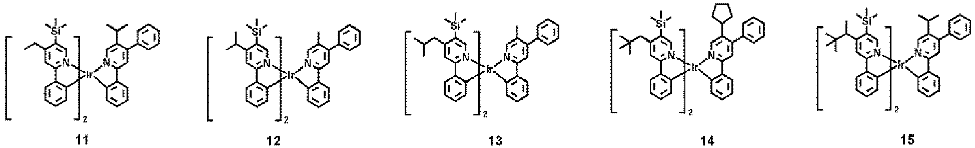

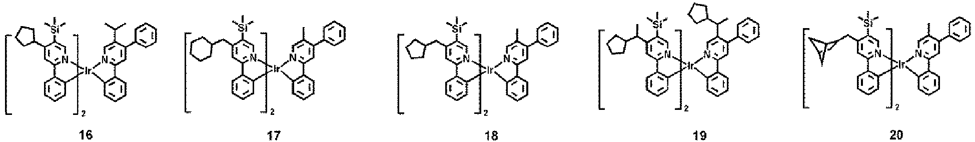

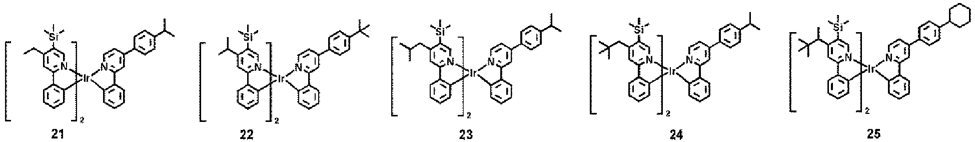

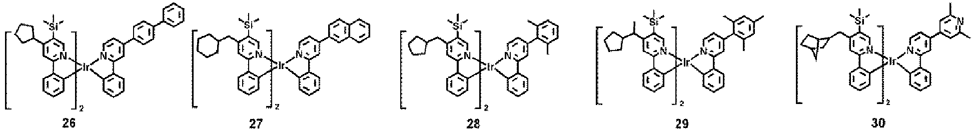

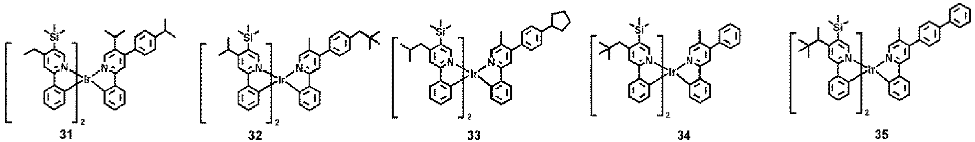

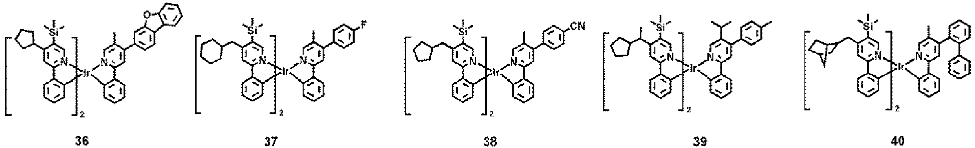

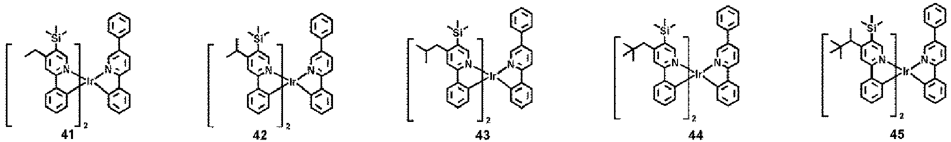

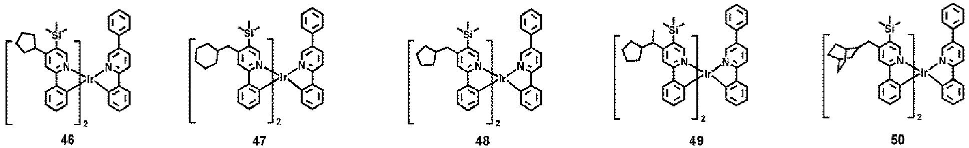

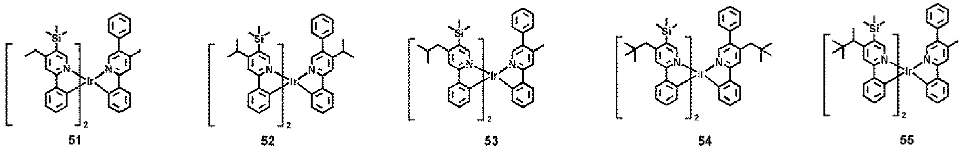

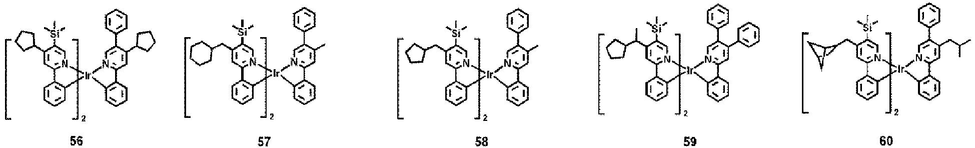

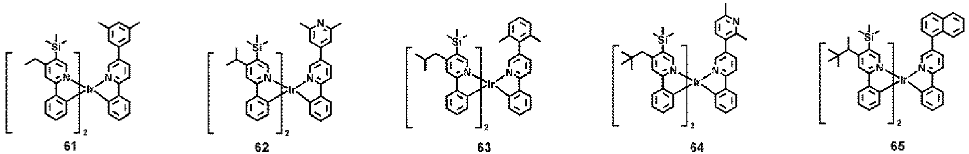

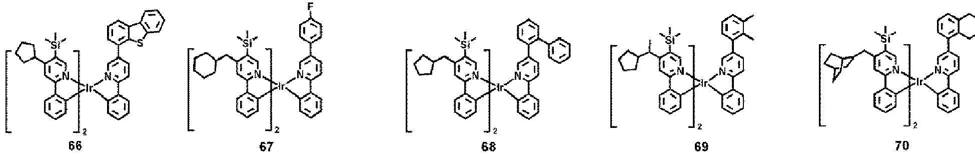

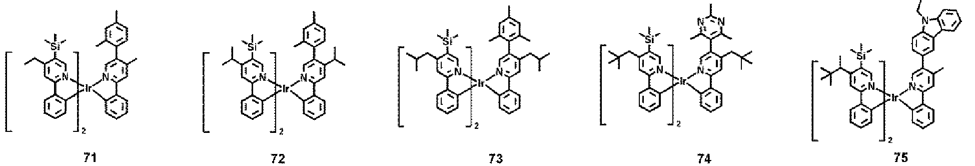

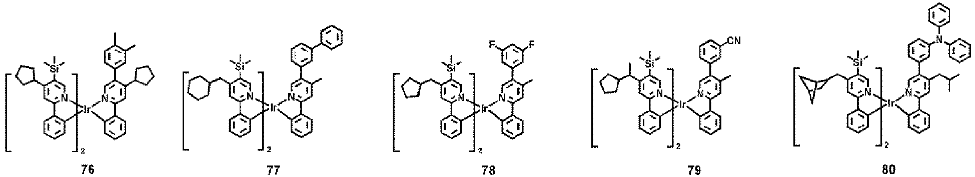

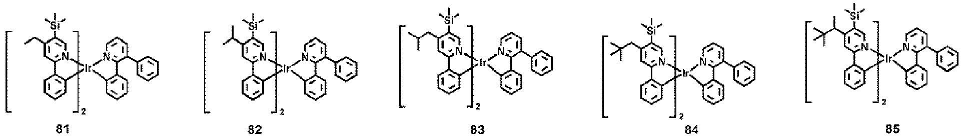

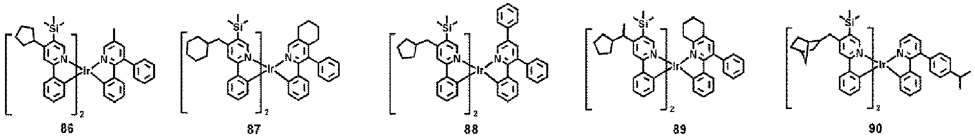

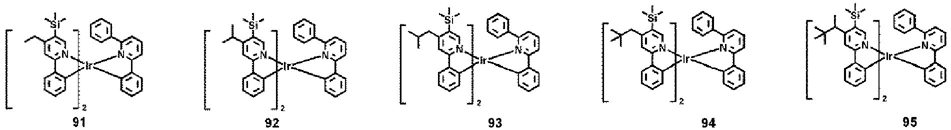

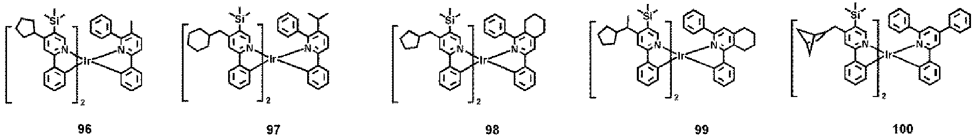

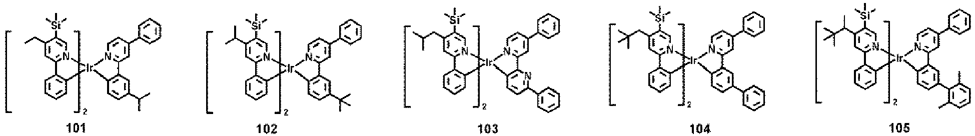

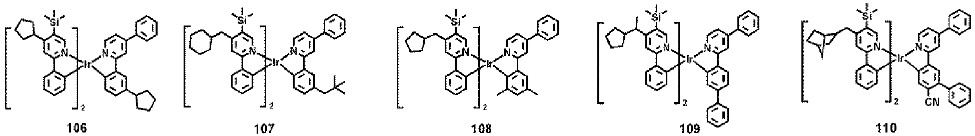

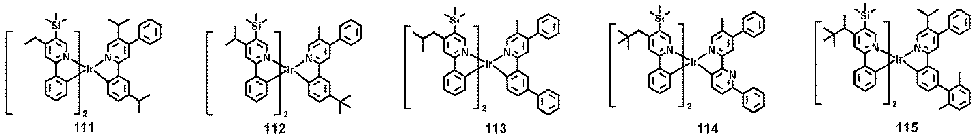

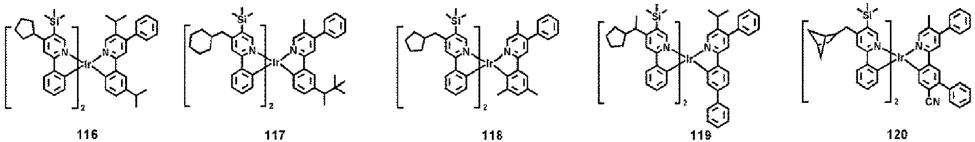

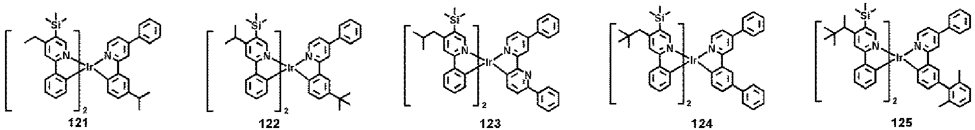

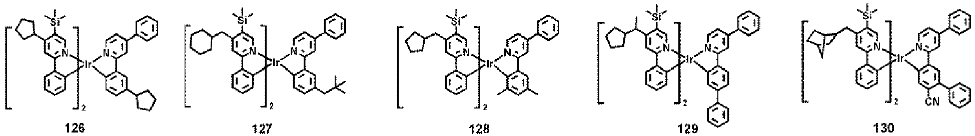

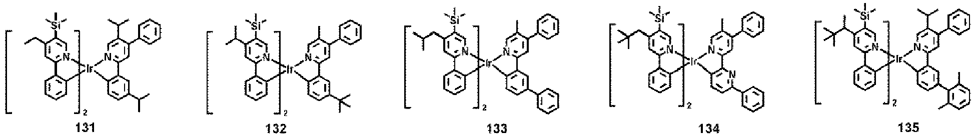

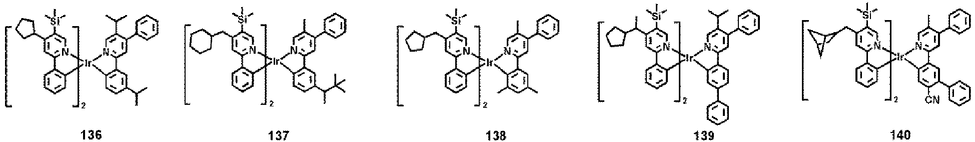

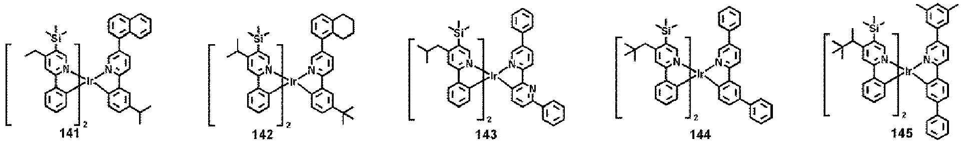

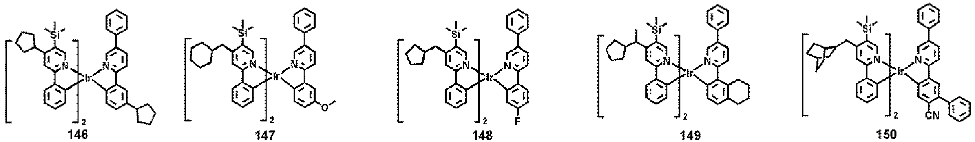

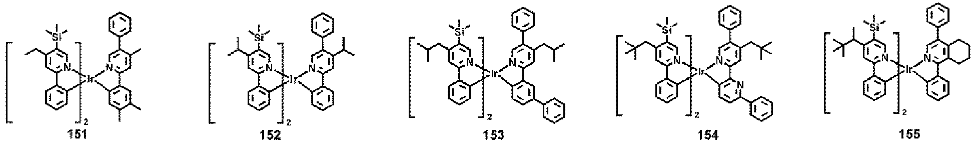

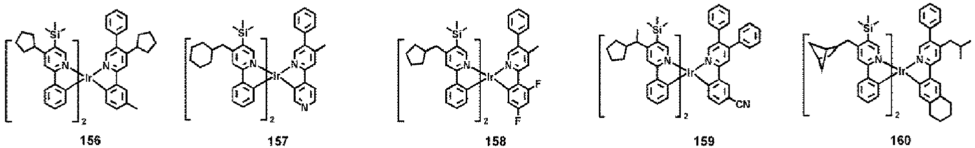

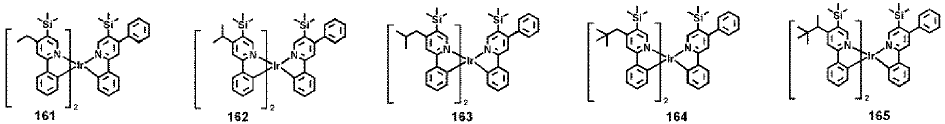

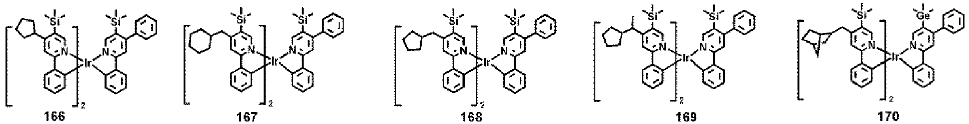

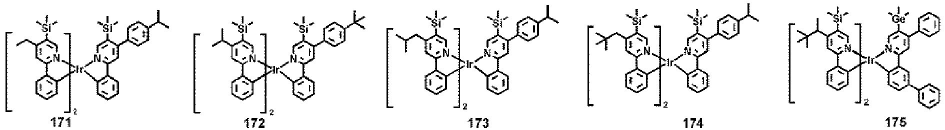

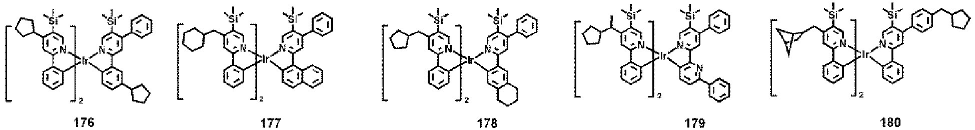

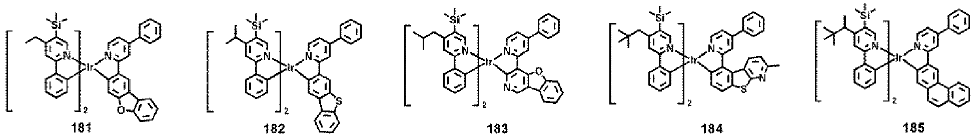

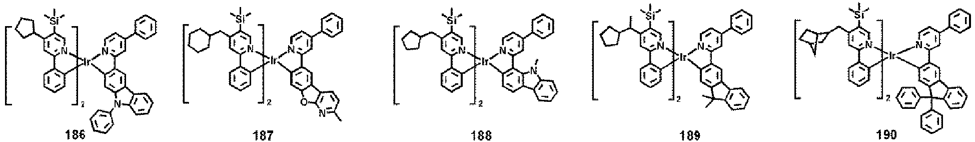

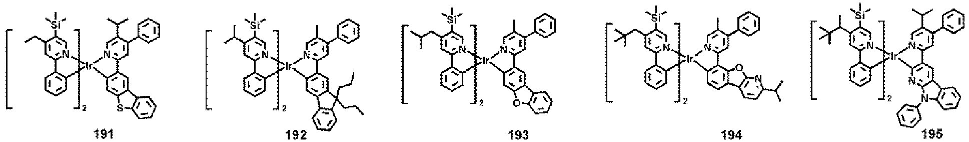

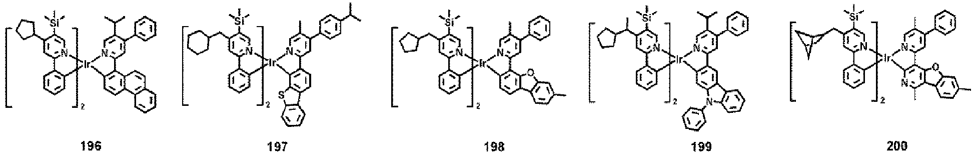

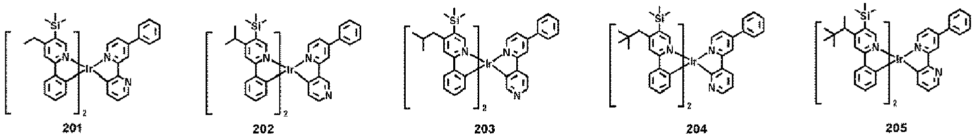

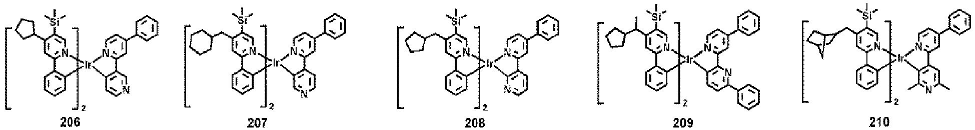

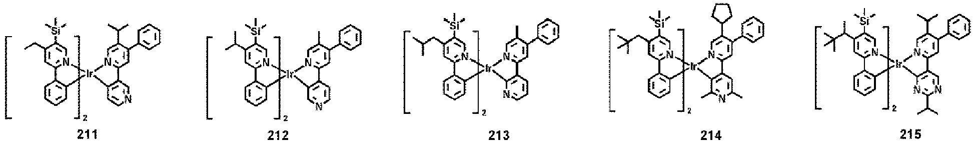

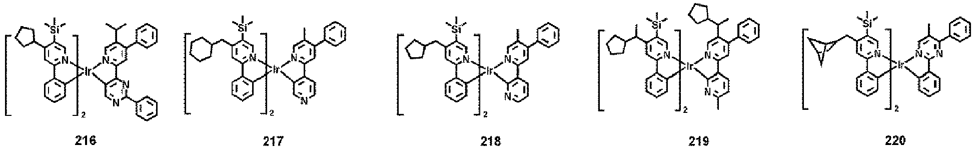

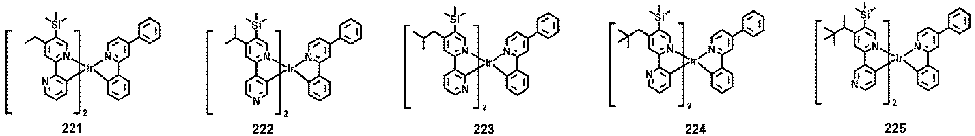

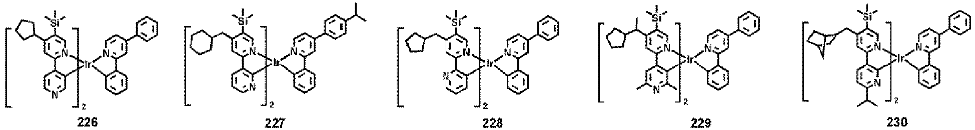

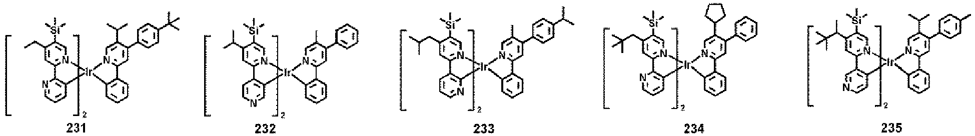

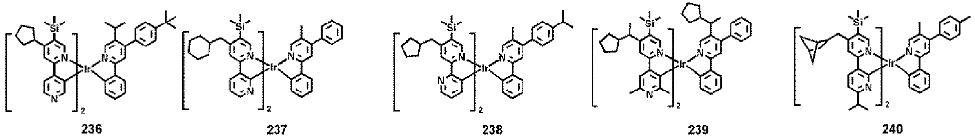

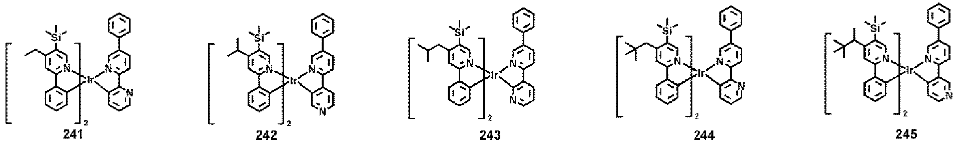

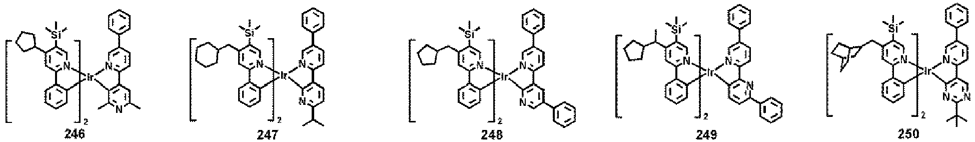

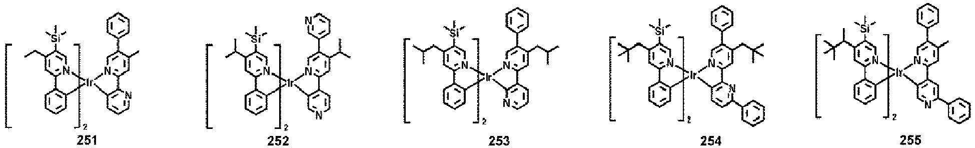

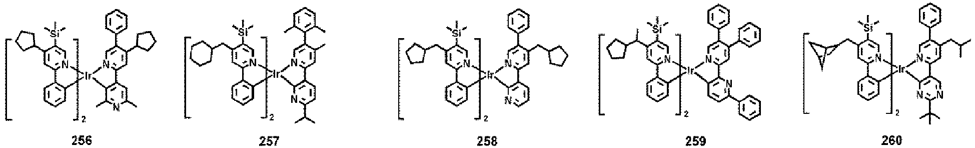

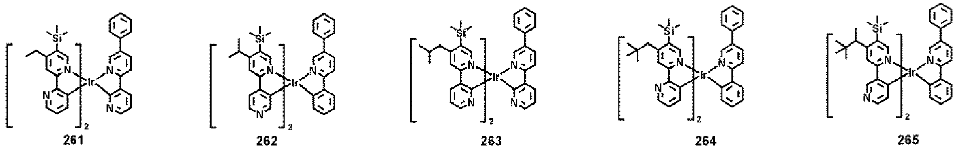

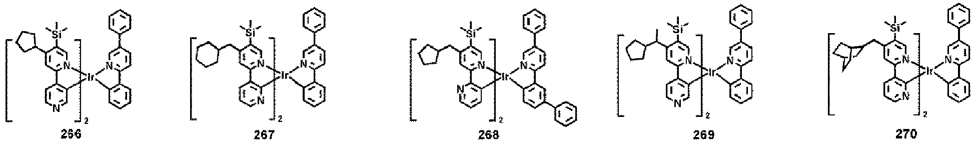

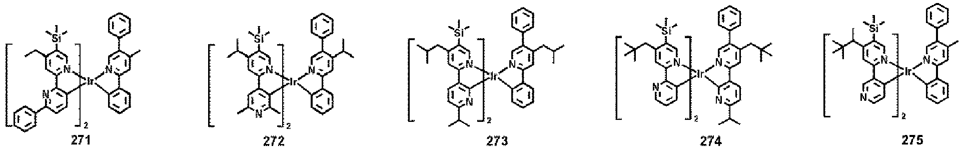

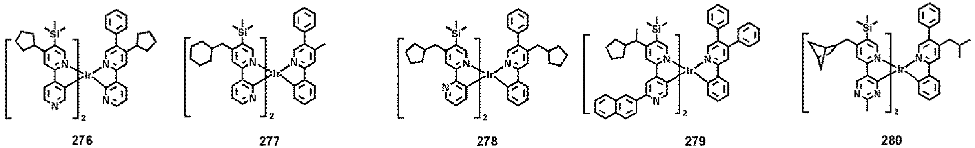

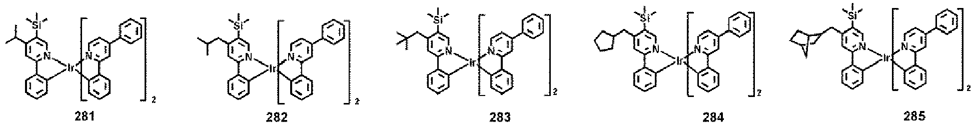

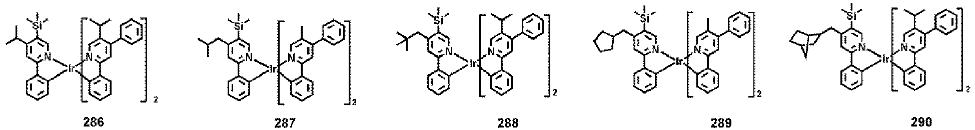

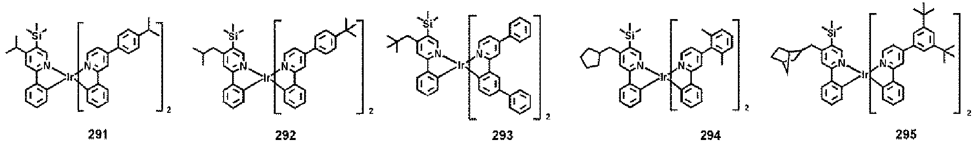

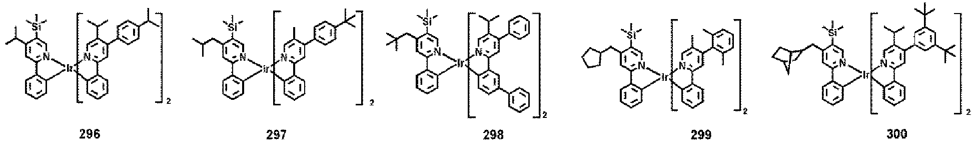

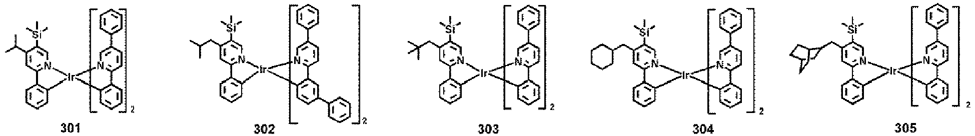

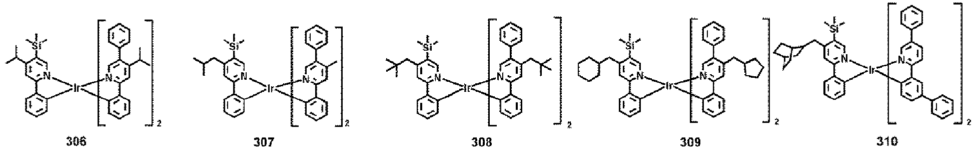

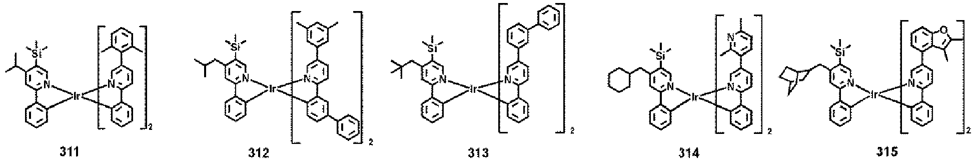

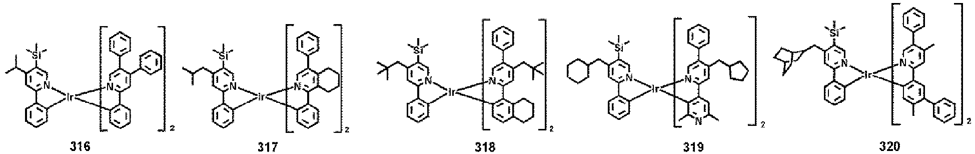

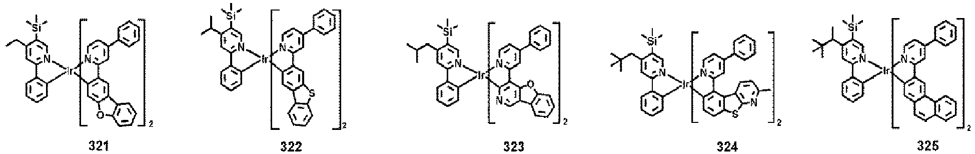

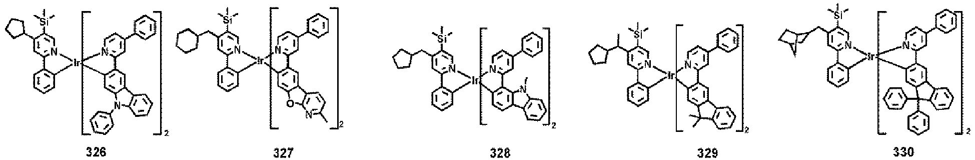

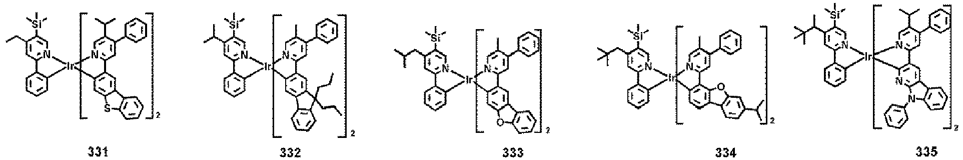

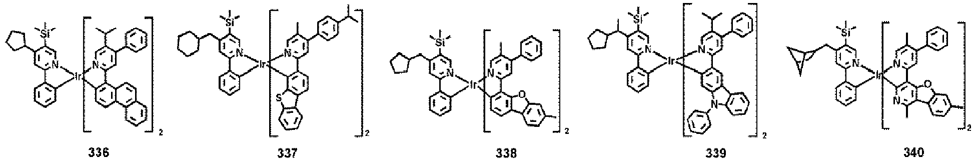

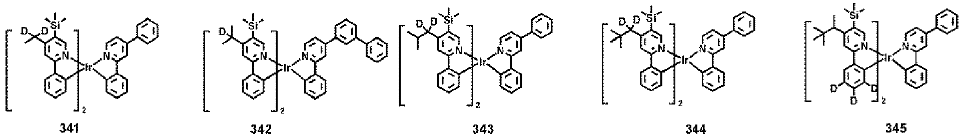

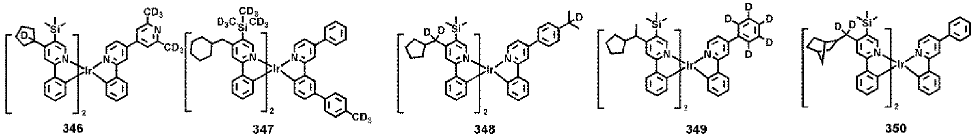

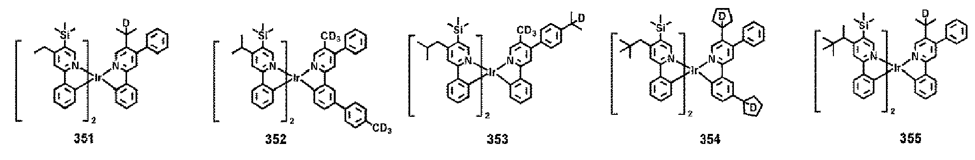

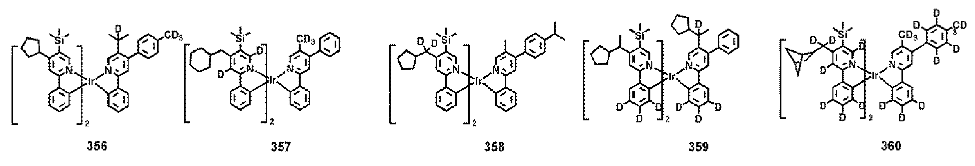

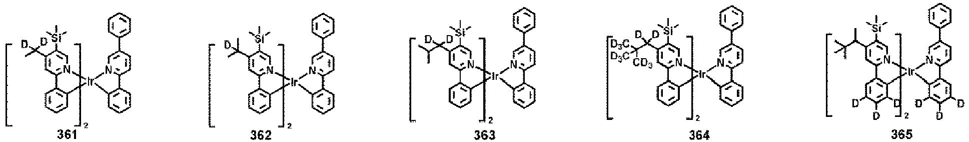

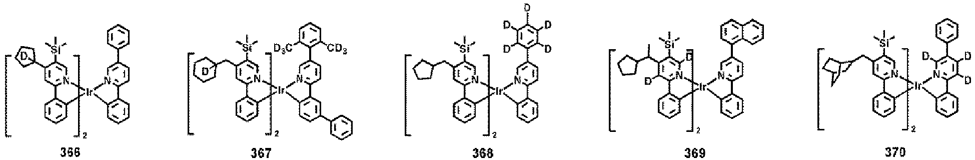

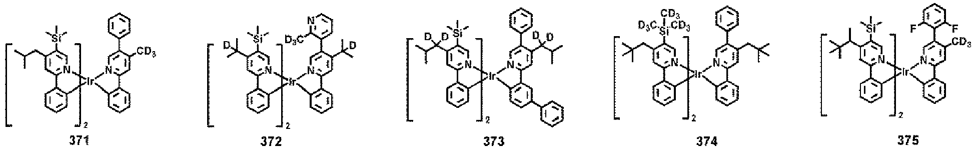

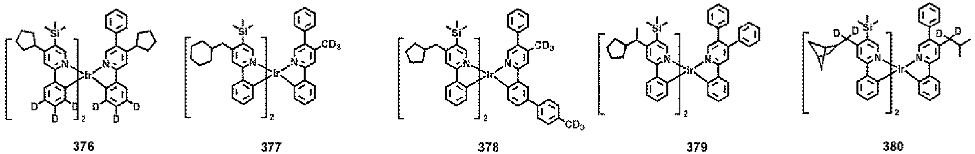

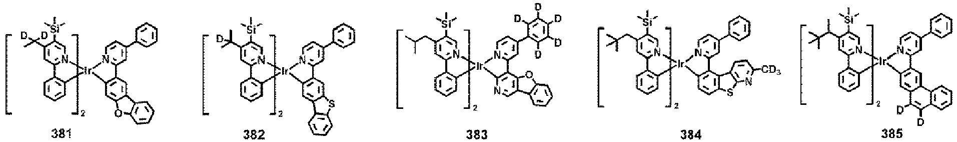

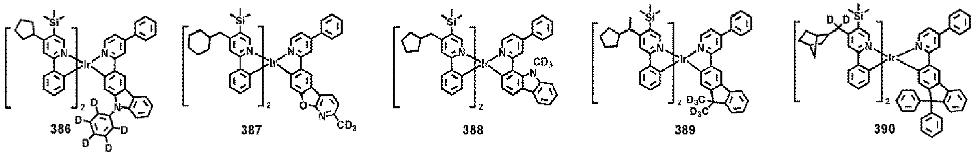

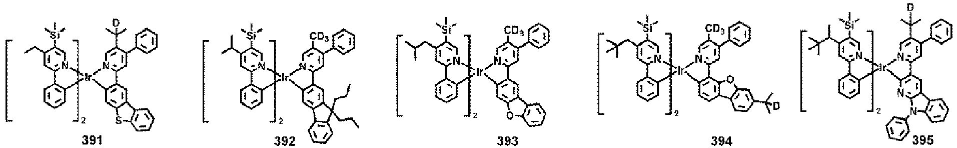

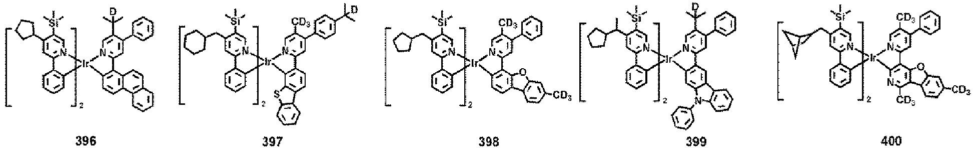

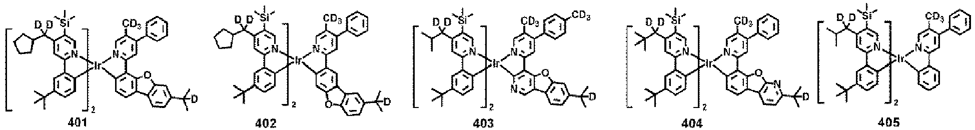

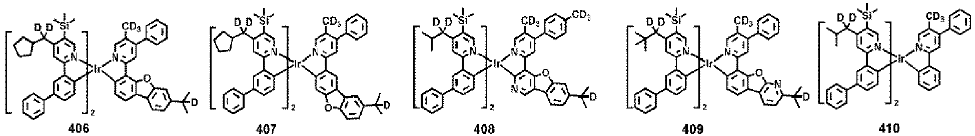

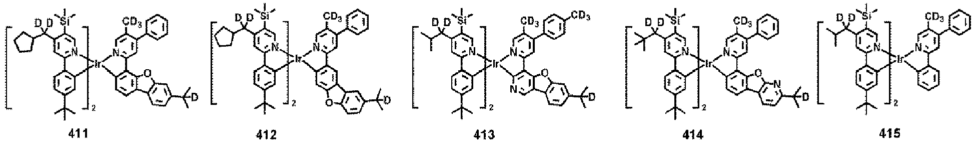

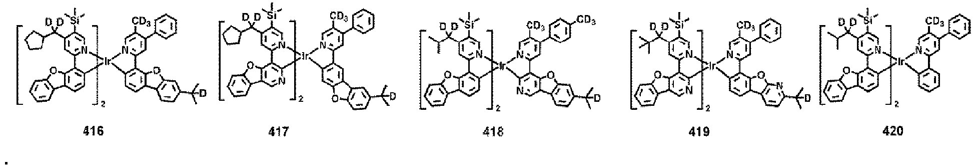

13. An organic light-emitting device comprising:
a first electrode,
a second electrode, and
an organic layer disposed between the first electrode and the second electrode and comprising an emission layer,
wherein the organic layer comprises at least one organometallic compound of any of claims 1-12;
preferably wherein
the first electrode is an anode,
the second electrode is a cathode,
the organic layer further comprises a hole transport region disposed between the first electrode and the emission layer and an electron transport region disposed between the emission layer and the second electrode,
the hole transport region comprises a hole injection layer, a hole transport layer, an electron blocking layer, a buffer layer, or a combination thereof, and
the electron transport region comprises a hole blocking layer, an electron transport layer, an electron injection layer, or a combination thereof.

14. The organic light-emitting device of claim 13, wherein
the emission layer comprises the at least one organometallic compound; preferably wherein
the emission layer further comprises a host, and an amount of the host is greater than an amount of the at least one organometallic compound.

15. A diagnostic composition comprising the organometallic compound of any of claims 1-12.

## Patentansprüche

1. Organometallische Verbindung dargestellt durch Formel 1:
Formel 1 M(L₁)ₙ₁(L₂)ₙ₂,
wobei in Formel 1
M ein Übergangsmetall ist,
L₁ ein Ligand dargestellt durch Formel 2A ist,
n1 1 oder 2 ist, wobei, wenn n1 2 ist, zwei Gruppen L₁ identisch zueinander oder unterschiedlich voneinander sind,
L₂ ein Ligand dargestellt durch Formel 2B ist,
n2 1 oder 2 ist, wobei, wenn n2 2 ist, zwei Gruppen L₂ identisch zueinander oder unterschiedlich voneinander sind,
die Summe von n1 und n2 2 oder 3 ist, und
L₁ und L₂ unterschiedlich voneinander sind: 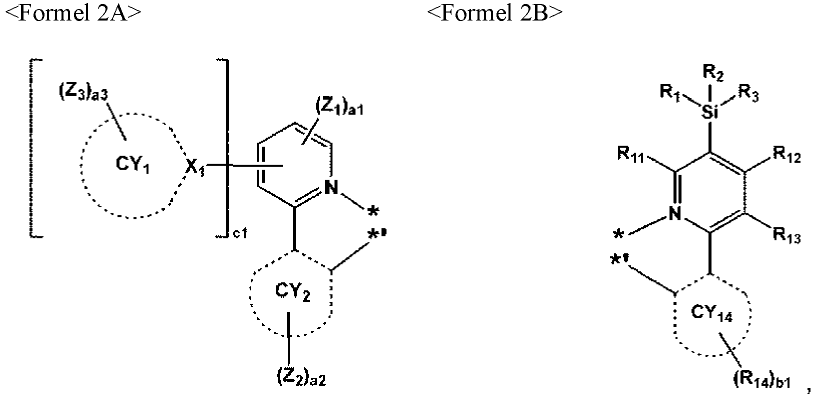 wobei in Formel 2A und 2B
X₁ C, N, Si oder P ist,
Ring CY₁, Ring CY₂ und Ring CY₁₄ jeweils unabhängig eine carbocyclische C₅-C₃₀-Gruppe oder eine heterocyclische C₁-C₃₀-Gruppe sind,
R₁ bis R₃ jeweils unabhängig eine C₁-C₆₀-Alkylgruppe oder eine C₆-C₆₀-Arylgruppe sind, jeweils unabhängig unsubstituiert oder substituiert mit zumindest einem ausgewählt aus Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon und einer C₁-C₁₀-Alkylgruppe,
Z₁ bis Z₃ und R₁₁ bis R₁₄ jeweils unabhängig ausgewählt sind aus Wasserstoff, Deuterium, -F, -Cl, -Br, -I, -SF₅, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer substituierten oder unsubstituierten C₁-C₆₀-Alkylgruppe, einer substituierten oder unsubstituierten C₂-C₆₀-Alkenylgruppe, einer substituierten oder unsubstituierten C₂-C₆₀-Alkynylgruppe, einer substituierten oder unsubstituierten C₁-C₆₀-Alkoxygruppe, einer substituierten oder unsubstituierten C₃-C₁₀-Cycloalkylgruppe, einer substituierten oder unsubstituierten C₁-C₁₀-Heterocycloalkylgruppe, einer substituierten oder unsubstituierten C₃-C₁₀-Cycloalkenylgruppe, einer substituierten oder unsubstituierten C₁-C₁₀-Heterocycloalkenylgruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₇-C₆₀-Arylalkylgruppe, einer substituierten oder unsubstituierten C₁-C₆₀-Heteroarylgruppe, einer substituierten oder unsubstituierten C₁-C₆₀-Heteroaryloxygruppe, einer substituierten oder unsubstituierten C₁-C₆₀-Heteroarylthiogruppe, einer substituierten oder unsubstituierten C₂-C₆₀-Heteroarylalkylgruppe, einer substituierten oder unsubstituierten monovalenten nichtaromatischen kondensierten polycyclischen Gruppe, einer substituierten oder unsubstituierten monovalenten nichtaromatischen kondensierten heteropolycyclischen Gruppe,-N(Q₁)(Q₂),
-Si(Q₃)(Q₄)(Q₅), -Ge(Q₃)(Q₄)(Q₅), -B(Q₆)(Q₇) und-P(=O)(Q₈)(Q₉), wobei R₁₂ weder Wasserstoff noch eine Methylgruppe ist,
a1 eine ganze Zahl von 0 bis 3 ist, wobei, wenn a1 zwei oder mehr ist, zwei oder mehr Gruppen Z₁ identisch zueinander oder unterschiedlich voneinander sind,
a2, a3 und b1 jeweils unabhängig eine ganze Zahl von 0 bis 20 sind, wobei, wenn a2 zwei oder mehr ist, zwei oder mehr Gruppen Z₂ identisch zueinander oder unterschiedlich voneinander sind, wenn a3 zwei oder mehr ist, zwei oder mehr Gruppen Z₃ identisch zueinander oder unterschiedlich voneinander sind, und wenn b1 zwei oder mehr ist, zwei oder mehr Gruppen R₁₄ identisch zueinander oder unterschiedlich voneinander sind,
c1 eine ganze Zahl von 1 bis 4 ist, wobei, wenn c1 2 oder mehr ist, zwei oder mehr Gruppen dargestellt durch 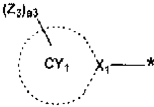 identisch zueinander oder unterschiedlich voneinander sind,
wenn a1 zwei oder mehr ist, zwei oder mehr Gruppen Z₁ in der Nummer von c1 optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
wenn a2 zwei oder mehr ist, zwei oder mehr Gruppen Z₂ in der Nummer von a2 optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
wenn a3 zwei oder mehr ist, zwei oder mehr Gruppen Z₃ in der Nummer von a3 optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
R₁₂ und R₁₃ optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
wenn b1 zwei oder mehr ist, zwei oder mehr Gruppen R₁₄(s) in der Nummer von b1 optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
zwei oder mehr Substituenten ausgewählt aus Z₁ und Z₂ optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
zwei oder mehr Substituenten ausgewählt aus R₁₁ bis R₁₄ optional verbunden sind, um eine carbocyclische C₅-C₃₀-Gruppe, die unsubstituiert oder substituiert mit zumindest einem von R₁₀ₐ ist, oder eine heterocyclische C₁-C₃₀-Gruppe zu bilden, die unsubstituiert oder substituiert mit zumindest einem R₁₀ₐ ist,
R₁₀ₐ ausgewählt ist aus Wasserstoff, Deuterium, -F, -Cl, -Br, -I, -SF₅, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer substituierten oder unsubstituierten C₁-C₆₀-Alkylgruppe, einer substituierten oder unsubstituierten C₂-C₆₀-Alkenylgruppe, einer substituierten oder unsubstituierten C₂-C₆₀-Alkynylgruppe, einer substituierten oder unsubstituierten C₁-C₆₀-Alkoxygruppe, einer substituierten oder unsubstituierten C₃-C₁₀-Cycloalkylgruppe, einer substituierten oder unsubstituierten C₁-C₁₀-Heterocycloalkylgruppe,einer substituierten oder unsubstituierten C₃-C₁₀-Cycloalkenylgruppe, einer substituierten oder unsubstituierten C₁-C₁₀-Heterocycloalkenylgruppe, einer substituierten oder unsubstituierten C₆-C₆₀-Arylgruppe, einer substituierten oder unsubstituierten C₆-C₆₀-Aryloxygruppe, einer substituierten oder unsubstituierten C₆-C₆₀-Arylthiogruppe, einer substituierten oder unsubstituierten C₇-C₆₀-Arylalkylgruppe, einer substituierten oder unsubstituierten C₁-C₆₀-Heteroarylgruppe, einer substituierten oder unsubstituierten C₁-C₆₀-Heteroaryloxygruppe, einer substituierten oder unsubstituierten C₁-C₆₀-Heteroarylthiogruppe, einer substituierten oder unsubstituierten C₂-C₆₀-Heteroarylalkylgruppe, einer substituierten oder unsubstituierten monovalenten nichtaromatischen kondensierten polycyclischen Gruppe, einer substituierten oder unsubstituierten monovalenten nichtaromatischen kondensierten heteropolycyclischen Gruppe, - N(Q₁)(Q₂), -Si(Q₃)(Q₄)(Q₅), -Ge(Q₃)(Q₄)(Q₅), -B(Q₆)(Q₇) und -P(=O)(Q₈)(Q₉),
* und in Formel 2A und 2B jeweils eine Bindungsstelle zu M in Formel 1 angeben, wobei zumindest ein Substituent der substituierten C₁-C₆₀-Alkylgruppe, der substituierten C₂-C₆₀-Alkenylgruppe, der substituierten C₂-C₆₀-Alkynylgruppe, der substituierten C₁-C₆₀-Alkoxygruppe, der substituierten C₃-C₁₀-Cycloalkylgruppe, der substituierten C₁-C₁₀-Heterocycloalkylgruppe, der substituierten C₃-C₁₀-Cycloalkenylgruppe, der substituierten C₁-C₁₀-Heterocycloalkenylgruppe, der substituierten C₆-C₆₀-Arylgruppe, der substituierten C₆-C₆₀-Aryloxygruppe, der substituierten C₆-C₆₀-Arylthiogruppe, der substituierten C₇-C₆₀-Arylalkylgruppe, der substituierten C₁-C₆₀-Heteroarylgruppe, der substituierten C₁-C₆₀-Heteroaryloxygruppe, der substituierten C₁-C₆₀-Heteroarylthiogruppe, der substituierten C₂-C₆₀-Heteroarylalkylgruppe, der substituierten monovalenten nichtaromatischen kondensierten polycyclischen Gruppe und der substituierten monovalenten nichtaromatischen kondensierten heteropolycyclischen Gruppe jeweils unabhängig ausgewählt sind aus:
Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkynylgruppe oder einer C₁-C₆₀-Alkoxygruppe;
einer C₁-C₆₀-Alkylgruppe, einer C₂-C₆₀-Alkenylgruppe, einer C₂-C₆₀-Alkynylgruppe oder einer C₁-C₆₀-Alkoxygruppe, jeweils substituiert mit zumindest einem ausgewählt aus Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer C₃-C₁₀-Cycloalkylgruppe, einer C₁-C₁₀-Heterocycloalkylgruppe,einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₇-C₆₀-Arylalkylgruppe, einer C₁-C₆₀-Heteroarylgruppe, einer C₁-C₆₀-Heteroaryloxygruppe, einer C₁-C₆₀-Heteroarylthiogruppe, einer C₂-C₆₀-Heteroarylalkylgruppe, einer monovalenten nichtaromatischen kondensierten polycyclischen Gruppe, einer monovalenten nichtaromatischen kondensierten heteropolycyclischen Gruppe, -N(Q₁₁)(Q₁₂), -Si(Q₁₃)(Q₁₄)(Q₁₅), -B(Q₁₆)(Q₁₇) und-P(=O)(Q₁₈)(Q₁₉);
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₇-C₆₀-Arylalkylgruppe, einer C₁-C₆₀-Heteroarylgruppe, einer C₁-C₆₀-Heteroaryloxygruppe, einer C₁-C₆₀-Heteroarylthiogruppe, einer C₂-C₆₀-Heteroarylalkylgruppe, einer monovalenten nichtaromatischen kondensierten polycyclischen Gruppe und einer monovalenten nichtaromatischen kondensierten heteropolycyclischen Gruppe;
einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₇-C₆₀-Arylalkylgruppe, einer C₁-C₆₀-Heteroarylgruppe, einer C₁-C₆₀-Heteroaryloxygruppe, einer C₁-C₆₀-Heteroarylthiogruppe, einer C₂-C₆₀-Heteroarylalkylgruppe, einer monovalenten nichtaromatischen kondensierten polycyclischen Gruppe oder einer monovalenten nichtaromatischen kondensierten heteropolycyclischen Gruppe, jeweils substituiert mit zumindest einem ausgewählt aus Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₇-C₆₀-Arylalkylgruppe, einer C₁-C₆₀-Heteroarylgruppe, einer C₁-C₆₀-Heteroaryloxygruppe, einer C₁-C₆₀-Heteroarylthiogruppe, einer C₂-C₆₀-Heteroarylalkylgruppe, einer monovalenten nichtaromatischen kondensierten polycyclischen Gruppe, einer monovalenten nichtaromatischen kondensierten heteropolycyclischen Gruppe, -N(Q₂₁)(Q₂₂), -Si(Q₂₃)(Q₂₄)(Q₂₅), -B(Q₂₆)(Q₂₇) und-P(=O)(Q₂₈)(Q₂₉); und
-N(Q₃₁)(Q₃₂), -Si(Q₃₃)(Q₃₄)(Q₃₅), -B(Q₃₆)(Q₃₇) oder -P(=O)(Q₃₈)(Q₃₉); und
Q₁ bis Q₉, Q₁₁ bis Q₁₉, Q₂₁ bis Q₂₉ und Q₃₁ bis Q₃₉ jeweils unabhängig ausgewählt sind aus Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Arylgruppe substituiert mit zumindest einem ausgewählt aus einer C₁-C₆₀-Alkylgruppe und einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten nichtaromatischen kondensierten polycyclischen Gruppe und einer monovalenten nichtaromatischen kondensierten heteropolycyclischen Gruppe.

2. Organometallische Verbindung nach Anspruch 1, wobei
in Formel 1, M Ir ist, und die Summe von n1 und n2 3 ist; oder
in Formel 1, M Pt ist, und die Summe von n1 und n2 2 ist.

3. Organometallische Verbindung nach Anspruch 1 oder 2, wobei
in Formel 2A und 2B, Ring CY₁, Ring CY₂ und Ring CY₁₄ jeweils unabhängig ausgewählt sind aus einer Cyclopentangruppe, einer Cyclohexangruppe, einer Cycloheptangruppe, einer Cyclopentengruppe, einer Cyclohexengruppe, einer Cycloheptengruppe, einer Benzolgruppe, einer Naphthalengruppe, einer Anthracengruppe, einer Phenanthrengruppe, einer Triphenylengruppe, einer Pyrengruppe, einer Chrysengruppe, einer Cyclopentadiengruppe, einer 1,2,3,4-Tetrahydronaphthalengruppe, einer Thiophengruppe, einer Furangruppe, einer Indolgruppe, einer Benzoborolgruppe, einer Benzophospholgruppe, einer Indengruppe, einer Benzosilolgruppe, einer Benzogermolgruppe, einer Benzothiophengruppe, einer Benzoselenophengruppe, einer Benzofurangruppe, einer Carbazolgruppe, einer Dibenzoborolgruppe, einer Dibenzophospholgruppe, einer Fluorengruppe, einer Dibenzosilolgruppe, einer Dibenzogermolgruppe, einer Dibenzothiophengruppe, einer Dibenzoselenophengruppe, einer Dibenzofurangruppe, einer Dibenzothiophen-5-oxidgruppe, einer 9H-Fluoren-9-on-Gruppe, einer Dibenzothiophen-5,5-dioxidgruppe, einer Azaindolgruppe, einer Azabenzoborolgruppe, einer Azabenzophospholgruppe, einer Azaindengruppe, einer Azabenzosilolgruppe, einer Azabenzogermolgruppe, einer Azabenzothiophengruppe, einer Azabenzoselenophengruppe, einer Azabenzofurangruppe, einer Azacarbazolgruppe, einer Azadibenzoborolgruppe, einer Azadibenzophospholgruppe, einer Azafluorengruppe, einer Azadibenzosilolgruppe, einer Azadibenzogermolgruppe, einer Azadibenzothiophengruppe, einer Azadibenzoselenophengruppe, einer Azadibenzofurangruppe, einer Azadibenzothiophen-5-oxidgruppe, einer Aza-9H-fluoren-9-on-Gruppe, einer Azadibenzothiophen-5,5-dioxidgruppe, einer Pyridingruppe, einer Pyrimidingruppe, einer Pyrazingruppe, einer Pyridazingruppe, einer Triazingruppe, einer Quinolingruppe, einer Isoquinolingruppe, einer Quinoxalingruppe, einer Quinazolingruppe, einer Phenanthrolingruppe, einer Pyrrolgruppe, einer Pyrazolgruppe, einer Imidazolgruppe, einer Triazolgruppe, einer Oxazolgruppe, einer Isoxazolgruppe, einer Thiazolgruppe, einer Isothiazolgruppe, einer Oxadiazolgruppe, einer Thiadiazolgruppe, einer Benzopyrazolgruppe, einer Benzimidazolgruppe, einer Benzoxazolgruppe, einer Benzothiazolgruppe, einer Benzoxadiazolgruppe, einer Benzothiadiazolgruppe, einer 5,6,7,8-Tetrahydroisoquinolingruppe und einer 5,6,7,8-Tetrahydroquinolingruppe; und/oder
wobei
in Formel 2B, R₁ bis R₃ jeweils unabhängig ausgewählt sind aus einer Methylgruppe, einer Ethylgruppe, einer n-Propylgruppe, einer Isopropylgruppe, einer n-Butylgruppe, einer sec-Butylgruppe, einer Isobutylgruppe, einer tert-Butylgruppe, einer n-Pentylgruppe, einer tert-Pentylgruppe, einer Neopentylgruppe, einer Isopentylgruppe, einer sec-Pentylgruppe, einer 3-Pentylgruppe, einer sec-Isopentylgruppe, einer n-Hexylgruppe, einer Isohexylgruppe, einer sec-Hexylgruppe, einer tert-Hexylgruppe, einer n-Heptylgruppe, einer Isoheptylgruppe, einer sec-Heptylgruppe, einer tert-Heptylgruppe, einer n-Octylgruppe, einer Isooctylgruppe, einer sec-Octylgruppe, einer tert-Octylgruppe, einer n-Nonylgruppe, einer Isononylgruppe, einer sec-Nonylgruppe, einer tert-Nonylgruppe, einer n-Decylgruppe, einer Isodecylgruppe, einer sec-Decylgruppe, einer tert-Decylgruppe, einer Phenylgruppe, einer Biphenylgruppe oder einer Naphthylgruppe, jeweils unsubstituiert oder substituiert mit zumindest einem ausgewählt aus Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon und einer C₁-C₁₀-Alkylgruppe.

4. Organometallische Verbindung nach einem der Ansprüche 1-3, wobei
in Formel 2A und 2B, Z₁ bis Z₃ und R₁₁ bis R₁₄ jeweils unabhängig ausgewählt sind aus:
Wasserstoff, Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, -SF₅, einer C₁-C₂₀-Alkylgruppe oder einer C₁-C₂₀-Alkoxygruppe;
einer C₁-C₂₀-Alkylgruppe und einer C₁-C₂₀-Alkoxygruppe, jeweils substituiert mit zumindest einem ausgewählt aus Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer C₁-C₁₀-Alkylgruppe, einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Bicyclo[1.1.1]pentylgruppe, einer Bicyclo[2.1.1]hexylgruppe, einer Bicyclo[2.2.1]heptylgruppe, einer Bicyclo[2.2.2]octylgruppe, einer Phenylgruppe, einer (C₁-C₂₀-Alkyl)phenylgruppe, einer Biphenylgruppe, einer Terphenylgruppe, einer Naphthylgruppe, einer Pyridinylgruppe oder einer Pyrimidinylgruppe;
einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Bicyclo(l.l.l)pentylgruppe, einer Bicyclo(2.1.1)hexylgruppe, einer Bicyclo(2.2.1)heptylgruppe, einer Bicyclo(2.2.2)octylgruppe, einer Phenylgruppe, einer (C₁-C₂₀-Alkyl)phenylgruppe, einer Biphenylgruppe, einer Terphenylgruppe, einer Naphthylgruppe, einer Fluorenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Quinolinylgruppe, einer Isoquinolinylgruppe, einer Benzoquinolinylgruppe, einer Quinoxalinylgruppe, einer Quinazolinylgruppe, einer Cinnolinylgruppe, einer Carbazolylgruppe, einer Phenanthrolinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyridinylgruppe, einer Imidazopyrimidinylgruppe, einer Azacarbazolylgruppe, einer Azadibenzofuranylgruppe oder einer Azadibenzothiophenylgruppe, jeweils unsubstituiert oder substituiert mit zumindest einem ausgewählt aus Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Bicyclo(l.l.l)pentylgruppe, einer Bicyclo(2.1.1)hexylgruppe, einer Bicyclo(2.2.1)heptylgruppe, einer Bicyclo(2.2.2)octylgruppe, einer Phenylgruppe, einer (C₁-C₂₀-Alkyl)phenylgruppe, einer Biphenylgruppe, einer Terphenylgruppe, einer Naphthylgruppe, einer Fluorenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Quinolinylgruppe, einer Isoquinolinylgruppe, einer Benzoquinolinylgruppe, einer Quinoxalinylgruppe, einer Quinazolinylgruppe, einer Cinnolinylgruppe, einer Carbazolylgruppe, einer Phenanthrolinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyridinylgruppe, einer Imidazopyrimidinylgruppe, einer Azacarbazolylgruppe, einer Azadibenzofuranylgruppe, einer Azadibenzothiophenylgruppe und - Si(Q₃₃)(Q₃₄)(Q₃₅); und
-N(Q₁)(Q₂), -Si(Q₃)(Q₄)(Q₅), -Ge(Q₃)(Q₄)(Q₅), -B(Q₆)(Q₇), -P(=O)(Q₈)(Q₉) oder-P(Q₈)(Q₉), wobei R₁₂ weder Wasserstoff noch eine Methylgruppe ist, und
Q₁ bis Q₉ und Q₃₃ bis Q₃₅ jeweils unabhängig ausgewählt sind aus:
-CH₃, -CD₃, -CD₂H, -CDH₂, -CH₂CH₃, -CH₂CD₃, -CH₂CD₂H, -CH₂CDH₂, -CHDCH₃,-CHDCD₂H, -CHDCDH₂, -CHDCD₃, -CD₂CD₃, -CD₂CD₂H oder -CD₂CDH₂; und
einer n-Propylgruppe, einer Isopropylgruppe, einer n-Butylgruppe, einer sec-Butylgruppe, einer Isobutylgruppe, einer tert-Butylgruppe, einer n-Pentylgruppe, einer tert-Pentylgruppe, einer Neopentylgruppe, einer Isopentylgruppe, einer sec-Pentylgruppe, einer 3-Pentylgruppe, einer sec-Isopentylgruppe, einer Phenylgruppe, einer Biphenylgruppe oder einer Naphthylgruppe, jeweils unsubstituiert oder substituiert mit zumindest einem ausgewählt aus Deuterium, einer C₁-C₁₀-Alkylgruppe oder einer Phenylgruppe; und/oder wobei
R₁₂ in Formel 2B ausgewählt ist aus:
einer C₂-C₂₀-Alkylgruppe oder einer C₂-C₂₀-Alkoxygruppe;
einer Methylgruppe oder einer Methoxygruppe, jeweils substituiert mit zumindest einem ausgewählt aus einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Bicyclo(l.l.l)pentylgruppe, einer Bicyclo(2.1.1)hexylgruppe, einer Bicyclo(2.2.1)heptylgruppe, einer Bicyclo(2.2.2)octylgruppe, einer Phenylgruppe, einer Naphthylgruppe, einer Pyridinylgruppe und einer Pyrimidinylgruppe;
einer Methylgruppe oder einer Methoxygruppe, jeweils substituiert mit i) zumindest einem ausgewählt aus einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Bicyclo(l.l.l)pentylgruppe, einer Bicyclo(2.1.1)hexylgruppe, einer Bicyclo(2.2.1)heptylgruppe, einer Bicyclo(2.2.2)octylgruppe, einer Phenylgruppe, einer Naphthylgruppe, einer Pyridinylgruppe und einer Pyrimidinylgruppe, und ii) zumindest einem Deuterium;
einer C₂-C₂₀-Alkylgruppe oder einer C₂-C₂₀-Alkoxygruppe, jeweils substituiert mit zumindest einem ausgewählt aus Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer C₁-C₁₀-Alkylgruppe, einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Bicyclo(1.1.1)pentylgruppe, einer Bicyclo(2.1.1)hexylgruppe, einer Bicyclo(2.2.1)heptylgruppe, einer Bicyclo(2.2.2)octylgruppe, einer Phenylgruppe, einer (C₁-C₂₀-Alkyl)phenylgruppe, einer Biphenylgruppe, einer Terphenylgruppe, einer Naphthylgruppe, einer Pyridinylgruppe und einer Pyrimidinylgruppe; und einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Bicyclo(l.l.l)pentylgruppe, einer Bicyclo(2.1.1)hexylgruppe, einer Bicyclo(2.2.1)heptylgruppe, einer Bicyclo(2.2.2)octylgruppe, einer Phenylgruppe, einer (C₁-C₂₀-Alkyl)phenylgruppe, einer Biphenylgruppe, einer Terphenylgruppe, einer Naphthylgruppe, einer Fluorenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Quinolinylgruppe, einer Isoquinolinylgruppe, einer Benzoquinolinylgruppe, einer Quinoxalinylgruppe, einer Quinazolinylgruppe, einer Cinnolinylgruppe, einer Carbazolylgruppe, einer Phenanthrolinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyridinylgruppe, einer Imidazopyrimidinylgruppe, einer Azacarbazolylgruppe, einer Azadibenzofuranylgruppe oder einer Azadibenzothiophenylgruppe, jeweils unsubstituiert oder substituiert mit zumindest einem ausgewählt aus Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carboxylsäuregruppe oder einem Salz davon, einer Sulfonsäuregruppe oder einem Salz davon, einer Phosphorsäuregruppe oder einem Salz davon, einer C₁-C₂₀-Alkylgruppe, einer C₁-C₂₀-Alkoxygruppe, einer Cyclopentylgruppe, einer Cyclohexylgruppe, einer Cycloheptylgruppe, einer Cycloctylgruppe, einer Adamantanylgruppe, einer Norbornanylgruppe, einer Norbornenylgruppe, einer Cyclopentenylgruppe, einer Cyclohexenylgruppe, einer Cycloheptenylgruppe, einer Bicyclo(l.l.l)pentylgruppe, einer Bicyclo(2.1.1)hexylgruppe, einer Bicyclo(2.2.1)heptylgruppe, einer Bicyclo(2.2.2)octylgruppe, einer Phenylgruppe, einer (C₁-C₂₀-Alkyl)phenylgruppe, einer Biphenylgruppe, einer Terphenylgruppe, einer Naphthylgruppe, einer Fluorenylgruppe, einer Phenanthrenylgruppe, einer Anthracenylgruppe, einer Fluoranthenylgruppe, einer Triphenylenylgruppe, einer Pyrenylgruppe, einer Chrysenylgruppe, einer Pyrrolylgruppe, einer Thiophenylgruppe, einer Furanylgruppe, einer Imidazolylgruppe, einer Pyrazolylgruppe, einer Thiazolylgruppe, einer Isothiazolylgruppe, einer Oxazolylgruppe, einer Isoxazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Quinolinylgruppe, einer Isoquinolinylgruppe, einer Benzoquinolinylgruppe, einer Quinoxalinylgruppe, einer Quinazolinylgruppe, einer Cinnolinylgruppe, einer Carbazolylgruppe, einer Phenanthrolinylgruppe, einer Benzimidazolylgruppe, einer Benzofuranylgruppe, einer Benzothiophenylgruppe, einer Isobenzothiazolylgruppe, einer Benzoxazolylgruppe, einer Isobenzoxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Benzocarbazolylgruppe, einer Dibenzocarbazolylgruppe, einer Imidazopyridinylgruppe, einer Imidazopyrimidinylgruppe, einer Azacarbazolylgruppe, einer Azadibenzofuranylgruppe und einer Azadibenzothiophenyl gruppe.

5. Organometallische Verbindung nach einem der Ansprüche 1-4, wobei
in Formel 2A und 2B, Z₁ bis Z₃, R₁₁, R₁₃ und R₁₄ jeweils unabhängig ausgewählt sind aus Wasserstoff, Deuterium, -F, einer Cyanogruppe, einer Nitrogruppe, -SF₅, -CH3, -CD₃, -CD₂H,-CDH₂, -CF₃, -CF₂H, -CFH₂, Gruppen dargestellt durch Formel 9-1 bis 9- 66, Gruppen dargestellt durch Formel 9-1 bis 9-66, wobei zumindest ein Wasserstoff substituiert ist mit Deuterium, Gruppen dargestellt durch Formel 10-1 bis 10-249 und Gruppen dargestellt durch Formel 10-1 bis 10-249, wobei zumindest ein Wasserstoff substituiert ist mit Deuterium, -N(Q₁)(Q₂), - Si(Q₃)(Q₄)(Q₅) und -Ge(Q₃)(Q₄)(Q₅), wobei Q₁ bis Q₅ jeweils unabhängig das gleiche wie in Anspruch 1 sind, und
R₁₂ in Formel 2B ausgewählt ist aus Gruppen dargestellt durch Formel 9-1 bis 9- 66, Gruppen dargestellt durch Formel 9-1 bis 9-66, wobei zumindest ein Wasserstoff substituiert ist mit Deuterium, Gruppen dargestellt durch Formel 10-1 bis 10-249, Gruppen dargestellt durch Formel 10-1 bis 10-249, wobei zumindest ein Wasserstoff substituiert ist mit Deuterium: 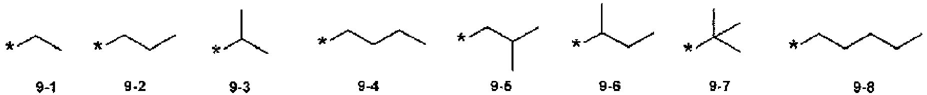 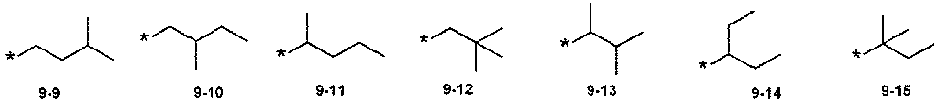 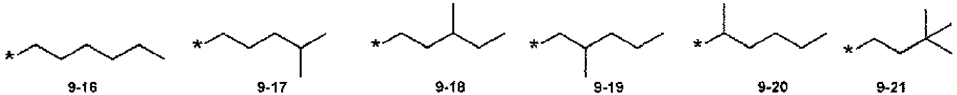 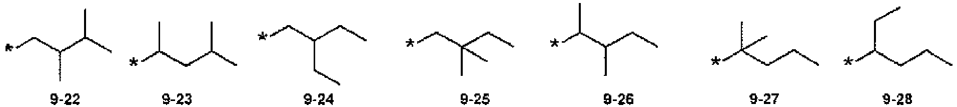 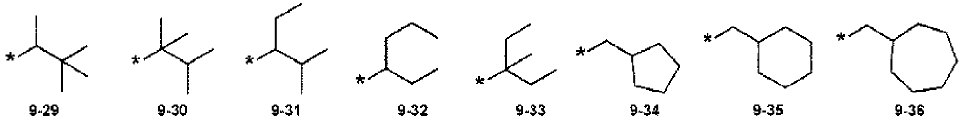 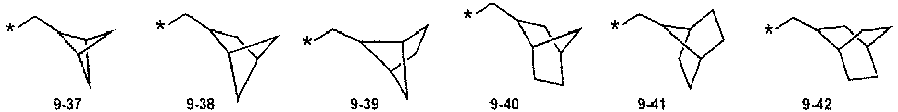 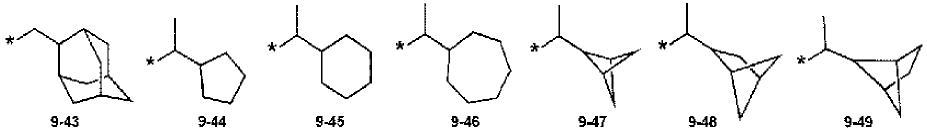 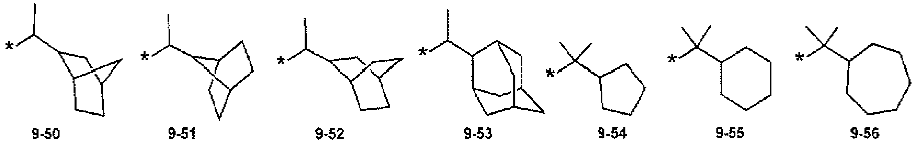 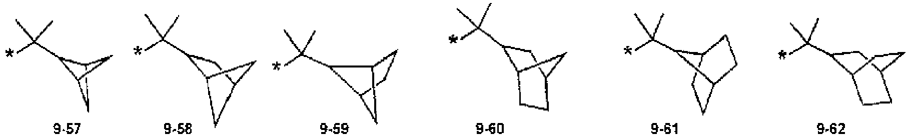 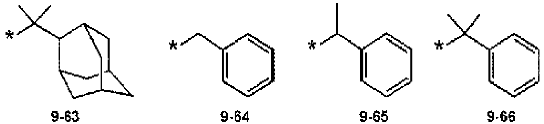 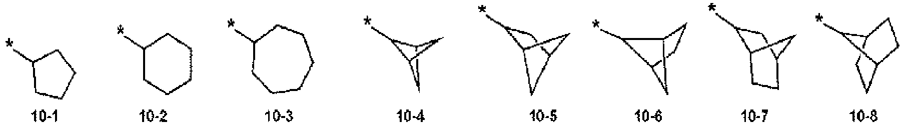 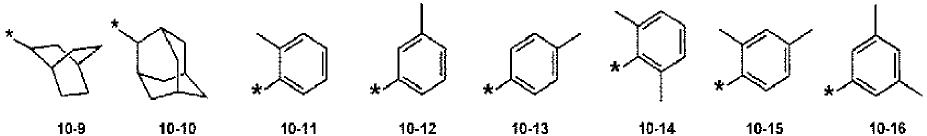 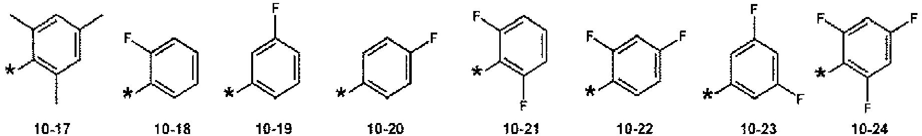 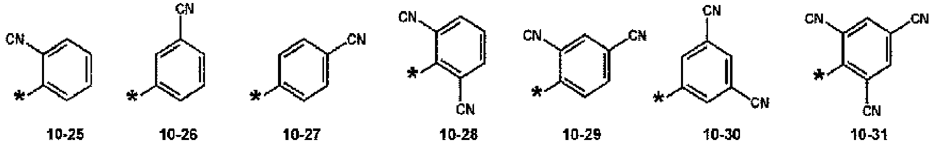 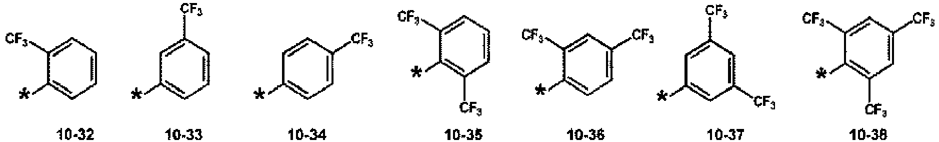 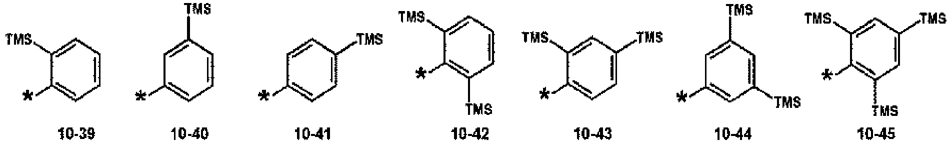 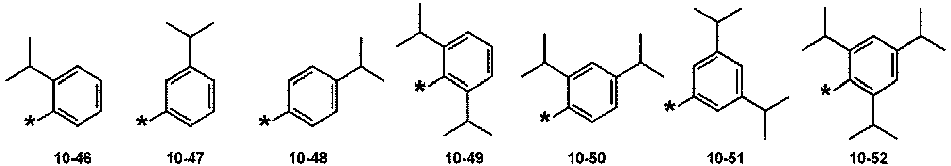 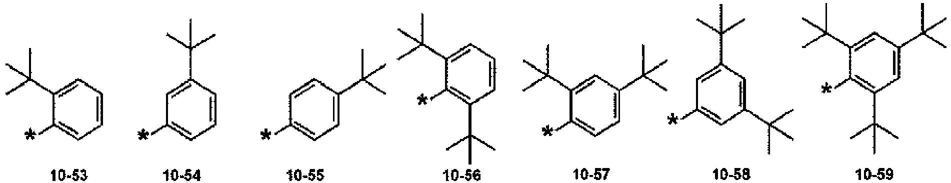 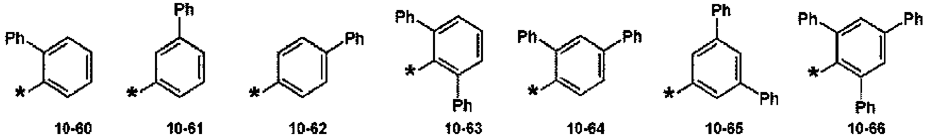 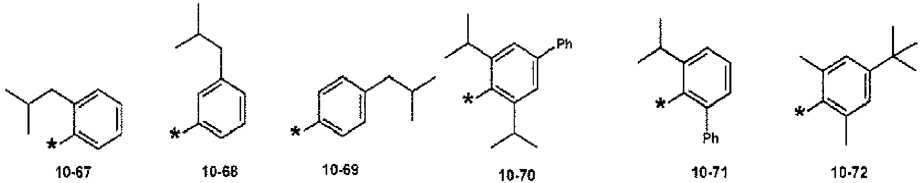 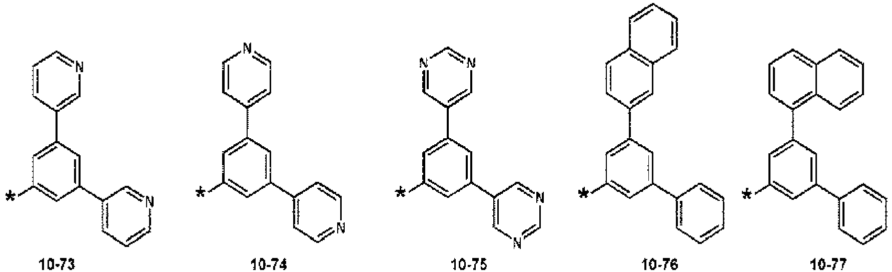 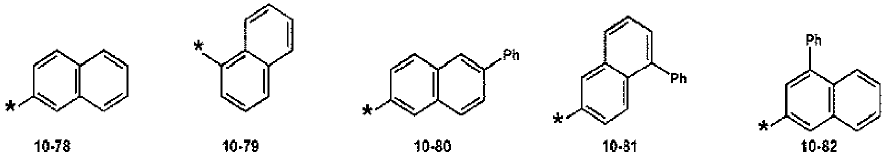 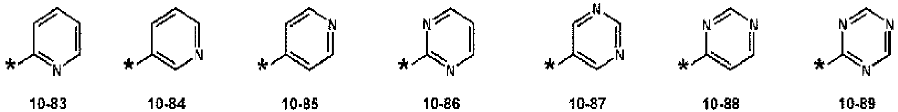 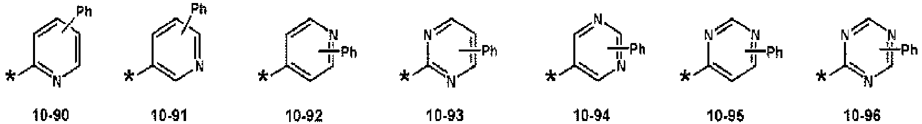 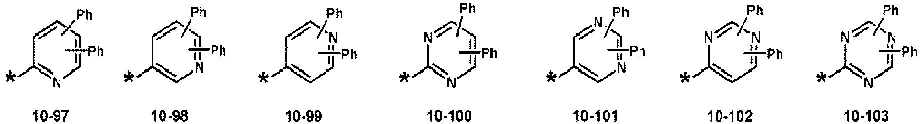 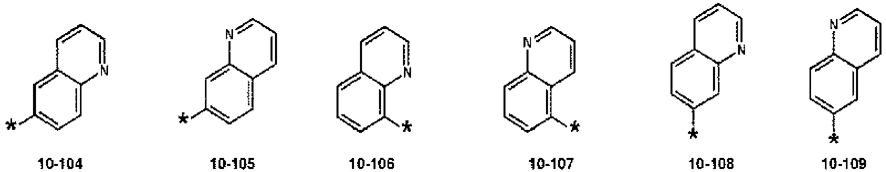 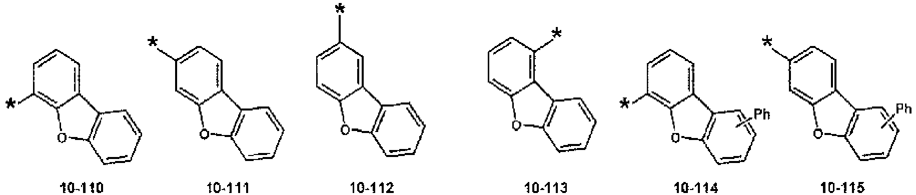 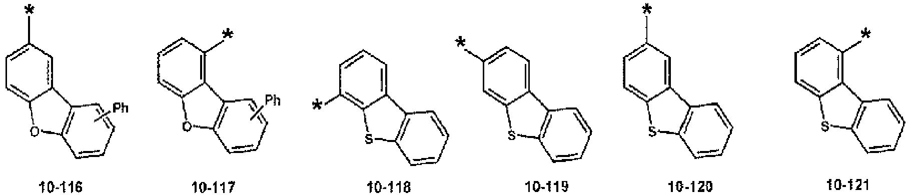 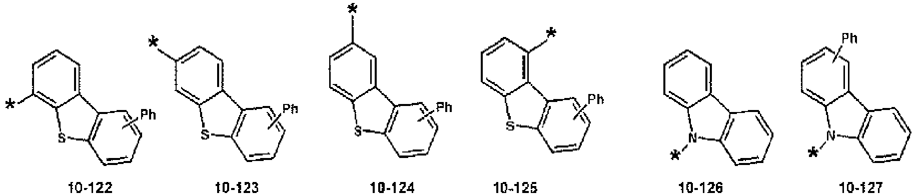 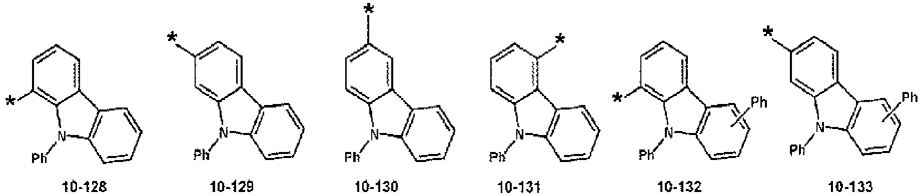 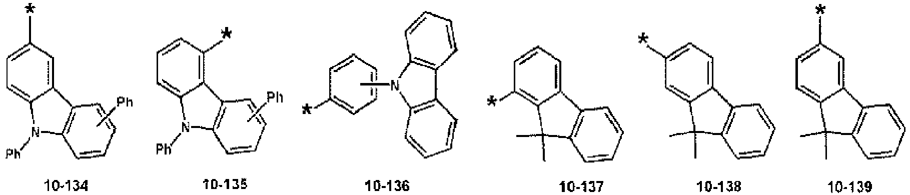 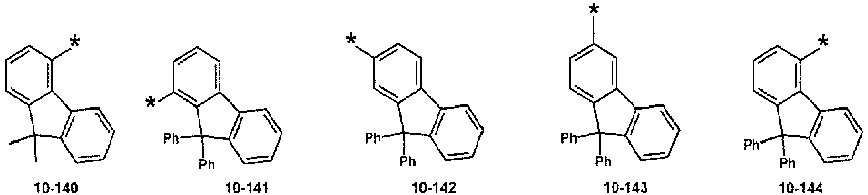 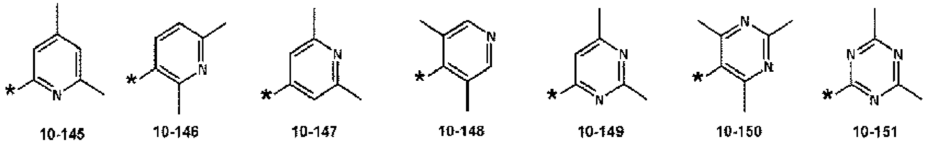 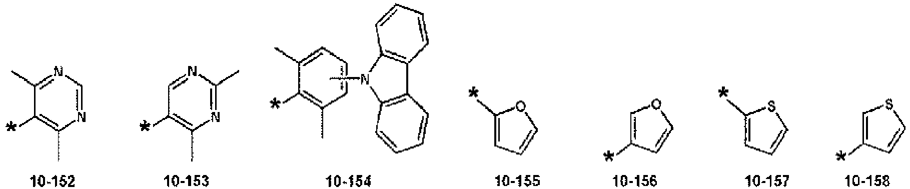 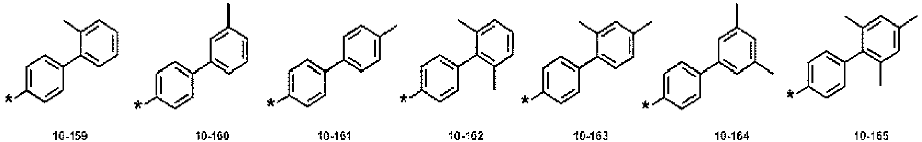 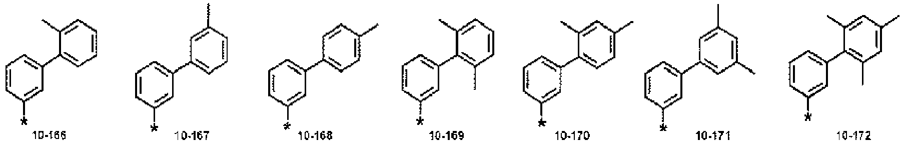 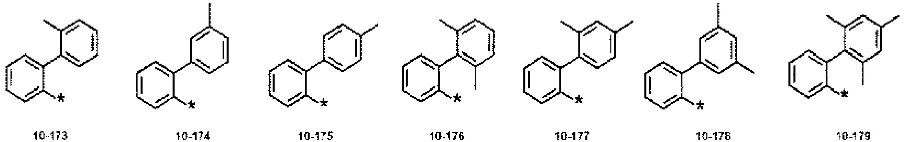 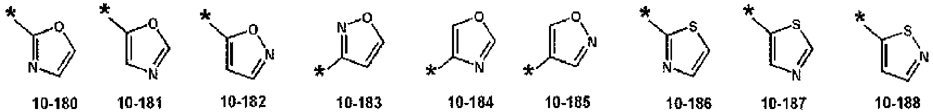 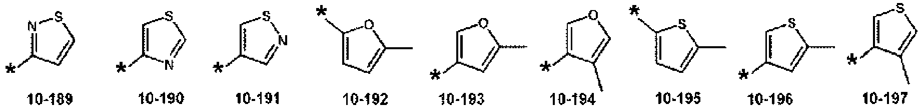 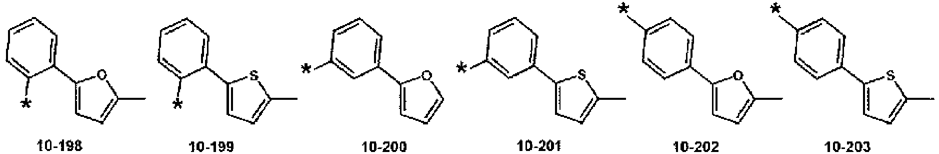 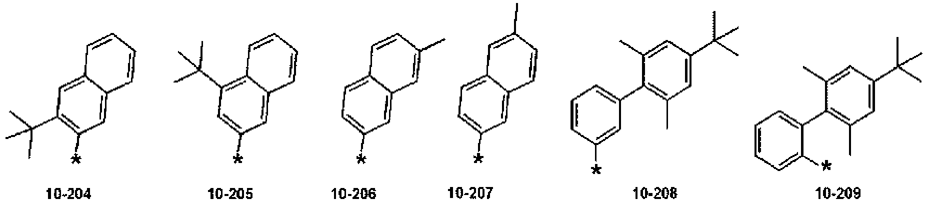 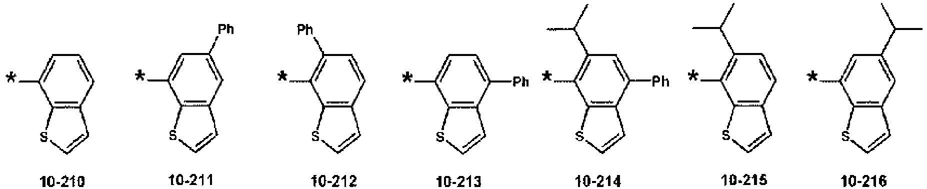 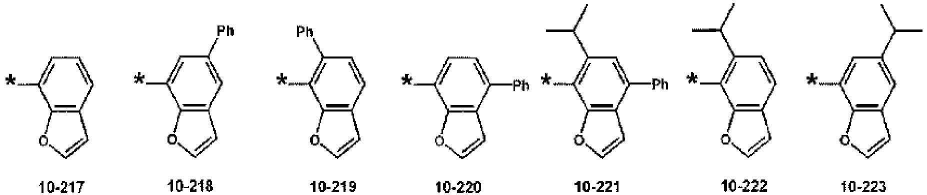 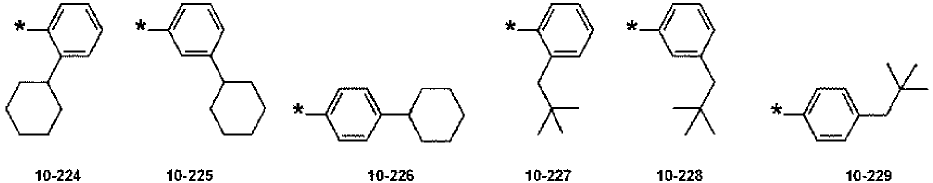 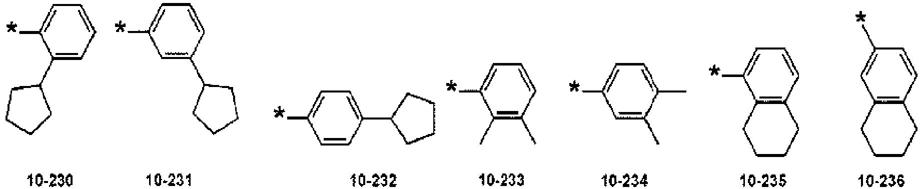 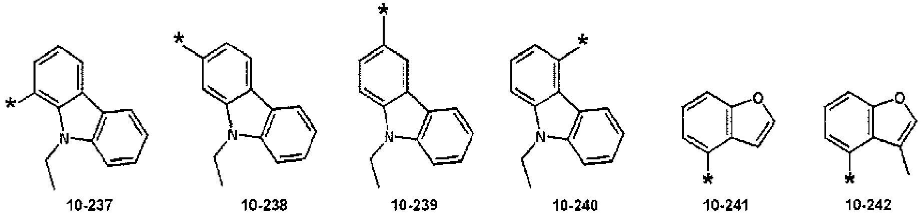 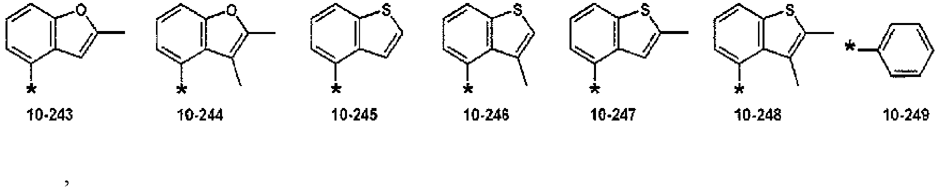 wobei in Formel 9-1 bis 9-66 und 10-1 bis 10-249 * eine Bindungsstelle zu einem benachbarten Atom angibt, Ph eine Phenylgruppe angibt und TMS eine Trimethylsilylgruppe angibt.

6. Organometallische Verbindung nach einem der Ansprüche 1-5, wobei
in Formel 2A, Z₁ ausgewählt ist aus Wasserstoff, Deuterium, -F, einer Cyanogruppe, einer substituierten oder unsubstituierten C₁-C₆₀-Alkylgruppe, einer substituierten oder unsubstituierten C₁-C₆₀-Alkoxygruppe, einer substituierten oder unsubstituierten C₃-C₁₀-Cycloalkylgruppe, einer substituierten oder unsubstituierten C₁-C₁₀-Heterocycloalkylgruppe, -N(Q₁)(Q₂), -Si(Q₃)(Q₄)(Q₅) und -Ge(Q₃)(Q₄)(Q₅), wobei Q₁ bis Q₅ jeweils unabhängig die gleichen wie in Anspruch 1 sind.

7. Organometallische Verbindung nach einem der Ansprüche 1-6, wobei c1 in Formel 2A 1 oder 2 ist; und/oder
wobei
die Gruppe dargestellt durch 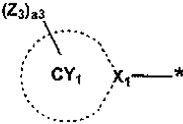 in Formel 2A eine Gruppe dargestellt durch eines ausgewählt aus Formel CY1 (1) bis CY1 (20) ist: 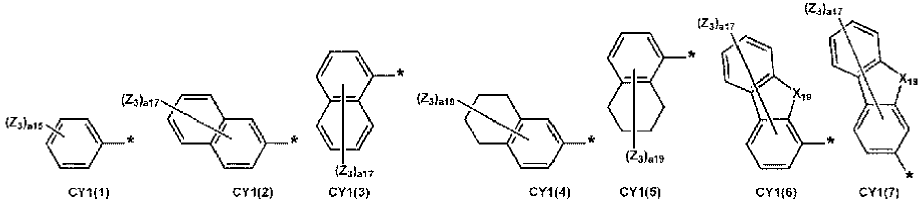 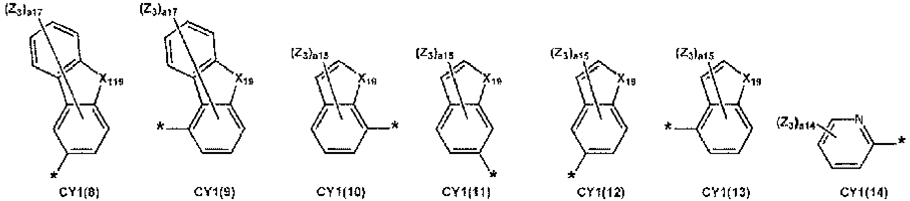 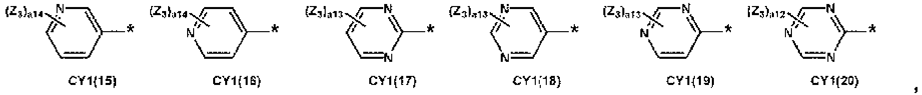 wobei in Formel CY1(1) bis CY1(20),
Z₃ das gleiche wie beschrieben in Anspruch 1 ist,
X₁₉ O, S, N(Z₄), C(Z₄)(Z₅) oder Si(Z₄)(Z₅) ist,
Z₄ und Z₅ jeweils unabhängig das gleiche wie beschrieben in Verbindung mit Z₃ sind,
a12 eine ganze Zahl von 0 bis 2 ist,
a13 eine ganze Zahl von 0 bis 3 ist,
a14 eine ganze Zahl von 0 bis 4 ist,
a15 eine ganze Zahl von 0 bis 5 ist,
a17 eine ganze Zahl von 0 bis 7 ist,
a19 eine ganze Zahl von 0 bis 9 ist, und
* eine Bindungsstelle zu einem benachbarten Kohlenstoffatom angibt.

8. Organometallische Verbindung nach einem der Ansprüche 1-7, wobei
die Gruppe dargestellt durch 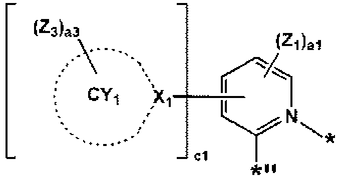 in Formel 2A eine Gruppe dargestellt durch eines ausgewählt aus Formel CY1-1 bis CY1-56 ist: 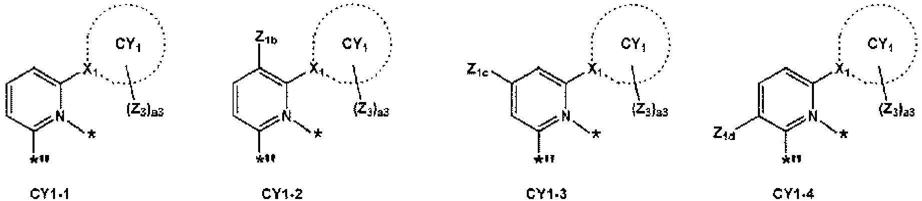 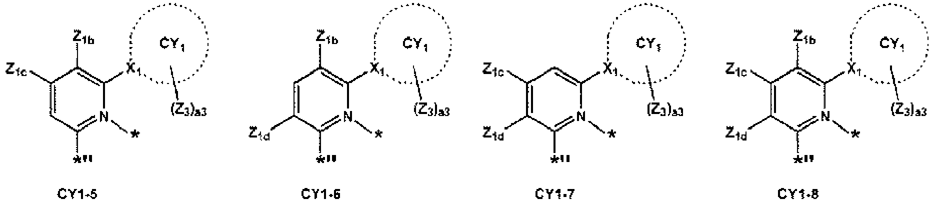 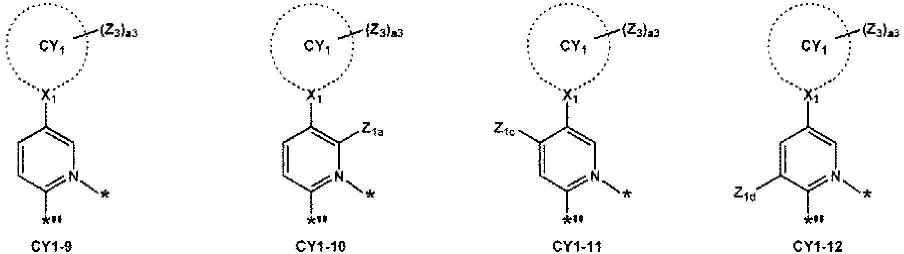 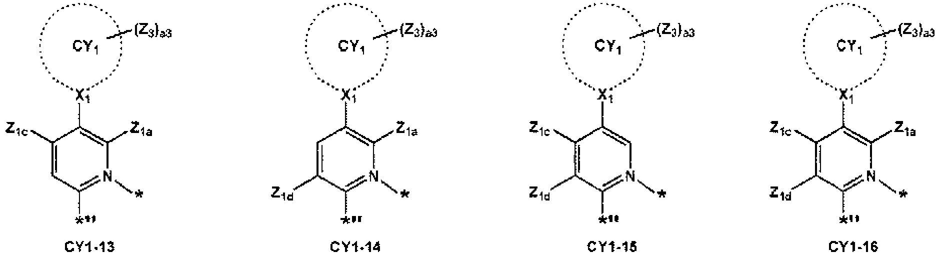 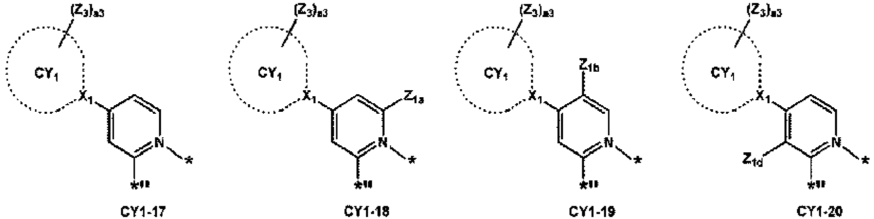 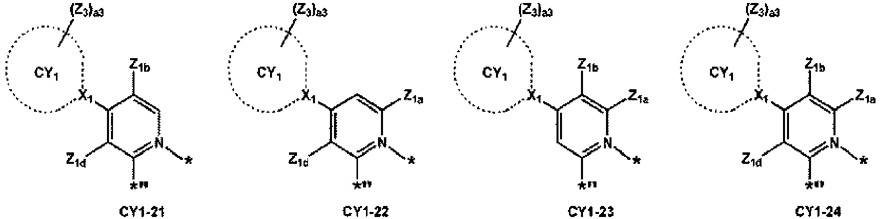 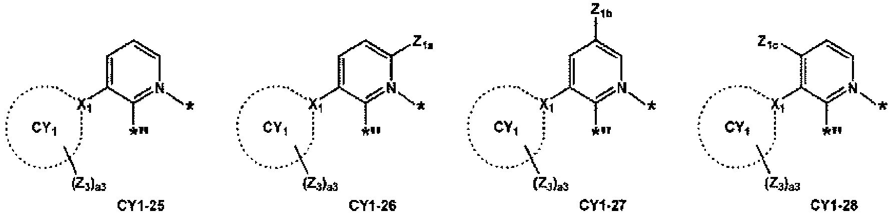 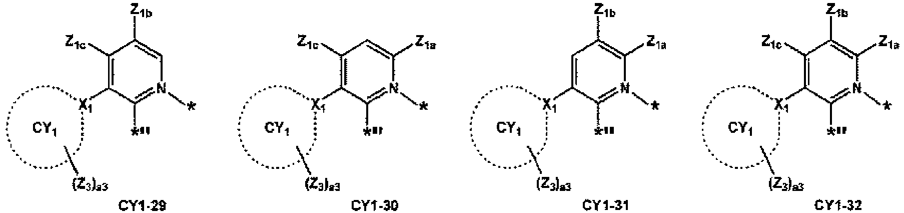 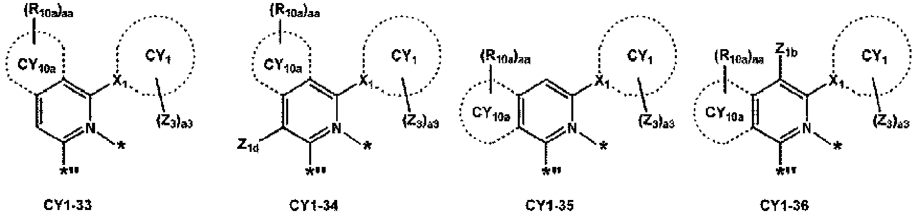 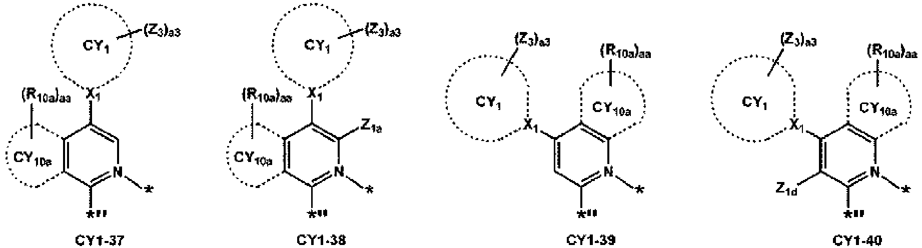 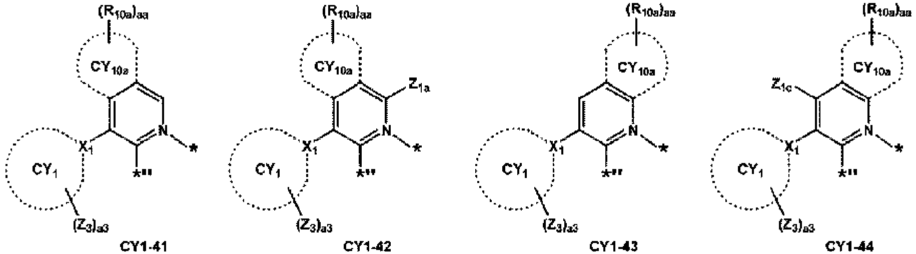 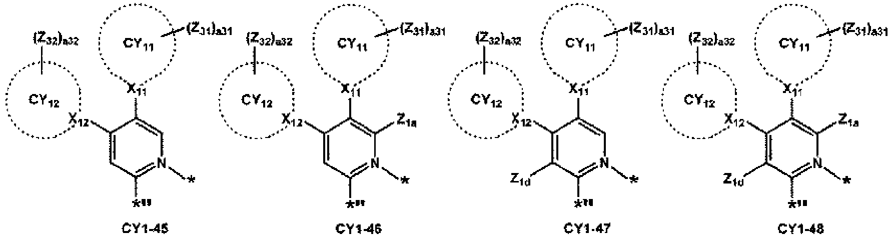 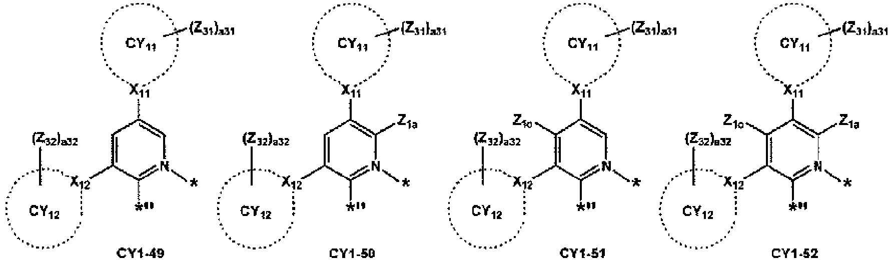 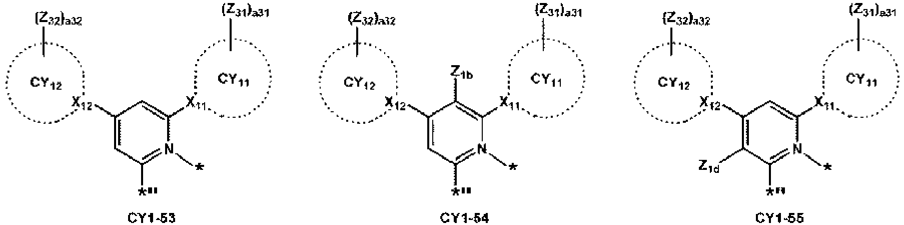 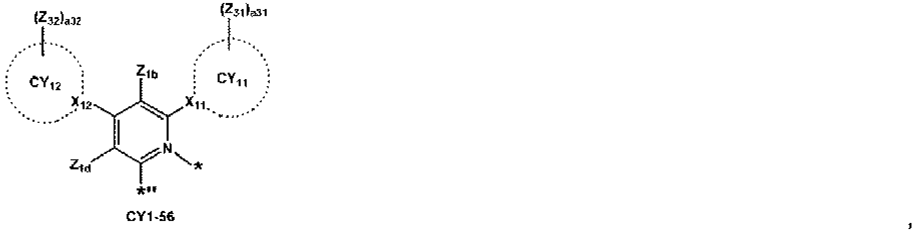 wobei in Formel CY1-1 bis CY1-56,
X₁, Ring CY₁, Z₃ und a3 jeweils unabhängig die gleichen wie beschrieben in Anspruch 1 sind,
Z₁ₐ bis Z_{1d} jeweils unabhängig die gleichen wie beschrieben in Verbindung mit Z₁ in Anspruch 1 sind, wobei Z₁ₐ bis Z_{1d} nicht Wasserstoff sind,
X₁₁ und X₁₂ jeweils unabhängig die gleichen wie beschrieben in Verbindung mit X₁ in Anspruch 1 sind,
Ring CY₁₁ und Ring CY₁₂ jeweils unabhängig die gleichen wie beschrieben in Verbindung mit Ring CY₁ in Anspruch 1 sind,
Z₃₁ und Z₃₂ jeweils unabhängig die gleichen wie beschrieben in Verbindung mit Z₃ in Anspruch 1 sind,
a31 und a32 jeweils unabhängig die gleichen wie beschrieben in Verbindung mit a3 in Anspruch 1 sind,
Ring CY₁₀ₐ eine carbocyclische C₅-C₃₀-Gruppe oder eine heterocyclische C₁-C₃₀-Gruppe ist,
R₁₀ₐ das gleiche wie beschrieben in Anspruch 1 ist,
aa eine ganze Zahl von 0 bis 10 ist,
* eine Bindungsstelle zu M in Formel 1 angibt, und
*" eine Bindungsstelle zu Ring CY₂ in Formel 2A angibt.

9. Organometallische Verbindung nach einem der Ansprüche 1-8, wobei
die Gruppe dargestellt durch 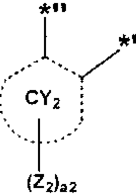 in Formel 2A eine Gruppe dargestellt durch eines ausgewählt aus Formel CY2-1 bis CY2-64 ist: 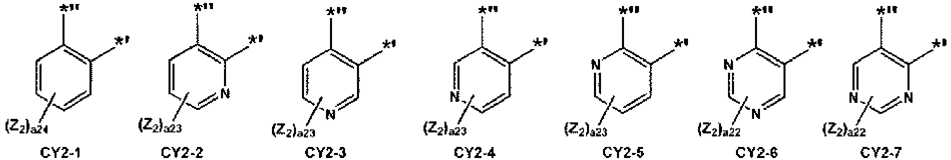 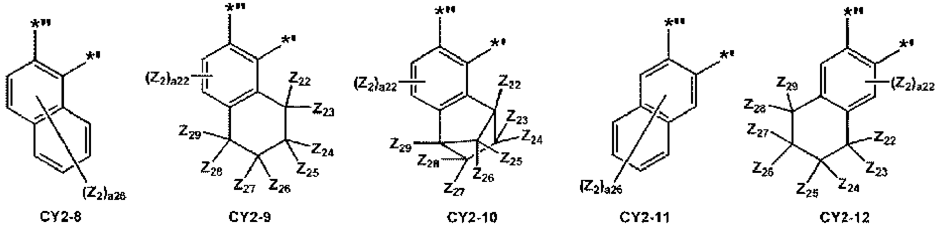 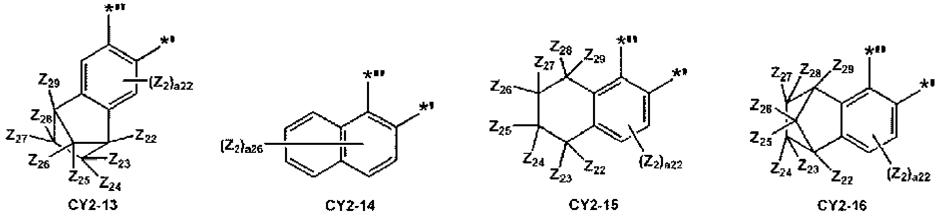 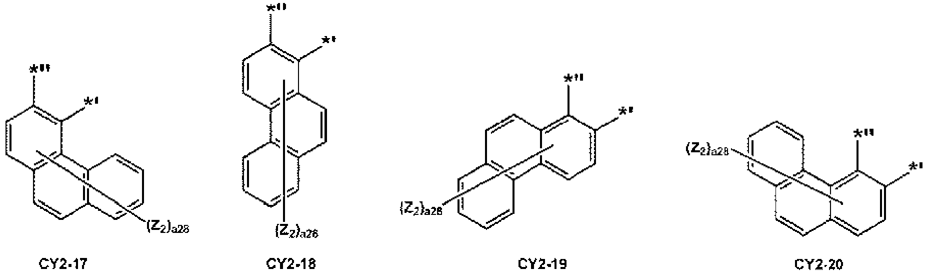 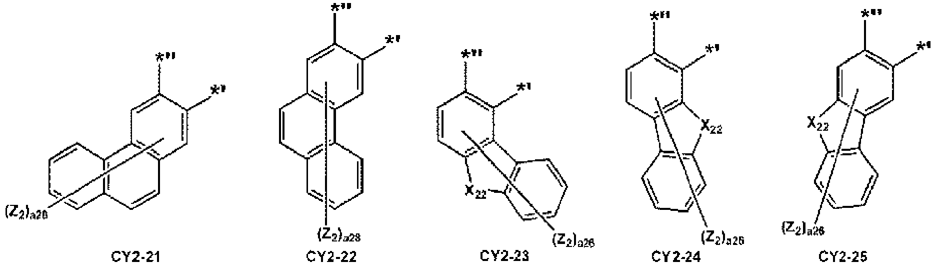 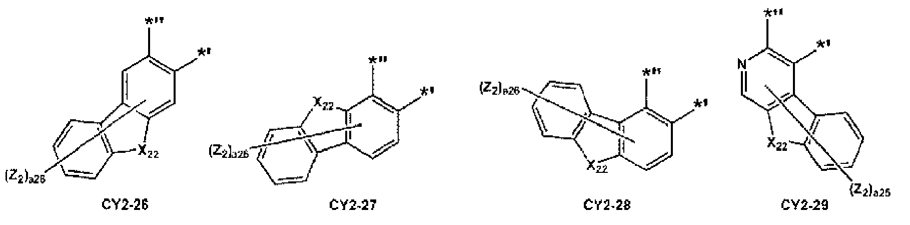 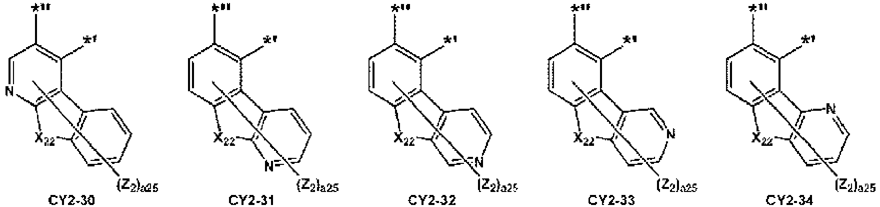 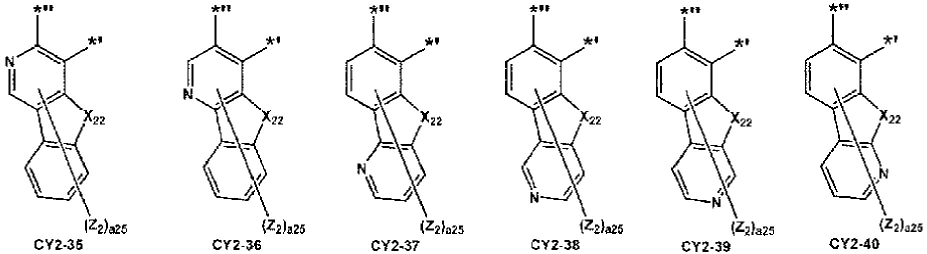 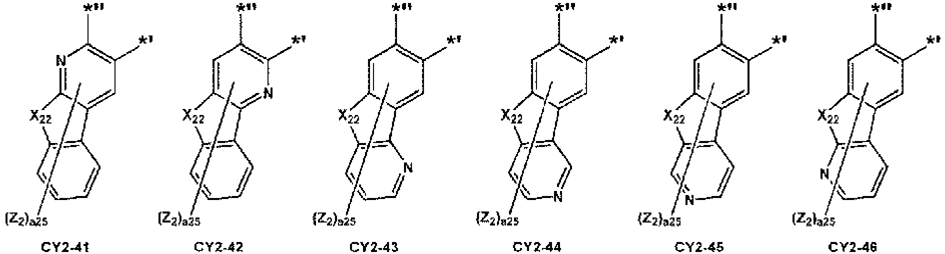 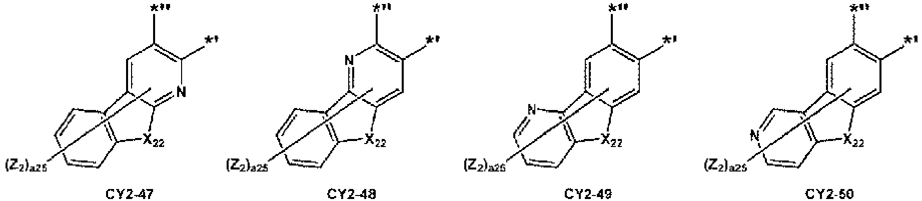 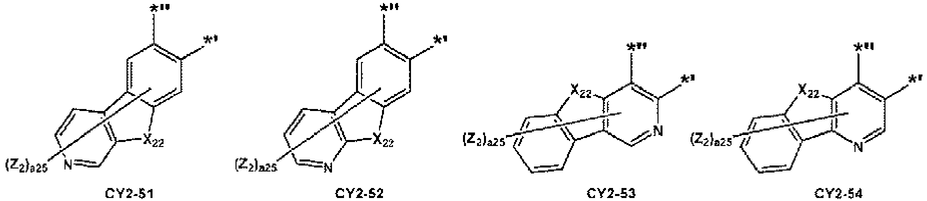 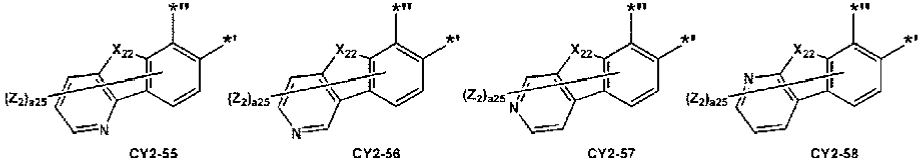 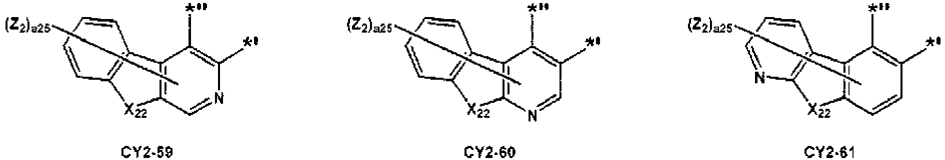 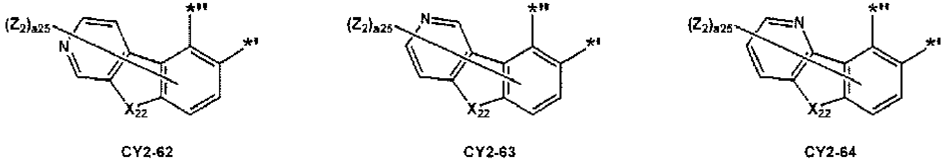 wobei in Formel CY2-1 bis CY2-64
Z₂ das gleiche wie beschrieben in Anspruch 1 ist,
X₂₂ C(Z₂₂)(Z₂₃), N(Z₂₂), O, S oder Si(Z₂₂)(Z₂₃) ist,
Z₂₂ bis Z₂₉ jeweils unabhängig die gleichen wie beschrieben in Verbindung mit Z₂ in Anspruch 1 sind,
a28 eine ganze Zahl von 0 bis 8 ist,
a26 eine ganze Zahl von 0 bis 6 ist,
a25 eine ganze Zahl von 0 bis 5 ist,
a24 eine ganze Zahl von 0 bis 4 ist,
a23 eine ganze Zahl von 0 bis 3 ist,
a22 eine ganze Zahl von 0 bis 2 ist,
*" eine Bindungsstelle zu einem Kohlenstoffatom eines benachbarten Pyridinrings in Formel 2A angibt, und
*' eine Bindungsstelle zu M in Formel 1 angibt.

10. Organometallische Verbindung nach einem der Ansprüche 1-9, wobei
die Gruppe dargestellt durch 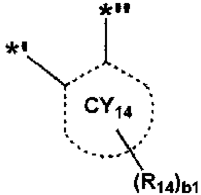 in Formel 2B eine Gruppe dargestellt durch eines ausgewählt aus Formel CY14-1 bis CY14-64 ist: 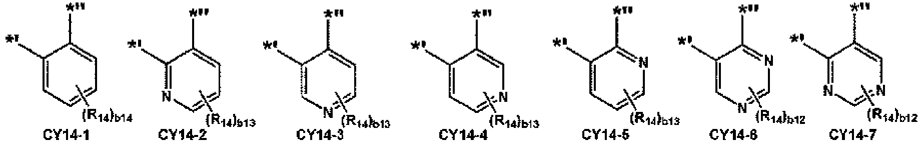 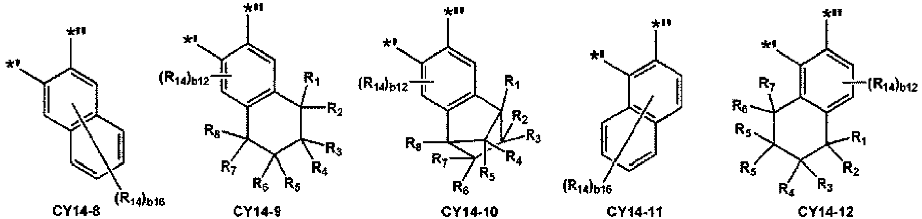 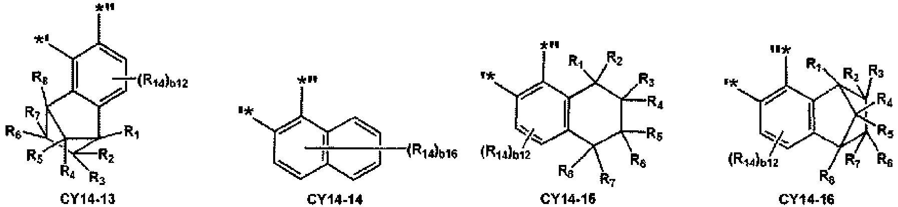 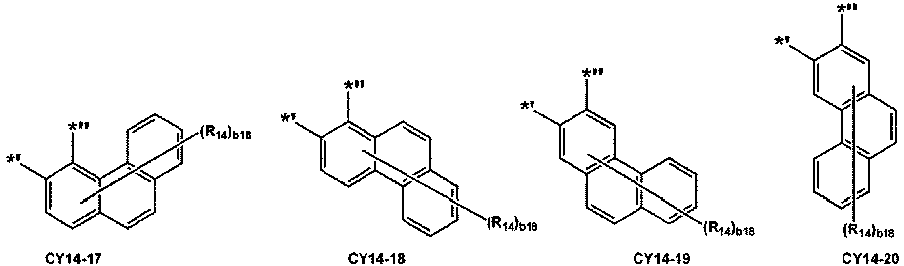 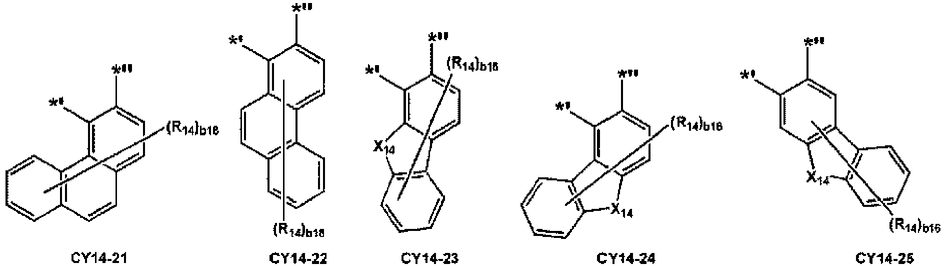 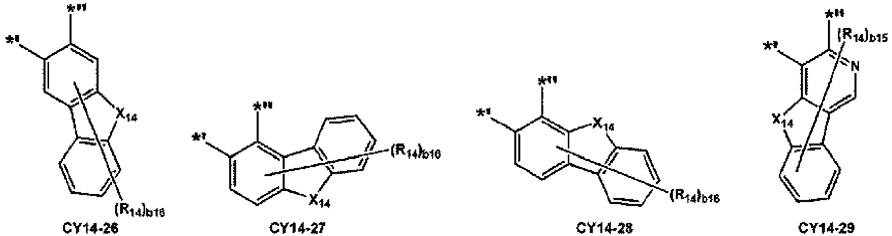 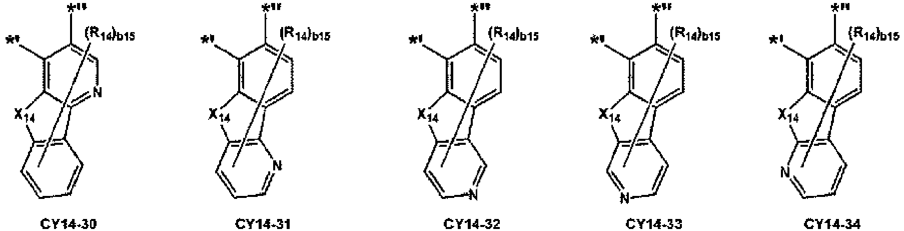 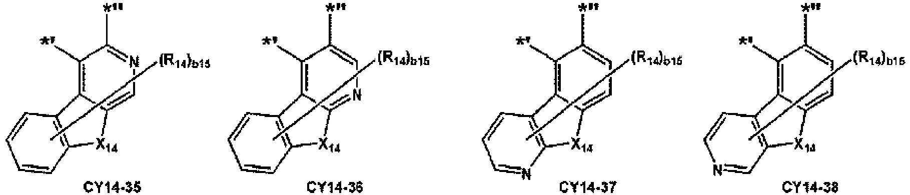 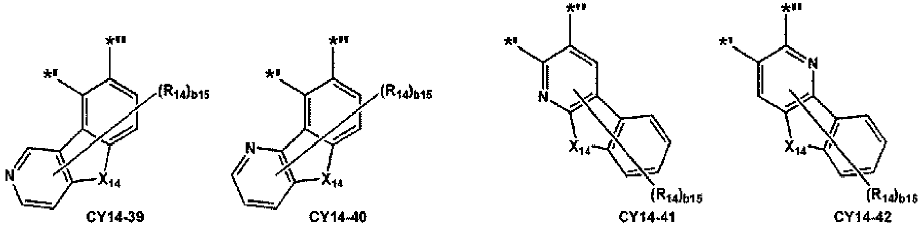 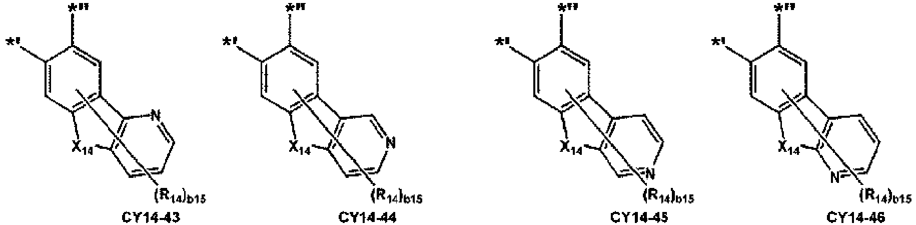 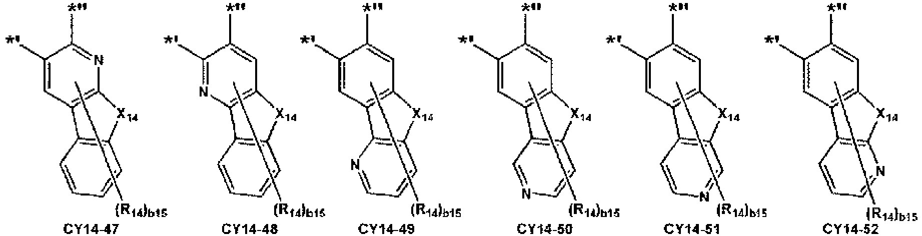 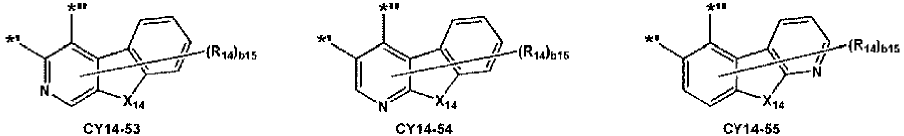 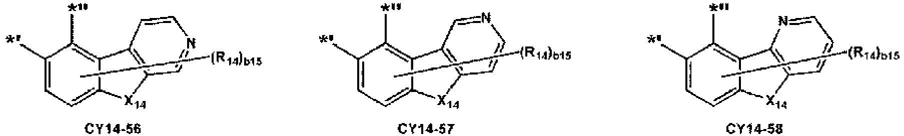 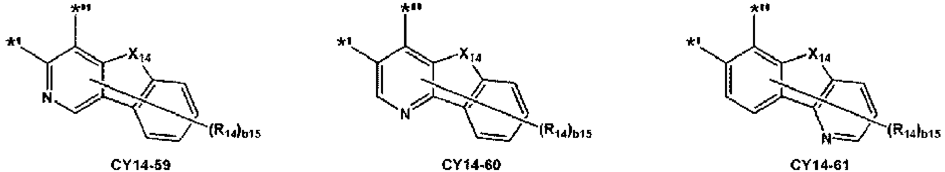 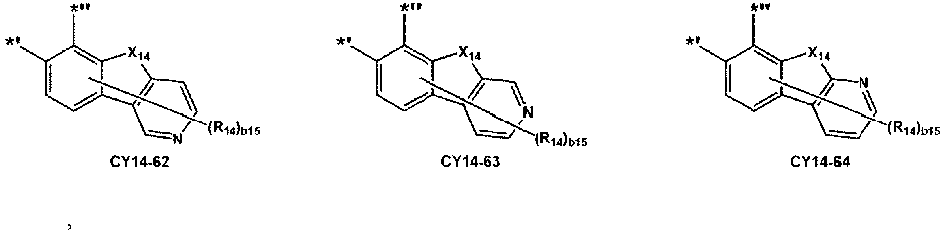 wobei in Formel CY14-1 bis CY14-64
R₁₄ das gleiche wie beschrieben in Anspruch 1 ist,
X₁₄ C(R₁)(R₂), N(R₁), O, S oder Si(R₁)(R₂) ist,
R₁ bis R₈ jeweils unabhängig die gleichen wie beschrieben in Verbindung mit R₁₄ in Anspruch 1 sind,
b18 eine ganze Zahl von 0 bis 8 ist,
b16 eine ganze Zahl von 0 bis 6 ist,
b15 eine ganze Zahl von 0 bis 5 ist,
b14 eine ganze Zahl von 0 bis 4 ist,
b13 eine ganze Zahl von 0 bis 3 ist,
b12 eine ganze Zahl von 0 bis 2 ist,
*" eine Bindungsstelle zu einem Kohlenstoffatom eines benachbarten Pyridinrings in Formel 2B angibt, und
*' eine Bindungsstelle zu M in Formel 1 angibt.

11. Organometallische Verbindung nach einem der Ansprüche 1-10, wobei die organometallische Verbindung dargestellt ist durch Formel 1A: 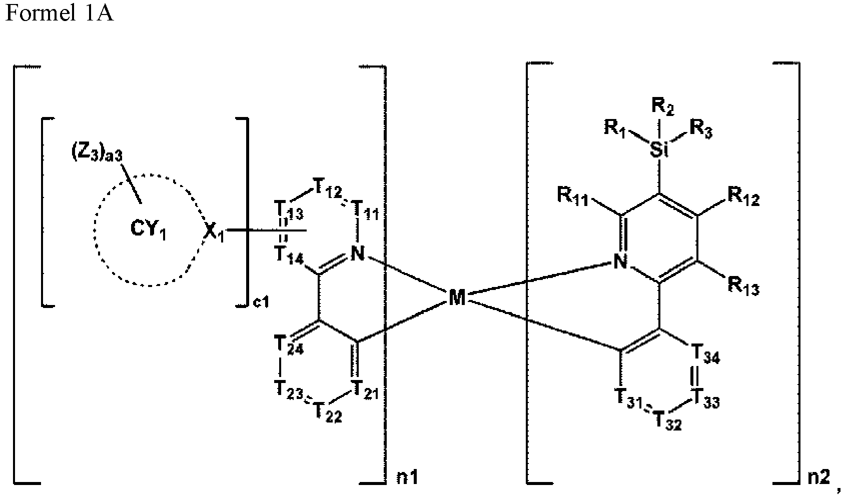 wobei in Formel 1A
M, n1, n2, X₁, Ring CY₁, R₁ bis R₃, Z₃, R₁₁ bis R₁₃ und c1 jeweils unabhängig die gleichen wie beschrieben in Anspruch 1 sind,
T₁₁ N, C(Z₁ₐ) oder Kohlenstoff verbunden mit Ring CY₁ ist, T₁₂ N, C(Z_{1b}) oder Kohlenstoff verbunden mit Ring CY₁ ist, T₁₃ N, C(Z_{1c}) oder Kohlenstoff verbunden mit Ring CY₁ ist, T₁₄ N, C(Z_{1d}) oder Kohlenstoff verbunden mit Ring CY₁ ist, zumindest eines ausgewählt aus Tu bis T₁₄ Kohlenstoff verbunden mit Ring CY₁ ist, und Z₁ₐ bis Z_{1d} jeweils unabhängig die gleichen wie beschrieben in Verbindung mit Z₁ in Anspruch 1 sind,
T₂₁ N oder C(Z₂ₐ) ist, T₂₂ N oder C(Z_{2b}) ist, T₂₃ N oder C(Z_{2c}) ist, T₂₄ N oder C(Z_{2d}) ist und Z₂ₐ bis Z_{2d} jeweils unabhängig die gleichen wie beschrieben in Verbindung mit Z₂ in Anspruch 1 sind,
T₃₁ N oder C(R₁₄ₐ) ist, T₃₂ N oder C(R_{14b}) ist, T₃₃ N oder C(R_{14c}) ist, T₃₄ N oder C(R_{14d}) ist und R₁₄ₐ bis R_{14d} jeweils unabhängig die gleichen wie beschrieben in Verbindung mit R₁₄ in Anspruch 1 sind,
zwei oder mehr Gruppen Z₁ₐ bis Z_{1d} optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
zwei oder mehr Gruppen Z₂ₐ bis Z_{2d} optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
wenn a3 zwei oder mehr ist, zwei oder mehr Gruppen Z₃ optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
R₁₂ und R₁₃ optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
zwei oder mehr Gruppen R₁₄ₐ bis R_{14d} optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
zwei oder mehr Gruppen Z₁ₐ bis Z_{1d}, Z₂ₐ bis Z_{2d}, R₁₁ bis R₁₃ und R₁₄ₐ bis R_{14d} optional verbunden sind, um eine carbocyclische C₅-C₃₀-Gruppe, die unsubstituiert oder substituiert mit zumindest einem R₁₀ₐ ist, oder eine heterocyclische C₁-C₃₀-Gruppe zu bilden, die unsubstituiert oder substituiert mit zumindest einem R₁₀ₐ ist,
R₁₀ₐ das gleiche wie beschrieben in Anspruch 1 ist,
die Bindungen zwischen N und M Koordinatenbindungen sind, und
die Bindungen zwischen C und M kovalente Bindungen sind.

12. Organometallische Verbindung nach Anspruch 1, wobei
die organometallische Verbindung eine ausgewählt aus Verbindung 1 bis 420 ist:

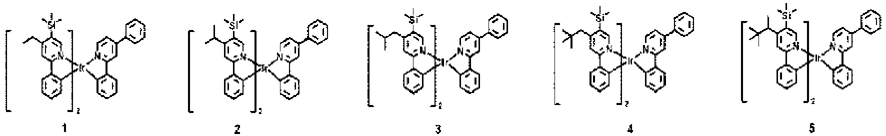

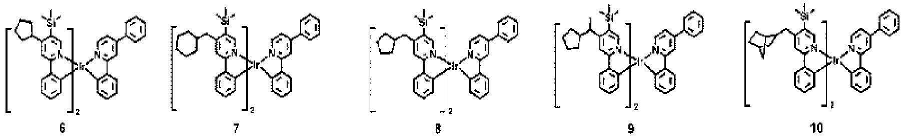

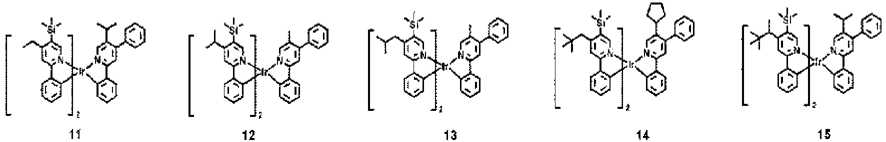

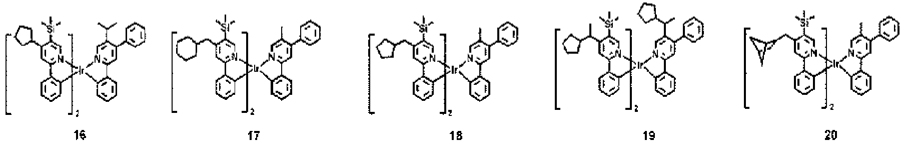

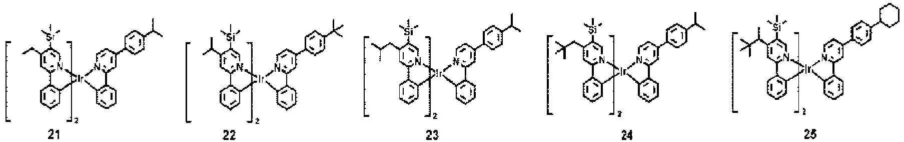

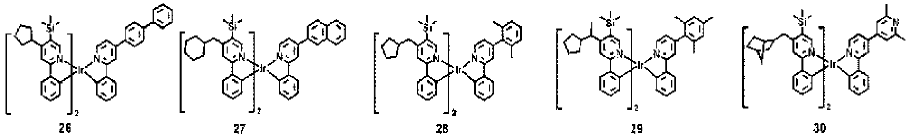

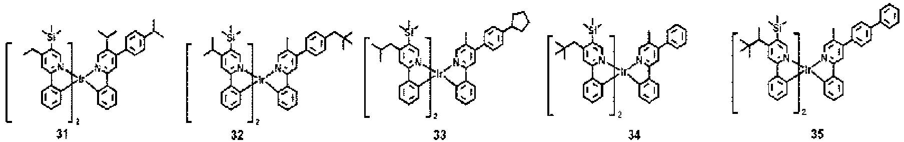

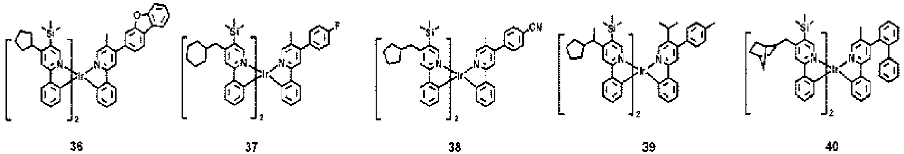

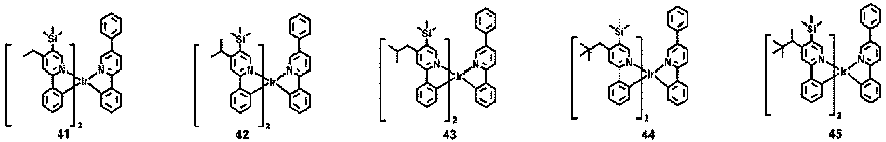

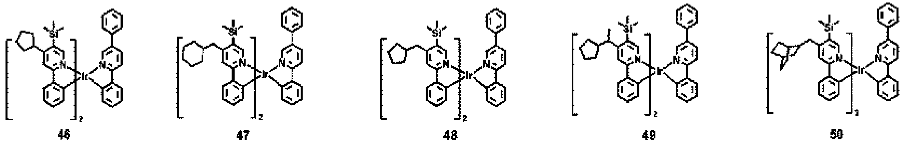

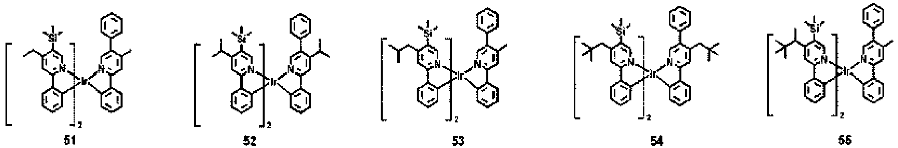

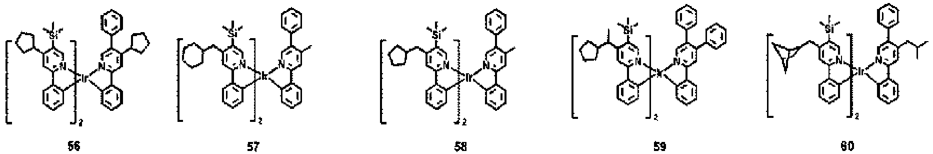

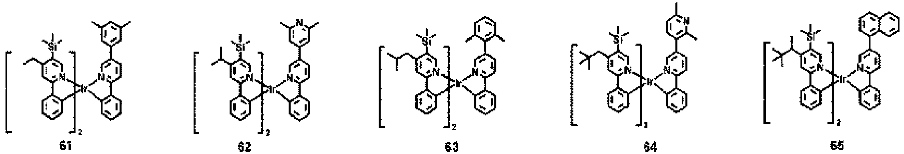

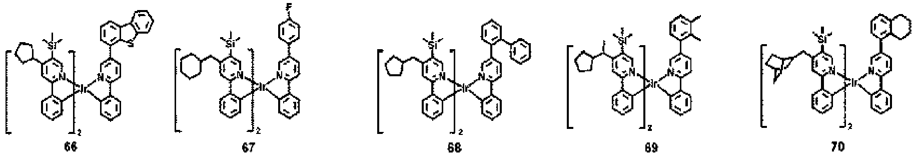

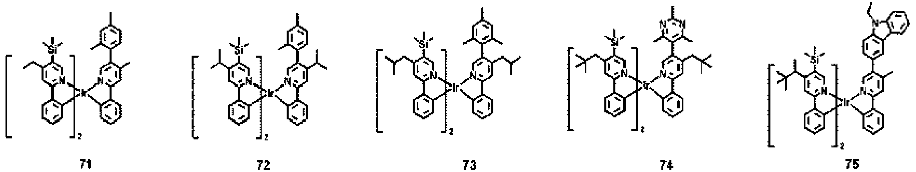

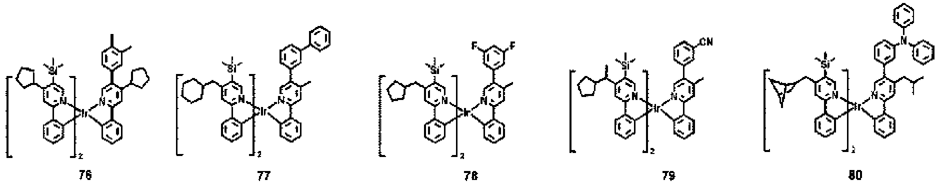

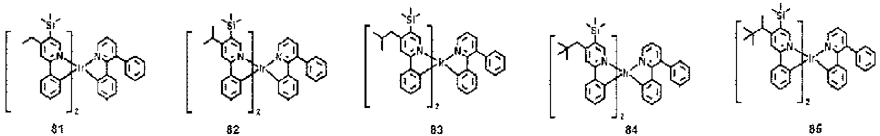

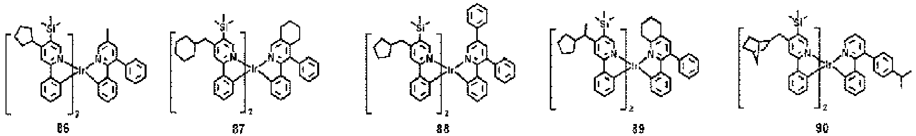

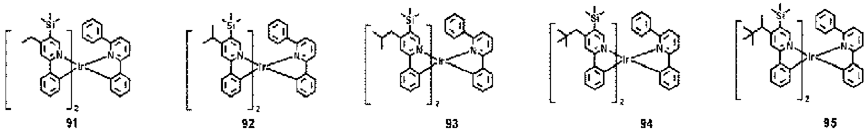

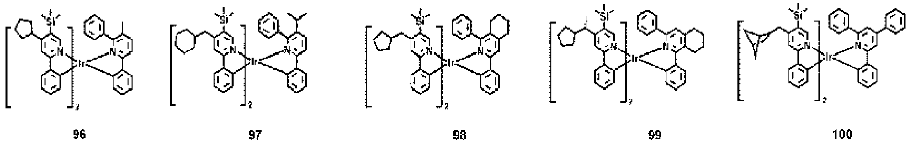

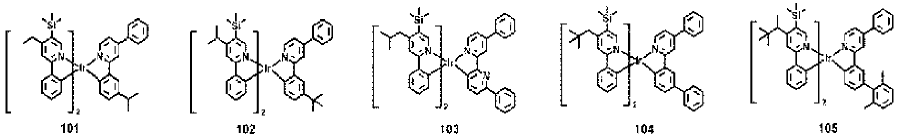

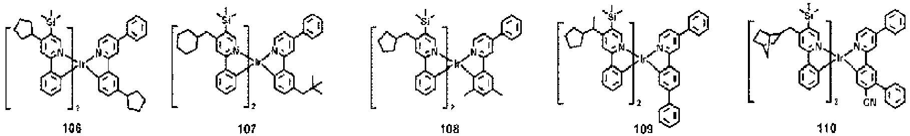

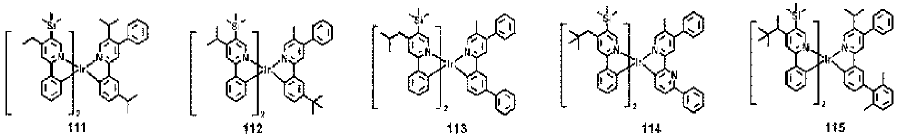

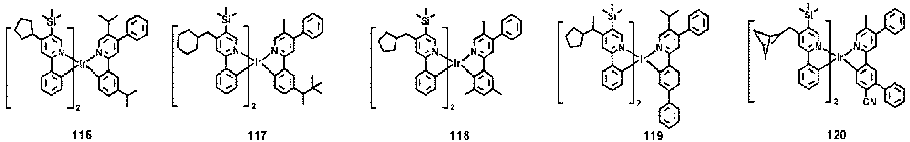

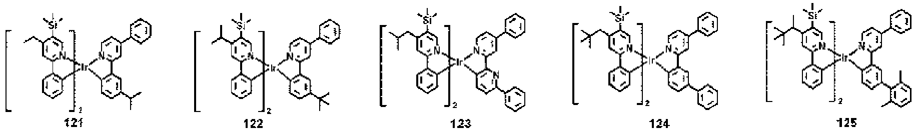

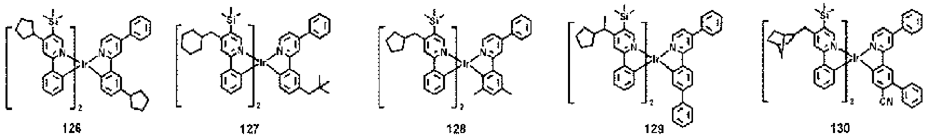

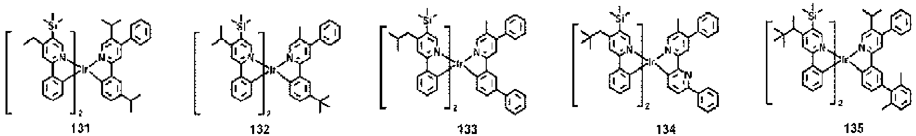

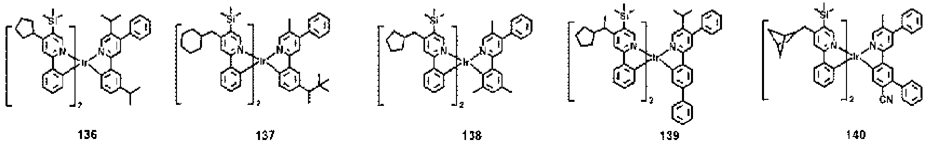

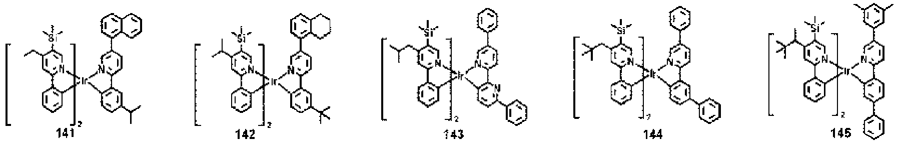

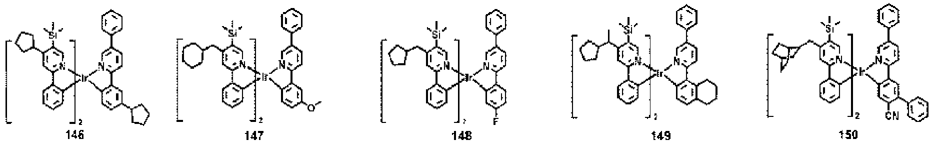

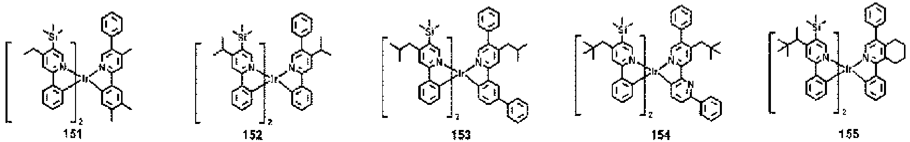

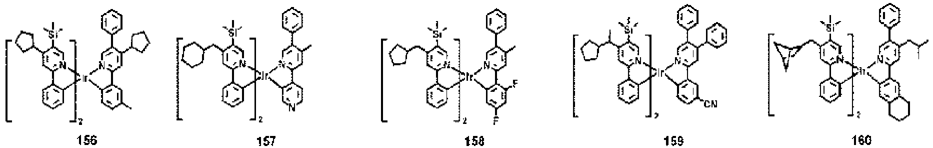

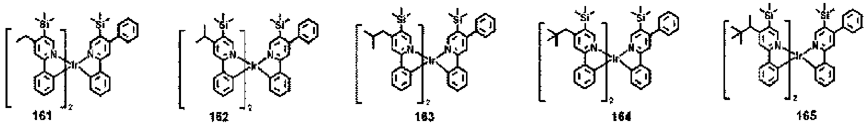

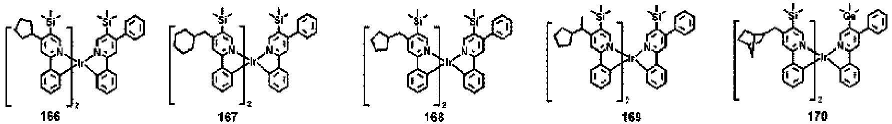

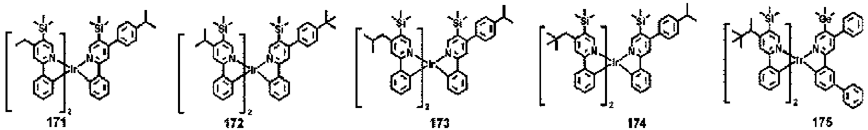

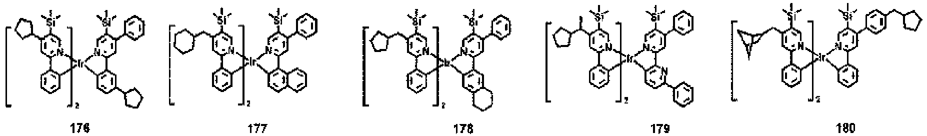

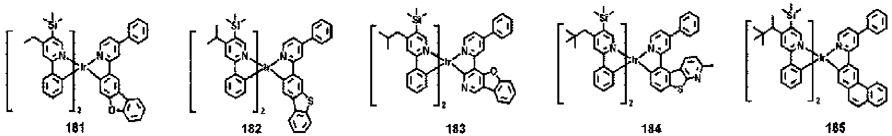

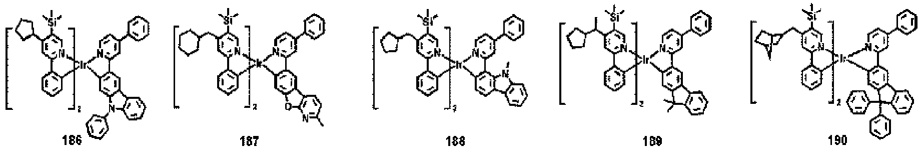

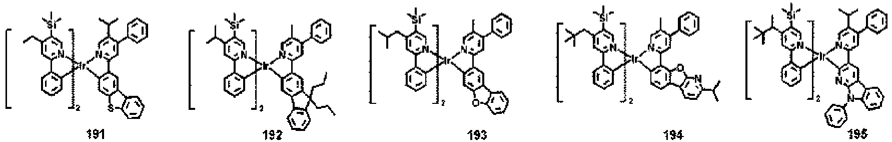

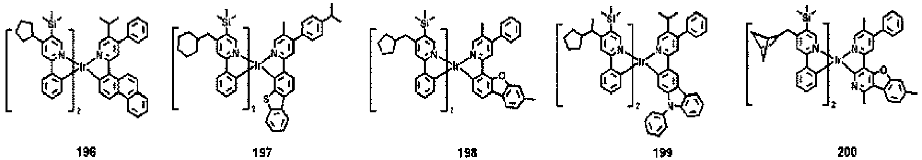

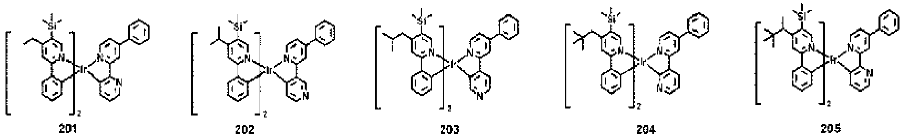

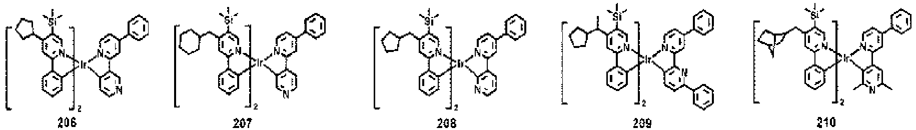

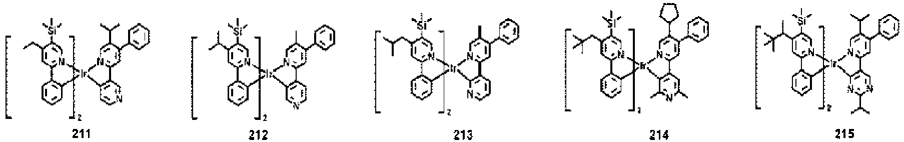

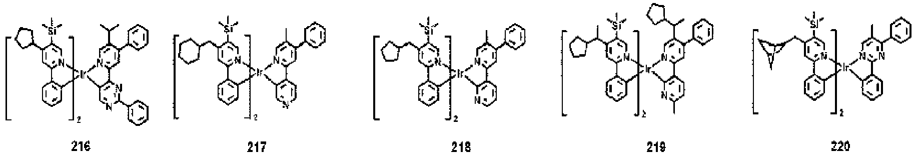

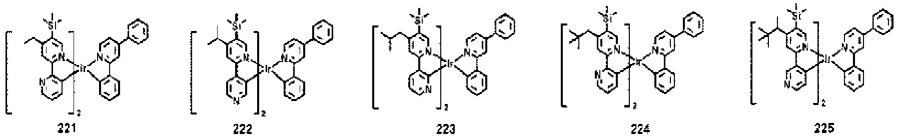

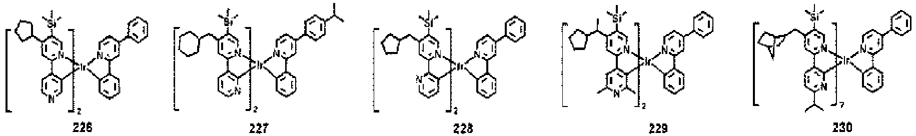

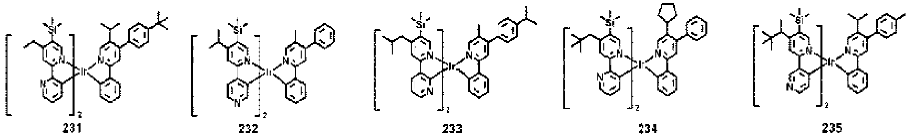

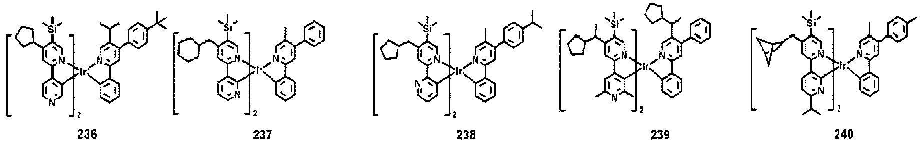

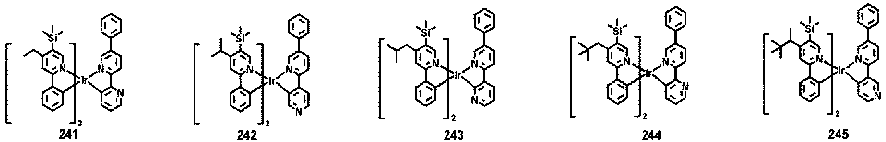

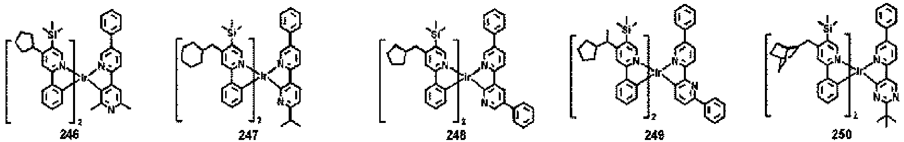

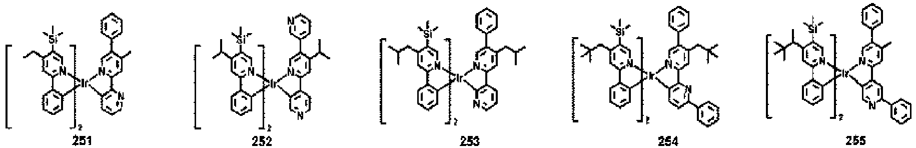

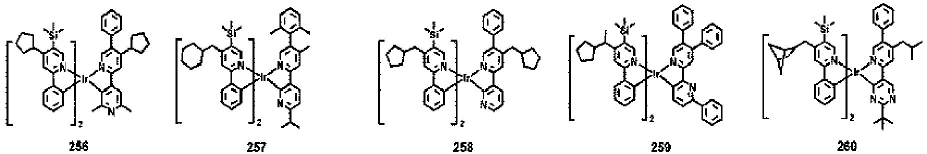

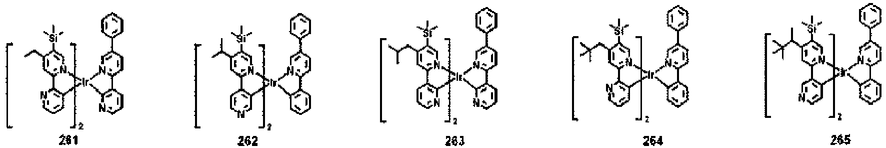

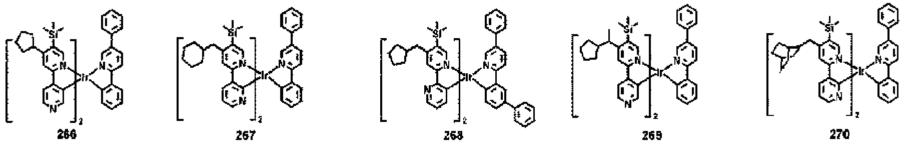

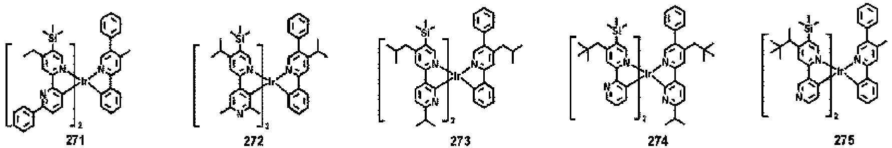

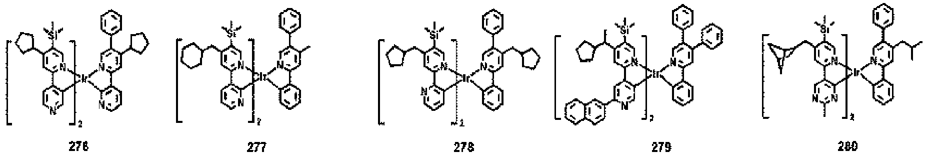

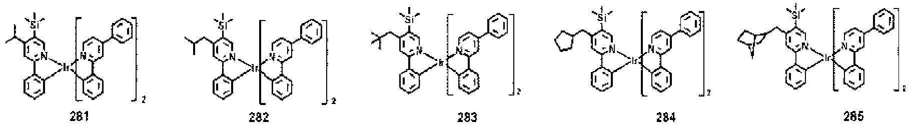

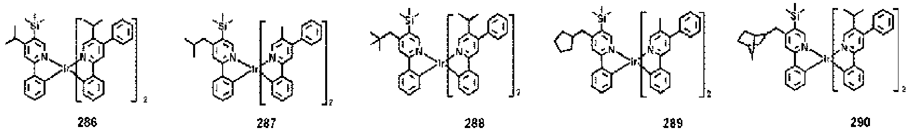

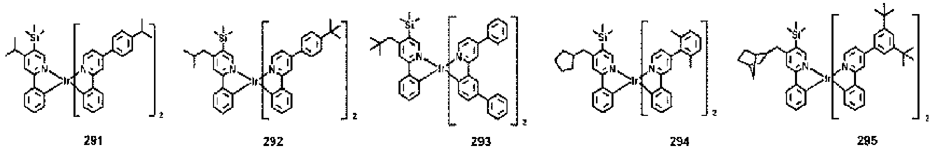

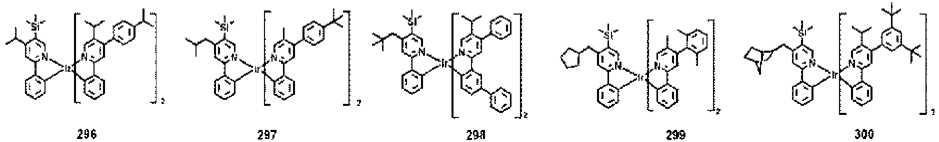

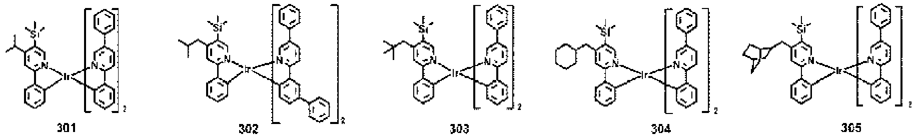

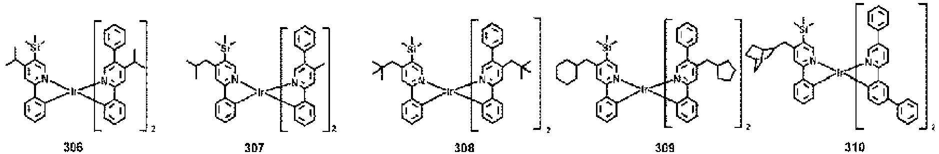

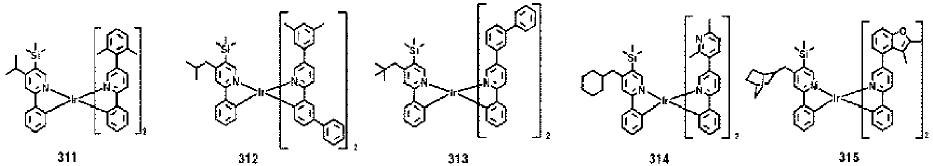

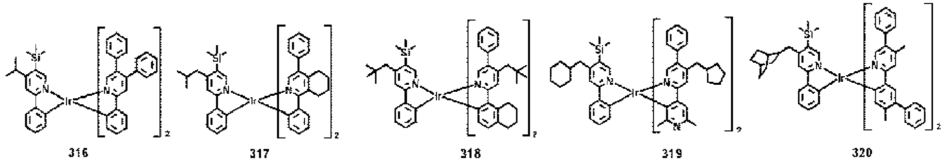

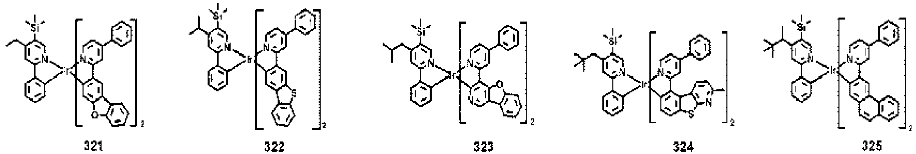

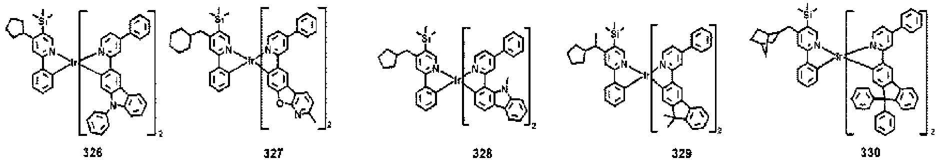

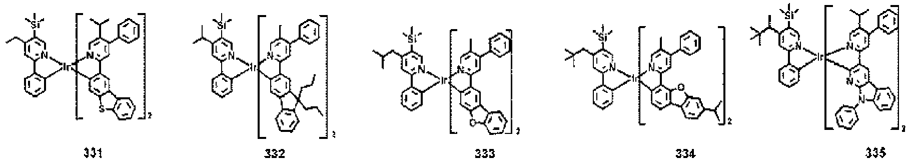

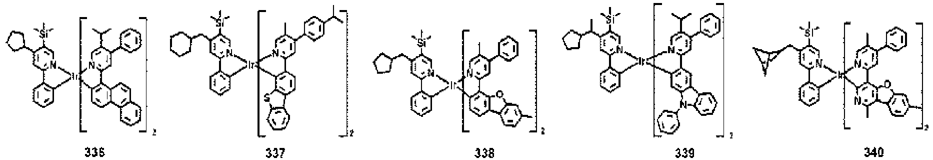

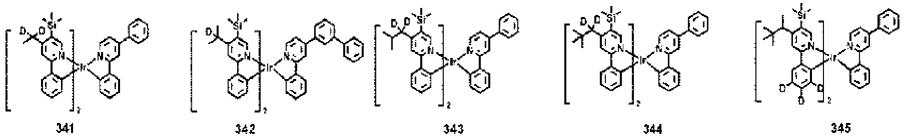

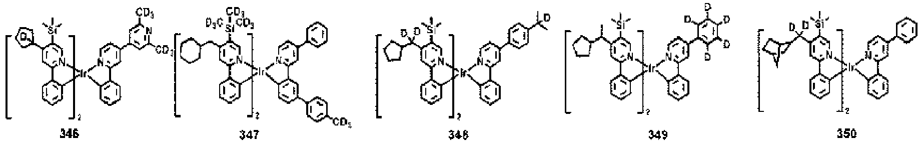

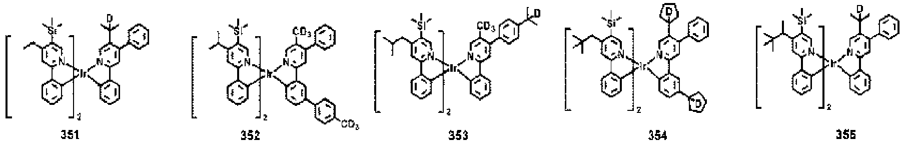

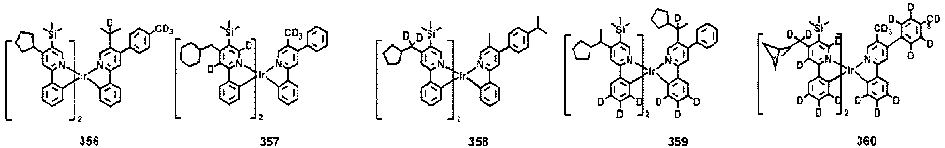

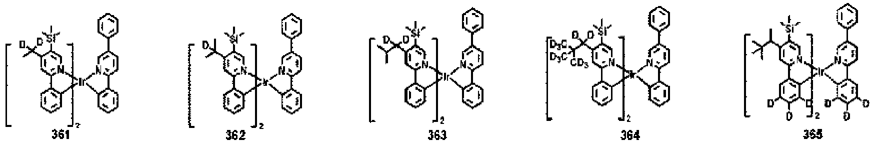

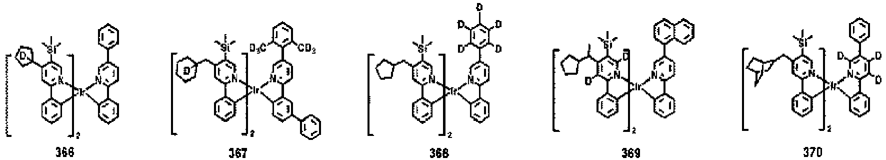

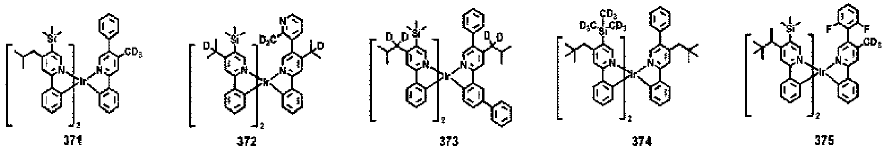

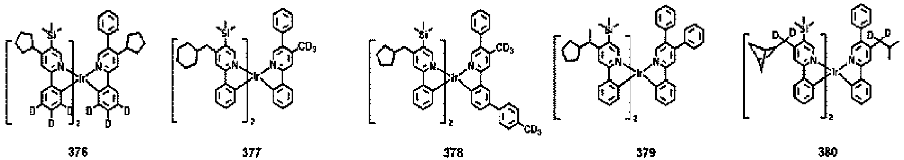

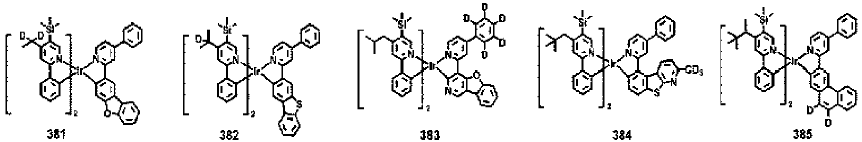

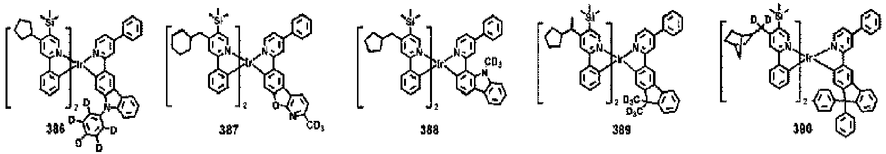

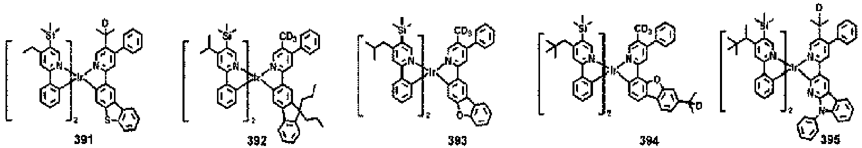

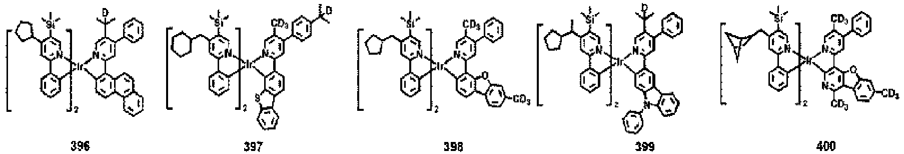

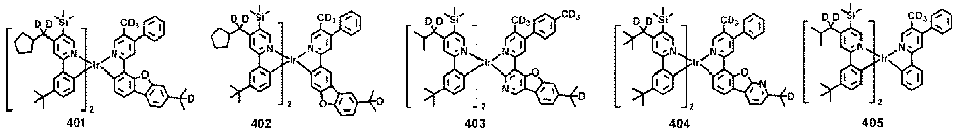

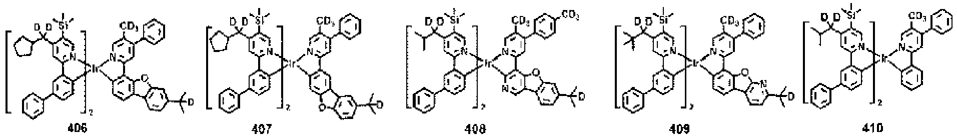

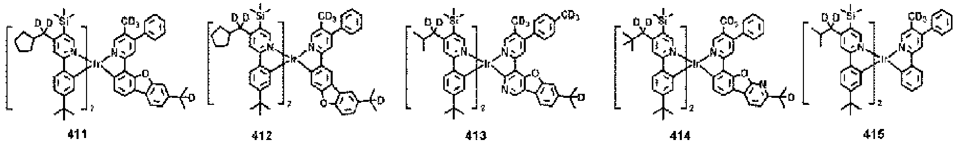

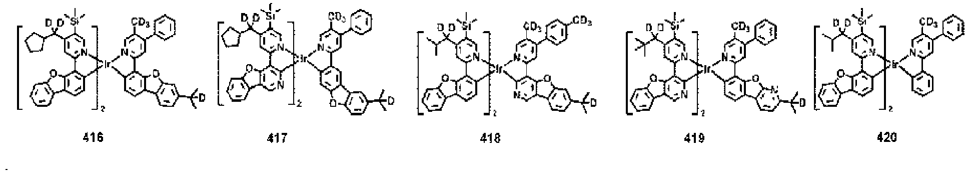

13. Organische lichtemittierende Vorrichtung, umfassend:
eine erste Elektrode,
eine zweite Elektrode, und
eine organische Schicht, die zwischen der ersten Elektrode und der zweiten Elektrode angeordnet ist und eine Emissionsschicht umfasst,
wobei die organische Schicht zumindest eine organometallische Verbindung nach einem der Ansprüche 1-12 umfasst;
wobei bevorzugt
die erste Elektrode eine Anode ist,
die zweite Elektrode eine Kathode ist,
die organische Schicht ferner einen Lochtransportbereich, der zwischen der ersten Elektrode und der Emissionsschicht angeordnet ist, und einen Elektronentransportbereich umfasst, der zwischen der Emissionsschicht und der zweiten Elektrode angeordnet ist,
wobei der Lochtransportbereich eine Locheinspritzschicht, eine Lochtransportschicht, eine Elektronenblockierschicht, eine Pufferschicht oder eine Kombination davon umfasst, und
wobei der Elektronentransportbereich eine Lochblockierschicht, eine Elektronentransportschicht, eine Elektroneneinspritzschicht oder eine Kombination davon umfasst.

14. Organische lichtemittierende Vorrichtung nach Anspruch 13, wobei die Emissionsschicht die zumindest eine organometallische Verbindung umfasst;
wobei bevorzugt
die Emissionsschicht ferner einen Wirt umfasst, und eine Menge des Wirts größer als eine Menge der zumindest einen organometallischen Verbindung ist.

15. Diagnostische Zusammensetzung, umfassend die organometallische Verbindung nach einem der Ansprüche 1-12.

## Revendications

1. Composé organométallique représenté par la Formule 1 :
Formule 1 M(L₁)ₙ₁(L₂)ₙ₂,
dans lequel, dans la Formule 1,
M est un métal de transition,
L₁ est un ligand représenté par la Formule 2A,
n1 vaut 1 ou 2, dans lequel, quand n1 vaut 2, deux groupes L₁ sont identiques ou différents l'un de l'autre,
L₂ est un ligand représenté par la Formule 2B,
n2 vaut 1 ou 2, dans lequel, quand n2 vaut 2, deux groupes L₂ sont identiques ou différents l'un de l'autre,
la somme de n1 et de n2 est 2 ou 3, et
L₁ et L₂ sont différents l'un de l'autre : 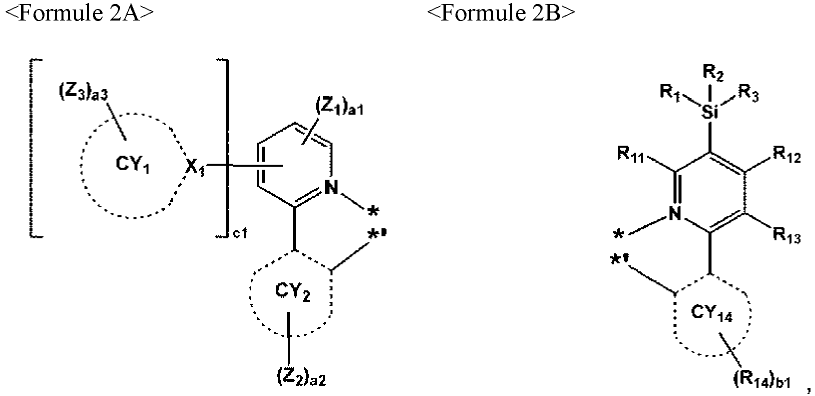 dans lequel, dans les Formules 2A et 2B,
X₁ est C, N, Si, ou P,
le noyau CY₁, le noyau CY₂, et le noyau CY₁₄ sont chacun indépendamment un groupe carbocyclique en C₅-C₃₀ ou un groupe hétérocyclique en C₁-C₃₀,
R₁ à R₃ sont chacun indépendamment un groupe alkyle en C₁-C₆₀ ou un groupe aryle en C₆-C₆₀, chacun indépendamment non substitué ou substitué par au moins un élément choisi parmi un deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, et un groupe alkyle en C₁-C₁₀,
Z₁ à Z₃ et R₁₁ à R₁₄ sont chacun indépendamment choisis parmi un hydrogène, un deutérium, -F, -Cl, -Br, -I, -SF₅, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀ substitué ou non substitué, un groupe alcényle en C₂-C₆₀ substitué ou non substitué, un groupe alcynyle en C₂-C₆₀ substitué ou non substitué, un groupe alcoxy en C₁-C₆₀ substitué ou non substitué, un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe arylalkyle en C₇-C₆₀ substitué ou non substitué, un groupe hétéroaryle en C₁-C₆₀ substitué ou non substitué, un groupe hétéroaryloxy en C₁-C₆₀ substitué ou non substitué, un groupe hétéroarylthio en C₁-C₆₀ substitué ou non substitué, un groupe hétéroarylalkyle en C₂-C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂),
-Si(Q₃)(Q₄)(Q₅), -Ge(Q₃)(Q₄)(Q₅), -B(Q₆)(Q₇), et -P(=O)(Q₈)(Q₉), dans lequel R₁₂ n'est ni un hydrogène ni un groupe méthyle,
a1 est un nombre entier allant de 0 à 3, dans lequel, quand a1 est deux ou plus, deux groupes Z₁ ou plus sont identiques ou différents les uns des autres,
a2, a3, et b1 sont chacun indépendamment un nombre entier allant de 0 à 20, dans lequel, quand a2 est deux ou plus, deux groupes Z₂ ou plus sont identiques ou différents les uns des autres, quand a3 est deux ou plus, deux groupes Z₃ ou plus sont identiques ou différents les uns des autres, et quand b1 est deux ou plus, deux groupes R₁₄ ou plus sont identiques ou différents les uns des autres,
c1 est un nombre entier allant de 1 à 4, dans lequel, quand c1 est 2 ou plus, deux groupes ou plus représentés par 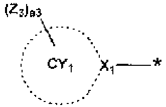 sont identiques ou différents les uns des autres,
quand a1 est deux ou plus, deux groupes Z₁ ou plus dans le nombre de c1 sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
quand a2 est deux ou plus, deux groupes Z₂ ou plus dans le nombre de a2 sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
quand a3 est deux ou plus, deux groupes Z₃ ou plus dans le nombre de a3 sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
R₁₂ et R₁₃ sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
quand b1 est deux ou plus, deux groupes R₁₄(s) ou plus dans le nombre de b1 sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
deux substituants ou plus choisis parmi Z₁ et Z₂ sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
deux substituants ou plus choisis parmi R₁₁ à R₁₄ sont facultativement liés pour former un groupe carbocyclique en C₅-C₃₀ qui est non substitué ou substitué par au moins un de R₁₀ₐ ou un groupe hétérocyclique en C₁-C₃₀ qui est non substitué ou substitué par au moins un R₁₀ₐ,
R₁₀ₐ est choisi parmi un hydrogène, un deutérium, -F, -Cl, -Br, -I, -SF₅, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀ substitué ou non substitué, un groupe alcényle en C₂-C₆₀ substitué ou non substitué, un groupe alcynyle en C₂-C₆₀ substitué ou non substitué, un groupe alcoxy en C₁-C₆₀ substitué ou non substitué, un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₁-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe arylalkyle en C₇-C₆₀ substitué ou non substitué, un groupe hétéroaryle en C₁-C₆₀ substitué ou non substitué, un groupe hétéroaryloxy en C₁-C₆₀ substitué ou non substitué, un groupe hétéroarylthio en C₁-C₆₀ substitué ou non substitué, un groupe hétéroarylalkyle en C₂-C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂), -Si(Q₃)(Q₄)(Q₅), -Ge(Q₃)(Q₄)(Q₅), -B(Q₆)(Q₇), et-P(=O)(Q₈)(Q₉),
* et *' dans les Formules 2A et 2B indiquent chacun un site de liaison à M dans la Formule 1,
au moins un substituant du groupe alkyle en C₁-C₆₀ substitué, du groupe alcényle en C₂-C₆₀ substitué, du groupe alcynyle en C₂-C₆₀ substitué, du groupe en alcoxy C₁-C₆₀ substitué, du groupe cycloalkyle en C₃-C₁₀ substitué, du groupe hétérocycloalkyle en C₁-C₁₀ substitué, du groupe cycloalcényle en C₃-C₁₀ substitué, du groupe hétérocycloalcényle en C₁-C₁₀ substitué, du groupe aryle en C₆-C₆₀ substitué, du groupe aryloxy en C₆-C₆₀ substitué, du groupe arylthio en C₆-C₆₀ substitué, du groupe arylalkyle en C₇-C₆₀ substitué, du groupe hétéroaryle en C₁-C₆₀ substitué, du groupe hétéroaryloxy en C₁-C₆₀ substitué, du groupe hétéroarylthio en C₁-C₆₀ substitué, du groupe hétéroarylalkyle en C₂-C₆₀ substitué, du groupe polycyclique condensé non aromatique monovalent substitué, et du groupe hétéropolycyclique condensé non aromatique monovalent substitué sont chacun indépendamment choisis parmi :
un deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀, un groupe alcényle en C₂-C₆₀, un groupe alcynyle en C₂-C₆₀, ou un groupe alcoxy en C₁-C₆₀ ;
un groupe alkyle en C₁-C₆₀, un groupe alcényle en C₂-C₆₀, un groupe alcynyle en C₂-C₆₀, ou un groupe alcoxy en C₁-C₆₀, chacun substitué par au moins un élément choisi parmi un deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe arylalkyle en C₇-C₆₀, un groupe hétéroaryle en C₁-C₆₀, un groupe hétéroaryloxy en C₁-C₆₀, un groupe hétéroarylthio en C₁-C₆₀, un groupe hétéroarylalkyle en C₂-C₆₀, un groupe polycyclique condensé non aromatique monovalent, un groupe hétéropolycyclique condensé non aromatique monovalent, -N(Q₁₁)(Q₁₂),-Si(Q₁₃)(Q₁₄)(Q₁₅), -B(Q₁₆)(Q₁₇), et -P(=O)(Q₁₈)(Q₁₉);
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe arylalkyle en C₇-C₆₀, un groupe hétéroaryle en C₁-C₆₀, un groupe hétéroaryloxy en C₁-C₆₀, un groupe hétéroarylthio en C₁-C₆₀, un groupe hétéroarylalkyle en C₂-C₆₀, un groupe polycyclique condensé non aromatique monovalent, et un groupe hétéropolycyclique condensé non aromatique monovalent ;
un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe arylalkyle en C₇-C₆₀, un groupe hétéroaryle en C₁-C₆₀, un groupe hétéroaryloxy en C₁-C₆₀, un groupe hétéroarylthio en C₁-C₆₀, un groupe hétéroarylalkyle en C₂-C₆₀, un groupe polycyclique condensé non aromatique monovalent, ou un groupe hétéropolycyclique condensé non aromatique monovalent, chacun substitué par au moins un élément choisi parmi un deutérium, -F, -Cl, -Br, -I, -CD₃, -CD₂H,-CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryloxy en C₆-C₆₀, un groupe arylthio en C₆-C₆₀, un groupe arylalkyle en C₇-C₆₀, un groupe hétéroaryle en C₁-C₆₀, un groupe hétéroaryloxy en C₁-C₆₀, un groupe hétéroarylthio en C₁-C₆₀, un groupe hétéroarylalkyle en C₂-C₆₀, un groupe polycyclique condensé non aromatique monovalent, un groupe hétéropolycyclique condensé non aromatique monovalent, -N(Q₂₁)(Q₂₂), -Si(Q₂₃)(Q₂₄)(Q₂₅),-B(Q₂₆)(Q₂₇), et -P(=O)(Q₂₈)(Q₂₉) ; et
-N(Q₃₁)(Q₃₂), -Si(Q₃₃)(Q₃₄)(Q₃₅), -B(Q₃₆)(Q₃₇), ou -P(=O)(Q₃₈)(Q₃₉) ; et
Q₁ à Q₉, Q₁₁ à Q₁₉, Q₂₁ à Q₂₉, et Q₃₁ à Q₃₉ sont chacun indépendamment choisis parmi un hydrogène, un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀, un groupe alcényle en C₂-C₆₀, un groupe alcynyle en C₂-C₆₀, un groupe alcoxy en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₁-C₁₀, un groupe aryle en C₆-C₆₀, un groupe aryle en C₆-C₆₀ substitué par au moins un groupe choisi parmi un groupe alkyle en C₁-C₆₀ et un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, et un groupe hétéropolycyclique condensé non aromatique monovalent.

2. Composé organométallique selon la revendication 1, dans lequel,
dans la Formule 1, M est Ir, et la somme de n1 et de n2 est 3 ; ou
dans la Formule 1, M est Pt, et la somme de n1 et de n2 est 2.

3. Composé organométallique selon la revendications 1 ou 2, dans lequel,
dans les Formules 2A et 2B, le noyau CY₁, le noyau CY₂, le noyau CY₁₄ sont chacun indépendamment choisis parmi un groupe cyclopentane, un groupe cyclohexane, un groupe cycloheptane, un groupe cyclopentène, un groupe cyclohexène, un groupe cycloheptène, un groupe benzène, un groupe naphtalène, un groupe anthracène, un groupe phénanthrène, un groupe triphénylène, un groupe pyrène, un groupe chrysène, un groupe cyclopentadiène, un groupe 1,2,3,4-tétrahydronaphtalène, un groupe thiophène, un groupe furane, un groupe indole, un groupe benzoborole, un groupe benzophosphole, un groupe indène, un groupe benzosilole, un groupe benzogermole, un groupe benzothiophène, un groupe benzosélénophène, un groupe benzofurane, un groupe carbazole, un groupe dibenzoborole, un groupe dibenzophosphole, un groupe fluorène, un groupe dibenzosilole, un groupe dibenzogermole, un groupe dibenzothiophène, un groupe dibenzosélénophène, un groupe dibenzofurane, un groupe 5-oxyde de dibenzothiophène, un groupe 9H-fluorène-9-one, un groupe 5,5-dioxyde de dibenzothiophène, un groupe azaindole, un groupe azabenzoborole, un groupe azabenzophosphole, un groupe azaindène, un groupe azabenzosilole, un groupe azabenzogermole, un groupe azabenzothiophène, un groupe azabenzosélénophène, un groupe azabenzofurane, un groupe azacarbazole, un groupe azadibenzoborole, un groupe azadibenzophosphole, un groupe azafluorène, un groupe azadibenzosilole, un groupe azadibenzogermole, un groupe azadibenzothiophène, un groupe azadibenzosélénophène, un groupe azadibenzofurane, un groupe 5-oxyde d'azadibenzothiophène, un groupe aza-9H-fluorène-9-one, un groupe 5,5-dioxyde d'azadibenzothiophène, un groupe pyridine, un groupe pyrimidine, un groupe pyrazine, un groupe pyridazine, un groupe triazine, un groupe quinoline, un groupe isoquinoline, un groupe quinoxaline, un groupe quinazoline, un groupe phénanthroline, un groupe pyrrole, un groupe pyrazole, un groupe imidazole, un groupe triazole, un groupe oxazole, un groupe isoxazole, un groupe thiazole, un groupe isothiazole, un groupe oxadiazole, un groupe thiadiazole, un groupe benzopyrazole, un groupe benzimidazole, un groupe benzoxazole, un groupe benzothiazole, un groupe benzoxadiazole, un groupe benzothiadiazole, un groupe 5,6,7,8-tétrahydroisoquinoline, et un groupe 5,6,7,8-tétrahydroquinoline ; et/ou
dans lequel,
dans la Formule 2B, R₁ à R₃ sont chacun indépendamment choisis parmi un groupe méthyle, un groupe éthyle, un groupe n-propyle, un groupe isopropyle, un groupe n-butyle, un groupe sec-butyle, un groupe isobutyle, un groupe tert-butyle, un groupe n-pentyle, un groupe tert-pentyle, un groupe néopentyle, un groupe isopentyle, un groupe sec-pentyle, un groupe 3-pentyle, un groupe sec-isopentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe iso-octyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe phényle, un groupe biphényle, ou un groupe naphtyle, chacun non substitué ou substitué par au moins un élément choisi parmi un deutérium, -F, -Cl, -Br, -I, -CD₃, -CD₂H,-CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci et un groupe alkyle en C₁-C₁₀.

4. Composé organométallique selon l'une quelconque des revendications 1 à 3, dans lequel,
dans les Formules 2A et 2B, Z₁ à Z₃ et R₁₁ à R₁₄ sont chacun indépendamment choisis parmi :
un hydrogène, un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, -SF₅, un groupe alkyle en C₁-C₂₀, ou un groupe alcoxy en C₁-C₂₀ ;
un groupe alkyle en C₁-C₂₀ et un groupe alcoxy en C₁-C₂₀, chacun substitué par au moins un élément choisi parmi un deutérium, -F, -Cl, -Br, -I, -CD₃, -CD₂H, -CDH₂, -CF₃, -CF₂H,-CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₁₀, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe (alkyle en C₁-C₂₀)phényle, un groupe biphényle, un groupe terphényle, un groupe naphtyle, un groupe pyridinyle, ou un groupe pyrimidinyle ;
un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe bicyclo(1.1.1)pentyle, un groupe bicyclo(2.1.1)hexyle, un groupe bicyclo(2.2.1)heptyle, un groupe bicyclo(2.2.2)octyle, un groupe phényle, un groupe (alkyle en C₁-C₂₀)phényle, un groupe biphényle, un groupe terphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, un groupe azacarbazolyle, un groupe azadibenzofuranyle, ou un groupe azadibenzothiophényle, chacun non substitué ou substitué par au moins un élément choisi parmi un deutérium, -F, -Cl, -Br, -I, -CD₃, -CD₂H, -CDH₂, -CF₃, - CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₂₀, un groupe alcoxy en C₁-C₂₀, un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe bicyclo(1.1.1)pentyle, un groupe bicyclo(2.1.1)hexyle, un groupe bicyclo(2.2.1)heptyle, un groupe bicyclo(2.2.2)octyle, un groupe phényle, un groupe (alkyle en C₁-C₂₀)phényle, un groupe biphényle, un groupe terphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, un groupe azacarbazolyle, un groupe azadibenzofuranyle, un groupe azadibenzothiophényle, et -Si(Q₃₃)(Q₃₄)(Q₃₅) ; et
-N(Q₁)(Q₂), -Si(Q₃)(Q₄)(Q₅), -Ge(Q₃)(Q₄)(Q₅), -B(Q₆)(Q₇), -P(=O)(Q₈)(Q₉), ou-P(Q₈)(Q₉), dans lequel R₁₂ n'est ni un hydrogène ni un groupe méthyle, et
Q₁ à Q₉ et Q₃₃ à Q₃₅ sont chacun indépendamment choisis parmi :
-CH₃, -CD₃, -CD₂H, -CDH₂, -CH₂CH₃, -CH₂CD₃, -CH₂CD₂H, -CH₂CDH₂, -CHDCH₃,-CHDCD₂H, -CHDCDH₂, -CHDCD₃, -CD₂CD₃, -CD₂CD₂H, ou -CD₂CDH₂ ; et
un groupe n-propyle, un groupe isopropyle, un groupe n-butyle, un groupe sec-butyle, un groupe isobutyle, un groupe tert-butyle, un groupe n-pentyle, un groupe tert-pentyle, un groupe néopentyle, un groupe isopentyle, un groupe sec-pentyle, un groupe 3-pentyle, un groupe sec-isopentyle, un groupe phényle, un groupe biphényle, ou un groupe naphtyle, chacun non substitué ou substitué par au moins un élément choisi parmi un deutérium, un groupe alkyle en C₁-C₁₀, ou un groupe phényle ; et/ou dans lequel
R₁₂ dans la Formule 2B est choisi parmi :
un groupe alkyle en C₂-C₂₀ ou un groupe alcoxy en C₂-C₂₀ ;
un groupe méthyle ou un groupe méthoxy, chacun substitué par au moins un élément choisi parmi un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe bicyclo(1.1.1)pentyle, un groupe bicyclo(2.1.1)hexyle, un groupe bicyclo(2.2.1)heptyle, un groupe bicyclo(2.2.2)octyle, un groupe phényle, un groupe naphtyle, un groupe pyridinyle, et un groupe pyrimidinyle ;
un groupe méthyle ou un groupe méthoxy, chacun substitué par i) au moins un élément choisi parmi un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe bicyclo(1.1.1)pentyle, un groupe bicyclo(2.1.1)hexyle, un groupe bicyclo(2.2.1)heptyle, un groupe bicyclo(2.2.2)octyle, un groupe phényle, un groupe naphtyle, un groupe pyridinyle, et un groupe pyrimidinyle, et ii) au moins un deutérium ;
un groupe alkyle en C₂-C₂₀ ou un groupe alcoxy en C₂-C₂₀, chacun substitué par au moins un élément choisi parmi un deutérium, -F, -Cl, -Br, -I, -CD₃, -CD₂H, -CDH₂, -CF₃, -CF₂H,-CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₁₀ alkyle, un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe bicyclo(1.1.1)pentyle, un groupe bicyclo(2.1.1)hexyle, un groupe bicyclo(2.2.1)heptyle, un groupe bicyclo(2.2.2)octyle, un groupe phényle, un groupe (alkyle en C₁-C₂₀)phényle, un groupe biphényle, un groupe terphényle, un groupe naphtyle, un groupe pyridinyle, un groupe pyrimidinyle ; et
un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe bicyclo(1.1.1)pentyle, un groupe bicyclo(2.1.1)hexyle, un groupe bicyclo(2.2.1)heptyle, un groupe bicyclo(2.2.2)octyle, un groupe phényle, un groupe (alkyle en C₁-C₂₀)phényle, un groupe biphényle, un groupe terphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, un groupe azacarbazolyle, un groupe azadibenzofuranyle, ou un groupe azadibenzothiophényle, chacun non substitué ou substitué par au moins un élément choisi parmi un deutérium, -F, -Cl, -Br, -I, -CD₃, -CD₂H, -CDH₂, -CF₃, - CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₂₀, un groupe alcoxy en C₁-C₂₀, un groupe cyclopentyle, un groupe cyclohexyle, un groupe cycloheptyle, un groupe cycloctyle, un groupe adamantanyle, un groupe norbornanyle, un groupe norbornényle, un groupe cyclopentényle, un groupe cyclohexényle, un groupe cycloheptényle, un groupe bicyclo(1.1.1)pentyle, un groupe bicyclo(2.1.1)hexyle, un groupe bicyclo(2.2.1)heptyle, un groupe bicyclo(2.2.2)octyle, un groupe phényle, un groupe (alkyle en C₁-C₂₀)phényle, un groupe biphényle, un groupe terphényle, un groupe naphtyle, un groupe fluorényle, un groupe phénanthrényle, un groupe anthracényle, un groupe fluoranthényle, un groupe triphénylényle, un groupe pyrényle, un groupe chrysényle, un groupe pyrrolyle, un groupe thiophényle, un groupe furanyle, un groupe imidazolyle, un groupe pyrazolyle, un groupe thiazolyle, un groupe isothiazolyle, un groupe oxazolyle, un groupe isoxazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe quinoxalinyle, un groupe quinazolinyle, un groupe cinnolinyle, un groupe carbazolyle, un groupe phénanthrolinyle, un groupe benzimidazolyle, un groupe benzofuranyle, un groupe benzothiophényle, un groupe isobenzothiazolyle, un groupe benzoxazolyle, un groupe isobenzoxazolyle, un groupe triazolyle, un groupe tétrazolyle, un groupe oxadiazolyle, un groupe triazinyle, un groupe dibenzofuranyle, un groupe dibenzothiophényle, un groupe benzocarbazolyle, un groupe dibenzocarbazolyle, un groupe imidazopyridinyle, un groupe imidazopyrimidinyle, un groupe azacarbazolyle, un groupe azadibenzofuranyle, et un groupe azadibenzothiophényle.

5. Composé organométallique selon l'une quelconque des revendications 1 à 4, dans lequel,
dans les Formules 2A et 2B, Z₁ à Z₃, R₁₁, R₁₃, et R₁₄ sont chacun indépendamment choisis parmi un hydrogène, un deutérium, -F, un groupe cyano, un groupe nitro, -SF₅, -CH3, - CD₃, -CD₂H, -CDH₂, -CF₃, -CF₂H, -CFH₂, des groupes représentés par les Formules 9-1 à 9-66, des groupes représentés par les Formules 9-1 à 9-66 dans lequel au moins un hydrogène est substitué par un deutérium, des groupes représentés par les Formules 10-1 à 10-249, et des groupes représentés par les Formules 10-1 à 10-249 dans lequel au moins un hydrogène est substitué par un deutérium, -N(Q₁)(Q₂), -Si(Q₃)(Q₄)(Q₅), et -Ge(Q₃)(Q₄)(Q₅), dans lequel Q₁ à Q₅ sont chacun indépendamment les mêmes que dans la revendication 1, et
R₁₂ dans la Formule 2B est choisi parmi des groupes représentés par les Formules 9-1 à 9-66, des groupes représentés par les Formules 9-1 à 9-66 dans lequel au moins un hydrogène est substitué par un deutérium, des groupes représentés par les Formules 10-1 à 10-249, des groupes représentés par les Formules 10-1 à 10-249 dans lequel au moins un hydrogène est substitué par un deutérium : 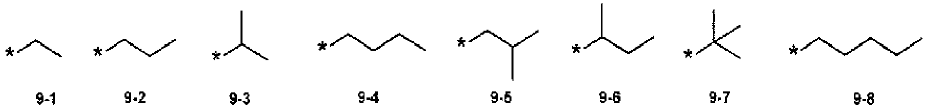 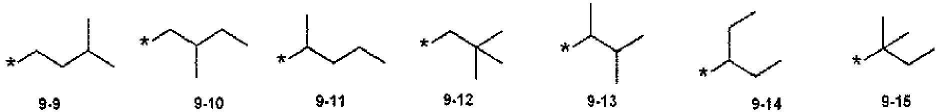 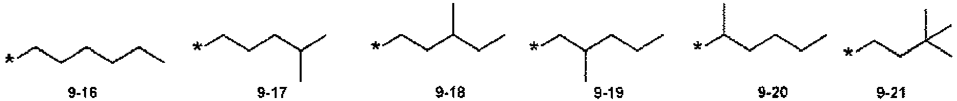 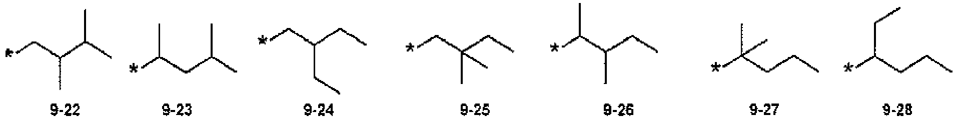 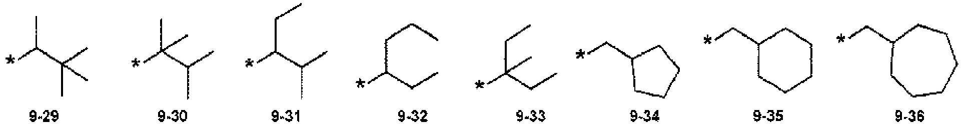 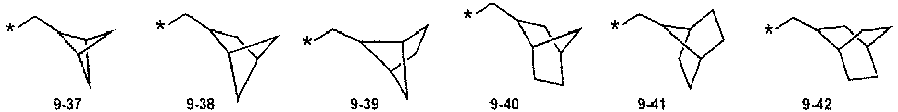 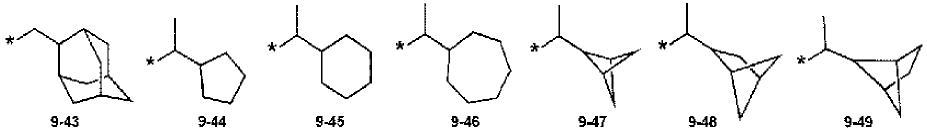 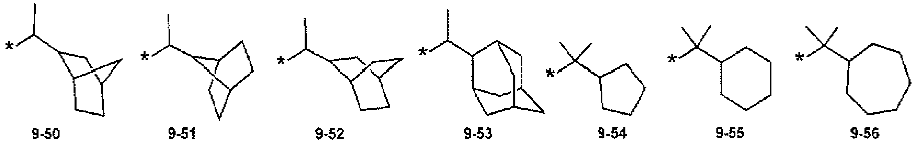 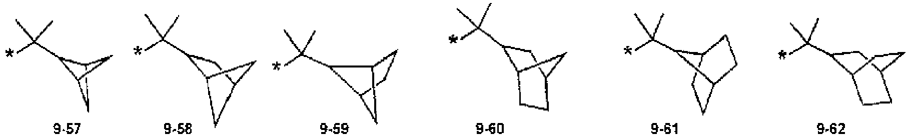 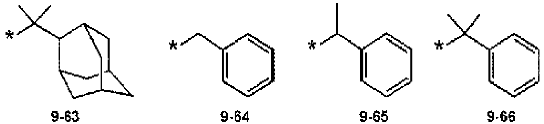 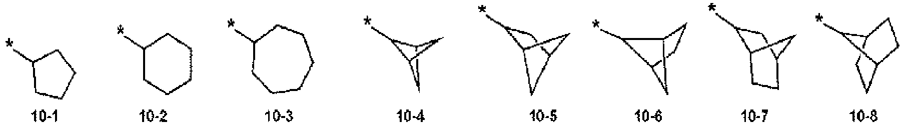 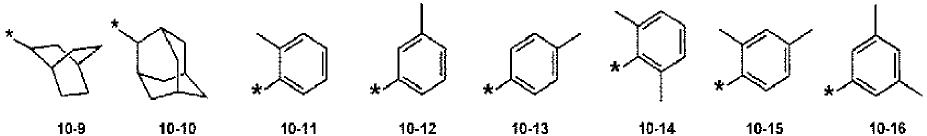 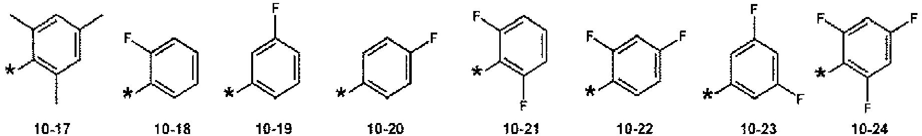 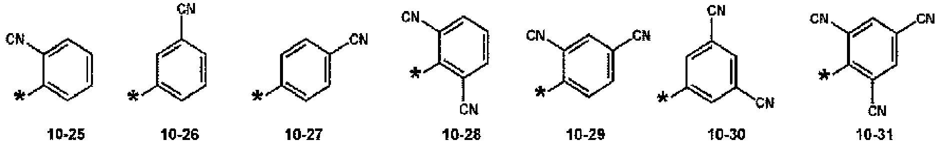 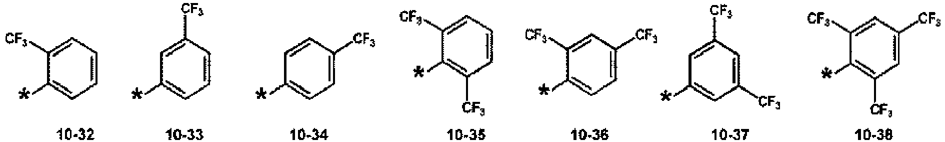 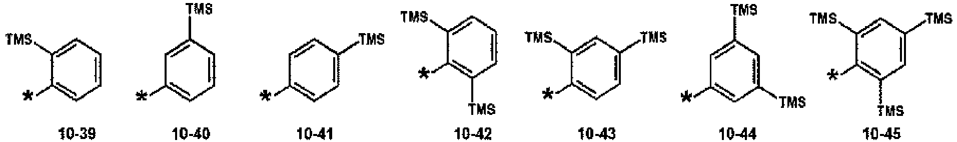 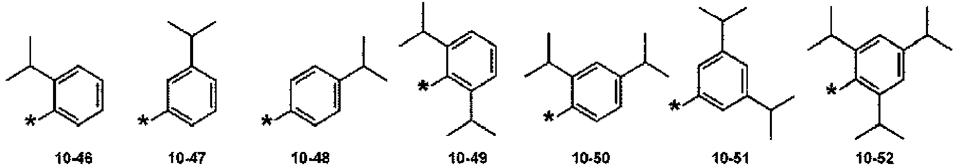 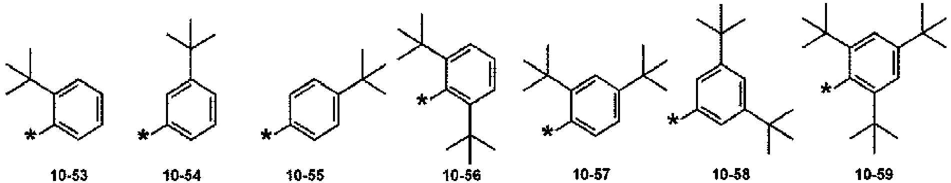 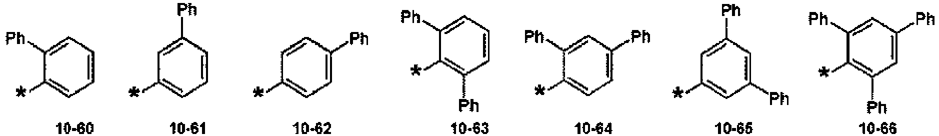 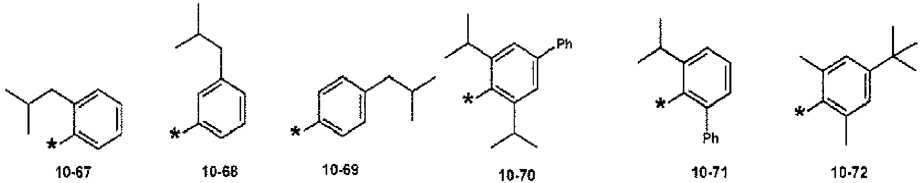 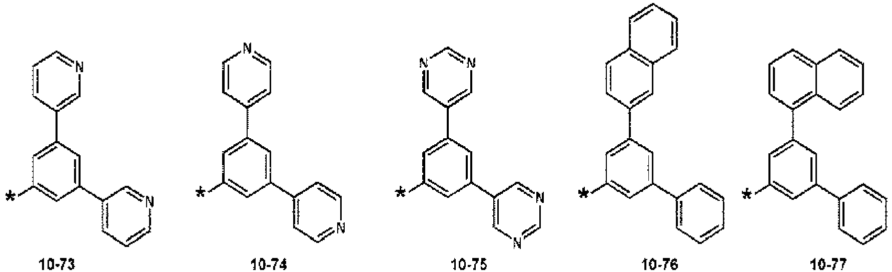 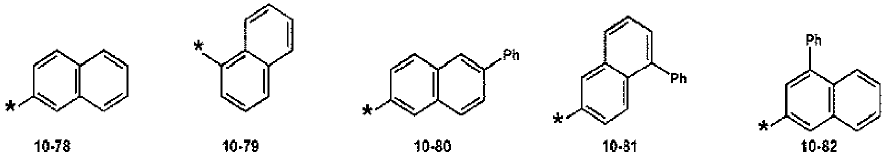 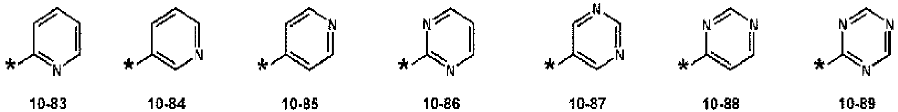 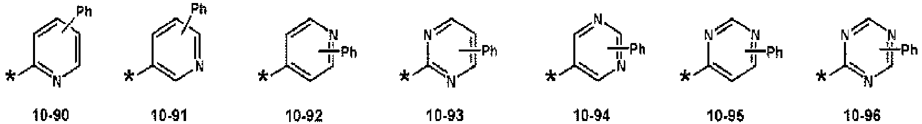 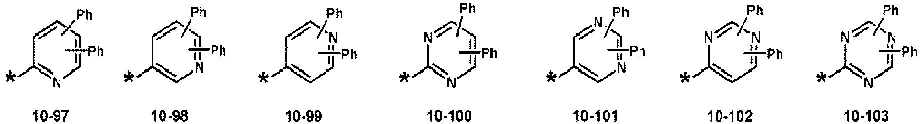 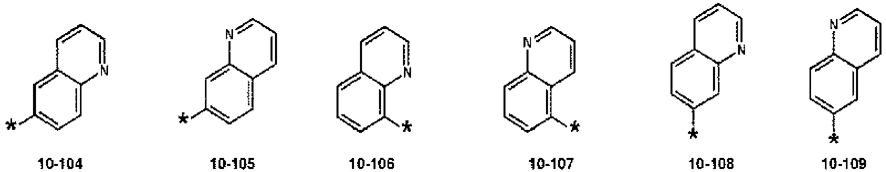 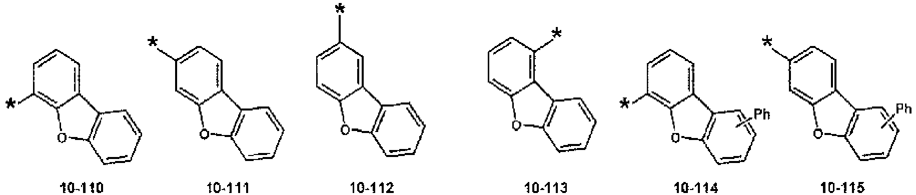 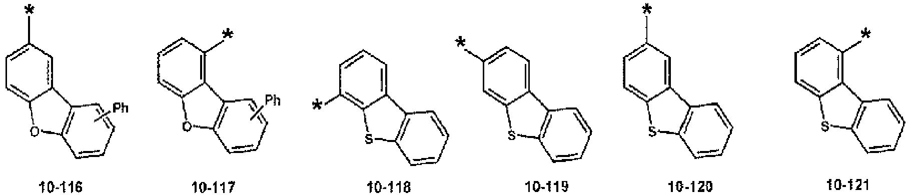 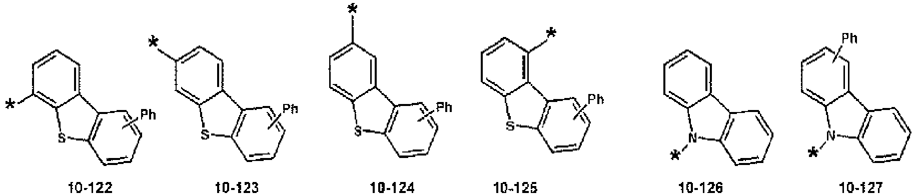 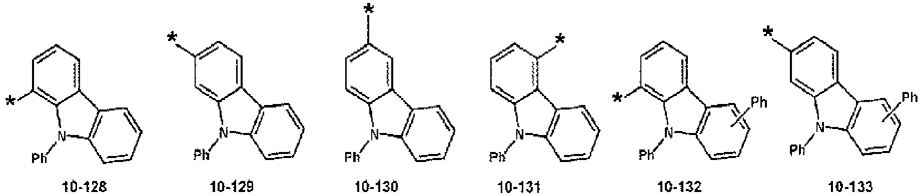 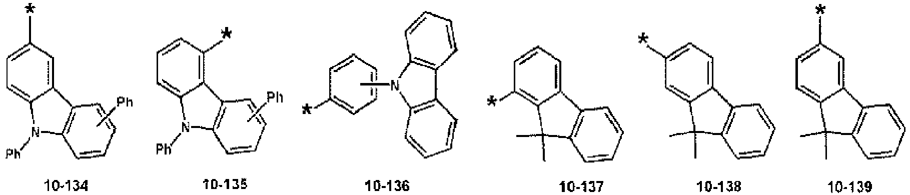 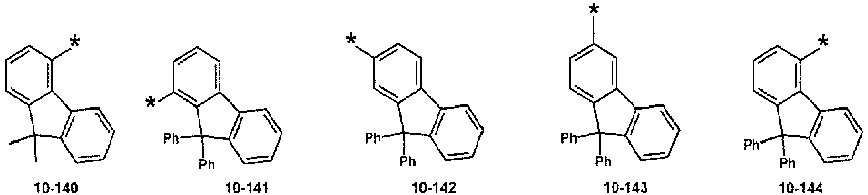 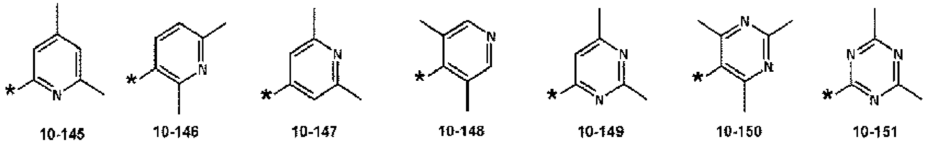 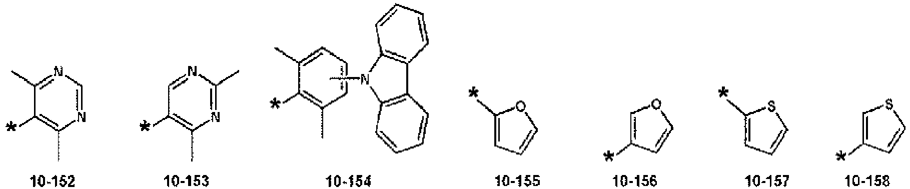 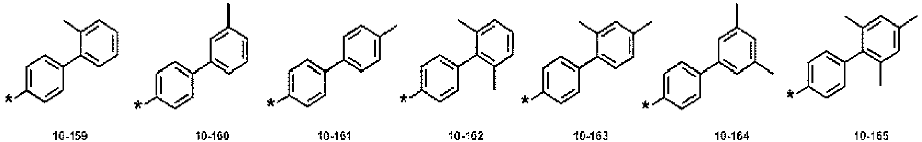 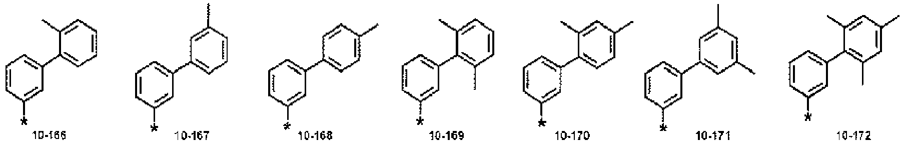 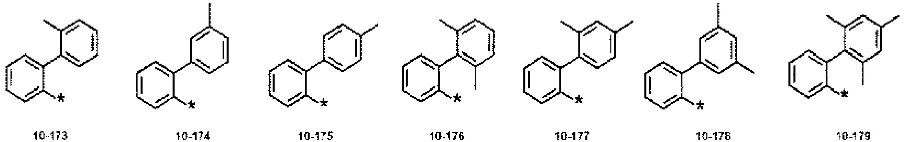 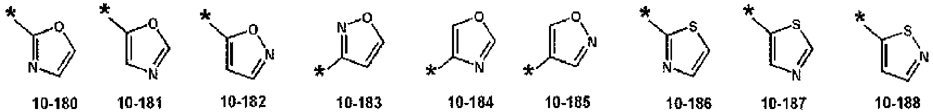 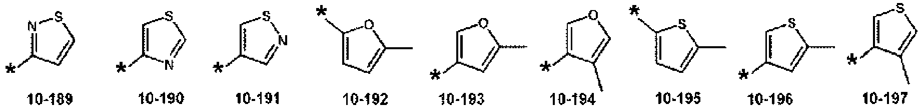 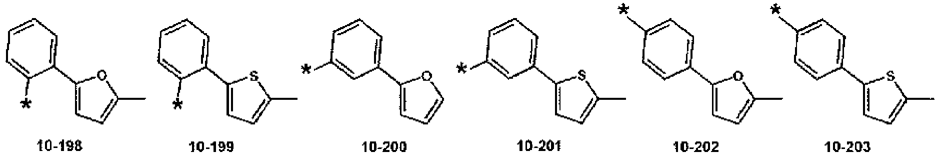 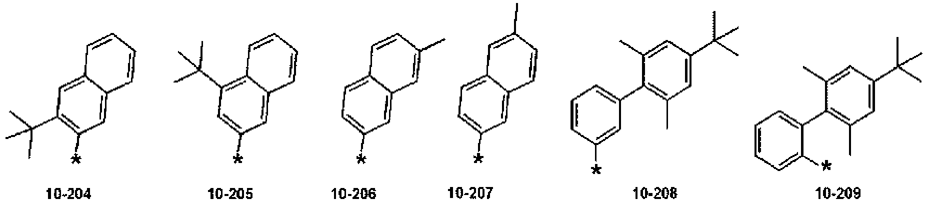 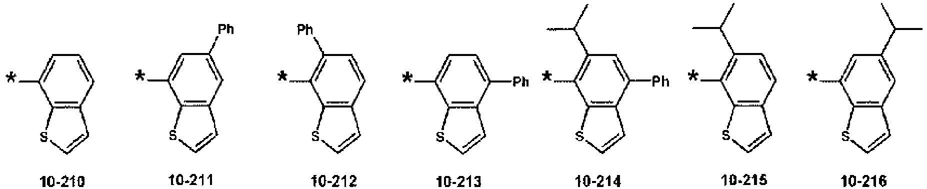 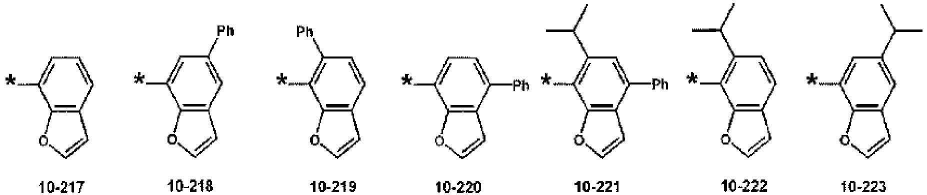 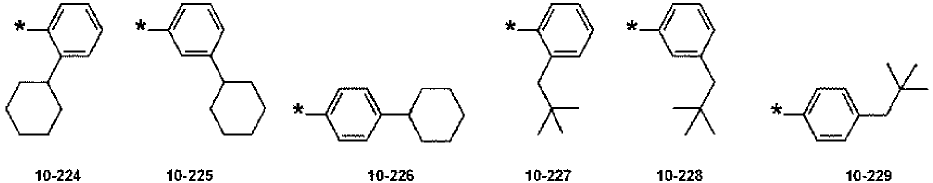 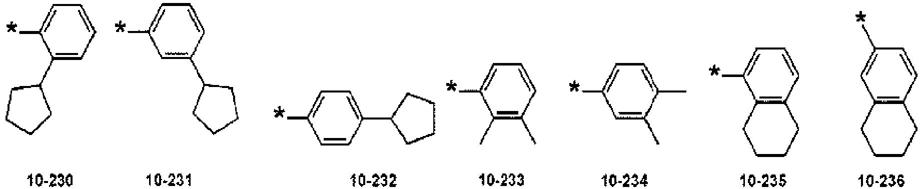 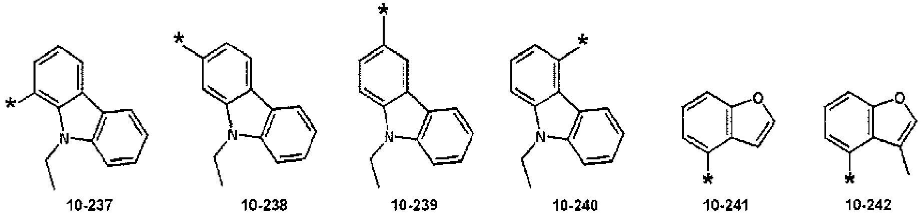 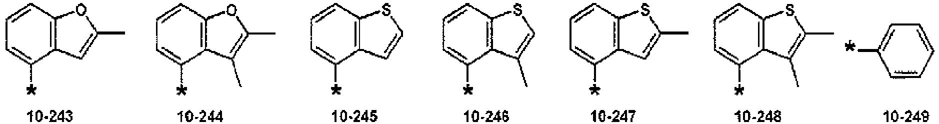 ,
dans lequel, dans les Formules 9-1 à 9-66 et 10-1 à 10-249, * indique un site de liaison à un atome voisin, Ph indique un groupe phényle, et TMS indique un groupe triméthylsilyle.

6. Composé organométallique selon l'une quelconque des revendications 1 à 5, dans lequel,
dans la Formule 2A, Z₁ est choisi parmi un hydrogène, un deutérium, -F, un groupe cyano, un groupe alkyle en C₁-C₆₀ substitué ou non substitué, un groupe alcoxy en C₁-C₆₀ substitué ou non substitué, un groupe cycloalkyle en C₃-C₁₀ substitué ou non substitué, un groupe hétérocycloalkyle en C₁-C₁₀ substitué ou non substitué, -N(Q₁)(Q₂), -Si(Q₃)(Q₄)(Q₅), et-Ge(Q₃)(Q₄)(Q₅), dans lequel Q₁ à Q₅ sont chacun indépendamment les mêmes que dans la revendication 1.

7. Composé organométallique selon l'une quelconque des revendications 1 à 6, dans lequel c1 dans la Formule 2A vaut 1 ou 2 ; et/ou
dans lequel
le groupe représenté par 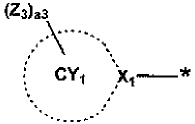 dans la Formule 2A est un groupe représenté par un groupe choisi parmi les Formules CY1(1) à CY1(20) : 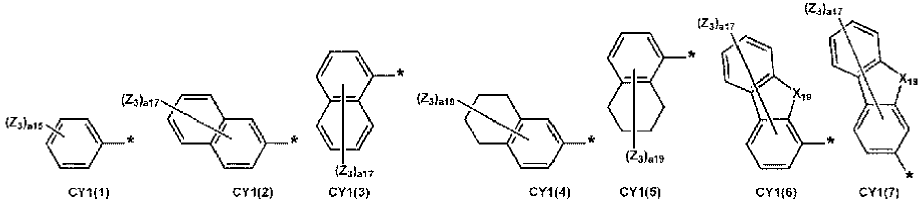 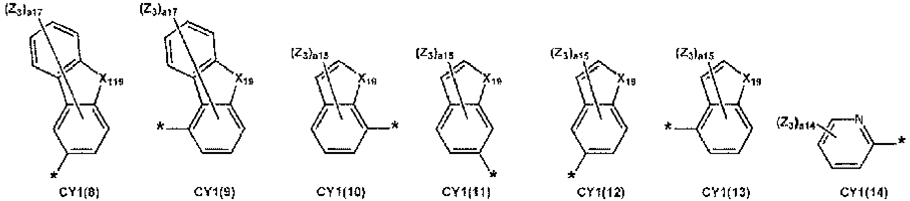 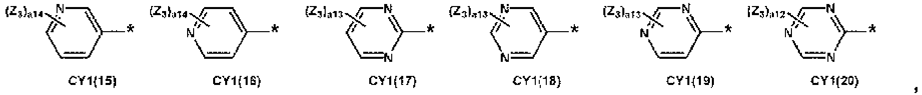 dans lequel, dans les Formules CY1(1) à CY1(20),
Z₃ est le même que celui décrit dans la revendication 1,
X₁₉ est O, S, N(Z₄), C(Z₄)(Z₅), ou Si(Z₄)(Z₅),
Z₄ et Z₅ sont chacun indépendamment les mêmes que ceux décrits par rapport à Z₃,
a12 est un nombre entier allant de 0 à 2,
a13 est un nombre entier allant de 0 à 3,
a14 est un nombre entier allant de 0 à 4,
a15 est un nombre entier allant de 0 à 5,
a17 est un nombre entier allant de 0 à 7,
a19 est un nombre entier allant de 0 à 9, et
* indique un site de liaison à un atome de carbone voisin.

8. Composé organométallique selon l'une quelconque des revendications 1 à 7, dans lequel
le groupe représenté par 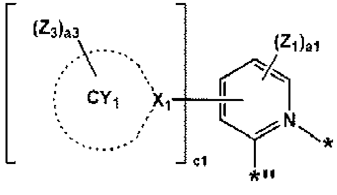 dans la Formule 2A est un groupe représenté par un groupe choisi parmi les Formules CY1-1 à CY1-56 : 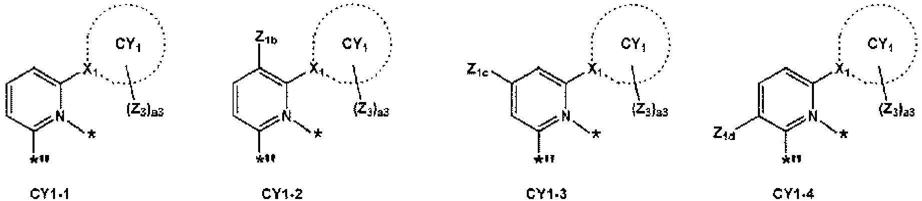 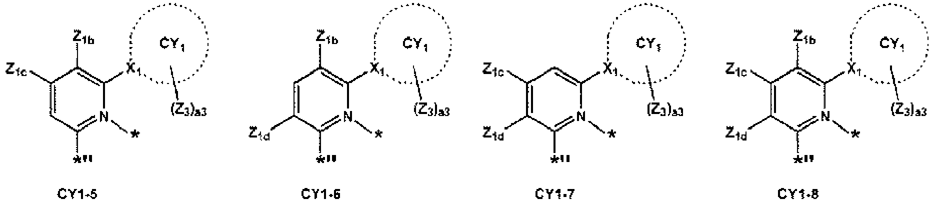 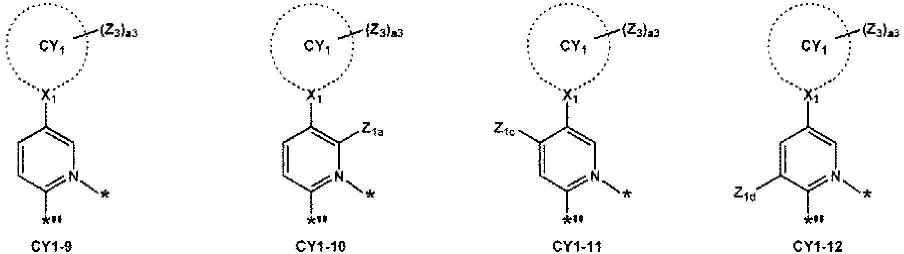 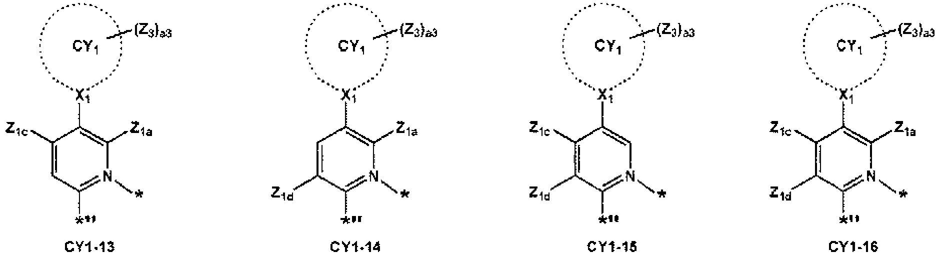 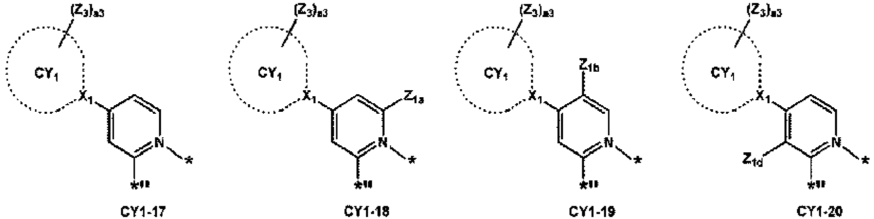 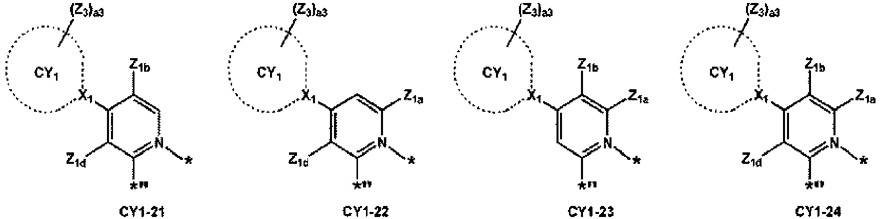 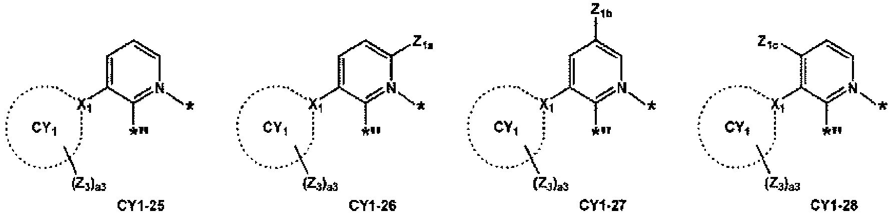 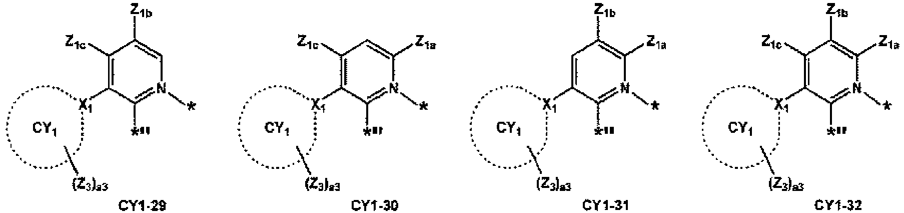 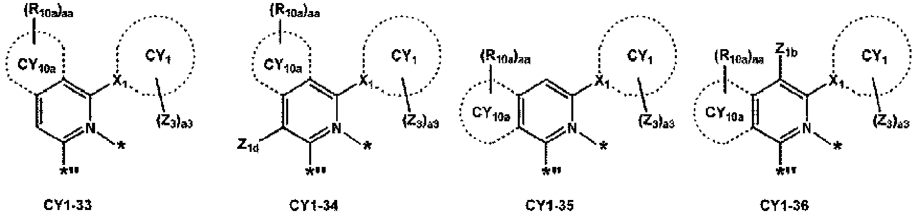 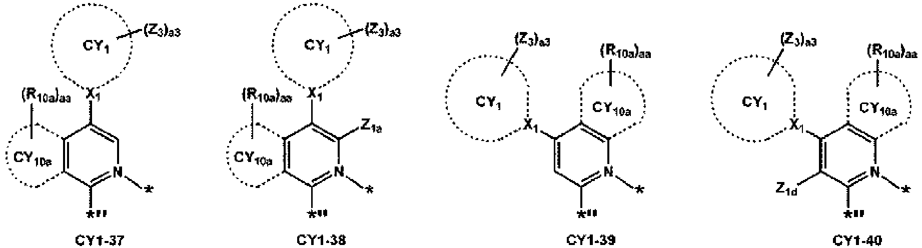 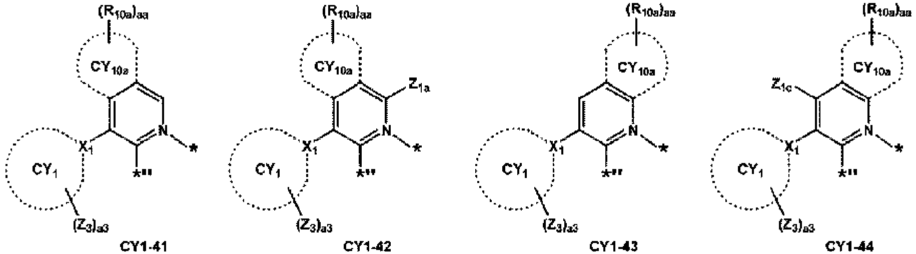 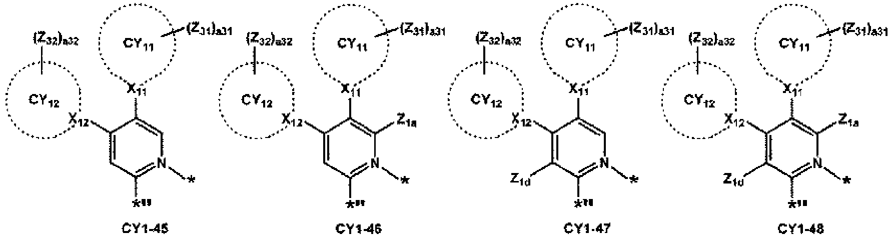 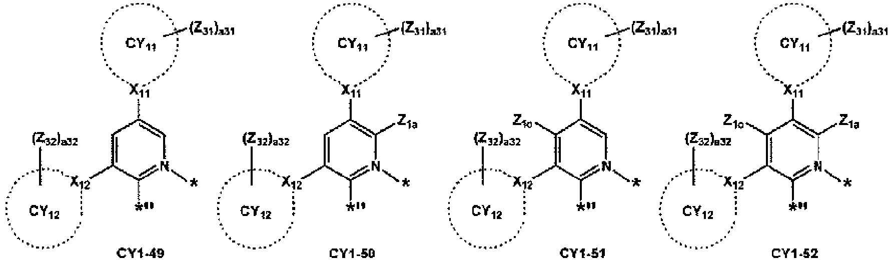 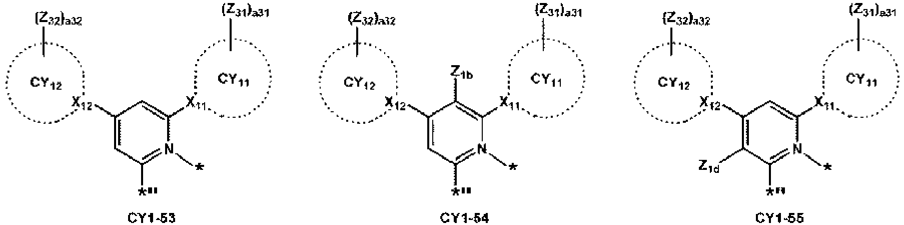 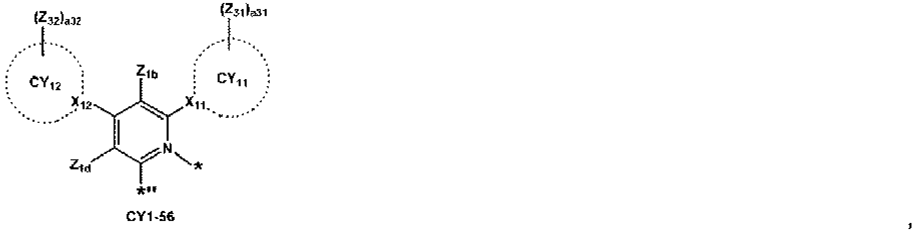 dans lequel, dans les Formules CY1-1 à CY1-56,
Xi, le noyau CY₁, Z₃, et a3 sont chacun indépendamment les mêmes que ceux décrits dans la revendication 1,
Z₁ₐ à Z_{1d} sont chacun indépendamment les mêmes que ceux décrits par rapport à Z₁ dans la revendication 1, dans lequel Z₁ₐ à Z_{1d} ne sont pas un hydrogène,
X₁₁ et X₁₂ sont chacun indépendamment les mêmes que ceux décrits par rapport à X₁ dans la revendication 1,
le noyau CY₁₁ et le noyau CY₁₂ sont chacun indépendamment les mêmes que ceux décrits par rapport au noyau CY₁ dans la revendication 1,
Z₃₁ et Z₃₂ sont chacun indépendamment les mêmes que ceux décrits par rapport à Z₃ dans la revendication 1,
a31 et a32 sont chacun indépendamment les mêmes que ceux décrits par rapport à a3 dans la revendication 1,
le noyau CY₁₀ₐ est un groupe carbocyclique en C₅-C₃₀ ou un groupe hétérocyclique en C₁-C₃₀,
R₁₀ₐ est le même que celui décrit dans la revendication 1,
aa est un nombre entier allant de 0 à 10,
* indique un site de liaison à M dans la Formule 1, et
*" indique un site de liaison au noyau CY₂ dans la Formule 2A.

9. Composé organométallique selon l'une quelconque des revendications 1 à 8, dans lequel
le groupe représenté par 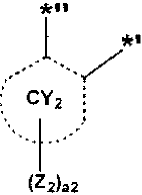 dans la Formule 2A est un groupe représenté par un groupe choisi parmi les Formules CY2-1 à CY2-64 : 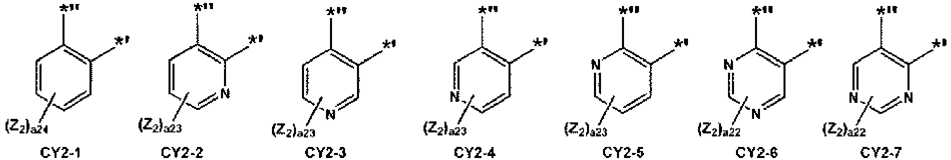 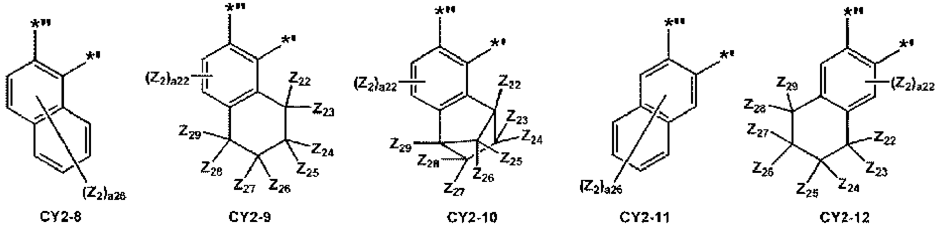 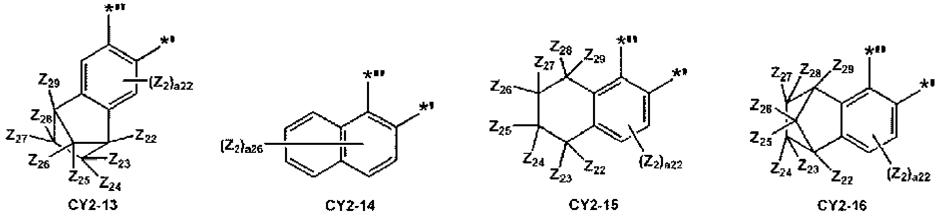 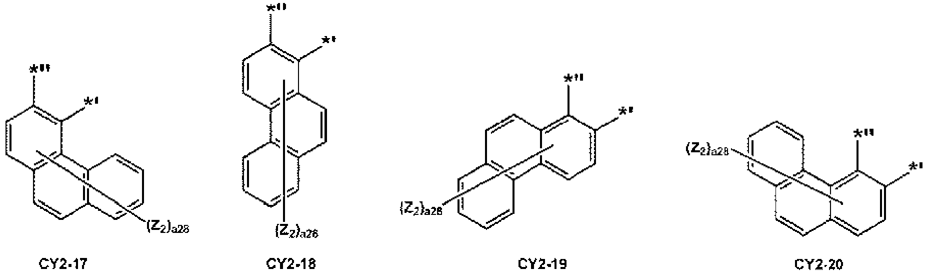 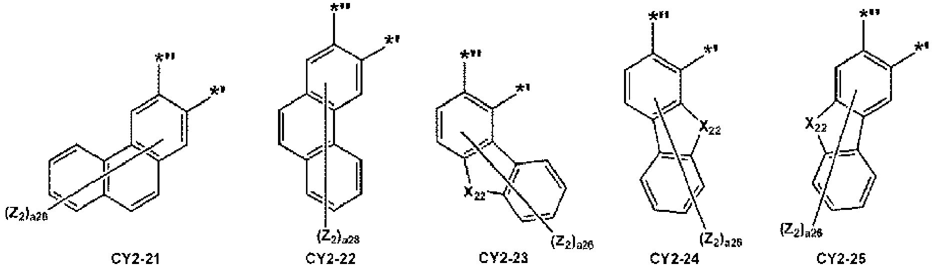 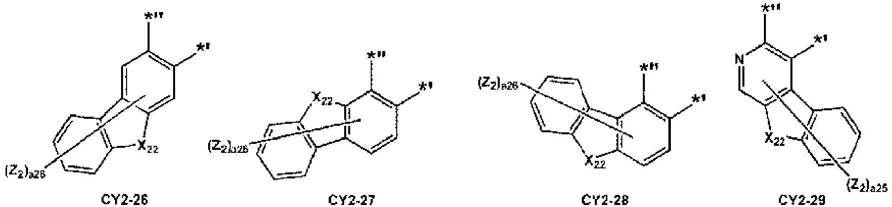 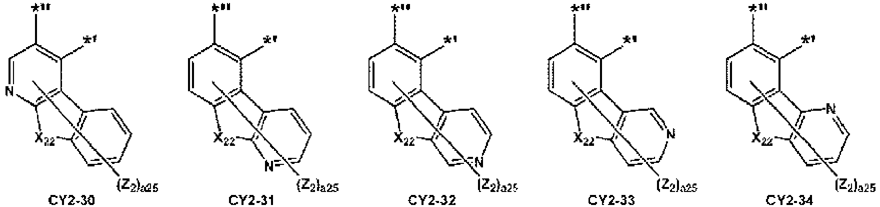 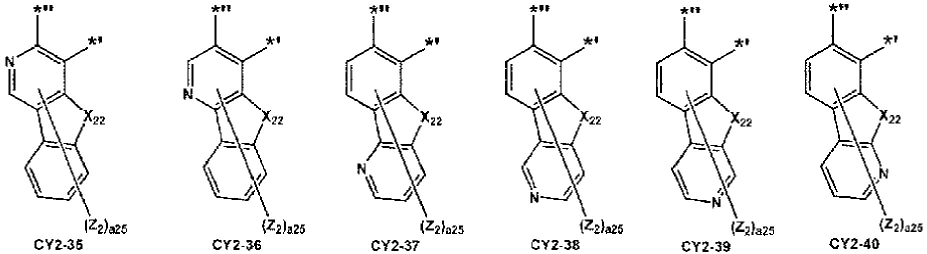 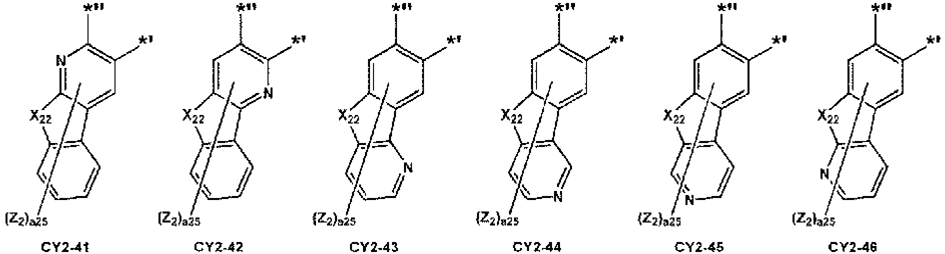 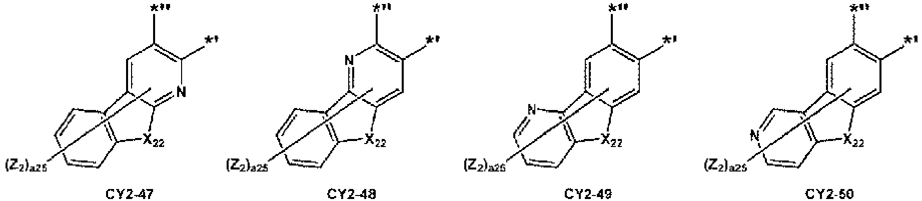 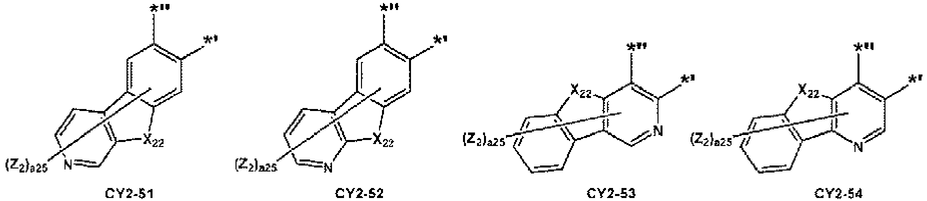 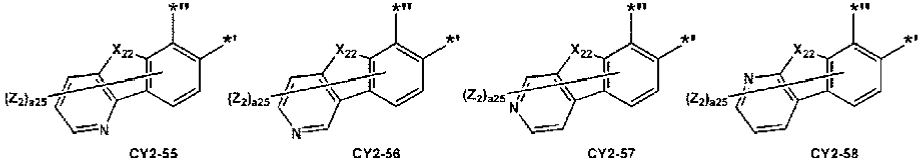 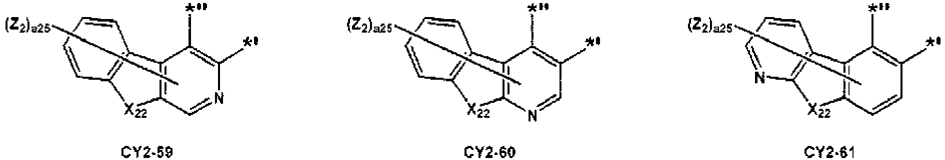 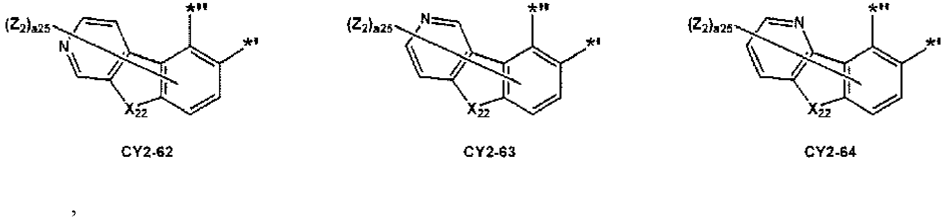 dans lequel, dans les Formules CY2-1 à CY2-64,
Z₂ est le même que celui décrit dans la revendication 1,
X₂₂ est C(Z₂₂)(Z₂₃), N(Z₂₂), O, S, ou Si(Z₂₂)(Z₂₃),
Z₂₂ à Z₂₉ sont chacun indépendamment les mêmes que ceux décrits par rapport à Z₂ dans la revendication 1,
a28 est un nombre entier allant de 0 à 8,
a26 est un nombre entier allant de 0 à 6,
a25 est un nombre entier allant de 0 à 5,
a24 est un nombre entier allant de 0 à 4,
a23 est un nombre entier allant de 0 à 3,
a22 est un nombre entier allant de 0 à 2,
*" indique un site de liaison à un atome de carbone d'un noyau pyridine voisin dans la Formule 2A, et
*' indique un site de liaison à M dans la Formule 1.

10. Composé organométallique selon l'une quelconque des revendications 1 à 9, dans lequel
le groupe représenté par 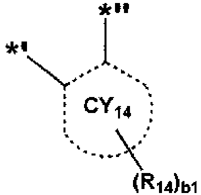 dans la Formule 2B est un groupe représenté par un groupe choisi parmi les Formules CY14-1 à CY14-64 : 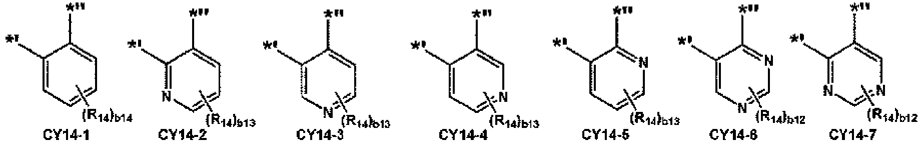 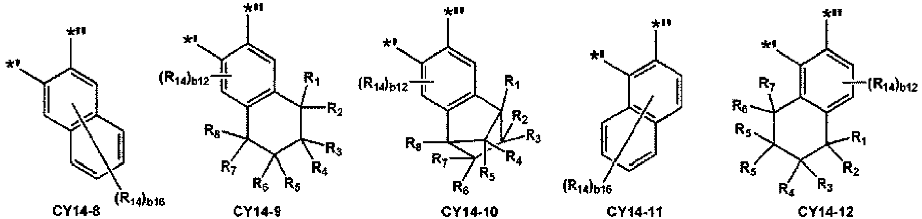 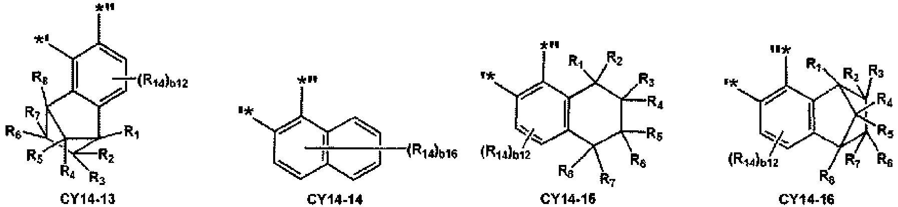 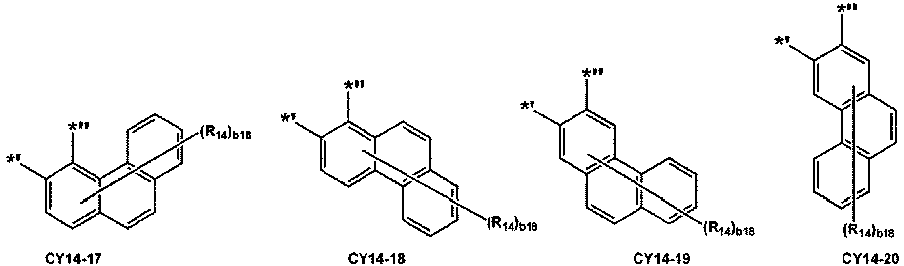 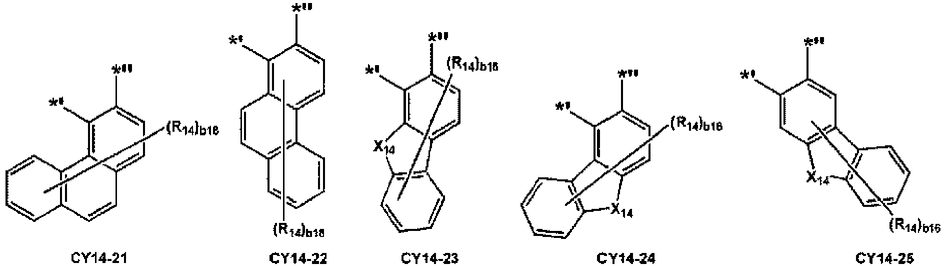 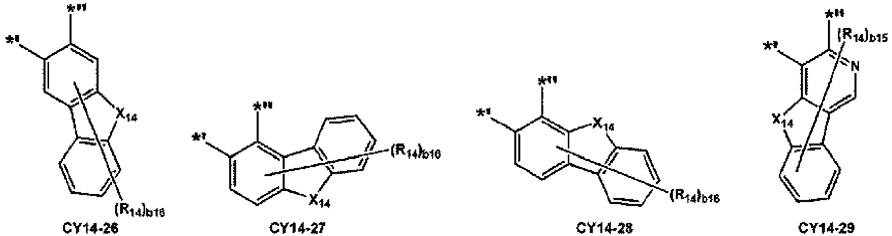 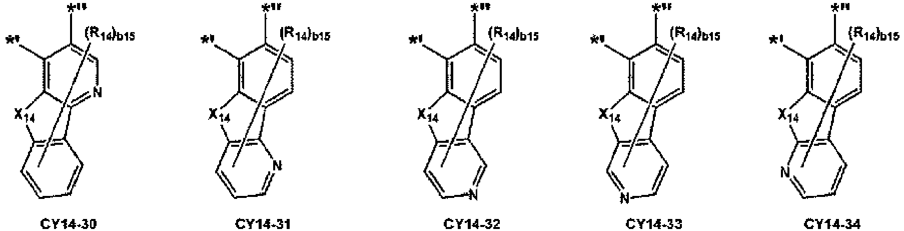 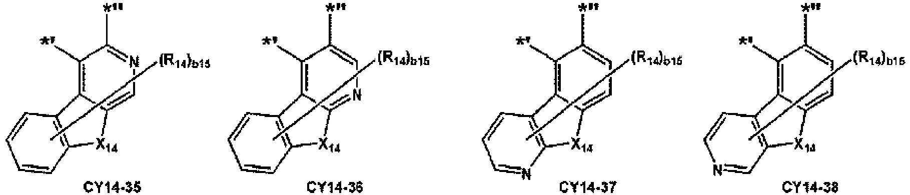 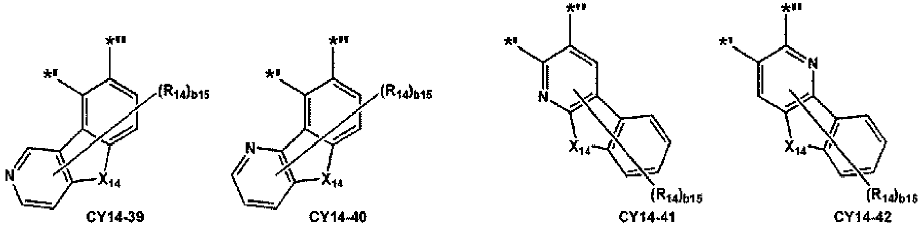 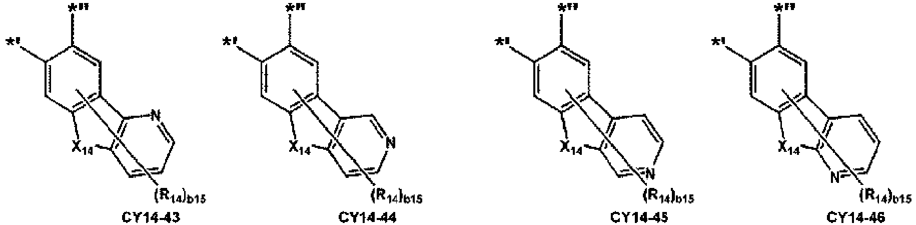 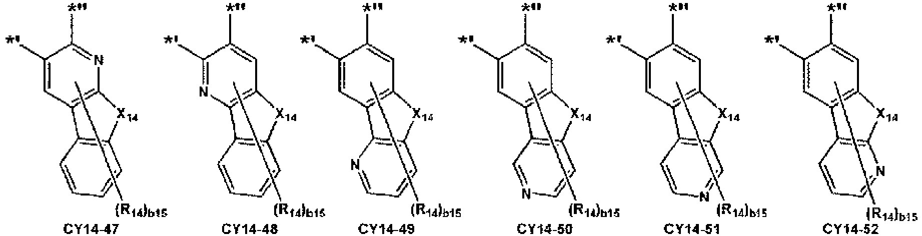 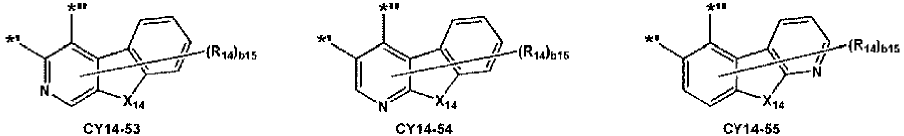 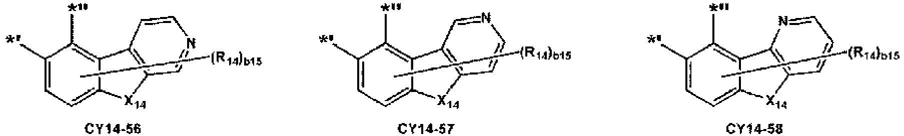 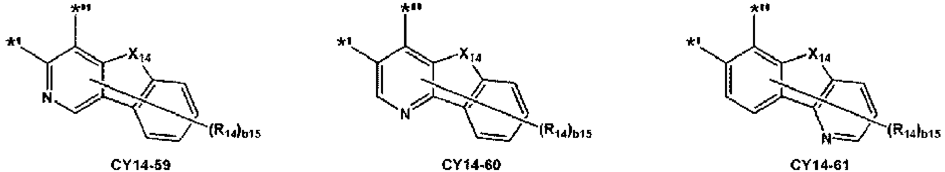 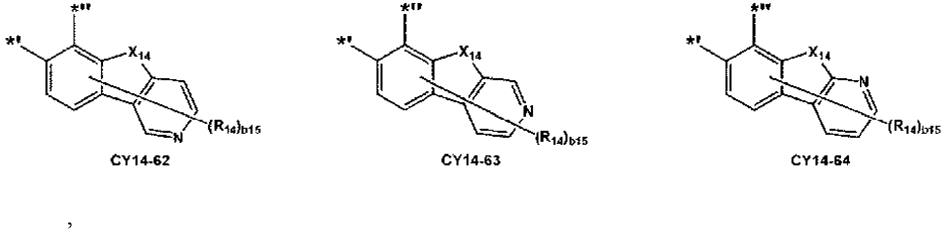 dans lequel, dans les Formules CY14-1 à CY14-64,
R₁₄ est le même que celui décrit dans la revendication 1,
X₁₄ est C(R₁)(R₂), N(R₁), O, S, ou Si(R₁)(R₂),
R₁ à R₈ sont chacun indépendamment les mêmes que ceux décrits par rapport à R₁₄ dans la revendication 1,
b 18 est un nombre entier allant de 0 à 8,
b 16 est un nombre entier allant de 0 à 6,
b 15 est un nombre entier allant de 0 à 5,
b14 est un nombre entier allant de 0 à 4,
b13 est un nombre entier allant de 0 à 3,
b 12 est un nombre entier allant de 0 à 2,
*" indique un site de liaison à un atome de carbone d'un noyau pyridine voisin dans la Formule 2B, et
*' indique un site de liaison à M dans la Formule 1.

11. Composé organométallique selon l'une quelconque des revendications 1 à 10, dans lequel le composé organométallique est représenté par la Formule 1A : 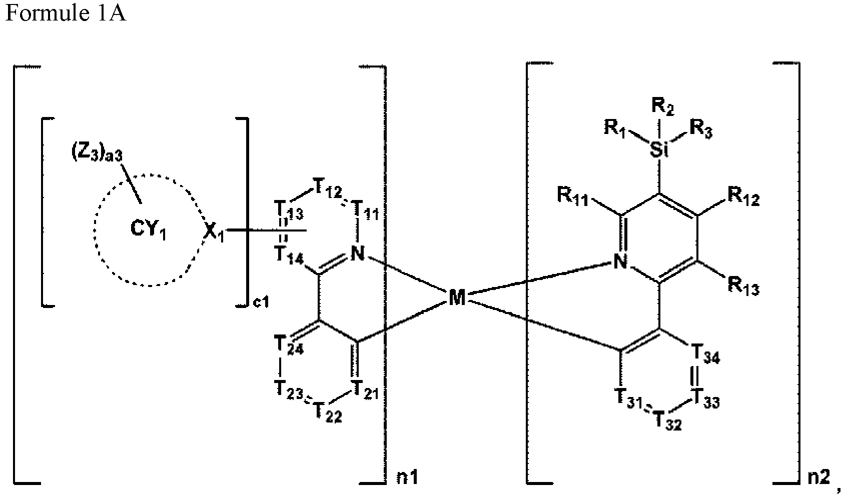 dans lequel, dans la Formule 1A,
M, n1, n2, X₁, le noyau CY₁, R₁ à R₃, Z₃, R₁₁ à R₁₃, et c1 sont chacun indépendamment les mêmes que ceux décrits dans la revendication 1,
Tu est N, C(Z₁ₐ), ou lié par un carbone au noyau CY₁, T₁₂ est N, C(Z_{1b}), ou lié par un carbone au noyau CY₁, T₁₃ est N, C(Z_{1c}), ou lié par un carbone au noyau CY₁, T₁₄ est N, C(Z_{1d}), ou lié par un carbone au noyau CY₁, au moins un choisi parmi T₁₁ à T₁₄ est lié par un carbone au noyau CY₁, et Z₁ₐ à Z_{1d} sont chacun indépendamment les mêmes que ceux décrits par rapport à Z₁ dans la revendication 1,
T₂₁ est N ou C(Z₂ₐ), T₂₂ est N ou C(Z_{2b}), T₂₃ est N ou C(Z_{2c}), T₂₄ est N ou C(Z_{2d}), et Z₂ₐ à Z_{2d} sont chacun indépendamment les mêmes que ceux décrits par rapport à Z₂ dans la revendication 1,
T₃₁ est N ou C(R₁₄ₐ), T₃₂ est N ou C(R_{14b}), T₃₃ est N ou C(R_{14c}), T₃₄ est N ou C(R_{14d}), et R₁₄ₐ à R_{14d} sont chacun indépendamment les mêmes que ceux décrits par rapport à R₁₄ dans la revendication 1,
deux groupes Z₁ₐ à Z_{1d} ou plus sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
deux groupes Z₂ₐ à Z_{2d} ou plus sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
quand a3 est deux ou plus, deux groupes Z₃ ou plus sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
R₁₂ et R₁₃ sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
deux groupes R₁₄ₐ à R_{14d} ou plus sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
deux groupes Z₁ₐ à Z_{1d}, Z₂ₐ à Z_{2d}, R₁₁ à R₁₃, et R₁₄ₐ à R_{14d} ou plus sont facultativement liés pour former un groupe carbocyclique en C₅-C₃₀ qui est non substitué ou substitué par au moins un R₁₀ₐ ou un groupe hétérocyclique en C₁-C₃₀ qui est non substitué ou substitué par au moins un R₁₀ₐ,
R₁₀ₐ est le même que celui décrit dans la revendication 1,
les liaisons entre N et M sont des liaisons de coordination, et
les liaisons entre C et M sont des liaisons covalentes.

12. Composé organométallique selon la revendication 1, dans lequel le composé organométallique est un composé choisi parmi les composés 1 à 420 :

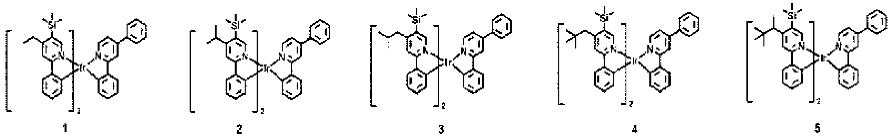

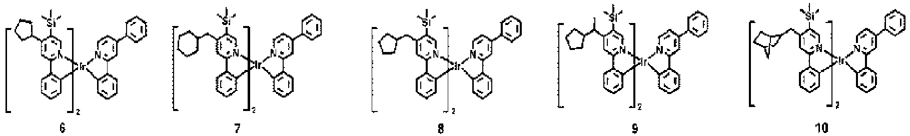

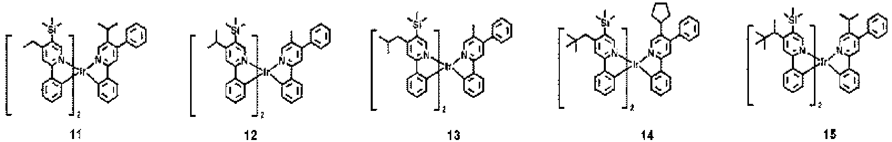

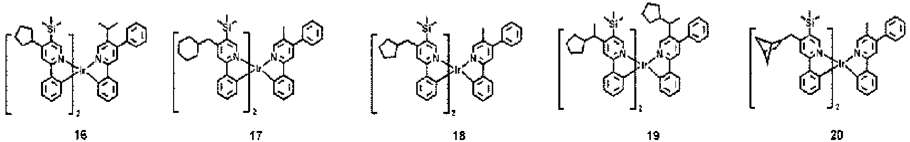

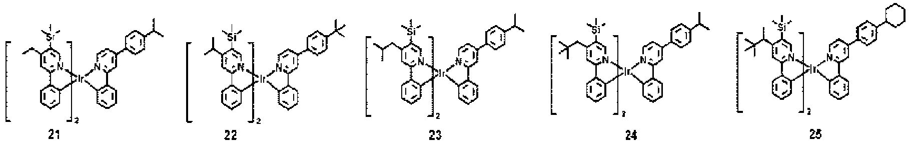

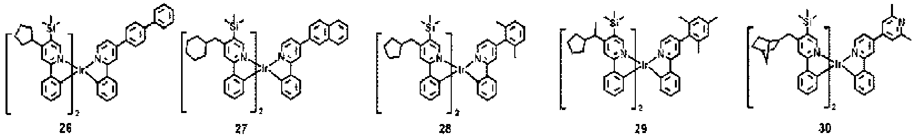

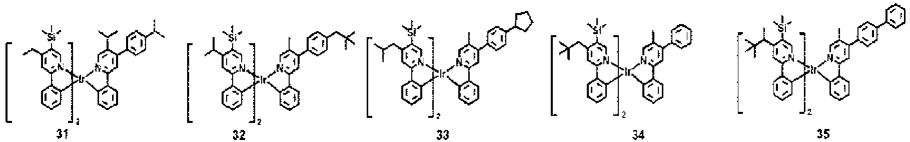

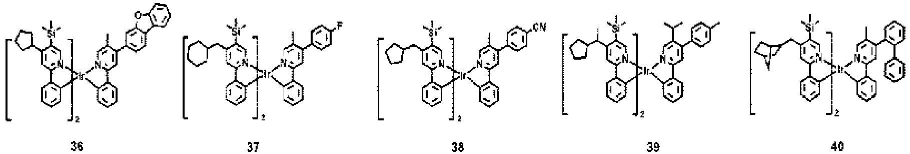

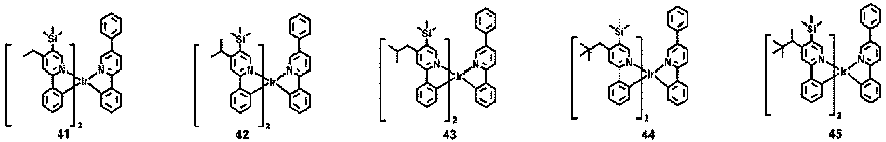

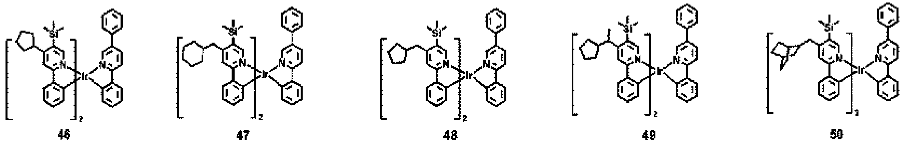

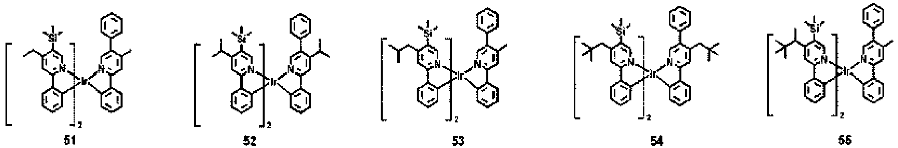

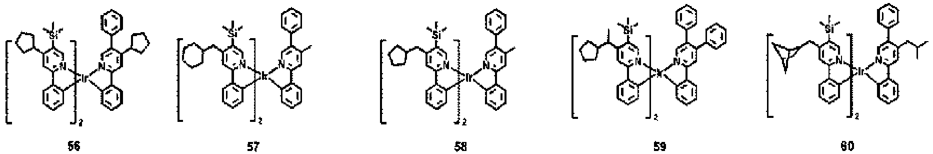

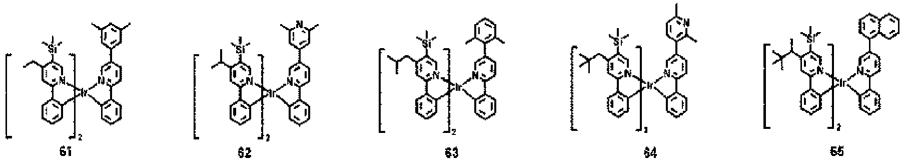

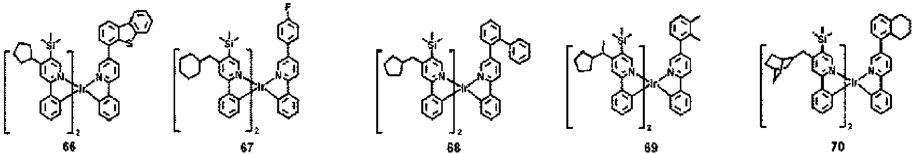

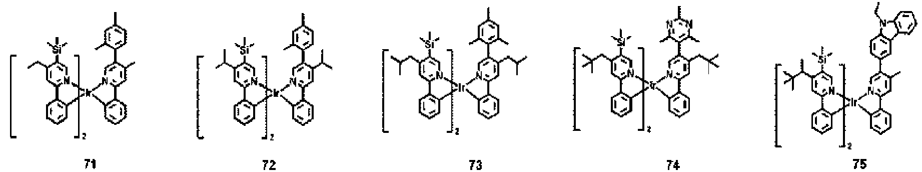

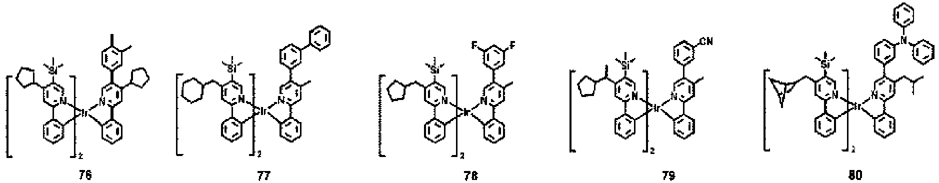

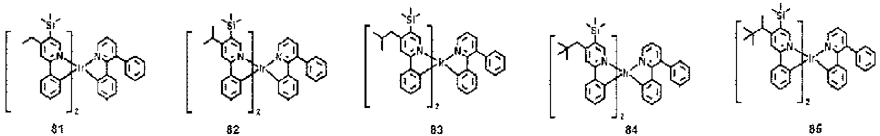

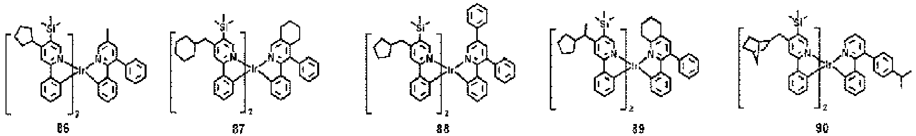

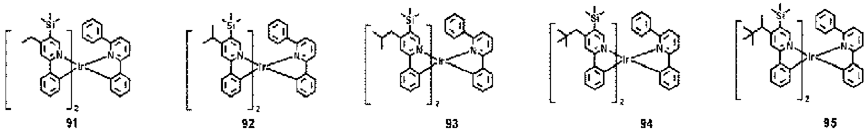

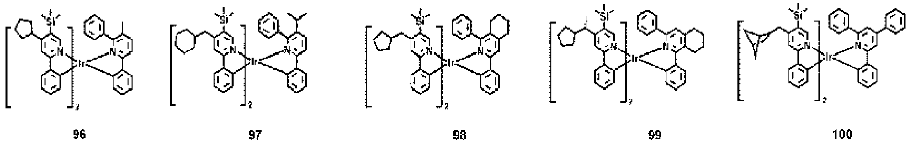

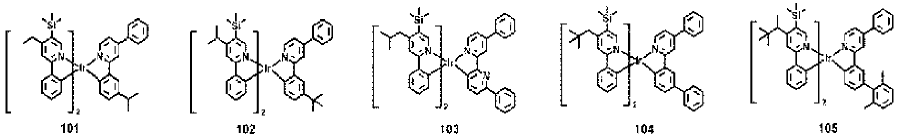

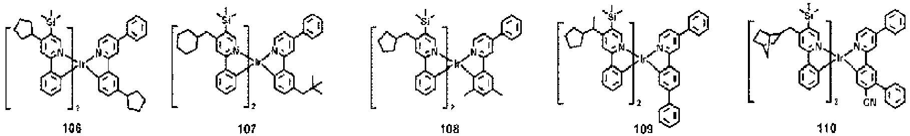

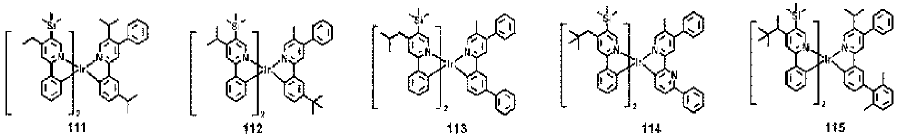

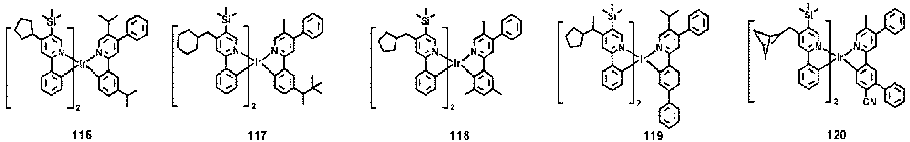

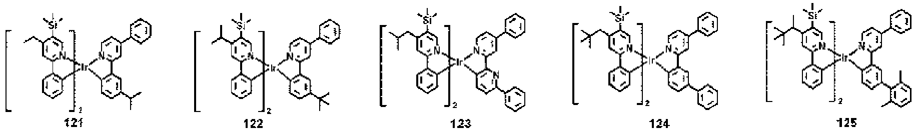

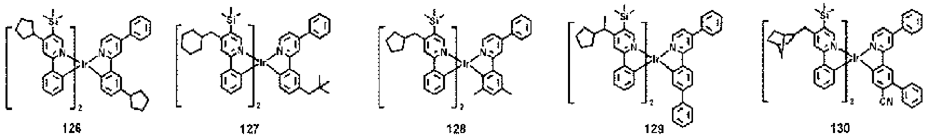

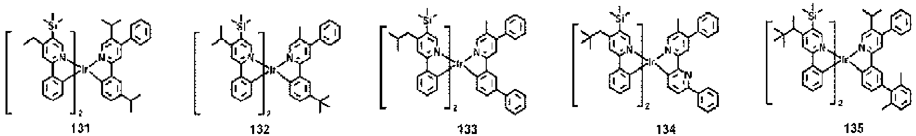

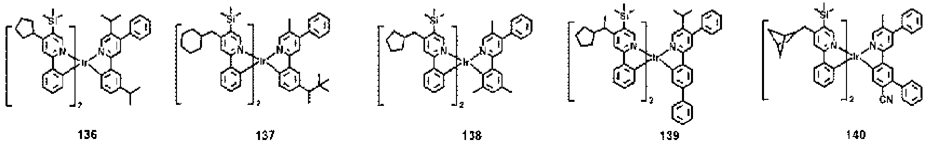

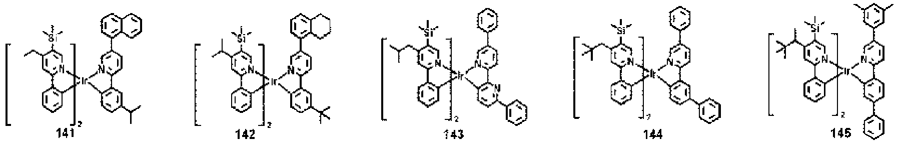

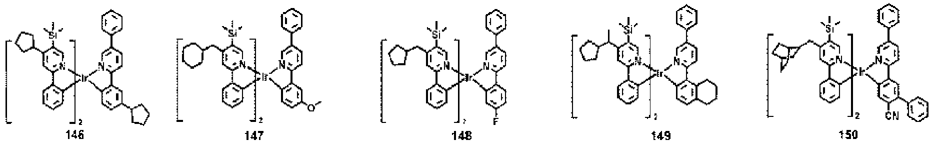

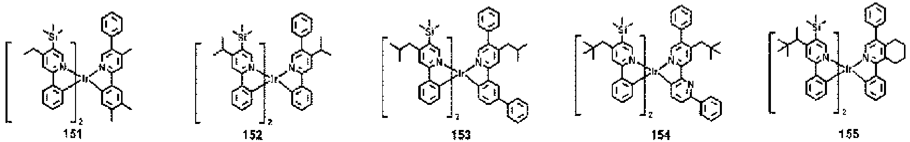

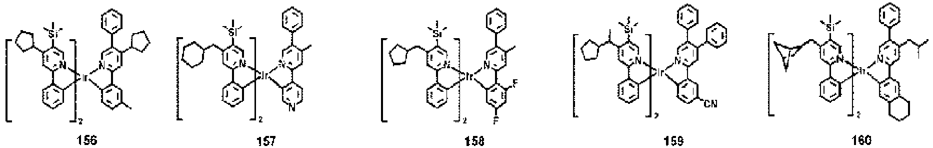

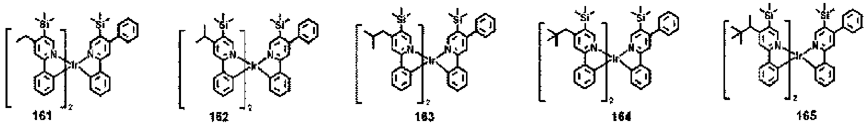

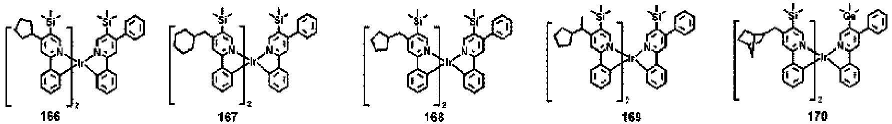

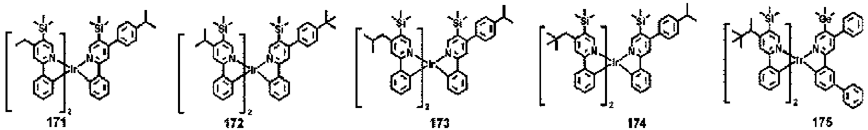

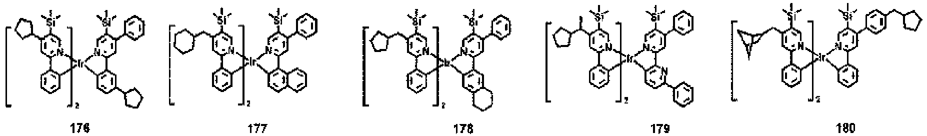

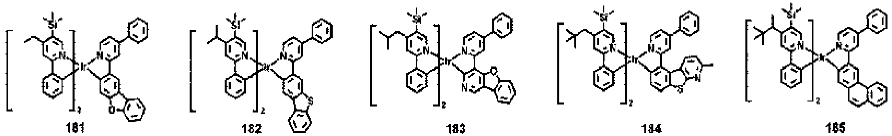

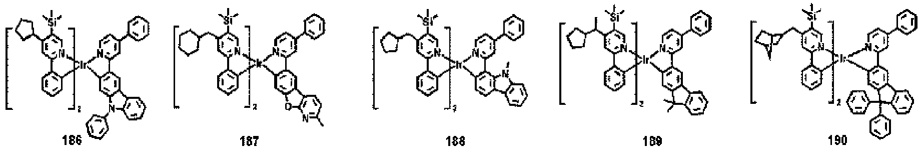

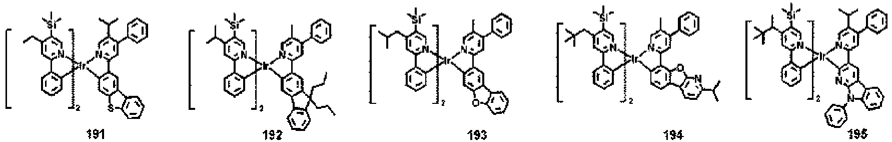

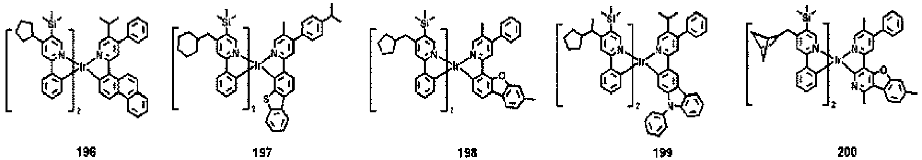

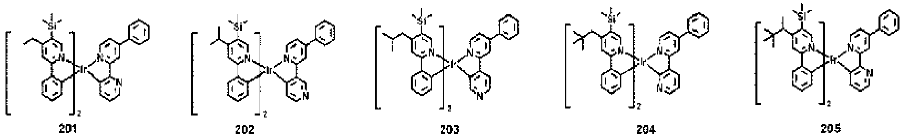

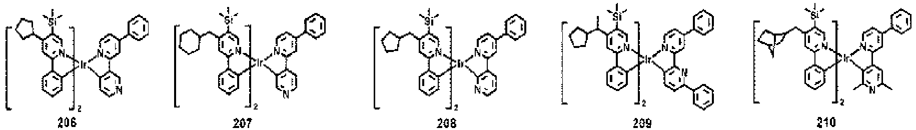

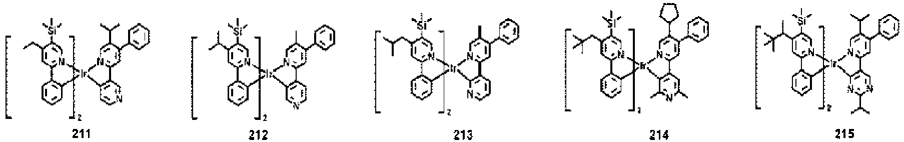

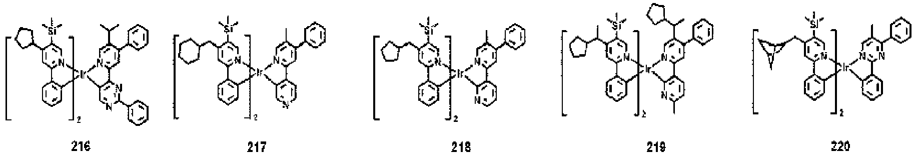

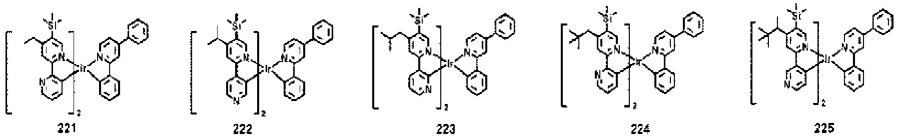

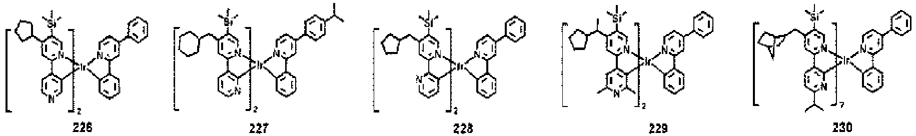

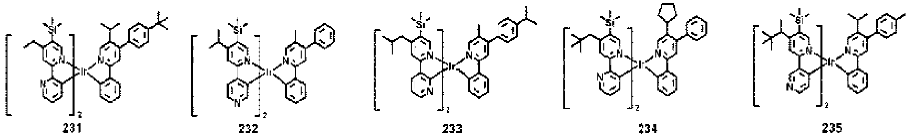

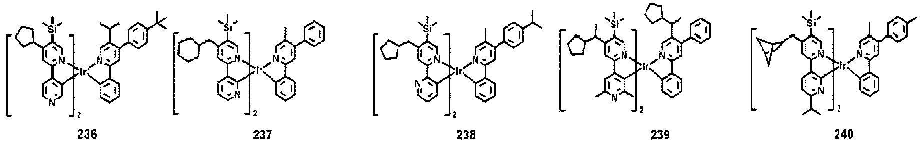

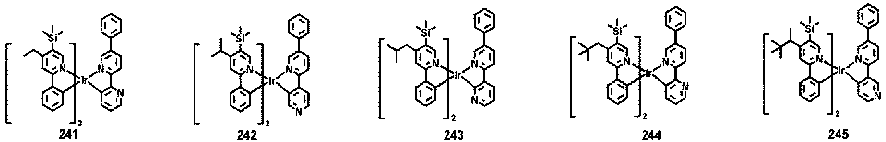

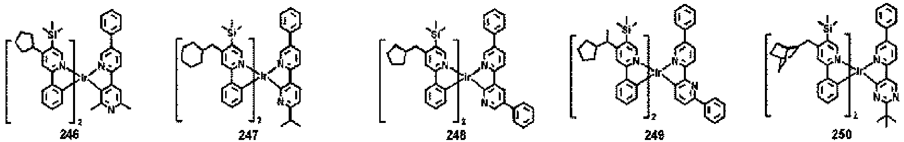

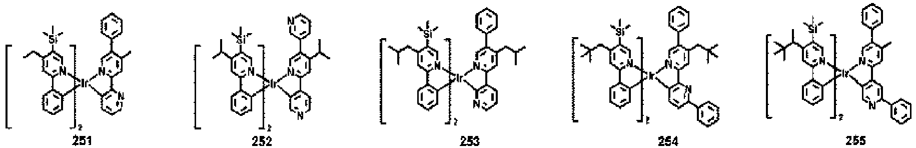

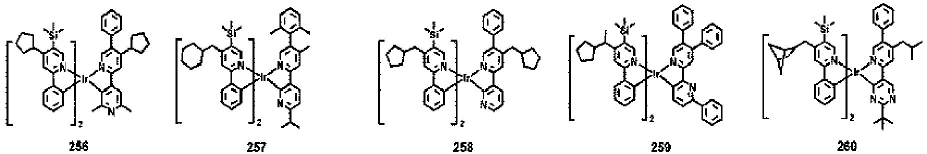

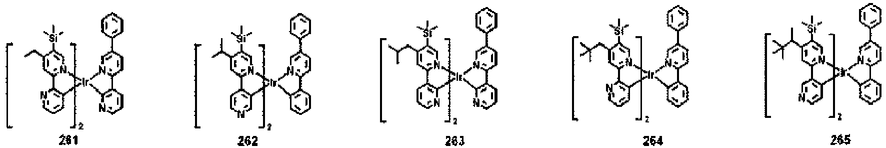

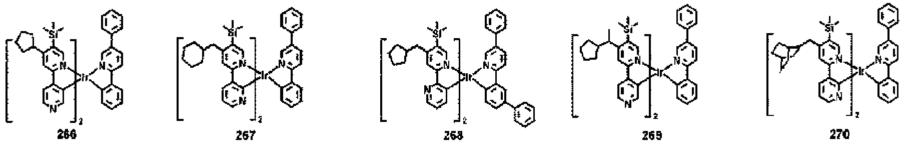

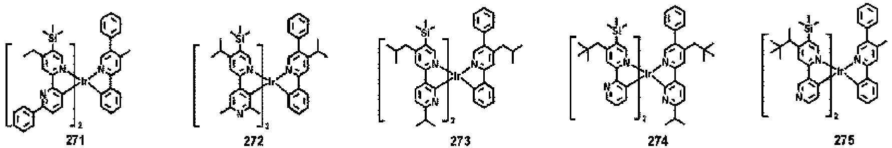

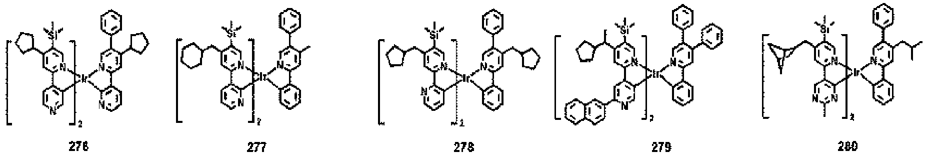

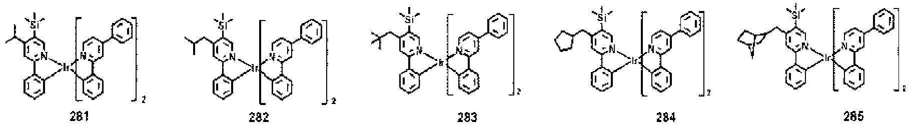

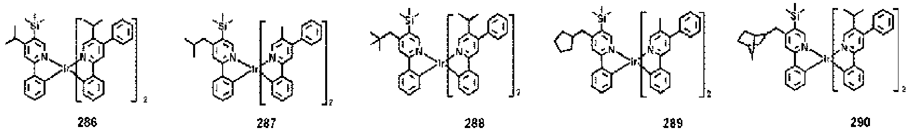

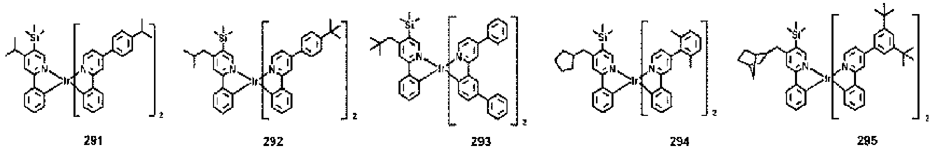

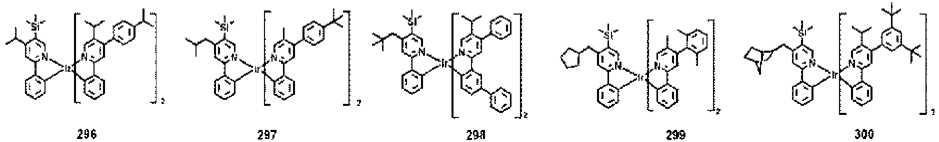

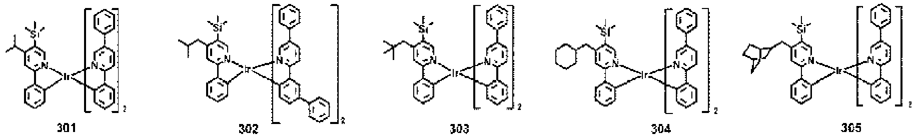

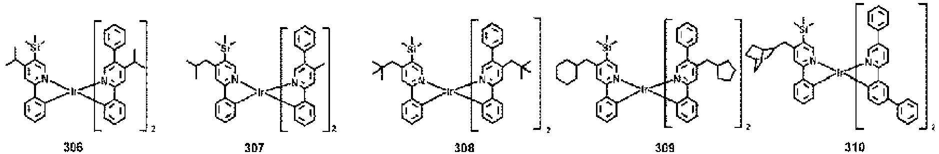

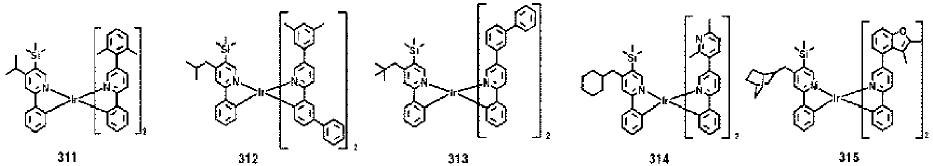

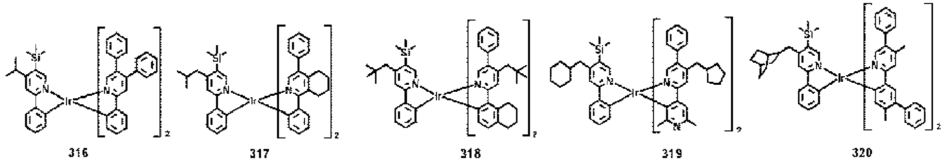

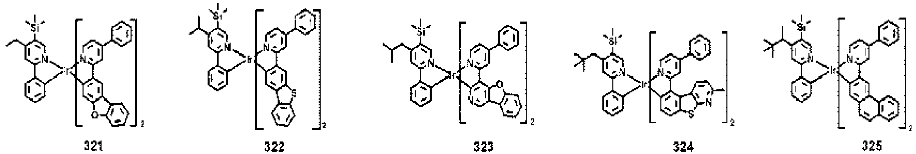

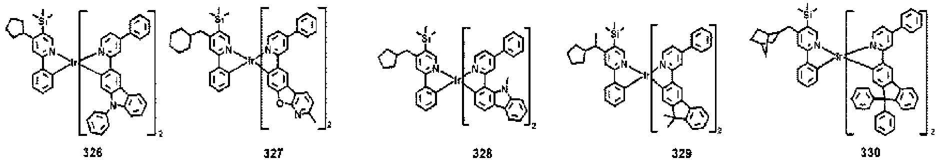

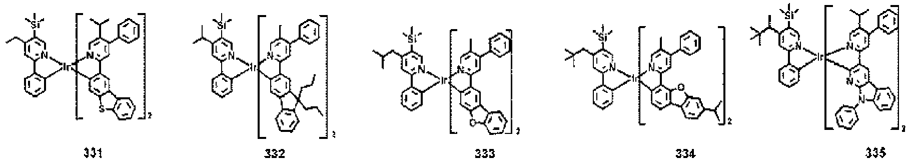

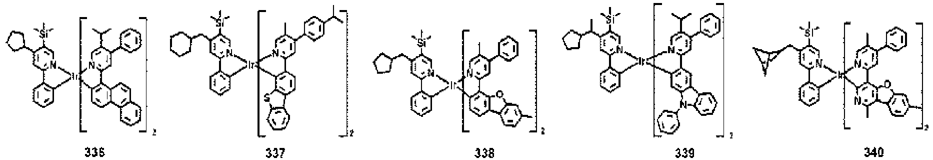

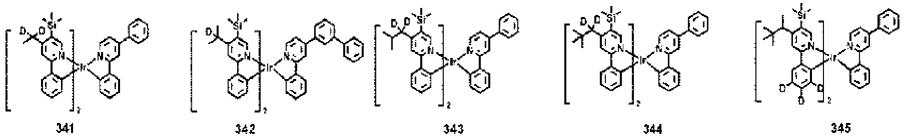

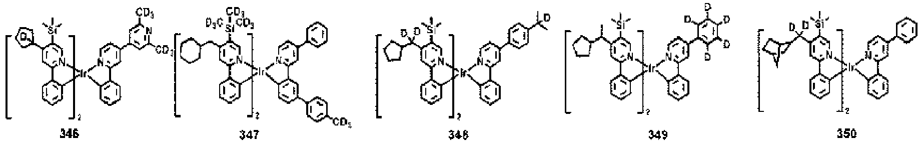

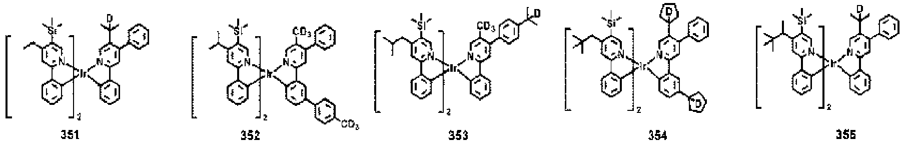

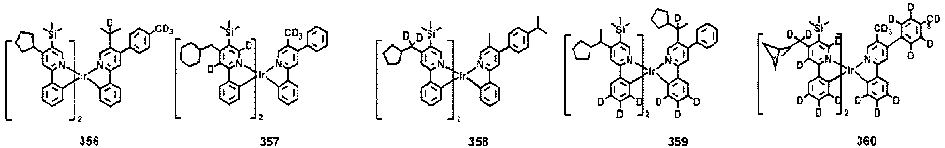

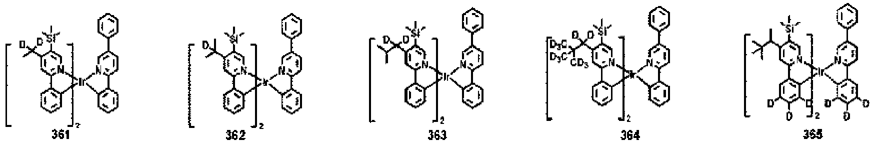

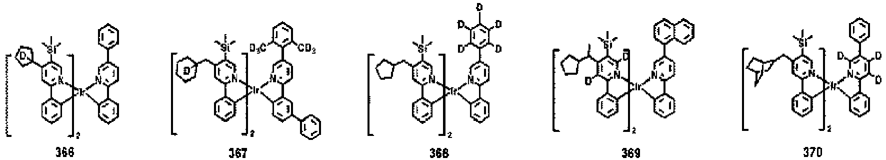

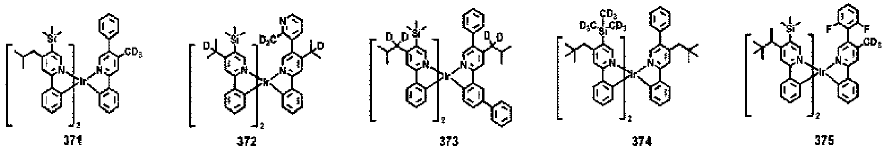

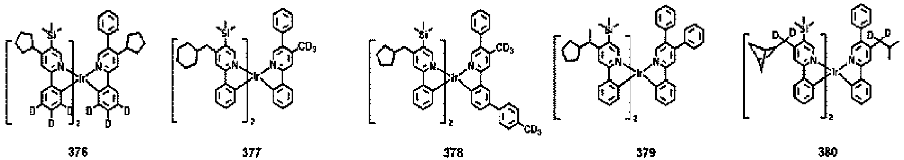

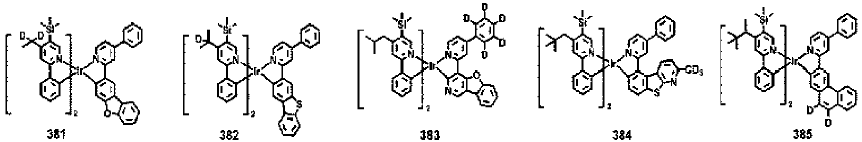

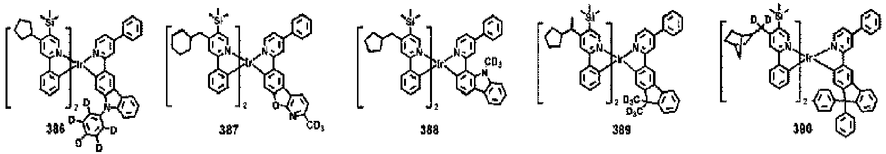

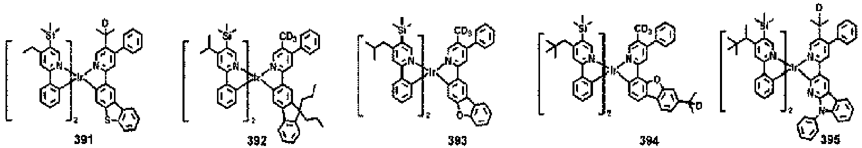

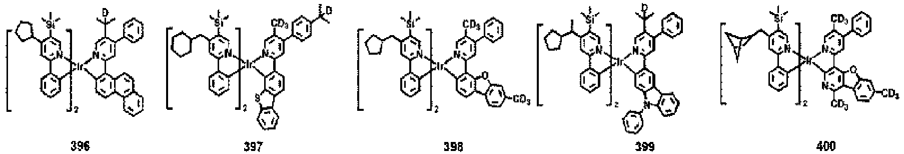

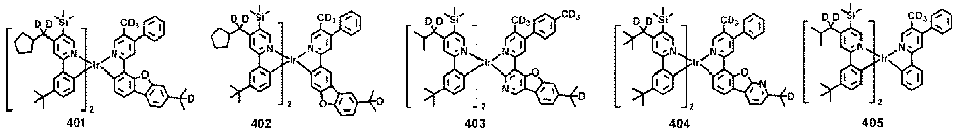

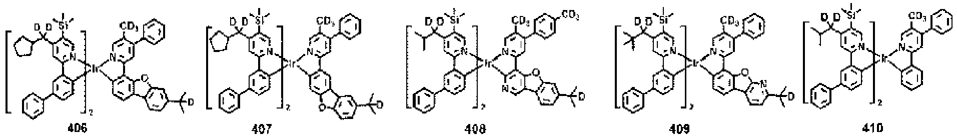

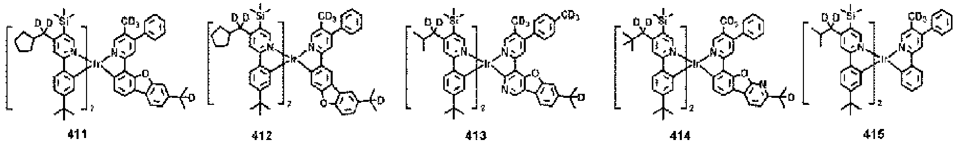

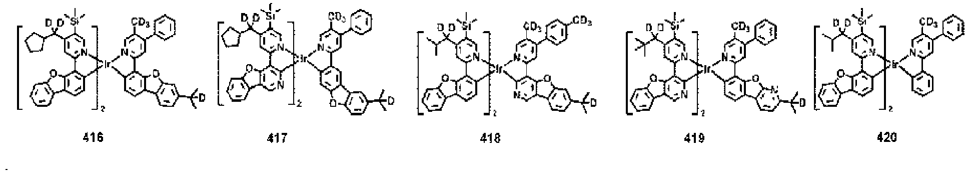

13. Dispositif émetteur de lumière organique comprenant :
une première électrode,
une deuxième électrode, et
une couche organique disposée entre la première électrode et la deuxième électrode et comprenant une couche d'émission,
dans lequel la couche organique comprend au moins un composé organométallique selon l'une quelconque des revendications 1 à 12 ;
préférablement dans lequel
la première électrode est une anode,
la deuxième électrode est une cathode,
la couche organique comprend en outre une zone de transport de trous disposée entre la première électrode et la couche d'émission et une zone de transport d'électrons disposée entre la couche d'émission et la deuxième électrode,
la zone de transport de trous comprend une couche d'injection de trous, une couche de transport de trous, une couche de blocage d'électrons, une couche tampon, ou une combinaison de celles-ci, et
la zone de transport d'électrons comprend une couche de blocage de trous, une couche de transport d'électrons, une couche d'injection d'électrons, ou une combinaison de celles-ci.

14. Dispositif émetteur de lumière organique selon la revendication 13, dans lequel la couche d'émission comprend l'au moins un composé organométallique ; préférablement dans lequel la couche d'émission comprend en outre un hôte, et une quantité de l'hôte est supérieure à une quantité de l'au moins un composé organométallique.

15. Composition de diagnostic comprenant le composé organométallique selon l'une quelconque des revendications 1 à 12.
